(19) Europäisches Patentamt
European Patent Office
Office européen des brevets

(11) Numéro de publication: **0 053 961**
**B1**

(12) **FASCICULE DE BREVET EUROPEEN**

(45) Date de publication du fascicule du brevet: **24.10.84**

(51) Int. Cl.³: **C 07 D 501/24,** A 61 K 31/545 // C07D317/28, C07D405/06

(21) Numéro de dépôt: **81401822.2**

(22) Date de dépôt: **19.11.81**

(54) **Nouvelles thiovinyl-3 céphalosporines, leur préparation et les compositions pharmaceutiques qui les contiennent.**

(30) Priorité: **20.11.80 FR 8024633**

(43) Date de publication de la demande: **16.06.82 Bulletin 82/24**

(45) Mention de la délivrance du brevet: **24.10.84 Bulletin 84/43**

(84) Etats contractants désignés: **AT BE CH DE FR GB IT LI LU NL SE**

(56) Documents cités:
**FR - A - 2 081 451**
**FR - A - 2 137 899**

(73) Titulaire: **RHONE-POULENC SANTE, Les Miroirs 18 Avenue d'Alsace, F-92400 Courbevoie Cedex (FR)**

(72) Inventeur: **Farge, Daniel, 30 rue des Pins Sylvestres, F-94320 Thiais (FR)**
Inventeur: **Le Roy, Pierre, 2 Allée des Cerisiers, F-94320 Thiais (FR)**
Inventeur: **Moutonnier, Claude, 3 rue Auguste Rodin, F-92350 Le Plessis Robinson (FR)**
Inventeur: **Peyronel, Jean-François, 36 parc d'Ardenay, F-91110 Palaiseau (FR)**

(74) Mandataire: **Gaumont, Robert et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

**Description**

La présente invention concerne de nouvelles thiovinyl-3 céphalosporines de formule générale:

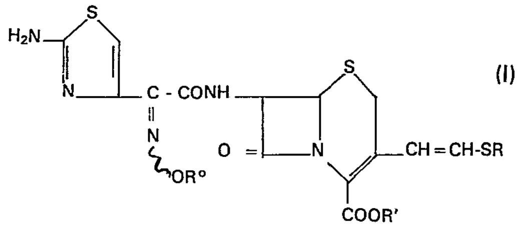

(I)

leurs sels, leur préparation et les compositions pharmaceutiques qui les contiennent.

Dans les demandes de brevet français publiées sous les numéros 2 081 451 et 2 137 899 on a décrit des céphalosporines portant en position —3 un radical vinyle qui peut être substitué par des radicaux aliphatiques ou aromatiques.

Les produits selon l'invention se caractérisent en ce que, dans la formule générale (I),
le symbole R' représente un atome d'hydrogène ou un radical, facilement éliminable par voie enzymatique, de formule générale

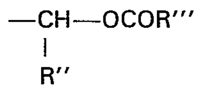

(II)

dans laquelle R'' représente un atome d'hydrogène ou un radical alcoyle et R''' représente un radical alcoyle ou le radical cyclohexyle et

α/ le symbole R est choisi parmi les significations suivantes:

1) alcoyle, L-amino-2 carboxy-2 éthyle, phényle,

2) pyridyle-2, pyridyle-3 ou pyridyle-4 et leurs N-oxydes,

3) pyrimidinyle-2; pyridazinyle-3 substitué en position —6 (par un radical alcoyle, méthoxy, amino ou acylamino) et éventuellement N-oxydé ou tétrazolo [4,5-b] pyridazinyle-6,

4) dioxo-5,6 tétrahydro-1,4,5,6 triazine-1,2,4 yle-3 substitué en position —4; triazol-1,3,4 yle-5 ou alcoyloxycarbonyl-2 triazol-1,3,4 yle-5 substitués en position —1

a) par un radical alcoyle, non substitué ou substitué par un radical alcoyloxy, alcoylthio, phényle, formyle, carbamoyle, alcoylcarbamoyle, dialcoylcarbamoyle, acyle, alcoyloxycarbonyle ou thiazolidinyle-2,

b) par un radical allyle; dihydroxy-2,3 propyle; dihydroxy-1,3 propyle-2; formyl-2 hydroxy-2 éthyle; formyloxy-3 hydroxy-2 propyle; bis-formyloxy-2,3 propyle ou bisformyloxy-1,3 propyle-2,

c) par un radical alcoyle contenant 2 à 4 atomes de carbone, substitué par un radical hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales:

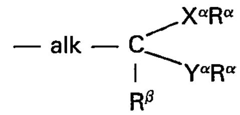

(III)

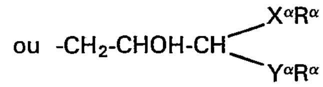

(IV)

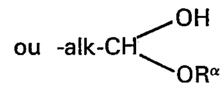

(V)

dans lesquelles alk est un radical alcoylène contenant 1 à 4 atomes de carbone, $X^\alpha$ et $Y^\alpha$ sont identiques et représentent des atomes d'oxygène ou de soufre et $R^\alpha$ représente un radical alcoyle, ou bien $X^\alpha$ et $Y^\alpha$ sont identiques ou différents et représentent des atomes d'oxygène ou de soufre et les radicaux $R^\alpha$ forment ensemble un radical alcoylène contenant 2 ou 3 atomes de carbone, et $R^\beta$ représente un atome d'hydrogène ou un radical alcoyle contenant 1 à 3 atomes de carbone,

e) par un radical alcoyle contenant 2 à 5 atomes de carbone substitué par un radical alcoyloxyimino ou hydroxyimino,

5) dialcoyl-1,4 dioxo-5,6 tétrahydro-1,4,5,6 triazine-1,2,4 yle-3; alcoyl-1 dioxo-5,6 tétrahydro-1,4,5,6 triazine-1,2,4 yle-3; alcoyl-2 dioxo-5,6 tétrahydro-1,2,5,6 triazine-1,2,4 yle-3,

6) triazol-1,3,4 yle-5; triazol-1,2,3 yle-5 ou alcoyl-1 triazol-1,2,4 yle-5 non substitué ou substitué en position —3 par alcoyloxycarbonyle,

7) a) — thiadiazol-1,3,4 yle-5 non substitué ou substitué par un radical alcoyle, trifluorométhyle, alcoyloxy, alcoylthio, hydroxyalcoylthio dont la partie alcoyle contient 2 à 4 atomes de carbone, alcoylsulfonyle, hydroxy, hydroxyalcoyle, carboxy, carboxyalcoyle, amino, alcoylamino, dialcoylamino, aminoalcoyle, alcoylaminoalcoyle, dialcoylaminoalcoyle, acylamino ou acylaminoalcoyle,

b) — thiadiazol-1,2,4 yle-5 substitué par un radical alcoyle ou alcoyloxy,

8) a) — oxadiazol-1,3,4 yle-5 non substitué ou substitué par un radical alcoyle, trifluorométhyle, phényle, aminoalcoyle, alcoylaminoalcoyle, dialcoylaminoalcoyle ou acylaminoalcoyle,

b) — oxazolyle-2 ou alcoyl-4 oxazolyle-2,

9) — tétrazolyle-5 non substitué ou substitué en position —1 par

a) — un radical alcoyle non substitué ou substitué par alcoyloxy, sulfo, carboxy, formyle ou sulfamoyle,

b) — un radical alcoyle contenant 2 à 4 atomes de carbone substitué par hydroxy, amino, alcoylamino, dialcoylamino, acylamino, carboxyalcoylamino, sulfamoylamino, sulfoamino, uréido, alcoyluréido ou dialcoyluréido,

c) — un radical alcoyle contenant 2 à 5 atomes

de carbone substitué par hydroxyimino ou alcoyloxyimino,

d) — un radical phényle, dihydroxy-2,3 propyle, dihydroxy-1,3 propyle-2; formyl-2 hydroxy-2 éthyle; formyloxy-3 hydroxy-2 propyle; bisformyloxy-2,3 propyle ou bisformyloxy-1,3 propyle-2, ou

e) — un radical de formule générale

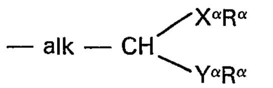

(IIIa)

dans laquelle alk, $X^\alpha$, $Y^\alpha$ et $R^\alpha$ sont définis comme ci-dessus, ou un radical de formule générale (IV), et le symbole R° représente un radical carboxyalcoyle de formule générale:

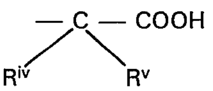

(VI)

dans laquelle les radicaux $R^{iv}$ et $R^{v}$, qui sont identiques ou différents, représentent des atomes d'hydrogène ou des radicaux alcoyle ou forment ensemble un radical alcoylène contenant 2 ou 3 atomes de carbone ou bien

β/ R est choisi parmi

1) dioxo-5,6 tétrahydro-1,4,5,6 triazine-1,2,4 yle-3 substitué en position —1, dioxo-5,6 tétrahydro-1,2,5,6 triazine-1,2,4 yle-3 substitué en position —2, triazol-1,2,4 yle-5 ou alcoyloxycarbonyl-3 triazol-1,2,4 yle-5 substitués en position —1

a) par un radical alcoyle substitué par un radical alcoyloxy, alcoylthio, phényle, formyle, carbamoyle, alcoylcarbamoyle, dialcoylcarbamoyle, hydroxyalcoylcarbamoyle (dont la partie alcoyle contient 2 à 4 atomes de carbone), acyle, alcoyloxycarbonyle ou thiazolidinyle-2,

b) par un radical allyle; dihydroxy-2,3 propyle; dihydroxy-1,3 propyle-2; formyl-2 hydroxy-2 éthyle; formyloxy-3 hydroxy-2 propyle; bis-formyloxy-2,3 propyle ou bisformyloxy-1,3 propyle-2,

c) par un radical alcoyle contenant 2 à 4 atomes de carbone, substitué par un radical hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales (III), (IV) ou (V) telles que définies précédemment ou

e) par un radical alcoyle contenant 2 à 5 atomes de carbone substitué par un radical alcoyloxyimino ou hydroxyimino, ou bien

2) dioxo-5,6 hydroxyalcoylcarbamoylalcoyl-4-tétrahydro-1,4,5,6 triazine-1,2,4 yle-3 dont la portion hydroxyalcoyle contient 2 à 4 atomes de carbone, ou bien

3) alcoyl-1 dioxo-5,6 tétrahydro-1,4,5,6 triazine-1,2,4 yle-3 substitué en position —4, alcoyl-1 dioxo-5,6 tétrahydro-1,2,5,6 triazine-1,2,4 yle-3 substitué en position —2, alcoyl-2 dioxo-5,6 tétrahydro-1,2,5,6 triazine-1,2,4 yle-3 substitué en position —1 ou alcoyl-4 dioxo-5,6 tétrahydro-1,4,5,6 triazine-1,2,4 yle-3 substitué en position —1 par un radical formylalcoyle ou par un radical de formule générale (IIIa), et

R° représente un atome d'hydrogène, un radical alcoyle, vinyle, cyanométhyle ou un radical de formule générale (VI).

Sauf mention spéciale, les portions ou radicaux alcoyles ou acyles cités dans la présente description contiennent 1 à 4 atomes de carbone en chaîne droite ou ramifiée.

Le substituant en position —3 des produits de formule générale (I) peut se présenter sous forme cis ou trans ou comme un mélange des formes cis et trans.

Dans ce qui suit la stéréoisomérie trans est désignée par E et la stéréoisomérie cis est désignée par Z.

Par ailleurs, le groupement OR° peut se trouver dans l'une des positions syn ou anti.

La forme syn est représentée par la formule:

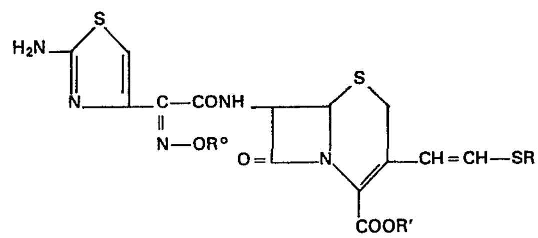

(Ia)

La forme anti est représentée par la formule:

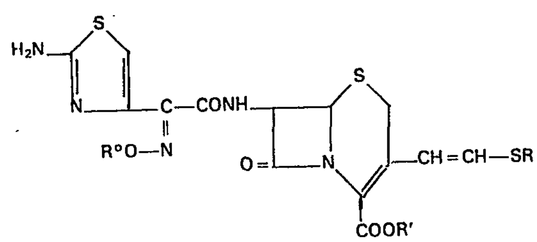

(Ib)

De même lorsque le radical R contient un substituant hydroxyiminoalcoyle ou alcoyloxyiminoalcoyle, ce groupement présente les isoméries syn et anti.

Lorsque le radical R est un radical tétrahydro-1,4,5,6 triazinyle substitué en position —1 ou —4 ou tétrahydro-1,2,5,6 triazinyle substitué en position —2, il est représenté par les formes tautomères:

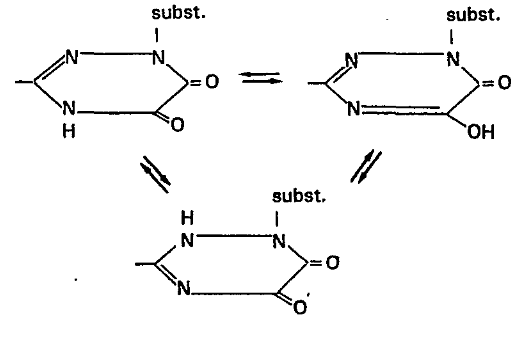

(VIIa)

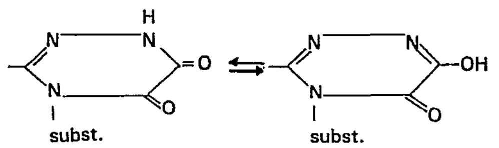
(VIIb)

ou

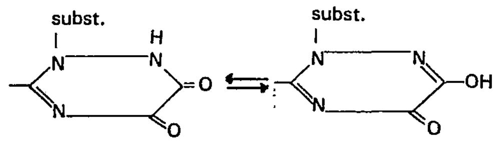
(VIIc)

De plus, lorsque dans la formule générale (VI) les symboles $R^{iv}$ et $R^{v}$ sont différents, il existe des diastéréoisomères.

Il est entendu que toutes les formes isomères et leurs mélanges entrent dans le cadre de la présente invention.

Lorsque le radical R contient un substituant formylalcoyle, il se présente sous sa forme aldéhyde libre ou hydrate d'aldéhyde. On observe notamment ces formes dans les conditions décrites ci-dessous.

Les études de résonance magnétique nucléaire montrent en particulier que lorsque R est dioxo-5,6 formylméthyl-4 tétrahydro-1,4,5,6 triazine-1,2,4 yle-3:
- en solvant acide, tel que l'acide formique ou trifluoroacétique (deutérés), en présence ou non d'eau (lourde) le produit se présente principalement sous la forme d'aldéhyde libre.
- en solvant basique tel que l'eau (lourde) additionnée de bicarbonate de sodium, il se présente principalement sous la forme d'hydrate d'aldéhyde.
- en solvant neutre tel que diméthylsulfoxyde ($d_6$), les formes aldéhyde libre et hydrate d'aldéhyde sont présentes, l'addition d'eau déplaçant l'équilibre en faveur de la forme hydrate d'aldéhyde.

Les produits de formule générale (Ia) sont les produits préférés.

Parmi les significations du symbole R° ci-dessus, on peut citer notamment: hydrogène, méthyle, éthyle, propyle, isopropyle, butyle, isobutyle, sec.butyle, vinyle, cyanométhyle, carboxyméthyle, carboxy-1 éthyle, carboxy-2 propyle-2, carboxy-1 cyclopropyle, carboxy-1 cyclobutyle.

Parmi les significations du symbole R ci-dessus, on peut citer notamment:

méthyl-1 thiadiazol-1,3,4 yle-5
(acétamido-2 éthyl)-2 thiadiazol-1,3,4 yle-5
diméthylaminométhyl-2 thiadiazol-1,3,4 yle-5
formylméthyl-1 triazol-1,2,4 yle-5
formylméthyl-1 triazol-1,3,4 yle-5
formylméthyl-1 méthoxycarbonyl-2 triazol-1,3,4 yle-5
méthyl-1 tétrazolyle-5
(acétylamino-2 éthyl)-1 tétrazolyle-5
formylméthyl-1 tétrazolyle-5
(hydroxy-2 éthyl)-1 tétrazolyle-5
(diméthylamino-2 éthyl)-1 tétrazolyle-5
pyrimidinyle-2
pyridyle-2
pyridyle-2 N-oxyde
pyridyle-3
pyridyle-4
méthyl-6 oxyde-1 pyridazinyle-3
dioxo-5,6 méthyl-1 tétrahydro-1,4,5,6 triazine-1,2,4 yle-3
dioxo-5,6 méthyl-2 tétrahydro-1,2,5,6 triazine-1,2,4 yle-3
dioxo-5,6 méthyl-4 tétrahydro-1,4,5,6 triazine-1,2,4 yle-3
dioxo-5,6 éthyl-1 tétrahydro-1,4,5,6 triazine-1,2,4 yle-3
dioxo-5,6 formylméthyl-1 tétrahydro-1,4,5,6 triazine-1,2,4 yle-3
dioxo-5,6 formylméthyl-2 tétrahydro-1,2,5,6 triazine-1,2,4 yle-3
dioxo-5,6 formylméthyl-4 tétrahydro-1,4,5,6 triazine-1,2,4 yle-3
dioxo-5,6 (hydroxy-2 éthyl)-1 tétrahydro-1,4,5,6 triazine-1,2,4 yle-3
dioxo-5,6 (hydroxy-2 éthyl)-2 tétrahydro-1,2,5,6 triazine-1,2,4 yle-3
dioxo-5,6 (hydroxy-2 éthyl)-4 tétrahydro-1,4,5,6 triazine-1,2,4 yle-3
dioxo-5,6 (méthoxy-2 éthyl)-1 tétrahydro-1,4,5,6 triazine-1,2,4 yle-3
dioxo-5,6 (méthoxy-2 éthyl)-2 tétrahydro-1,2,5,6 triazine-1,2,4 yle-3
dioxo-5,6 (méthoxy-2 éthyl)-4 tétrahydro-1,4,5,6 triazine-1,2,4 yle-3
dioxo-5,6 (méthylthio-2 éthyl)-1 tétrahydro-1,4,5,6 triazine-1,2,4 yle-3
dioxo-5,6 (méthylthio-2 éthyl)-2 tétrahydro-1,2,5,6 triazine-1,2,4 yle-3
dioxo-5,6 (méthylthio-2 éthyl)-4 tétrahydro-1,4,5,6 triazine-1,2,4 yle-3
(acétamido-2 éthyl)-1 dioxo-5,6 tétrahydro-1,4,5,6 triazine-1,2,4 yle-3
(acétamido-2 éthyl)-2 dioxo-5,6 tétrahydro-1,2,5,6 triazine-1,2,4 yle-3
(acétamido-2 éthyl)-4 dioxo-5,6 tétrahydro-1,4,5,6 triazine-1,2,4 yle-3
dioxo-5,6 (formyl-2 hydroxy-2 éthyl)-1 tétrahydro-1,4,5,6 triazine-1,2,4 yle-3
dioxo-5,6 (formyl-2 hydroxy-2 éthyl)-2 tétrahydro-1,2,5,6 triazine-1,2,4 yle-3
dioxo-5,6 (formyl-2 hydroxy-2 éthyl)-4 tétrahydro-1,4,5,6 triazine-1,2,4 yle-3
(dihydroxy-2,3 propyl)-1 dioxo-5,6 tétrahydro-1,4,5,6 triazine-1,2,4 yle-3
(dihydroxy-2,3 propyl)-4 dioxo-5,6 tétrahydro-1,4,5,6 triazine-1,2,4 yle-3
dioxo-5,6 (méthoxy-2 hydroxy-2 éthyl)-4 tétrahydro-1,4,5,6 triazine-1,2,4 yle-3
dioxo-5,6 (éthoxy-2 hydroxy-2 éthyl)-4 tétrahydro-1,4,5,6 triazine-1,2,4 yle-3
dioxo-5,6 (formyloxy-2 éthyl)-4 tétrahydro-1,4,5,6 triazine-1,2,4 yle-3
(carbamoyloxy-2 éthyl)-4 dioxo-5,6 tétrahydro-1,4,5,6 triazine-1,2,4 yle-3
allyl-4 dioxo-5,6 tétrahydro-1,4,5,6 triazine-1,2,4 yle-3
allyl-1 dioxo-5,6 tétrahydro-1,4,5,6 triazine-1,2,4 yle-3
allyl-2 dioxo-5,6 tétrahydro-1,2,5,6 triazine-1,2,4 yle-3
dioxo-5,6 (glycylamino-2 éthyl)-4 tétrahydro-1,4,5,6 triazine-1,2,4 yle-3
dioxo-5,6 (glycylamino-2 éthyl)-1 tétrahydro-1,4,5,6 triazine-1,2,4 yle-3
dioxo-5,6 (méthanesulfonylamino-2 éthyl)-4 tétrahydro-1,4,5,6 triazine-1,2,4 yle-3
dioxo-5,6 (méthanesulfonylamino-2 éthyl)-1 tétrahydro-1,4,5,6 triazine-1,2,4 yle-3
dioxo-5,6 [(méthyl-3 uréido)-2 éthyl]-4 tétrahydro-1,4,5,6 triazine-1,2,4 yle-3
dioxo-5,6 [(méthyl-3 uréido)-2 éthyl]-1 tétrahydro-1,4,5,6 triazine-1,2,4 yle-3
carbamoylméthyl-4 dioxo-5,6 tétrahydro-1,4,5,6 triazine-1,2,4 yle-3
diméthylcarbamoylméthyl-4 dioxo-5,6 tétrahydro-1,4,5,6 triazine-1,2,4 yle-3
N-(hydroxy-2 éthyl) carbamoylméthyl-4 dioxo-5,6 tétrahydro-1,4,5,6 triazine-1,2,4 yle-3
dioxo-5,6 formylméthyl-1 méthyl-4 tétrahydro-1,4,5,6 triazine-1,2,4 yle-3
dioxo-5,6 formylméthyl-2 méthyl-1 tétrahydro-1,2,5,6 triazine-1,2,4 yle-3
dioxo-5,6 formylméthyl-4 méthyl-1 tétrahydro-1,4,5,6 triazine-1,2,4 yle-3
dioxo-5,6 formylméthyl-1 méthyl-2 tétrahydro-1,2,5,6 triazine-1,2,4 yle-3
dioxo-5,6 formylméthyl-2 méthyl-1 tétrahydro-1,2,5,6 triazine-1,2,4 yle-3
dioxo-5,6 éthyl-1 formylméthyl-4 tétrahydro-1,4,5,6 triazine-1,2,4 yle-3
dioxo-5,6 éthyl-4 formylméthyl-1 tétrahydro-1,4,5,6 triazine-1,2,4 yle-3

Parmi les significations du symbole R' ci-dessus, peuvent être cités notamment hydrogène, pivaloyloxyméthyle et acétoxyméthyle.

A/ Selon l'invention les produits de formule générale (I) dans laquelle R est autre qu'un radical triazinyle ou triazolyle substitués par un groupement de formule générale (V) peuvent être préparés par action d'un acide de formule générale:

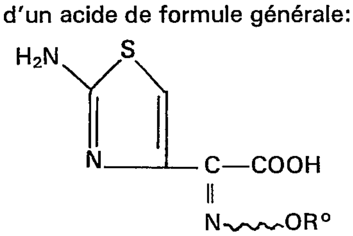

(VIII)

dans laquelle R° est défini comme précédemment, et dont la fonction amine est préalablement protégée [ainsi que l'oxime ou le radical carboxyalcoyle lorsque R° représente l'hydrogène ou un radical de formule générale (VI)], ou d'un dérivé réactif de cet acide, sur une amino-7 céphalosporine de formule générale:

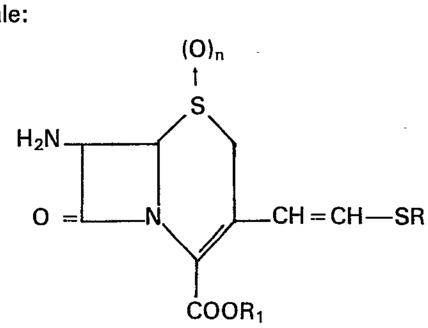

(IX)

[dans laquelle R est défini comme précédemment, à l'exception de représenter un radical substitué par un groupement de formule générale (V), $R_1$ représente un atome d'hydrogène, un radical de formule générale (II) ou un radical protecteur facilement éliminable, par exemple méthoxyméthyle, t.butyle, benzhydryle, nitrobenzyle ou p.méthoxybenzyle, et n représente 0 ou 1],
suivie de la réduction du sulfoxyde obtenu lorsque n = 1, puis de l'élimination des radicaux protecteurs.

Il est entendu que l'acide de formule générale (VIII), sous forme syn ou anti ou leurs mélanges, conduit respectivement aux produits de formule générale (I) de forme syn ou anti ou leurs mélanges.

a/ Lorsque l'on utilise le produit de formule générale (VIII) sous forme acide la protection du groupement amino s'effectue par toute méthode connue en soi pour le blocage d'une fonction amine sans toucher au reste de la molécule. Il est nécessaire d'utiliser un groupement facilement éliminable, tel qu'un groupement t-butoxycarbonyle, trichloro-2,2,2 éthoxycarbonyle, chloracétyle, trichloracétyle, trityle, benzyle, dibenzyle, benzyloxycarbonyle, p.nitrobenzyloxycarbonyle, p.méthoxybenzyloxycarbonyle, formyle ou trifluoroacétyle.

Lorsque R° représente un atome d'hydrogène, la protection de l'oxime peut être effectuée par toute méthode connue et qui n'altère pas le reste de la molécule. On utilise notamment les groupements trityle, tétrahydropyrannyle ou méthoxy-2 propyle-2.

Lorsque R° contient un radical carboxy, il est nécessaire de protéger ce groupement par toute méthode connue qui n'altère pas le reste de la molécule. On utilise notamment un radical protecteur facilement éliminable tel que méthoxyméthyle, t-butyle, benzhydryle, benzyle, nitrobenzyle ou p.méthoxybenzyle.

Généralement on effectue la condensation du produit de formule générale (VIII), dont la fonction amine a été préalablement protégée, sur l'amino-7 céphalosporine de formule générale (IX) dans laquelle, R et n étant définis comme précédemment, $R_1$ représente un radical de formule générale (II) ou un radical protecteur facilement éliminable tel que méthoxyméthyle, t.butyle, benzhydryle, nitrobenzyle ou p.méthoxybenzyle, en opérant dans un solvant organique tel que le diméthylformamide, l'acétonitrile, le tétrahydrofuranne, le dichlorométhane ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre —20 et 40°C, puis on réduit l'oxyde obtenu lorsque l'on a utilisé une amino-7 céphalosporine de formule générale (IX) dans laquelle n = 1, et on élimine les groupements protecteurs de la fonction amine et le cas échéant des fonctions acide et de l'oxime.

Il est entendu que les groupements amino, alcoylamino, carboxy et hydroxy qui existent dans certains radicaux R sont ou peuvent être protégés par tous groupements protecteurs habituellement utilisés pour la protection des amines, des acides carboxyliques ou des alcools et dont la mise en oeuvre n'altère pas le reste de la molécule.

A titre d'exemples,

— les groupements amino et alcoylamino sont protégés par des radicaux tels que t.butoxycarbonyle, trichloro-2,2,2 éthoxycarbonyle, trichloracétyle, trityle, benzyle, dibenzyle, benzyloxycarbonyle, p.nitrobenzyloxycarbonyle, p.méthoxybenzyloxycarbonyle, chloracétyle, formyle ou trifluoracétyle
— les groupements carboxy peuvent être protégés par des radicaux tels que méthoxyméthyle, t.butyle, benzhydryle, nitrobenzyle ou p.méthoxybenzyle
— les groupements hydroxy peuvent être protégés par des radicaux tels que trityle, tétrahydropyrannyle ou méthoxy-2 propyle-2 ou bien sous forme d'un radical diméthyl-2,2 dioxolannyl-4 méthyle ou diméthyl-2,2 dioxannyle-5 lorsqu'il s'agit de la protection des radicaux dihydroxy-2,3 propyle ou dihydroxy-1,3 propyle-2.

Il est également entendu que lorsque dans la formule générale (IX) le radical R contient un groupement hydroxy, sulfo, sulfinyle ou sulfonyle, il est préférable de mettre en oeuvre un produit dans la formule duquel n = 0.

Lorsque l'on veut obtenir un produit de formule générale (I) dans laquelle R contient un radical formylalcoyle ou acylalcoyle, ce radical peut être éventuellement protégé à l'état d'acétal, sous forme d'un radical de formule générale (III), (IIIa) ou (IV) tel que défini précédemment.

L'élimination du radical protecteur de R est effectuée après la réduction de l'oxyde, avant, simultanément ou après l'élimination des autres radicaux protecteurs. Il en est de même pour les autres radicaux protecteurs présents dans la molécule.

La réduction du S-oxyde s'effectue par exemple dans les conditions décrites dans la demande de brevet allemand 2 637 176. L'élimination des différents radicaux protecteurs peut s'effectuer simultanément ou successivement.

A titre d'exemple,

1/ l'élimination des groupements protecteurs d'amines s'effectue
— lorsqu'il s'agit d'un radical t.butoxycarbonyle, trityle, p.méthoxybenzyloxycarbonyle ou formyle: par traitement en milieu acide. De préférence on utilise l'acide trifluoracétique en opérant à une température comprise entre 0 et 20°C, ou bien on utilise l'acide formique, phosphorique ou polyphosphorique purs ou en présence d'eau, à température comprise entre 20 et 60°C, ou encore l'acide paratoluènesulfonique ou méthanesulfonique dans l'acétone à la température de reflux du mélange réactionnel. Dans ces conditions le produit de formule générale (I) peut être obtenu sous forme de trifluoroacétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique
— Lorsqu'il s'agit d'un radical trichloro-2,2,2 éthoxycarbonyle ou p.nitrobenzyloxycarbonyle: par réduction (notamment traitement par le zinc dans l'acide acétique)
— lorsqu'il s'agit d'un radical chloracétyle ou trichloracétyle: par application de la méthode décrite dans le brevet français publié sous le n° 2 243 199
— lorsqu'il s'agit d'un radical benzyle, dibenzyle ou benzyloxycarbonyle: par hydrogénation catalytique
— lorsqu'il s'agit d'un radical trifluoracétyle: par traitement en milieu basique.

2/ L'élimination des groupements protecteurs des radicaux carboxy s'effectue:
— lorsqu'il s'agit d'un groupement t.butyle, p.méthoxybenzyle ou benzhydryle: par traitement en milieu acide, dans les conditions décrites ci-dessus pour l'élimination du radical trityle protecteur d'amino. Dans le cas du radical benzhydryle, on peut opérer en présence d'anisole
— lorsqu'il s'agit d'un groupement méthoxyméthyle: par traitement en milieu acide dilué
— lorsqu'il s'agit d'un groupement nitrobenzyle: par réduction (notamment traitement par le zinc dans l'acide acétique ou hydrogénolyse).

3/ L'élimination des groupements protecteurs de l'oxime et/ou des radicaux hydroxy s'effectue
— lorsqu'il s'agit de groupement trityle ou tétrahydropyrannyle ou des radicaux diméthyl-2,2 dioxolannyl-4 méthyle ou diméthyl-2,2 dioxannyle-5: par acidolyse, par exemple par l'acide trifluoracétique, l'acide formique aqueux ou non, ou l'acide paratoluènesulfonique. Lorsqu'on utilise l'acide formique, aqueux ou non, la libération des radicaux hydroxy protégés à l'état d'acétal cyclique peut conduire au moins partiellement aux mono ou diesters formiques correspondants, qui peuvent être séparés le cas échéant par chromatographie
— lorsqu'il s'agit du groupement méthoxy-2 propyle-2: selon la méthode décrite dans le brevet belge 875 379.

4/ La libération des radicaux formylalcoyle ou acylalcoyle protégés sous forme de groupement de formule générale (III), (IIIa) ou (IV) (lorsque l'on veut obtenir un produit de formule générale (I) dans laquelle R contient un radical formylalcoyle ou acylalcoyle) s'effectue
— en présence d'un acide sulfonique (acide méthanesulfonique ou acide paratoluènesulfonique par exemple) dans un solvant organique (acétonitrile ou acétone par exemple), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel
— ou bien, lorsque le radical R est un radical dioxo-5,6 tétrahydro-1,4,5,6 triazine-1,2,4 yle-3, ou dioxo-5,6 tétrahydro-1,2,5,6 triazine-1,2,4 yle-3, par action d'acide formique pur ou aqueux (contenant de préférence moins de 10% d'eau), soit en présence ou non de silice, soit par transacétalisation en présence d'un réactif acétalisable tel que défini ci-dessus.

b/ Lorsque l'on utilise un dérivé réactif de l'acide de formule générale (VIII), il est possible de mettre en oeuvre l'anhydride, un anhydride mixte ou un ester réactif de formule générale:

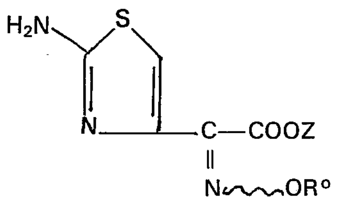

(X)

dans laquelle R° est défini comme ci-dessus et Z représente un radical succinimido, benzotriazolyle-1, nitro-4 phényle, dinitro-2,4 phényle, pentachlorophényle ou phtalimido; la fonction amine de tels dérivés et le cas échéant le radical R° ayant été préalablement protégés (par exemple comme décrit précédemment en a). Il est également possible de mettre en oeuvre des dérivés réactifs tels qu'un thioloester tel que défini ci-après par la formule générale (XV) ou un halogénure d'acide. Dans ce dernier cas, on peut faire réagir le chlorhydrate du chlorure d'acide sur l'amino-7 céphalosporine de formule générale (IX).

Lorsque l'on met en oeuvre l'anhydride, un anhydride mixte ou un halogénure d'acide (qui peuvent être préparés in situ), on effectue la condensation dans un solvant organique inerte tel qu'un éther (tétrahydrofuranne ou dioxanne par exemple), un solvant chloré (chloroforme ou dichlorométhane par exemple), un amide (diméthylformamide ou diméthylacétamide par exemple) ou une cétone (acétone par exemple), ou dans des mélanges de tels solvants, en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (triéthylamine par exemple), ou dans un milieu hydroorganique en présence d'un agent alcalin de condensation tel que le bicarbonate de sodium, et l'on opère à une température comprise entre —40 et 40°C, puis on réduit, le cas échéant, le S-oxyde obtenu, et on remplace éventuellement les groupements protecteurs par des atomes d'hydrogène.

Lorsque l'on met en oeuvre un ester réactif de formule générale (X) ou un thioloester de formule générale (XV) on opère généralement en présence d'une trialcoylamine (triéthylamine) dans un solvant organique tel que le diméthylformamide à une température comprise entre 0 et 40°C, puis on réduit, le cas échéant, le S-oxyde obtenu et on remplace les groupements protecteurs par des atomes d'hydrogène.

B/ Selon l'invention, les produits de formule générale (I) dans laquelle R, R° et R' sont définis comme ci-dessus, à l'exception pour R de contenir un substituant de formule générale (V), peuvent également être préparés par action d'un thiol (ou d'un de ses sels alcalins ou alcalinoterreux) de formule générale:

$$R — SH \qquad (XI)$$

[[dans laquelle R, qui est défini comme ci-dessus, est protégé à l'état d'acétal [tel que défini par les formules générales (III), (IIIa) ou (IV)] lorsque l'on veut obtenir une céphalosporine de formule générale (I) dans laquelle R contient un radical formyle ou acylalcoyle]] sur un dérivé de céphalosporine (ou le cas échéant sur un mélange des isomères de ce dérivé) de formule générale:

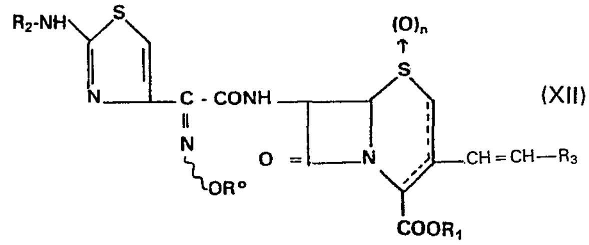

(XII)

dans laquelle, R°, $R_1$ et n étant définis comme précédemment, lorsque n = 0 le produit se présente sous forme bicyclooctène-2 ou -3 et lorsque n = 1 le produit se présente sous forme bicyclooctène-2 (selon la nomenclature des Chemical Abstracts),
le substituant sur l'atome de carbone en position —3 du bicyclooctène présente la stéréoisomérie E ou Z,
$R_2$ représente un atome d'hydrogène ou un radical protecteur du groupement amino, tel que défini précédemment an A/ et
$R_3$ représente un atome d'halogène choisi parmi chlore, brome et iode, ou un radical de formule générale:

$$- O - SO_2 - R'_3 \qquad (XIIIa)$$

$$ou \quad - O - CO - R''_3 \qquad (XIIIb)$$

dans lesquelles $R'_3$ est un radical alcoyle, trifluorométhyle, trichlorométhyle ou un radical phényle éventuellement substitué par un atome d'halogène ou par un radical alcoyle ou nitro, et $R''_3$ est défini comme $R'_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle, suivie de la réduction de l'oxyde obtenu (lorsque n = 1) puis éventuellement de l'élimination des radicaux protecteurs.

Il est entendu que, lorsque le radical R du produit de formule générale (XI) contient un groupement susceptible d'interférer avec la réaction, il est préférable de protéger ce groupement dans les conditions décrites précédemment (notamment lorsque R contient un radical amino, alcoylamino, hydroxy ou carboxy).

Il est également entendu que, lorsque R° représente l'hydrogène, ou un radical de formule générale (VI), il est préférable de protéger l'oxime ou le radical carboxy dans les conditions décrites précédemment.

Il est également entendu que, lorsque le radical R risque d'interférer avec la réaction de réduction, il est préférable d'utiliser un produit de formule générale (XII) pour lequel n = 0 (notamment lorsque R comprend un groupement hydroxy, sulfo, sulfinyle ou sulfonyle).

On opère généralement en présence d'une base organique, telle qu'une pyridine ou une base organique tertiaire de formule générale:

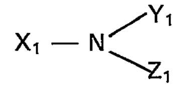

(XIV)

dans laquelle $X_1$, $Y_1$ et $Z_1$ représentent des radicaux alcoyle ou phényle, ou éventuellement deux d'entre eux forment un cycle avec l'atome d'azote auquel ils

sont rattachés. On utilise par exemple la diisopropyléthylamine, ou la diéthylphénylamine.

Lorsque l'on fait agir un sel alcalin ou alcalino-terreux du thiol de formule générale (XI), il n'est pas nécessaire d'opérer en présence d'une base organique telle que définie ci-dessus.

La réaction s'effectue avantageusement dans un solvant organique tel que le diméthylformamide, le tétrahydrofuranne, l'éthanol, le méthanol, ou l'acétonitrile, ou un mélange de tels solvants.

Il est également possible d'opérer en présence de bicarbonate alcalin dans un solvant tel que cité ci-dessus, éventuellement en présence d'eau.

On opère à une température comprise entre —20°C et la température de reflux du mélange réactionnel, la température choisie étant variable selon le thiol employé. De même, selon le thiol employé, le temps de réaction peut varier de 5 minutes à 48 heures.

Eventuellement on opère sous azote.

De préférence, lorsque l'on veut utiliser un bicyclooctène-3 de formule générale (XII), on met en oeuvre un tel produit pour lequel $R_1$ est autre que l'hydrogène.

La réduction de l'oxyde et l'élimination des groupements protecteurs s'effectuent selon les méthodes décrites précédemment.

C/ Selon l'invention, les produits de formule générale (I) dans laquelle R, R° et R' sont définis comme ci-dessus, R étant autre qu'un radical triazinyle ou triazolyle substitués par un groupement de formule générale (V), peuvent également être préparés par action d'un thioloester de formule générale:

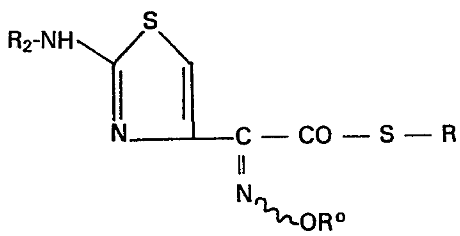

(XV)

dans laquelle R° et $R_2$ sont définis comme précédemment (étant entendu que lorsque R° contient un radical carboxy, celui-ci est protégé et lorsque R° représente un atome d'hydrogène l'oxime est préalablement protégée) et R est défini comme ci-dessus [étant entendu que lorsqu'il contient un substituant amino ou alcoylamino celui-ci est protégé, lorsqu'il contient un substituant hydroxy ou carboxy celui-ci est libre ou protégé et lorsqu'il contient un substituant formyle ou acylalcoyle celui-ci est protégé à l'état d'acétal comme dans la formule générale (III), (IIIa) ou (IV)], sur une amino-7 céphalosporine de formule générale:

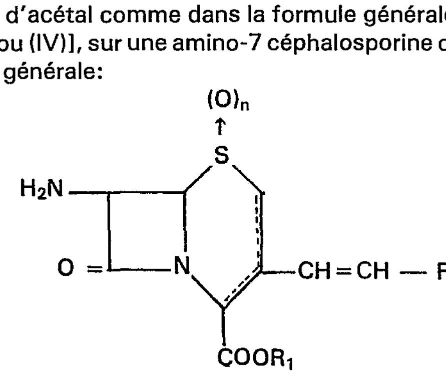

(XVI)

dans laquelle $R_1$, $R_3$ et n sont définis comme précédemment et qui présente la stéréoisomérie définie précédemment pour le produit de formule générale (XII), suivie de la réduction du sulfoxyde obtenu lorsque n = 1 et le cas échéant de l'élimination des radicaux protecteurs.

De même que pour le procédé A/, il est entendu que les thioloesters de forme syn ou anti ou leurs mélanges conduisent respectivement aux produits de formule générale (I) de forme syn ou anti ou leurs mélanges.

De même, ainsi que pour les procédés décrits précédemment, lorsque R contient un substituant hydroxy, sulfo, sulfinyle ou sulfonyle, on préfère mettre en oeuvre un produit de formule générale (XVI) dans laquelle n = 0.

La protection et l'élimination des radicaux protecteurs s'effectuent dans les conditions décrites précédemment.

La réaction du thioloester avec l'amino-7 céphalosporine de formule générale (XVI) s'effectue généralement en présence d'un accepteur d'acide tel qu'une base organique, plus particulièrement en présence d'une pyridine ou d'une base organique tertiaire de formule générale (XIV), notamment la triéthylamine, la NN-diisopropyl N-éthylamine, la diéthylphénylamine ou la N-méthylmorpholine.

La réaction s'effectue avantageusement dans un solvant organique tel qu'un amide (par exemple diméthylformamide, diméthylacétamide), un éther (par exemple tétrahydrofuranne, dioxanne), un solvant chloré (par exemple chloroforme, dichlorométhane), une cétone (par exemple acétone) ou un nitrile (par exemple acétonitrile), ou bien dans un mélange de tels solvants. Il est également possible d'opérer en présence d'un bicarbonate alcalin dans l'un des solvants cités ci-dessus, éventuellement en présence d'eau.

On opère à une température comprise entre —20°C et la température de reflux du mélange réactionnel. La réaction s'effectue éventuellement sous azote.

La réduction du S-oxyde s'effectue dans les conditions décrites précédemment.

D/ Selon l'invention, les produits de formule générale (I) [dans laquelle R° et R' sont définis comme ci-dessus à l'exception pour R° de représenter le radical vinyle et R est défini comme ci-dessus à l'exception de pouvoir représenter un radical triazinyle ou triazolyle substitués par un groupement de formule générale (V)], peuvent être préparés par action d'une thiourée de formule générale:

$$R_2NH — CS — NH_2 \qquad (XVII)$$

(dans laquelle $R_2$ est défini comme précédemment en B/ à l'exception de représenter chloracétyle ou trichloracétyle) sur un produit de formule générale:

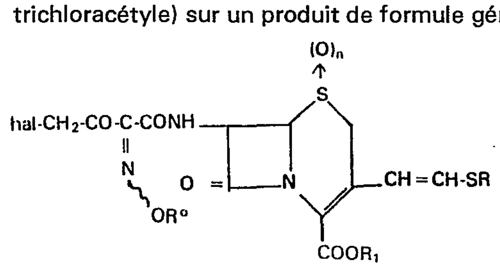

(XVIII)

dans laquelle R°, R, $R_1$ et n sont définis comme ci-dessus, et hal représente un atome de chlore ou de brome, suivie s'il y a lieu de la réduction du sulfoxyde et de l'élimination des radicaux protecteurs.

On opère généralement en milieu aqueux, organique ou hydroorganique par exemple dans des solvants ou des mélanges de solvants tels que des alcools (méthanol, éthanol), des cétones (acétone), des solvants chlorés (chloroforme, dichlorométhane), des nitriles (acétonitrile), des amides (diméthylformamide, diméthylacétamide), des éthers (tétrahydrofuranne, dioxanne), des esters (acétate d'éthyle) ou des acides (acide acétique, acide formique), en présence ou non d'une base telle que la soude, la potasse, des carbonates, des carbonates acides de métaux alcalins, des sels d'acides carboxyliques et de métaux alcalins (formiate de sodium, acétate de sodium) ou des amines tertiaires (triéthylamine, triméthylamine ou pyridine), à une température comprise entre —30 et 60°C.

Lorsque l'on opère en présence d'une base, selon la nature de celle-ci et la quantité introduite on isole ou non l'intermédiaire de formule générale:

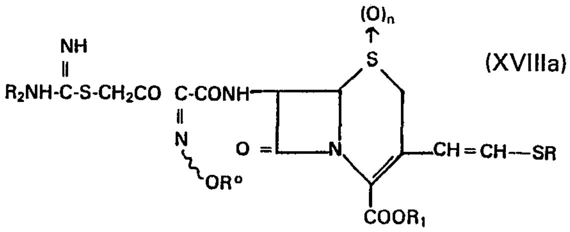

(XVIIIa)

dans laquelle R°, R, $R_1$, $R_2$ et n sont définis comme précédemment, qui peut alors être cyclisé en milieu acide.

Il est entendu que lorsque R° contient un radical carboxy, celui-ci peut être libre ou protégé.

Lorsque l'on veut obtenir un produit de formule générale (I) dans laquelle R contient un radical formylalcoyle ou acylalcoyle, ce radical peut être protégé à l'état d'acétal, sous forme d'un radical de formule générale (III), (IIIa) ou (IV) tel que défini précédemment.

La réduction du sulfoxyde et l'élimination des radicaux protecteurs s'effectuent dans les conditions décrites précédemment.

E/ Selon l'invention, les produits de formule générale (I), dans laquelle, R° et R' étant définis comme ci-dessus, R représente un radical dioxo-5,6 tétrahydro-1,4,5,6 triazine-1,2,4 yle-3 substitué en position —1 ou —4, dioxo-5,6 tétrahydro-1,2,5,6 triazine-1,2,4 yle-3 substitué en position —2 ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position —1, par un radical alcoyle contenant 2 à 4 atomes de carbone substitué par un groupement carbamoyloxy ou acyloxy (dont la partie acyle est éventuellement substituée par un radical amino, alcoylamino ou dialcoylamino), qui sont des esters de l'alcool correspondant de formule générale (I) dans laquelle R° et R' sont définis comme précédemment et R est un radical -(R)-alk'-OH choisi parmi dioxo-5,6 hydroxyalcoyle-1 (ou -4) tétrahydro-1,4,5,6 triazine-1,2,4 yle-3, dioxo-5,6 hydroxyalcoyl-2 tétrahydro-1,2,5,6 triazine-1,2,4 yle-3 ou bien hydroxyalcoyl-1 triazol-1,3,4 yle-5, alcoyloxycarbonyl-2 hydroxyalcoyl-1 triazol-1,3,4 yle-5, hydroxyalcoyl-1 triazol-1,2,4 yle-5 ou alcoyloxycarbonyl-3 hydroxyalcoyl-1 triazol-1,2,4 yle-5, peuvent être obtenus par carbamatation ou estérification d'un alcool de formule générale

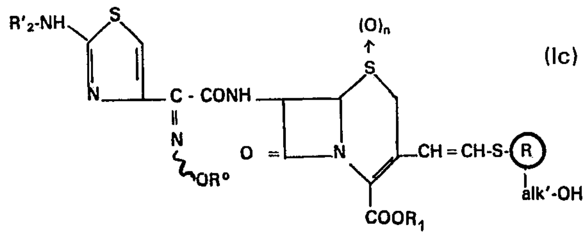

(Ic)

(dans laquelle R°, $R_1$, (R)-alk'-OH et n sont définis comme précédemment étant entendu que R° peut être libre ou protégé, et $R'_2$ est défini comme $R_2$ à l'exception de représenter l'hydrogène), par toute méthode connue pour obtenir un ester ou un carbamate à partir d'un alcool sans toucher au reste de la molécule, puis s'il y a lieu, réduction du sulfoxyde obtenu et élimination des radicaux protecteurs.

L'estérification s'effectue à une température comprise entre —50°C et la température de reflux du mélange réactionnel, notamment par condensation de l'anhydride de l'acide (ou d'un autre dérivé réactif, par exemple halogénure) dans un solvant organique inerte tel qu'un éther (par exemple tétrahydrofuranne), un solvant chloré (par exemple dichlorométhane), ou un mélange de ces solvants, en présence d'une base azotée comme la pyridine, la diméthylamino-4 pyridine ou une trialcoylamine (triéthylamine) ou d'un agent alcalin de condensation (par exemple bicarbonate de sodium) puis, le cas échéant, réduction du S-oxyde obtenu et élimination des groupements protecteurs selon les méthodes décrites précédemment.

Lorsque le groupement acyloxy contient un atome de carbone et est substitué par amino (groupement carbamoyloxy), on opère notamment par action d'isocyanate de chlorosulfonyle ou de trichloracétyle dans un solvant organique inerte, par exemple le tétrahydrofuranne ou l'acétonitrile, à une température comprise entre —80 et 20°C, puis élimination des groupements protecteurs.

Les produits de formule générale (Ic) peuvent être obtenus selon l'un ou l'autre des procédés décrits précédemment.

F/ Selon l'invention, les produits de formule générale (I) dans laquelle, R° et R' étant définis comme précédemment, R représente un radical dioxo-5,6 tétrahydro-1,4,5,6 triazine-1,2,4 yle-3 substitué en position —1 ou —4, dioxo-5,6 tétrahydro-1,2,5,6 triazine-1,2,4 yle-3 substitué en position —2 ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitué en position —1, [par un radical alcoyle contenant 2 à 4 atomes de carbone substitué par un groupement sulfoamino, alcoylsulfonylamino, sulfamoylamino, acylamino

(dont la partie acyle est éventuellement substituée par hydroxy, amino, alcoylamino ou dialcoylamino), alcoyloxycarbonylamino, uréido, alcoyluréido ou dialcoyluréido], ou représente un radical thiadiazol-1,3,4 yle-5 substitué par un radical acylamino ou acylaminoalcoyle, ou représente un radical oxadiazol-1,3,4 yle-5 substitué par un radical acylaminoalcoyle, ou représente un radical tétrazolyle-5 substitué en position —1 par un radical alcoyle contenant 2 à 4 atomes de carbone substitué par un groupement acylamino, sulfamoylamino, sulfoamino, uréido, alcoyluréido ou dialcoyluréido, qui sont tous des dérivés fonctionnels de l'amine correspondante, peuvent être obtenus par traitement d'une amine de formule générale:

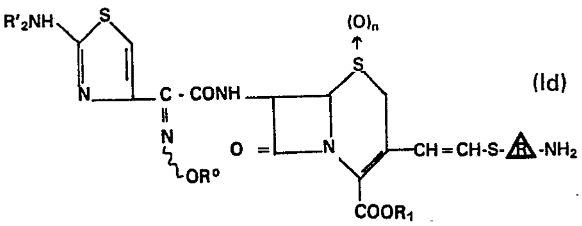

(Id)

dans laquelle R°, $R_1$, $R'_2$ et n sont définis comme précédemment, et -$\triangle$R-$NH_2$ représente un radical dioxo-5,6 tétrahydro-1,4,5,6 triazine-1,2,4 yle-3 substitué en position —1 ou —4, dioxo-5,6 tétrahydro-1,2,5,6 triazine-1,2,4 yle-3 substitué en position —2, ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitué en position —1, par un radical aminoalcoyle dont la partie alcoyle contient 2 à 4 atomes de carbone, ou un radical thiadiazol-1,3,4 yle-5 substitué par un radical amino ou aminoalcoyle, ou un radical oxadiazol-1,3,4 yle-5 substitué par un radical aminoalcoyle, ou un radical tétrazolyle-5 substitué en position —1 par un radical aminoalcoyle dont la partie alcoyle contient 2 à 4 atomes de carbone, par toute méthode connue en soi pour former une fonction amide, sulfamide, carbamate ou urée, sans toucher au reste de la molécule, puis le cas échéant réduction du sulfoxyde et élimination des groupements protecteurs.

Il est entendu que les produits qui contiennent un groupement sulfo, sulfonyle ou sulfamoyle sont préparés de préférence à partir d'un produit de formule générale (Id) dans laquelle n = 0.

Par ailleurs, lorsque l'on veut préparer un produit dont le radical R contient un groupement amino ou hydroxy, il est nécessaire de protéger ces radicaux dans le réactif utilisé. De même, lorsque R° représente l'atome d'hydrogène, il est nécessaire de protéger l'oxime. Lorsque R° contient un radical carboxy, ce groupement peut être libre ou protégé.

Lorsque l'on veut préparer un produit de formule générale (I) dans laquelle le radical R contient un substituant alcoylsulfonylamino, sulfamoylamino, acylamino (substitué ou non), alcoyloxycarbonylamino ou dialcoyluréido, la réaction est effectuée avantageusement par action, respectivement, du dérivé chlorosulfonyle, du chlorure d'acide, du chloroformiate ou du chlorure de dialcoylcarbamoyle correspondant dans les conditions décrites précédemment pour la réaction du chlorure de l'acide de formule générale (VIII) sur l'amino-7 céphalosporine de formule générale (IX).

Lorsque l'on veut préparer un produit de formule générale (I) dans laquelle le radical R contient un substituant sulfoamino, alcoylsulfonylamino ou acylamino (substitué ou non), on peut effectuer la réaction au moyen de l'anhydride de l'acide correspondant, dans les conditions décrites précédemment pour faire réagir le produit de formule générale (VIII) sous forme d'anhydride.

Lorsque l'on veut obtenir un produit de formule générale (I) pour lequel R contient un radical acylamino (substitué ou non), il est également possible de faire agir l'acide correspondant, dans les conditions opératoires décrites précédemment pour l'emploi de l'acide de formule générale (VIII).

Lorsque l'on veut obtenir un produit de formule générale (I) dans laquelle R contient un radical uréido ou alcoyluréido, on fait agir respectivement un isocyanate alcalin ou un isocyanate d'alcoyle sur le produit correspondant de formule générale (Id) en milieu hydroorganique ou organique (par exemple dans le tétrahydrofuranne) à une température comprise entre —20 et 60°C.

La réduction et l'élimination des radicaux protecteurs s'effectuent dans les conditions décrites précédemment.

G/ Selon la présente invention, les produits de formule générale (I) dans laquelle R° et R' sont définis comme précédemment et R représente un radical dioxo-5,6 tétrahydro-1,4,5,6 triazine-1,2,4 yle-3 substitué en position —1 ou —4, dioxo-5,6 tétrahydro-1,2,5,6 triazine-1,2,4 yle-3 substitué en position —2 ou bien triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position —1, par un radical thiazolidinyl-2 alcoyle, par un radical de formule générale (V) ou par un radical hydroxyiminoalcoyle ou alcoyloxyiminoalcoyle dont la partie iminoalcoyle contient 2 à 5 atomes de carbone ou bien représente un radical tétrazolyle-5 substitué en position —1 par un radical hydroxyiminoalcoyle ou alcoyloxyiminoalcoyle dont la partie iminoalcoyle contient 2 à 5 atomes de carbone, qui sont des dérivés d'addition du produit de formule générale (I) dans laquelle R° et R' sont définis comme précédemment et R est l'un des hétérocycles cités cidessus substitué par un radical formylalcoyle (ou sa forme hydrate), peuvent être obtenus à partir d'un aldéhyde de formule générale:

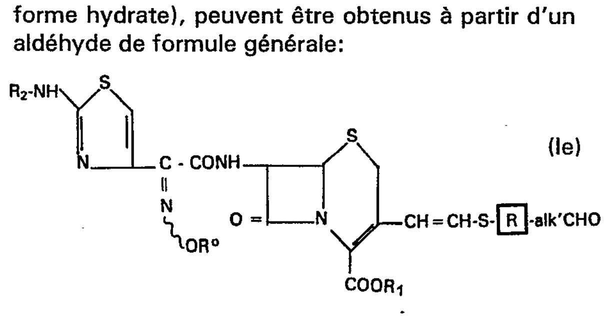

(Ie)

dans laquelle R°, $R_1$ et $R_2$ sont définis comme précédemment (lorsque R° contient un radical carboxy, celui-ci est libre ou protégé) et -$\boxed{R}$-alk'CHO repré-

sente un radical dioxo-5,6 formylalcoyl-1 (ou -4) tétrahydro-1,4,5,6 triazine-1,2,4 yle-3, dioxo-5,6 formylalcoyl-2 tétrahydro-1,2,5,6 triazine-1,2,4 yle-3 ou bien formylalcoyl-1 triazine-1,3,4 yle-5, alcoyloxycarbonyl-2 formylalcoyl-1 triazol-1,3,4 yle-5, formylalcoyl-1 triazol-1,2,4 yle-5, alcoyloxycarbonyl-3 formylalcoyl-1 triazol-1,2,4 yle-5 ou formylalcoyl-1 tétrazolyle-5, par addition respectivement de cystéamine, d'un alcool, d'hydroxylamine ou d'une alcoyloxyamine selon les méthodes connues pour former des dérivés d'addition de fonctions carbonylées, puis s'il y a lieu élimination des radicaux protecteurs.

La réaction s'effectue généralement dans un solvant organique à une température comprise entre 20°C et la température de reflux du mélange réactionnel.

Les solvants organiques sont choisis en fonction de la solubilité des produits. Lorsque l'on met en oeuvre un produit de formule générale (Ie) dans laquelle $R_1$ et $R_2$ sont autres que l'hydrogène et que le radical carboxy contenu par R° est protégé, on utilise avantageusement des solvants tels que le tétrahydrofuranne, l'acétonitrile, des alcools, des cétones. Lorsque l'on met en oeuvre un produit de formule générale (Ie) dans laquelle $R_1$ et $R_2$ sont des atomes d'hydrogène, et R° contient un radical carboxy libre on opère avantageusement dans des solvants tels que la pyridine, le diméthylsulfoxyde ou le diméthylformamide.

Lorsque l'on veut préparer un produit de formule générale (I) pour lequel le radical R contient un substituant de formule générale (V), on opère en milieu acide.

H/ Selon la présente invention, les produits de formule générale (I) dans laquelle R' représente un radical de formule générale (II) dans laquelle R'' et R''' sont définis comme précédemment, peuvent être obtenus par estérification d'un produit de formule générale (I) dans laquelle R' représente un atome d'hydrogène et dont la fonction amine et le cas échéant le radical carboxy contenu par R° ont été préalablement protégés, par toute méthode connue en soi pour préparer un ester à partir d'un acide sans toucher au reste de la molécule.

Généralement on fait réagir un sel de métal alcalin ou un sel d'amine tertiaire d'un produit de formule générale (I) tel que défini ci-dessus dont la fonction amine et la fonction acide de R° ont été préalablement protégées et dont, le cas échéant, le radical R et l'oxime sont également protégés, sur un produit de formule générale:

$$Z_2 - \underset{\displaystyle R''}{\underset{|}{CH}} - OCO - R''' \quad (XIX)$$

dans laquelle R'' et R''' sont définis comme précédemment et $Z_2$ représente un atome d'halogène, dans un solvant inerte tel que le diméthylformamide, à une température comprise entre 0 et 30°C.

I/ Selon la présente invention, les produits de formule générale (I) dans laquelle R° représente un radical de formule générale (VI) et R est autre qu'un radical triazinyle ou triazolyle substitués par un groupement de formule générale (V) peuvent être également obtenus par action d'un produit de formule générale:

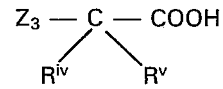

(VIa)

(dans laquelle $R^{iv}$ et $R^{v}$ sont définis comme précédemment, $Z_3$ représente un atome d'halogène ou un radical sulfate ou sulfonate, et la fonction acide a été préalablement protégée) sur un produit de formule générale:

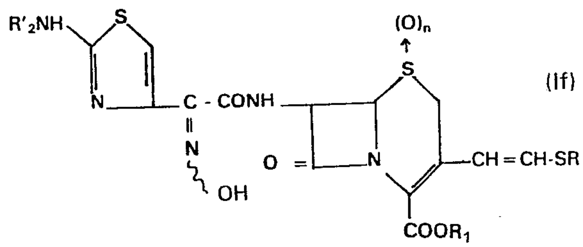

(If)

dans laquelle R, $R_1$, $R'_2$ et n sont définis comme précédemment puis, le cas échéant, réduction du sulfoxyde obtenu et élimination des groupements protecteurs.

La fonction acide du produit de formule générale (VIa) peut être protégée par tout groupement protecteur défini précédemment pour la protection des radicaux carboxy.

Il est entendu que les groupements amino ou alcoylamino qui existent dans certains radicaux R sont protégés et les groupements formyle sont libres ou protégés.

La réaction s'effectue généralement en présence d'une base minérale ou organique (par exemple la soude, la potasse, l'hydrure de sodium, le carbonate de potassium ou une base azotée comme la triéthylamine), dans un solvant organique tel qu'un solvant chloré (par exemple dichlorométhane, dichloroéthane), un éther (par exemple tétrahydrofuranne, dioxanne), une cétone (par exemple acétone), un amide (par exemple diméthylformamide), éventuellement en présence d'eau.

La réduction du sulfoxyde et l'élimination des radicaux protecteurs s'effectuent dans les conditions décrites précédemment.

J/ Selon la présente invention, les produits de formule générale (I) pour lesquels R ne contient pas de substituant de formule générale (V) peuvent également être préparés à partir d'un produit (ou du mélange des isomères du produit) de formule générale:

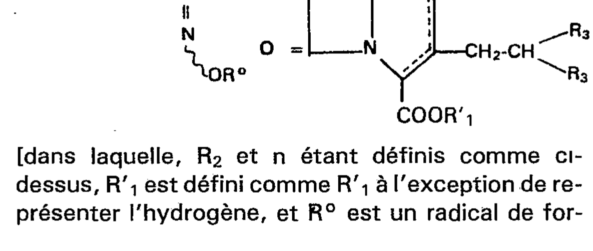

(XIIa)

[dans laquelle, $R_2$ et n étant définis comme ci-dessus, $R'_1$ est défini comme $R'_1$ à l'exception de représenter l'hydrogène, et R° est un radical de formule générale (VI) protégé, un radical alcoyle, vinyle, cyanométhyle ou un radical protecteur d'oxime; lorsque n = 0 le produit se présente sous forme bicyclooctène-2 ou -3 et lorsque n = 1 le produit se présente sous forme bicyclooctène-2, et $R_3$ représente un atome de chlore ou de brome] par action d'un thiol (ou d'un de ses sels alcalins ou alcalino terreux) de formule générale (XI) dans laquelle R, qui est défini comme ci-dessus, est éventuellement protégé dans les conditions définies dans le procédé B, puis éventuellement réduction du sulfoxyde obtenu lorsque n = 1 et élimination des groupements protecteurs.

La réaction s'effectue dans les conditions décrites précédemment pour la préparation des produits de formule générale (I) à partir d'un produit de formule générale (XII) et d'un produit de formule générale (XI). Les conditions de protection (et de libération) des différents radicaux sont également les mêmes que pour le procédé B.

Les produits de formule générale (XIX) peuvent être préparés selon la méthode décrite dans la demande de brevet allemand 2 350 230.

Les produits de formule générale (VIII) pour lesquels R° représente un atome d'hydrogène ou un radical alcoyle peuvent être préparés selon la méthode décrite dans le brevet belge 850 662.

Les produits de formule générale (VIII) dans laquelle R° représente un radical vinyle peuvent être préparés selon la méthode décrite dans le brevet belge 869 079.

Les produits de formule générale (VIII) dans laquelle R° représente un radical cyanométhyle peuvent être préparés selon la méthode dans la demande de brevet allemand 2 812 625.

Les produits de formule générale (VIII) dans laquelle R° est un radical de formule générale (VI) peuvent être préparés selon les méthodes décrites dans les brevets belges 864 810, 865 298, 876 541 et 876 542.

Les produits de formule générale (IX) peuvent être obtenus à partir d'un produit de formule générale:

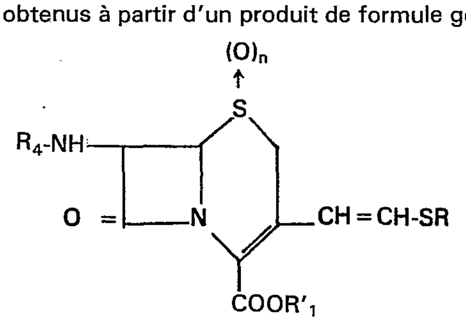

(XX)

(dans laquelle R et $R'_1$ sont définis comme ci-dessus, à l'exception de représenter un radical triazinyle ou triazolyle substitué par un radical de formule générale (V), n est défini comme précédemment et $R_4$ représente un radical facilement éliminable) par élimination du radical $R_4$ ou éventuellement élimination successive ou simultanée du radical $R_4$ et des autres radicaux protecteurs.

Il n'est pas nécessaire d'isoler le produit de formule générale (IX) pour l'utiliser dans la suite de la synthèse.

Par radical $R_4$ facilement éliminable on entend un radical benzhydryle ou trityle, un radical trichloro-2,2,2 éthyle, un radical acyle de formule générale:

$$R_5 — CO — \qquad (XXIa)$$

(dans laquelle $R_5$ est un atome d'hydrogène ou un radical alcoyle [éventuellement substitué par un ou plusieurs atomes d'halogène ou par un radical phényle ou phénoxy] ou phényle), ou bien un radical de formule générale:

$$R_6 — O — CO — \qquad (XXIb)$$

[[dans laquelle $R_6$ est un radical alcoyle ramifié non substitué, un radical alcoyle droit ou ramifié (portant un ou plusieurs substituants choisis parmi les atomes d'halogène, un radical cyano, trialcoylsilyle, phényle, phényle substitué (par un ou plusieurs radicaux alcoyloxy, nitro ou phényle)], vinyle, allyle ou quinolyle]] ou nitrophénylthio. De plus le radical $R_4NH$- peut être remplacé par un radical méthylèneamino dans lequel le radical méthylène est substitué par un groupement dialcoylamino ou aryle (ce dernier éventuellement substitué par un ou plusieurs radicaux méthoxy ou nitro).

Comme exemples de radicaux $R_4$ pouvant être utilisés on peut citer les radicaux suivants:

formyle, acétyle, chloracétyle, trichloracétyle, phénylacétyle, phénoxyacétyle, benzoyle,
t.butoxycarbonyle
chloro-2 diméthyl-1,1 éthoxycarbonyle
trichloro-2,2,2 éthoxycarbonyle
trichloro-2,2,2 diméthyl-1,1 éthoxycarbonyle
cyano-2 diméthyl-1,1 éthoxycarbonyle
triméthylsilyl-2 éthoxycarbonyle
benzyloxycarbonyle
p.méthoxybenzyloxycarbonyle
diméthoxy-3,5 benzyloxycarbonyle
p.nitrobenzyloxycarbonyle
diphénylméthoxycarbonyle
(biphénylyl-4)-2 isopropyloxycarbonyle
vinyloxycarbonyle
allyloxycarbonyle
quinolyl-8 oxycarbonyle
o.nitrophénylthio
p.nitrophénylthio.

Comme exemples de radicaux méthylèneamino, on peut citer:

diméthylaminométhylèneamino
diméthoxy-3,4 benzylidèneamino
nitro-4 benzylidèneamino.

L'élimination du radical protecteur $R_4$ s'effectue par toute méthode connue pour libérer une fonction amine sans toucher au reste de la molécule.

A titre d'exemple, on peut citer les méthodes suivantes:

— lorsque $R_4$ représente trityle, benzhydryle, trichloracétyle, chloracétyle, t.butoxycarbonyle, trichloréthoxycarbonyle, benzyloxycarbonyle, p.méthoxybenzyloxycarbonyle et p.nitrobenzyloxycarbonyle: selon les méthodes citées ci-dessus pour la libération du radical amino du produit de formule générale (I); dans le cas du radical t.butoxycarbonyle, on opère avantageusement par utilisation de l'acide

p.toluènesulfonique dans l'acétonitrile, à une température comprise entre 0 et 50°C,

— lorsque $R_4$ représente formyle, chloro-2 diméthyl-1,1 éthoxycarbonyle, cyano-2 diméthyl-1,1 éthoxycarbonyle, diméthoxy-3,5 benzyloxycarbonyle, diphénylméthoxycarbonyle, (biphénylyl-4)-2 isopropyloxycarbonyle, vinyloxycarbonyle, allyloxycarbonyle, quinolyl-8 oxycarbonyle, o.nitrophénylthio, p.nitrophénylthio, et lorsque $R_4NH$- est remplacé par diméthylaminométhylèneamino, diméthoxy-3,4 benzylidèneamino ou nitro-4 benzylidèneamino: par hydrolyse en milieu acide,

— lorsque $R_4$ représente trichloro-2,2,2 éthyle ou trichloro-2,2,2 diméthyl-1,1 éthoxycarbonyle: par traitement par le zinc dans l'acide acétique,

— lorsque $R_4$ représente acétyle, benzoyle, phénylacétyle ou phénoxyacétyle: selon la méthode décrite dans le brevet belge BE 758 800,

— lorsque $R_4$ représente triméthylsilyléthoxycarbonyle: selon la méthode décrite par H. GERLACH, Helv. Chim. Acta *60* (8), 3039 (1977),

— lorsque $R_4$ représente p.nitrobenzyloxycarbonyle: par hydrogénolyse en présence de palladium.

Les produits de formule générale (XX) peuvent être obtenus par action d'un thiol de formule générale (XI) tel que défini ci-avant, dont le radical R est éventuellement protégé, ou d'un de ses sels alcalins ou alcalino-terreux, sur un dérivé de céphalosporine (ou le cas échéant sur un mélange d'isomères d'un dérivé) de formule générale:

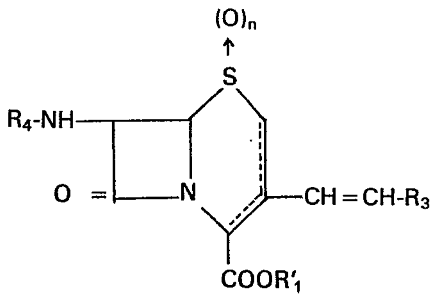

(XXII)

dans laquelle, $R'_1$, $R_3$, $R_4$ et n étant définis comme précédemment, lorsque n = 0 le produit se présente sous forme bicyclooctène-2 ou -3, lorsque n = 1 le produit se présente sous forme bicyclooctène-2 et le substituant sur l'atome de carbone en position —3 du bicyclooctène présente la stéréoisomérie E ou Z.

La réaction s'effectue généralement dans les conditions décrites précédemment pour l'obtention d'une thiovinyl-3 céphalosporine de formule générale (I) à partir d'un thiol de formule générale (XI) et d'un produit de formule générale (XII).

Les thiols de formule générale (XI) (qui peuvent être mis en oeuvre sous leur forme tautomère), peuvent être préparés par application de l'une des méthodes suivantes selon la signification du radical R:

— lorsque R est un radical pyridyle-3: selon la méthode décrite par H.M. WUEST et E.H. SAKAL, J. Am. Chem. Soc., *73,* 1210 (1951),

— lorsque R est un radical oxyde-1 pyridyle-3: selon la méthode décrite par B. BLANK et coll., J. Med. Chem., *17,* 1065 (1974),

— lorsque R est un radical oxyde-1 pyridyle-4: selon la méthode décrite par R.A.Y. JONES et coll., J. Chem. Soc. 2937 (1960),

— lorsque R est un radical pyridazinyle-3 substitué par alcoyle ou méthoxy et éventuellement N-oxydé: selon la méthode décrite dans le brevet belge 787 635,

— lorsque R est un radical pyridazinyle-3 substitué par amino et éventuellement N-oxydé: selon la méthode décrite dans le brevet belge 579 291,

— lorsque R est un radical pyridazinyle-3 substitué par acylamino et éventuellement N-oxydé: par application des méthodes décrites par M. KUMAGAI et M. BANDO, Nippon Kagaku Zasshi, *84* 995 (1963) et par T. HORIE et T. UEDA, Chem. Pharm. Bull., *11,* 114 (1963),

— lorsque R est un radical tétrazolo [4,5-b] pyridazinyle-6: selon la méthode décrite dans le brevet belge 804 251,

— lorsque R est un radical dioxo-5,6 tétrahydro-1,4,5,6 triazine-1,2,4 yle-3 substitué en position —4 ou alcoyloxycarbonyl-2 triazol-1,3,4 yle-5 substitué en position —1 par un radical $R^\gamma$ choisi parmi:

a) un radical allyle, alcoyle (1 à 4 atomes de carbone, lui-même éventuellement substitué par un radical alcoyloxy, alcoylthio, phényle, carbamoyle, alcoylcarbamoyle, dialcoylcarbamoyle, acyle, alcoyloxycarbonyle ou thiazolidinyle-2)

b) un radical dihydroxy-2,3 propyle ou dihydroxy-1,3 propyle-2 (éventuellement protégé sous forme d'acétal cyclique)

c) un radical alcoyle [2 à 4 atomes de carbone, lui-même substitué par hydroxy, carbamoyloxy, dialcoylamino, alcoylsulfinyle, alcoylsulfonyle, alcoylsulfonylamino, sulfamoylamino, acylamino (éventuellement substitué), alcoyloxycarbonylamino, uréido, alcoyluréido, dialcoyluréido],

d) un radical de formule générale (III) ou (IV),

e) un radical hydroxyiminoalcoyle ou alcoyloxyiminoalcoyle; ou lorsque R est un radical dioxo-5,6 tétrahydro-1,4,5,6 triazine-1,2,4 yle-3 substitué en position —1, dioxo-5,6 tétrahydro-1,2,5,6 triazine-1,2,4 yle-3 substitué en position —2 ou alcoyloxycarbonyl-3 triazol-1,2,4 yle-5 substitué en position —1 par un radical $R^\gamma$ tel que défini ci-dessus (à l'exception de représenter un radical alcoyle non substitué) ou par un radical hydroxyalcoylcarbamoylalcoyle dont la portion hydroxyalcoyle contient 2 à 4 atomes de carbone, ou encore lorsque R est un radical dioxo-5,6 hydroxyalcoylcarbamoylalcoyl-4 tétrahydro-1,4,5,6 triazine-1,2,4 yle-3 dont la portion hydroxyalcoyle contient 2 à 4 atomes de carbone: en faisant agir un oxalate d'alcoyle ou un halogénure de monooxalate d'alcoyle sur une thiosemicarbazide de formule générale:

$$H_2N\text{-}CS\text{-}NH\text{-}NH\text{-}R^\gamma \qquad \text{(XXIIIa)}$$

$$H_2N\text{-}CS\text{-}N(R^\gamma)\text{-}NH_2 \qquad \text{(XXIIIb)}$$

$$R^{\gamma 1}\text{-}NH\text{-}CS\text{-}NH\text{-}NH_2 \qquad \text{(XXIIIc)}$$

(dans lesquelles $R^\gamma$ est défini comme ci-dessus et $R^{\gamma 1}$ représente un radical hydroxyalcoylcarbamoylalcoyle ou un radical $R^\gamma$).

On effectue la réaction en présence d'un alcoolate alcalin, par exemple l'éthylate ou le méthylate de sodium ou le t.butylate de potassium, par application de la méthode décrite par M. PESSON et M. ANTOINE, Bull. Soc. Chim. France 1590 (1970).

Il n'est pas absolument nécessaire de purifier le produit obtenu (ni de libérer les radicaux protégés) pour le mettre en oeuvre pour la préparation des produits de formule générale (I).

Les thiosemicarbazides de formules générales (XXIIIa) à (XXIIIc) peuvent être préparés selon l'une des méthodes décrites par K.A. JENSEN et coll., Acta Chem. Scand., *22,* 1 (1968), ou par application de la méthode décrite par Y. KAZAKOV et J.Y. POTOVSKII, Doklady Acad. Nauk. SSSR *134,* 824 (1960), étant entendu que, lorsque $R^{\gamma}$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t-butoxycarbonyle, qui peut être éliminé par hydrolyse acide.

— Lorsque R est un radical triazol-1,3,4 yle-5 substitué en position —1
par un radical alcoyle, allyle ou alcoyloxyalcoyle,
par un radical alcoyle (1 à 4 atomes de carbone) luimême substitué comme défini ci-dessus en a) à l'exception d'un radical thiazolidinyle-2,
par un radical tel que défini ci-dessus en c), ou
par un radical alcoyloxyiminoalcoyle:
par application de l'une des méthodes décrites par M. PESSON et M. ANTOINE, Bull. Soc. Chim. France 1590 (1970).

— Lorsque R est un radical triazol-1,3,4 yle-5 substitué en position —1
par thiazolidinyl-2 alcoyle ou hydroxyiminoalcoyle:
par action respectivement de cystéamine ou d'hydroxylamine sur un dialcoyloxyalcoyl-1 mercapto-5 triazole-1,3,4 qui peut être obtenu par application de la méthode décrite par M. KANAOKA, J. Pharm. Soc. Japan, *75,* 1149 (1955), à partir d'une dialcoyloxyalcoyl-4 thiosemicarbazide.

— Lorsque R est un radical triazol-1,3,4 yle-5 substitué en position —1
par dihydroxy-2,3 propyle ou dihydroxy-1,3 propyle-2 (éventuellement protégés sous forme d'acétal cyclique), ou représente un radical de formule générale (III) ou (IV):
par application de la méthode décrite par M. KANAOKA, J. Pharm. Soc. Japan, *75,* 1149 (1955).

— Lorsque R est un radical dioxo-5,6 tétrahydro-1,4,5,6 triazine-1,2,4 yle-3 substitué en position —4 ou un radical alcoyloxycarbonyl-2 triazol-1,3,4 yle-5 ou triazol-1,3,4 yle-5 substitués en position —1
par acyloxyalcoyle (éventuellement substitué):
par acylation respectivement de la dioxo-5,6 hydroxyalcoyl-4 mercapto-3 tétrahydro-1,4,5,6 triazine-1,2,4, de l'alcoyloxycarbonyl-2 hydroxyalcoyl-1 mercapto-5 triazole-1,3,4 ou de l'hydroxyalcoyl-1 mercapto-5 triazole-1,3,4 dont le radical mercapto a été préalablement protégé [par exemple selon C.G. KRUSE et coll., Tet. Lett. 1725 (1976)], par toute méthode connue pour acyler un alcool sans toucher au reste de la molécule, puis libération du groupement mercapto en milieu acide.

— Lorsque R est un radical dioxo-5,6 tétrahydro-1,4,5,6 triazine-1,2,4 yle-3 substitué en position —4 ou alcoyloxycarbonyl-2 triazol-1,3,4 yle-5 ou triazol-1,3,4 yle-5 substitué en position —1 par aminoalcoyle ou alcoylaminoalcoyle:
par libération de la fonction amine du produit correspondant, protégé par exemple par un groupement t.butoxycarbonyle.

— Lorsque R est un radical dioxo-5,6 tétrahydro-1,4,5,6 triazine-1,2,4 yle-3 substitué en position —4, alcoyloxycarbonyl-2 triazol-1,3,4 yle-5 ou triazol-1,3,4 yle-5 substitué en position —1 par sulfoaminoalcoyle:
à partir du produit correspondant substitué par un radical t.butoxycarbonylaminoalcoyle, par analogie avec la méthode décrite dans le brevet belge 847 237.

— Lorsque R est un radical dialcoyl-1,4 dioxo-5,6 tétrahydro-1,4,5,6 triazine-1,2,4 yle-3 ou alcoyl-1 dioxo-5,6 tétrahydro-1,4,5,6 triazine-1,2,4 yle-3:
selon la méthode décrite dans le brevet belge 830 455.

— Lorsque R est un radical alcoyl-2 dioxo-5,6 tétrahydro-1,2,5,6 triazine-1,2,4 yle-3 ou alcoyl-1 alcoyloxycarbonyl-3 triazol-1,2,4 yle-5:
selon la méthode décrite par M. PESSON et M. ANTOINE, C.R. Acad. Sci., Ser C, 267, 25, 1726 (1968).

— Lorsque R est un radical triazol-1,2,3 yle-5:
selon la méthode décrite dans la demande de brevet français 2 215 942.

— Lorsque R est un radical triazol-1,3,4 yle-5:
selon la méthode décrite par M. KANAOKA, J. Pharm. Soc. Jap. *75,* 1149 (1955).

— Lorsque R est un radical thiadiazol-1,3,4 yle-5 éventuellement substitué par alcoyle, alcoyloxy, alcoylthio, alcoylsulfonyle, amino, alcoylamino, dialcoylamino ou acylamino:
selon les méthodes décrites dans le brevet belge 830 821.

— Lorsque R est un radical thiadiazol-1,3,4 yle-5 substitué par hydroxyalcoyle, aminoalcoyle, alcoylaminoalcoyle ou dialcoylaminoalcoyle:
selon la méthode décrite dans la demande de brevet allemand 2 446 254.

— Lorsque R est un radical thiadiazol-1,3,4 yle-5 substitué par un radical carboxyalcoyle:
par application de la méthode décrite dans la demande de brevet allemand 1 953 861.

— Lorsque R est un radical thiadiazol-1,3,4 yle-5 substitué par un radical trifluorométhyle:
selon la méthode décrite dans la demande de brevet allemand 2 162 575.

— Lorsque R est un radical thiadiazol-1,3,4 yle-5 substitué par un radical carboxy:
selon la méthode décrite dans la demande de brevet japonais 77 48666.

— Lorsque R est un radical thiadiazol-1,3,4 yle-5 substitué par un radical acylaminoalcoyle:
selon la méthode décrite dans la demande de brevet japonais 76 80857.

— Lorsque R est un radical thiadiazol-1,3,4 yle-5

substitué par un radical hydroxyalcoylthio: par application de la méthode décrite par G. NANNINI, Arz. Forsch. *27* (2), 343 (1977).

— Lorsque R est un radical thiadiazol-1,2,4 yle-5 substitué par alcoyle ou alcoyloxy: selon la méthode décrite dans la demande de brevet allemand 2 806 226 ou selon Chem. Ber. *90,* 184 (1957).

— Lorsque R est un radical oxadiazol-1,3,4 yle-5 tel que défini dans la formule (I) en $\alpha$ 8 a/: par application de la méthode décrite par E. HOGGARTH, J. Chem. Soc. 4811 (1952).

— Lorsque R est un radical oxazolyle-2 ou alcoyl-4 oxazolyle-2: par application de la méthode décrite précédemment par C. BRADSHER, J. Org. Chem. *32,* 2079 (1967).

— Lorsque R est un radical tétrazolyle-5 éventuellement substitué en position —1 par alcoyle, hydroxyalcoyle ou phényle: selon les méthodes décrites dans le brevet belge 830 821.

— Lorsque R est un radical tétrazolyle-5 substitué en position —1 par alcoyloxyalcoyle: par addition d'azoture de sodium sur un isothiocyanatoalcoyloxyalcoyle en opérant dans un solvant organique tel que l'éthanol, à la température de reflux du mélange réactionnel.

L'isothiocyanatoalcoyloxyalcoyle peut être obtenu par application de la méthode décrite par E. Schmidt et coll., Chem. Ber. *73* 286 (1940).

— Lorsque R est un radical tétrazolyle-5 substitué en position —1 par un radical carboxyalcoyle: selon la méthode décrite dans le brevet belge 858 112.

— Lorsque R est un radical tétrazolyle-5 substitué en position —1 par un radical sulfoalcoyle: selon la méthode décrite dans le brevet belge 856 498 ou décrite par D.A. BERGES et coll., J. Het. Chem. *15,* 981 (1978).

— Lorsque R est un radical tétrazolyle-5 substitué en position —1 par un radical aminoalcoyle, alcoylaminoalcoyle, dialcoylaminoalcoyle: par application de la méthode décrite dans la demande de brevet allemand 2 738 711.

— Lorsque R est un radical tétrazolyle-5 substitué en position —1 par un radical sulfamoylalcoyle, sulfamoylaminoalcoyle ou sulfoaminoalcoyle: selon la méthode décrite dans le brevet belge 856 636.

— Lorsque R est un radical tétrazolyle-5 substitué par un radical acylaminoalcoyle ou thiadiazol-1,3,4 yle-5 substitué par hydroxy: selon la méthode décrite dans le brevet US 4 117 123.

— Lorsque R est un radical tétrazolyle-5 substitué en position —1 par un radical uréidoalcoyle, alcoyluréidoalcoyle ou dialcoyluréidoalcoyle: à partir du produit correspondant substitué par aminoalcoyle (dont le radical mercapto a été préalablement protégé), par traitement par un isocyanate alcalin, par un isocyanate d'alcoyle ou par un halogénure de dialcoylcarbamoyle, puis libération du groupement mercapto dans les conditions décrites dans le brevet belge 847 237.

— Lorsque R est un radical tétrazolyle-5 substitué en position —1 par un radical carboxyalcoylaminoalcoyle: selon la méthode décrite dans la demande de brevet allemand 2 715 597.

— Lorsque R est un radical tétrazolyle-5 substitué en position —1 par un radical dihydroxy-2,3 propyle: selon la méthode décrite dans le brevet US 4 064 242.

— Lorsque R est un radical tétrazolyle-5 substitué en position —1 par un radical dihydroxy-1,3 propyle-2: par addition d'azoture de sodium sur un isothiocyanate de diméthyl-2,2 dioxolanne-1,3 yle-5 (suivie éventuellement de la libération des groupements hydroxy).

— Lorsque R est un radical tétrazolyle-5 substitué en position —1 par un radical de formule générale (IIIa) ou (IV) tels que définis précédemment ou un radical défini précédemment en $\alpha$ 9c/: par action d'azoture de sodium sur l'isothiocyanate correspondant, par analogie avec la méthode décrite par R.E. ORTH, J. Pharm. Sci. *52* (9), 909 (1963), étant entendu que dans le cas où R contient un substituant hydroxy ou hydroxyiminoalcoyle, l'alcool ou l'oxime sont éventuellement protégés par exemple par un groupement tétrahydropyrannyle.

— Lorsque R est un radical triazol-1,2,4 yle-5 substitué en position —1 par un radical allyle ou alcoyloxyalcoyle,
par un radical alcoyle (1 à 4 atomes de carbone) lui-même substitué comme défini ci-dessus en a) à l'exception d'un radical thiazolidinyle-2, ou
par un radical hydroxyalcoylcarbamoylalcoyle dont la portion hydroxyalcoyle contient 2 à 4 atomes de carbone,
par un radical tel que défini ci-dessus en c), ou
par un radical alcoyloxyiminoalcoyle:
par analogie avec les méthodes décrites par M. PESSON et M. ANTOINE, Bull. Soc. Chim. France 1599 (1970) ou C.R. Acad. Sci., Ser C, *267,* 25, 1726 (1968).

— Lorsque R est un radical triazol-1,2,4 yle-5 substitué en position —1 par thiazolidinyl-2 alcoyle ou par hydroxyiminoalcoyle: par action respectivement de cystéamine ou d'hydroxylamine sur un dialcoyloxyalcoyl-1 mercapto-5 triazole-1,2,4 qui peut être obtenu par analogie avec la méthode décrite par M. KANAOKA, J. Pharm. Soc. Japan, *75,* 1149 (1955), à partir d'une dialcoyloxyalcoyl-4 thiosemicarbazide.

— Lorsque R est un radical triazol-1,2,4 yle-5 substitué en position —1 par dihydroxy-2,3 propyle ou dihydroxy-1,3 propyle-2 (éventuellement protégés sous forme d'acétal cyclique), ou par un radical de formule générale (III) ou (IV): par analogie avec la méthode décrite par M. KANAOKA, J. Pharm. Soc. Japan, *75,* 1149 (1955).

— Lorsque R est un radical dioxo-5,6 tétrahydro-1,4,5,6 triazine-1,2,4 yle-3 substitué en position —1, dioxo-5,6 tétrahydro-1,2,5,6 triazine-1,2,4 yle-3 substitué en position —2 ou un radical alcoyloxycarbonyl-3 triazol-1,2,4 yle-5 ou triazol-1,2,4 yle-5 substitués en position —1, par acyloxyalcoyle (éventuellement substitué):

par acylation respectivement de la dioxo-5,6 hydroxyalcoyl-1 mercapto-3 tétrahydro-1,4,5,6 triazine-1,2,4, de la dioxo-5,6 hydroxyalcoyl-2 mercapto-3 tétrahydro-1,2,5,6 triazine-1,2,4, de l'alcoyloxycarbonyl-3 hydroxyalcoyl-1 mercapto-5 triazole-1,2,4 ou de l'hydroxyalcoyl-1 mercapto-5 triazole-1,2,4 dont le radical mercapto a été préalablement protégé [par exemple selon C.G. KRUSE et coll., Tet. Lett. 1725 (1976)], par toute méthode connue pour acyler un alcool sans toucher au reste de la molécule, puis libération du groupement mercapto en milieu acide.

— Lorsque R est un radical dioxo-5,6 tétrahydro-1,4,5,6 triazine-1,2,4 yle-3 substitué en position —1, dioxo-5,6 tétrahydro-1,2,5,6 triazine-1,2,4 yle-3 substitué en position —2 ou alcoyloxycarbonyl-3 triazol-1,2,4 yle-5 ou triazol-1,2,4 yle-5 substitués en position —1, par aminoalcoyle ou alcoylaminoalcoyle:

par libération de la fonction amine du produit correspondant, protégée par exemple par un groupement t.butoxycarbonyle.

— Lorsque R est un radical dioxo-5,6 tétrahydro-1,4,5,6 triazine-1,2,4 yle-3 substitué en position —1, dioxo-5,6 tétrahydro-1,2,5,6 triazine-1,2,4 yle-3 substitué en position —2, alcoyloxycarbonyl-3 triazol-1,2,4 yle-5 ou triazol-1,2,4 yle-5 substitués en position —1, par sulfoaminoalcoyle:

à partir du produit correspondant substitué par un radical t.butoxycarbonylaminoalcoyle, par analogie avec la méthode décrite dans le brevet belge 847 237.

— Lorsque R est un radical alcoyl-1 dioxo-5,6 tétrahydro-1,4,5,6 triazine-1,2,4 yle-3 substitué en position —4, alcoyl-1 dioxo-5,6 tétrahydro-1,2,5,6 triazine-1,2,4 yle-3 substitué en position —2, alcoyl-2 dioxo-5,6 tétrahydro-1,2,5,6 triazine-1,2,4 yle-3 substitué en position —1 ou alcoyl-4 dioxo-5,6 tétrahydro-1,4,5,6 triazine-1,2,4 yle-3 substitué en position —1, par un radical formylalcoyle ou par un radical de formule générale (IIIa):

en faisant agir un oxalate d'alcoyle sur une thiosemicarbazide, par analogie avec la méthode décrite par M. PESSON et M. ANTOINE, C.R. Acad. Sci. *267*, 15C, 904 (1968) ou C.R. Acad. Sci. *267*, 25, 1726 (1968).

Les produits de formule générale (XII) ou (XXII) peuvent être préparés soit par action d'un dérivé activé des acides $R'_3SO_3H$ et $R''_3COOH$ de formule générale:

$$\left.\begin{array}{ll} (R'_3SO_2)_2O & (a) \\ R'_3SO_2Hal & (b) \\ (R''_3CO)_2O & (c) \\ R''_3COHal & (d) \end{array}\right\} \quad (XXIV)$$

[dans ces formules $R'_3$ et $R''_3$ sont définis comme précédemment et Hal représente un atome d'halogène], soit par action d'un agent d'halogénation, sur un produit (ou un mélange des isomères) de formule générale:

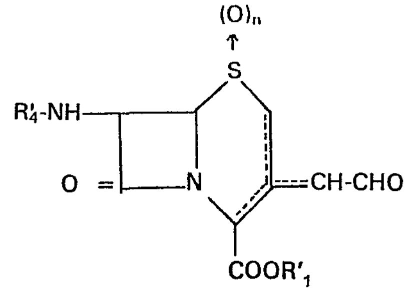

(XXV)

[dans laquelle, n et $R'_1$ étant définis comme précédemment,
lorsque n = 0 le produit se présente sous forme bicyclooctène-2 ou -3 ou oxoéthylidène-3 bicyclooctane et
lorsque n = 1 le produit se présente sous forme bicyclooctène-2 ou oxoéthylidène-3 bicyclooctane et $R'_4$ représente un radical de formule générale:

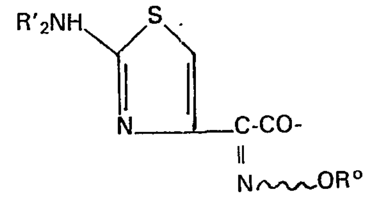

(VIIIa)

dans laquelle R° est défini comme dans la formule générale (I) et $R'_2$ est défini comme $R_2$, à l'exception de représenter l'hydrogène, ou bien $R'_4$ représente un radical $R_4$ tel que défini précédemment], suivie éventuellement de la réduction du sulfoxyde obtenu et éventuellement de l'élimination des radicaux protecteurs de la fonction amine et des fonctions acides (lorsque l'on veut obtenir un produit de formule générale (XII) pour lequel $R_1$ et/ou $R_2$ sont hydrogène).

Il est entendu que, lorsque $R'_4$ est un radical de formule générale (VIIIa) dans laquelle R° est un atome d'hydrogène, il est nécessaire de protéger l'oxime. La protection et la libération s'effectuent selon les méthodes décrites précédemment.

De même lorsque R° contient un radical carboxy, il est préférable de protéger ce groupement. Notamment lorsque l'on veut obtenir un produit de formule générale (XII) ou (XXII) pour lequel $R_3$ est un atome d'halogène, on met en oeuvre un produit de formule générale (XXV) dans laquelle le cas échéant le groupement carboxy contenu par $R'_4$ est protégé.

On opère généralement en présence d'une base tertiaire telle que définie par la formule générale (XIV) par exemple la triéthylamine ou la NN-diméthylaniline dans un solvant organique chloré (par exemple dichlorométhane), dans un ester (acétate d'éthyle), dans un éther (par exemple dioxanne, tétrahydrofuranne), dans un amide (par exemple diméthylacétamide, diméthylformamide) dans l'acétonitrile ou la N-méthylpyrrolidone ou dans un mélange de tels solvants ou directement dans un solvant basique comme la pyridine, ou bien lorsque $R_3$ est autre qu'un atome d'halogène, on peut opérer en milieu hydroorganique en présence d'un agent alcalin de condensation (par exemple bicarbonate alcalin, soude ou potasse), à une température comprise entre —78 °C et la température de reflux du mélange réactionnel.

Eventuellement on opère sous azote.

Il n'est pas absolument nécessaire d'avoir purifié l'intermédiaire de formule générale (XXV) pour mettre en oeuvre cette réaction.

Le cas échéant, l'élimination des radicaux protecteurs de la fonction amine et de la fonction acide s'effectue selon les méthodes décrites précédemment pour l'obtention du produit de formule générale (I).

Lorsque l'on veut préparer un produit de formule générale (XII) ou (XXII) dans laquelle $R_3$ est un atome d'halogène, l'agent d'halogénation peut être choisi parmi des dérivés halogénés du phosphore, notamment les

— composés d'addition d'halogène et de triarylphosphite, ou bien

— le trichlorure de phosphore, l'oxychlorure de phosphore, le pentachlorure de phosphore, le dichlorotriphénylphosphorane ou le catéchyltrichlorophosphorane lorsque $R_3$ est un atome de chlore, ou

— le tribromure de phosphore, l'oxybromure de phosphore, le pentabromure de phosphore ou le catéchyltribromophosphorane lorsque $R_3$ est un atome de brome.

Lorsque l'on utilise le trichlorure (ou le tribromure) de phosphore, on fait agir ce réactif sur un produit de formule générale (XXV) dans laquelle n = 0.

Le catéchyltrichloro (ou tribromo) phosphorane, qui peut être préparé in situ, peut être obtenu selon la méthode décrite par H. GROSS et U. KARSH, J. Prakt. Chem., *29,* 315 (1965).

Les composés d'addition d'halogène et de triarylphosphite, qui peuvent être formés in situ, sont décrits par H.N. RYDON et B.L. TONGE, J. Chem. Soc., 3043 (1956), par J. MICHALSKI et coll., J. Org. Chem., *45,* 3122 (1980) ou dans le brevet belge 881 424 et peuvent être préparés selon les méthodes mentionnées dans ces documents.

La préparation des dérivés halogénés de formule générale (XII) ou (XXII) s'effectue en milieu anhydre.

Lorsque l'on veut préparer un produit de formule générale (XII) ou (XXII), dans laquelle $R_3$ est un atome de chlore ou de brome, selon les conditions opératoires on peut isoler l'intermédiaire dihalogéné de formule générale:

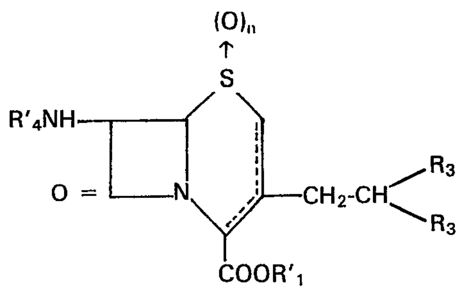

(XIIb)

[dans laquelle, n, $R'_4$, $R'_1$ et $R_3$ étant définis comme ci-dessus, le produit présente la même isomérie que le produit de formule générale (XIIa)] qui est ensuite déhydrohalogéné.

Lorsque l'on veut isoler l'intermédiaire dihalogéné, on opère par action d'un agent d'halogénation, dans un solvant organique tel qu'un solvant chloré (par exemple dichlorométhane, chloroforme, tétrachlorure de carbone, dichloro-1,2 éthane), un éther (par exemple éther éthylique, oxyde de propylène, tétrahydrofuranne, dioxanne), un amide (par exemple diméthylacétamide, diméthylpropionamide, diméthylformamide, N-acétylmorpholine, N-acétylpipéridine, N-méthylpyrrolidone) ou un mélange de tels solvants, à une température un peu moins élevée que pour préparer le dérivé halogénovinyle correspondant, c'est-à-dire comprise entre —78 et 30°C.

Il est également possible d'opérer en présence d'une base telle que la pyridine dans un solvant cité ci-dessus, à une température comprise entre —78 et 0°C.

La déhydrohalogénation s'effectue en présence d'une base tertiaire telle que définie précédemment, d'une amine aromatique (par exemple pyridine, picoline, quinoléine) ou d'une base minérale (telle que la soude, la potasse, un carbonate ou un bicarbonate alcalin ou alcalino-terreux), en milieu organique ou hydroorganique dans les solvants cités précédemment, à une température comprise entre —20°C et la température de reflux du mélange réactionnel.

Il n'est pas absolument nécessaire d'avoir purifié l'intermédiaire dihalogéné pour procéder à sa déhydrohalogénation.

Les produits de formule générale (XXV) dans laquelle $R'_4$ est autre qu'un radical de formule générale (VIIIa) et n est égal à 0, peuvent être obtenus par hydrolyse d'une énamine (ou du mélange d'énamines isomères) de formule générale:

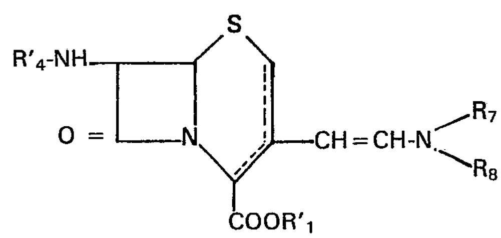

(XXVI)

dans laquelle, $R'_1$ et $R'_4$ étant définis comme précédemment, le produit se présente sous forme bicyclooctène-2 ou -3, et le substituant sur l'atome de carbone en position —3 du bicyclooctène présente la stéréoisomérie E ou Z, et

$R_7$ et $R_8$ qui sont identiques ou différents représentent des radicaux alcoyle (éventuellement substitués par un radical hydroxy, alcoyloxy, amino, alcoylamino ou dialcoylamino) ou phényle, ou forment ensemble avec l'atome d'azote auquel ils sont rattachés un hétérocycle saturé à 5 ou 6 chaînons, contenant éventuellement un autre hétéroatome choisi parmi l'azote, l'oxygène ou le soufre et éventuellement substitué par un radical alcoyle.

De préférence on hydrolyse une énamine de formule générale (XXVI) dans laquelle $R_7$ et $R_8$ représentent chacun un radical méthyle.

On opère généralement dans un acide organique (acide formique, acide acétique par exemple) ou minéral (acide chlorhydrique, acide sulfurique par exemple) en présence ou non d'un solvant, en milieu aqueux ou organique, à une température comprise entre —20°C et la température de reflux du mélange réactionnel. Lorsque l'on opère en milieu organique, l'hydrolyse est réalisée par addition d'eau au mélange réactionnel, puis on traite éventuellement par une base minérale (par exemple bicarbonate alcalin)

ou organique (par exemple amine tertiaire ou pyridine).

Lorsque l'on opère en présence d'un solvant, il n'est pas nécessaire que le solvant soit miscible à la phase aqueuse acide. Le contact est alors réalisé par agitation vive.

Parmi les solvants utilisables, peuvent être cités les solvants chlorés, l'acétate d'éthyle, le tétrahydrofuranne, l'acétonitrile, le diméthylformamide, les alcools. Il n'est pas absolument nécessaire d'avoir purifié l'intermédiaire de formule générale (XXVI) pour mettre en oeuvre cette réaction.

Les produits de formule générale (XXV), dans laquelle $R'_4$ est un radical de formule générale (VIIa) et n est égal à 0, peuvent être obtenus par hydrolyse d'une énamine (ou du mélange d'énamines isomères) de formule générale:

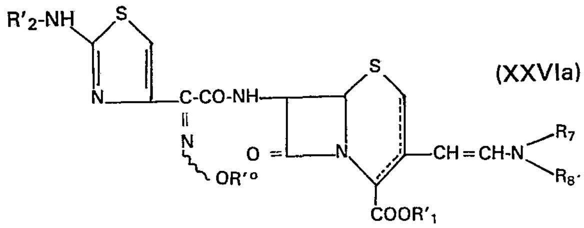
(XXVIa)

qui se présente sous forme bicyclooctène-2 ou -3 et dans laquelle le substituant sur l'atome de carbone en position —3 du bicyclooctène présente la stéréoisomérie E ou Z,
$R'_1$ et $R'_2$ sont définis comme précédemment,
$R^o$ représente un atome d'hydrogène, un radical alcoyle, vinyle, cyanométhyle ou un radical de formule générale (VI) protégé et
$R_7$ et $R_8$ sont définis comme dans la formule générale (XXVI).

On opère dans les conditions indiquées ci-dessus pour l'hydrolyse de l'énamine de formule générale (XXVI).

Lorsque l'on veut préparer un produit de formule générale (XXV) contenant un radical de formule générale (VI), il est nécessaire d'utiliser une énamine de formule générale (XXVIa) dans laquelle les radicaux protecteurs des fonctions acides (de $R'^o$ et $-COOR'_1$) sont différents et éliminables sélectivement.

L'élimination du radical protecteur de la fonction acide du radical de formule générale (VI) s'effectue ensuite dans les conditions décrites précédemment.

Les produits de formule générale (XXV) dans laquelle n est égal à 1 peuvent être obtenus par oxydation des produits de formule générale (XXV) pour lesquels n est égal à 0, par application de la méthode décrite dans la demande de brevet allemand 2 637 176.

De même les produits de formules générales (IX), (XII), (XIIa), (XIIb), (XVI), (XVIII) ou (XXII) dans lesquelles n est égal à 1 peuvent être obtenus respectivement par oxydation des produits de formules générales (IX), (XII), (XIIa), (XIIb), (XVI), (XVIII) ou (XXII) dans lesquelles n est égal à 0, par application de la méthode décrite dans la demande de brevet allemand 2 637 176.

Les produits de formule générale (XXVI) ou (XXVIa) dans laquelle $R_7$ et $R_8$ ont la définition précédente, à l'exception de représenter alcoyle substitué par hydroxy, amino ou alcoylamino, peuvent être obtenus par action d'un produit de formule générale:

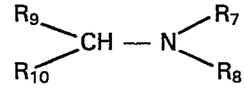
(XXVII)

éventuellement préparé in situ, [pour lequel $R_7$ et $R_8$ sont définis comme précédemment et $R_9$ et $R_{10}$, qui sont identiques ou différents, soit représentent des groupements de formule générale:

$$-X_2R_{11} \qquad \text{(XXVIIa)}$$

dans laquelle $X_2$ est un atome d'oxygène et $R_{11}$ représente un radical alcoyle ou phényle,
soit représentent, l'un un radical de formule générale (XXVIIa) (dans laquelle $X_2$ représente un atome d'oxygène ou de soufre et $R_{11}$ est alcoyle ou phényle), et l'autre un radical amino de formule générale:

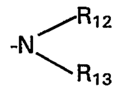
(XXVIIb)

dans laquelle $R_{12}$ et $R_{13}$ sont définis comme $R_7$ et $R_8$, soit encore $R_9$ et $R_{10}$ représentent chacun un radical de formule générale (XXVIIb)] sur un dérivé de céphalosporine de formule générale:

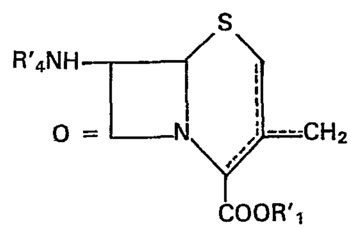
(XXVIII)

dans laquelle, $R'_1$ et $R'_4$ étant définis comme précédemment, le produit se présente sous forme méthyl-3 bicyclooctène-2 ou -3 ou méthylène-3 bicyclooctane.

Lorsque l'on utilise un produit de formule générale (XXVII) dans laquelle le radical (XXVIIb) est différent de $-NR_7R_8$, il est préférable de choisir un tel produit de manière que l'amine $HNR_{12}R_{13}$ soit plus volatile que $HNR_7R_8$.

On opère généralement dans un solvant organique tel que le diméthylformamide, l'hexaméthylphosphorotriamide, le diméthylacétamide, l'acétonitrile, l'acétate d'éthyle, le dioxanne ou un solvant chloré (par exemple dichloro-1,2 éthane) ou dans un mélange de tels solvants, à une température comprise entre 20°C et la température de reflux du mélange réactionnel.

Il est entendu que lorsque $R'^o$ est un atome d'hydrogène, il est préférable que l'oxime soit protégée dans les conditions décrites précédemment.

Les produits de formule générale (XXVI), dans laquelle $R'_1$ et $R'_4$ sont définis comme précédemment et $R_7$ et $R_8$ représentent des radicaux alcoyle substitués par hydroxy, amino ou alcoylamino, peuvent être obtenus par transénamination à partir d'un produit de formule générale (XXVI) dans laquelle $R_7$ et $R_8$ représentent des radicaux alcoyle, de préférence méthyle.

La réaction s'effectue par action d'une amine de formule générale:

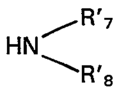

(XXIX)

(dans laquelle $R'_7$ et $R'_8$, qui sont identiques ou différents, représentent des radicaux alcoyle substitués par hydroxy, amino ou alcoylamino) sur le produit de formule générale (XXVI) et l'on opère dans les conditions décrites précédemment pour l'action d'un produit de formule générale (XXVII) sur un dérivé de formule générale (XXVIII).

Les produits de formule générale (XXVII) peuvent être préparés selon les méthodes décrites par H. BREDERECK et coll., Chem. Ber. *101* 41 (1968), Chem. Ber. *101,* 3058 (1968) et Chem. Ber. *106,* 3725 (1973).

Les dérivés de la céphalosporine de formule générale (XXVIII), dans laquelle $R'_4$ représente un radical de formule générale (VIIIa), peuvent être préparés à partir des produits de formule générale:

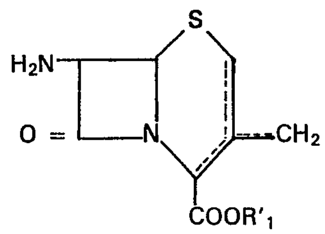

(XXX)

dans laquelle $R'_1$ est défini comme précédemment, en opérant par action d'un acide de formule générale (VIII) ou d'un de ses dérivés réactifs, dans les conditions décrites précédemment pour l'obtention des produits de formule générale (I).

L'introduction des groupements protecteurs $R'_1$ et $R'_4$ du produit de formule générale (XXVIII) [ou (XXX) pour $R'_1$] peut être effectuée sur une amino-7 céphalosporine de formule générale:

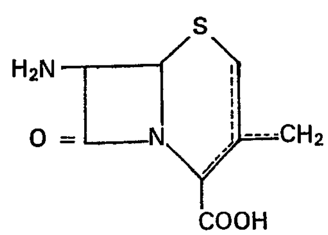

(XXXa)

dans laquelle la position de la double liaison est définie comme précédemment, selon les méthodes connues ou décrites dans la littérature:

— lorsque $R'_4$ est un radical formyle: selon J.C. SHEEHAN et coll., J. Amer. Chem. Soc. *80* 1156 (1958),

— lorsque $R'_4$ est acétyle, chloracétyle, trichloracétyle, phénylacétyle, phénoxyacétyle ou benzoyle: selon E.H. FLYNN, Cephalosporins and Penicillins, Ac. Press (1972),

— lorsque $R'_4$ est un radical t.butoxycarbonyle: selon L. MORODER et coll., Hoppe Seyler's Z. Physiol. Chem. *357* 1651 (1976),

— lorsque $R'_4$ est trichloro-2,2,2 diméthyl-1,1 éthoxycarbonyle: selon J. UGI et coll., Angew. Chem. In. Ed. Engl. *17(5)* 361 (1978),

— lorsque $R'_4$ est trichloro-2,2,2 éthoxycarbonyle, chloro-2 diméthyl-1,1 éthoxycarbonyle, cyano-2 diméthyl-1,1 éthoxycarbonyle, triméthylsilyl-2 éthoxycarbonyle, banzyloxycarbonyle, p.méthoxybenzyloxycarbonyle, diméthoxy-3,5 benzyloxycarbonyle, p.nitrobenzyloxycarbonyle, vinyloxycarbonyle: par action d'un chloroformiate en milieu hydroorganique en présence d'un bicarbonate alcalin, ou selon le brevet belge 788 885,

— lorsque $R'_4$ est diphénylméthoxycarbonyle: par action de l'azidoformiate correspondant en milieu hydroorganique, en présence d'un bicarbonate alcalin,

— lorsque $R'_4$ est (biphénylyl-4)-2 isopropyloxycarbonyle: par analogie avec la méthode décrite par Helv. Chim. Acta, *51,* 924 (1968),

— lorsque $R'_4$ est quinolyl-8 oxycarbonyle ou allyloxycarbonyle: par action du carbonate correspondant en milieu hydroorganique basique,

— lorsque $R'_4$ est o.nitrophénylthio ou p.nitrophénylthio: par analogie avec la méthode décrite par L. ZERVAS et coll., J. Amer. Chem. Soc. *85,* 3660 (1963),

— lorsque $R'_4NH$- est remplacé par diméthylaminométhylèneimino: par analogie avec la méthode décrite par J.F. FITT, J. Org. Chem. *42(15),* 2639 (1977),

— lorsque $R'_4NH$- est remplacé par nitro-4 benzylidèneimino ou diméthoxy-3,4 benzilidèneimino: selon la méthode décrite par R.A. FIRESTONE, Tetrahedron Lett., 375 (1972),

— lorsque $R'_1$ est méthoxyméthyle: selon S. SEKI et coll., Tetrahedron Lett., *33,* 2915 (1977),

— lorsque $R'_1$ est t.butyle: selon R.J. STEDMAN, J. Med. Chem., *9,* 444 (1966),

— lorsque $R'_1$ est benzhydryle: selon la demande de brevet néerlandais 73 03263,

— lorsque $R'_1$ est p.nitrobenzyle ou p.méthoxybenzyle: selon R.R. CHAUVETTE et coll., J. Org. Chem., *38(17)* 2994 (1973).

Les dérivés de la céphalosporine de formules générales (XXVIII) et (XXX) dans lesquelles $R'_1$ représente un radical de formule générale (II) peuvent être obtenus par estérification des acides correspondants selon la méthode décrite précédemment pour l'obtention d'un produit de formule générale (I) dans laquelle $R_1$ est un radical de formule générale (II) à partir d'un produit de formule générale (I) dans laquelle $R_1$ est un atome d'hydrogène.

Les produits de formule générale (XII) peuvent également être obtenus par action d'un acide de formule générale (VIII) dont la fonction amine et le cas échéant la fonction acide de $R°$ sont préalablement protégées ou par action de ses dérivés réactifs, sur un produit de formule générale (XVI) dans laquelle,

$R_1$, $R_3$ et n étant définis comme précédemment,

lorsque n = 0 le produit se présente sous forme bicyclooctène-2 ou -3,

lorsque n = 1 le produit se présente sous forme bicyclooctène-2 et le substituant sur l'atome de carbone en position —3 du bicyclooctène présente la stéréoisomérie E ou Z, ou le cas échéant sur un mélange des isomères de ce produit, suivie éventuellement de la réduction de l'oxyde obtenu, puis éventuellement de l'élimination des radicaux protecteurs.

La réaction s'effectue dans les conditions décrites précédemment pour l'action d'un acide de formule générale (VIII) sur une amino-7 céphalosporine de formule générale (IX).

Le cas échéant la réduction de l'oxyde ainsi que l'élimination des radicaux protecteurs peuvent être effectuées dans les conditions décrites pour obtenir le produit de formule générale (I).

Le produit de formule générale (XVI) peut être obtenu par élimination du radical protecteur $R_4$ d'un produit de formule générale (XXII), ou éventuellement par élimination simultanée des radicaux $R_4$ et $R'_1$ lorsque l'on veut obtenir un produit de formule générale (XVI) dans laquelle $R_1$ est hydrogène.

On opère généralement dans les conditions décrites précédemment pour l'obtention d'un produit de formule générale (IX) à partir d'un produit de formule générale (XX).

Les produits de formule générale (XII) dans laquelle R° est autre que vinyle peuvent également être préparés à partir d'un produit de formule générale:

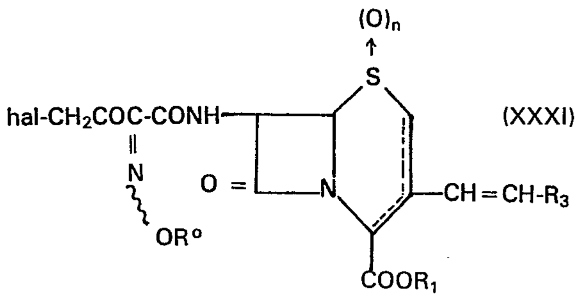

(XXXI)

[dans laquelle R°, $R_1$, $R_3$ et n sont définis comme précédemment à l'exception pour R° de représenter un radical vinyle et hal est défini comme dans la formule générale (XVIII)], par analogie avec la méthode décrite pour la préparation des produits de formule générale (I) dans le procédé D.

Les produits de formule générale (XXXI) peuvent être préparés à partir d'un produit de formule générale (XVI) par application des méthodes décrites ci-après pour la préparation de produits de formule générale (XVIII).

Les thioloesters de formule générale (XV) peuvent être préparés par action d'un acide ou d'un dérivé réactif d'un acide de formule générale:

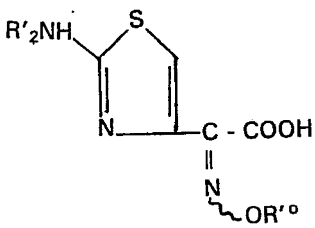

(VIIIb)

[dans laquelle R'° représente un radical alcoyle, vinyle, cyanométhyle, un radical de formule générale (VI) protégé ou un radical protecteur et dans laquelle $R'_2$ est défini comme $R_2$ à l'exception de représenter l'atome d'hydrogène (ou peut représenter l'atome d'hydrogène lorsque le dérivé réactif est le chlorure d'acide)] sur un thiol de formule générale (XI) [dans laquelle R est défini comme pour la formule (XV)] ou sur un de ses sels alcalins ou alcalino-terreux suivie de l'élimination du radical $R'_2$ protecteur du radical amino, lorsque l'on veut obtenir un produit pour lequel $R_2$ est un atome d'hydrogène, et éventuellement des autres radicaux protecteurs.

Lorsque l'on veut obtenir un produit de formule générale (XV) pour lequel R° est un atome d'hydrogène, la protection de l'oxime peut être effectuée par toute méthode connue qui n'altère pas le reste de la molécule. On utilise notamment le groupement trityle.

Par ailleurs, lorsque l'on veut obtenir un produit de formule générale (XV) pour lequel R contient un radical carboxy ou sulfo, il est préférable de faire agir un dérivé réactif de l'acide de formule générale (VIIIb) sur le thiol correspondant.

Lorsque l'on veut obtenir un produit pour lequel R contient un radical hydroxy, il est préférable de protéger préalablement ce radical, par exemple par un groupement trityle.

Il est avantageux de n'éliminer ces groupements protecteurs qu'après la réaction des thioloesters de formule générale (XV) avec les produits de formule générale (XVI) ou (IX)

a/ Lorsque l'on utilise le produit de formule générale (VIIIb) sous forme acide, la condensation s'effectue généralement dans un solvant organique tel que le diméthylformamide, l'acétonitrile, le tétrahydrofuranne, le dichlorométhane, le chloroforme ou l'acétate d'éthyle, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre —20 et 40°C, puis on élimine éventuellement les groupements protecteurs.

b/ Lorsque l'on utilise un dérivé réactif de l'acide de formule générale (VIIIb), il est possible de mettre en oeuvre l'anhydride, un anhydride mixte, un halogénure d'acide ou un ester réactif de formule générale:

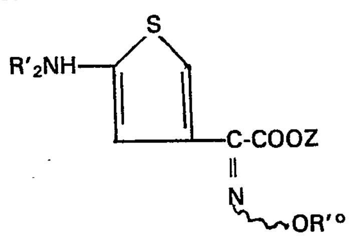

(Xa)

dans laquelle R'° et $R'_2$ sont définis comme ci-dessus et Z représente un radical tel que succinimido, benzotriazolyle-1, nitro-4 phényle, dinitro-2,4 phényle, pentachlorophényle ou phtalimido.

c/ Lorsque l'on veut obtenir un produit de formule générale (XV) dans laquelle $R_2$ est un atome d'hydrogène, il est également possible de mettre en oeuvre un halogénure d'acide, par exemple le chlorure d'acide, en faisant réagir le chlorhydrate du chlorure de l'acide de formule générale (VIII) sur le thiol, ou sur un de ses sels.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou dichlorométhane), un amide (par exemple diméthylformamide ou diméthylacétamide) ou une cétone (par exemple acétone), ou dans des mélanges de tels solvants, en présence d'un accepteur d'acide tel qu'un époxyde (par exemple oxyde de propylène) ou tel qu'une base organique azotée comme la pyridine, la N-méthylmorpholine ou une trialcoylamine (par exemple triéthylamine) ou dans un milieu hydroorganique en présence d'un agent alcalin de condensation tel que le bicarbonate de sodium, et l'on opère à une température comprise entre —40 et +40°C, puis on élimine éventuellement le ou les groupements protecteurs.

Lorsque l'on met en oeuvre un ester réactif de formule générale (Xa), on opère généralement en présence d'une trialcoylamine (par exemple triéthylamine) dans un solvant organique tel que le diméthylformamide à une température comprise entre 0 et 60°C, puis éventuellement on élimine le ou les groupements protecteurs.

A titre d'exemple, la libération des différents radicaux protégés peut être effectuée dans les conditions suivantes:

— lorsque l'on veut obtenir un produit de formule générale (XV) dans laquelle $R_2$ est hydrogène, on élimine le radical t.butoxycarbonyle protecteur de l'aminothiazole par traitement en milieu acide anhydride. Dans ce cas on obtient le produit soit à l'état de sel, soit à l'état de solvate avec l'acide employé. De préférence on utilise l'acide trifluoracétique en opérant entre 0 et 20°C. Il est également possible d'éliminer le radical benzyle protecteur de l'aminothiazole par hydrogénation catalytique.

— Lorsque l'on veut obtenir un produit de formule générale (XV) dans laquelle le radical R comprend un groupement hydroxy et/ou dans laquelle R° est un atome d'hydrogène, on élimine le ou les groupements trityle par acidolyse par l'acide trifluoracétique anhydride. L'élimination s'effectue avant, simultanément ou après l'élimination du radical protecteur de l'aminothiazole.

Selon l'invention les thioloesters de formule générale (XV) dans laquelle R contient un radical carbamoyloxyalcoyle ou acyloxyalcoyle (dont la partie acyle est éventuellement substituée par un radical amino ou alcoylamino protégés ou dialcoylamino), peuvent également être préparés à partir du thioloester correspondant de formule générale (XV) dans laquelle R contient un radical hydroxyalcoyle et dans laquelle le radical $R_2$ et le radical R° sont autres que l'atome d'hydrogène, par toute méthode connue pour obtenir un carbamate ou un ester à partir d'un alcool sans toucher au reste de la molécule.

On opère généralement selon les méthodes décrites pour l'obtention de produits de formule générale (I) contenant de tels substituants, à partir d'un produit de formule générale (Ic).

Les produits de formule générale (XVIII) dans laquelle R° est autre que l'atome d'hydrogène, peuvent être obtenus par action d'un halogénure d'acide de formule générale:

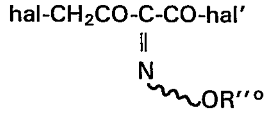

(XXXII)

dans laquelle hal est défini comme précédemment, R''° est défini comme R'° ci-dessus pour la formule générale (VIIIb) à l'exception de représenter un radical vinyle et hal' représente le chlore ou le brome, sur une amino-7 céphalosporine de formule générale (IX), suivie éventuellement de la réduction du sulfoxyde obtenu (lorsque n = 1) et de l'élimination des radicaux protecteurs.

La réaction s'effectue généralement en milieu hydroorganique par exemple eau-éther (tétrahydrofuranne, dioxanne), eau-cétone (acétone) ou eau-solvant chloré (chloroforme, dichlorométhane), en présence d'un agent alcalin de condensation tel qu'un bicarbonate alcalin (par exemple bicarbonate de sodium) à une température comprise entre —40 et 40°C.

Il est également possible d'opérer par analogie avec la méthode décrite dans la demande de brevet français 2 399 418.

Il est entendu que, lorsque le radical R de l'amino-7 céphalosporine contient un radical amino ou alcoylamino, celui-ci est protégé, et lorsque le radical R contient un radical hydroxy, carboxy, formyle ou acylalcoyle, ce dernier est libre ou protégé.

La protection et l'élimination des radicaux protecteurs s'effectue dans les conditions décrites précédemment.

Les produits de formule générale (XXXII) peuvent être obtenus par halogénation d'un produit de formule générale:

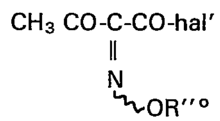

(XXXIII)

dans laquelle R''° et hal' sont définis comme ci-dessus, par toute méthode connue en soi, pour la préparation de dérivés halogénés, qui n'altère pas le reste de la molécule.

Lorsque l'on veut obtenir un produit de formule générale (XXXII) dans laquelle hal représente un atome de brome, on fait agir le brom en présence d'un catalyseur, soit un catalyseur acide tel que l'acide bromhydrique, l'acide chlorhydrique, les acides sulfoniques (acide méthanesulfonique, acide p.toluènesulfonique anhydride ou acide benzènesulfonique), soit en présence de lumière ultra-violette.

Lorsque l'on veut obtenir un produit de formule générale (XXXII) dans laquelle hal est un atome de chlore, on fait agir le chlore en présence d'un catalyseur tel que cité ci-dessus ou le chlorure de sulfuryle.

L'halogénation s'effectue dans un solvant organique tel que les solvants chlorés (par exemple dichlorométhane, chloroforme, tétrachlorure de carbone, dichloroéthane ou trichloroéthane) ou les

éthers (par exemple éther éthylique ou dioxanne) ou dans un mélange de tels solvants, à une température comprise entre —40°C et la température de reflux du mélange réactionnel.

Les produits de formule générale (XXXIII) peuvent être préparés par action d'un agent d'halogénation sur l'acide, un sel ou un ester silylique de l'acide de formule générale:

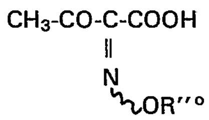

(XXXIV)

dans laquelle R''° est défini comme ci-dessus, en présence d'un produit de formule générale:

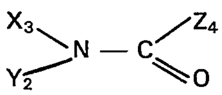

(XIVa)

dans laquelle $X_3$, $Y_2$ et $Z_4$ sont identiques ou différents et représentent des radicaux alcoyle ou phényle, ou 2 d'entre eux forment ensemble avec les atomes auxquels ils sont rattachés un hétérocycle à 5 ou 6 chaînons contenant éventuellement un autre hétéroatome choisi parmi l'oxygène, l'azote ou le soufre, ou bien $X_3$ et $Y_2$ représentent des radicaux alcoyle tels que définis précédemment et $Z_4$ représente un atome d'hydrogène ou un radical dialcoylamino.

L'agent d'halogénation peut être choisi parmi le pentachlorure de phosphore, l'oxychlorure de phosphore, le chlorure d'oxalyle ou le phosgène lorsque l'on veut obtenir le chlorure d'acide, ou bien parmi le pentabromure de phosphore, l'oxybromure de phosphore ou le bromure de thionyle lorsque l'on veut obtenir le bromure d'acide.

Parmi les produits de formule générale (XIVa) on utilise avantageusement le diméthylacétamide, le diéthylacétamide, le diméthylpropionamide, le diéthylpropionamide, le diméthylformamide, la N-acétylmorpholine, la N-acétylpipéridine, la N-acétyl N-méthylaniline, la N-méthylpyrrolidone ou la tétraméthylurée.

Lorsque l'on fait agir un sel de l'acide de formule générale (XXXIV), on met en oeuvre de préférence un sel alcalin ou un sel d'amine tertiaire (par exemple de triéthylamine).

Lorsque l'on fait agir un ester silylique, on met en oeuvre avantageusement un ester de triméthylsilyle ou de t.butyldiméthylsilyle. Cet ester peut éventuellement être préparé par application de la méthode décrite par A. WISSNER et coll., J. Org. Chem., *43* (20), 3972 (1978), ou formé in situ.

L'halogénation de l'acide de formule générale (XXXIV) s'effectue généralement dans un solvant organique tel qu'un solvant chloré (par exemple dichlorométhane, chloroforme), dans un ester (par exemple acétate d'éthyle), un hydrocarbure aromatique (par exemple toluène, xylène), ou dans un éther (par exemple éther éthylique, éther isopropylique ou tétrahydrofuranne), à une température comprise entre —70 et +30°C.

Il est entendu que les acides de formule générale (XXXIV) de forme syn conduisent aux halogénures d'acide de forme syn et que les acides de formule générale (XXXIV) de forme anti conduisent aux halogénures d'acide de formule générale (XXXIII) de forme anti.

Les acides de formule générale (XXXIV) pour lesquels R''° est autre qu'un radical de formule générale (VI) peuvent être obtenus par hydrolyse acide ou par saponification d'un ester de formule générale:

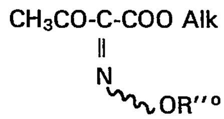

(XXXV)

dans laquelle R''° est défini comme ci-dessus et Alk représente un radical alcoyle.

On opère généralement en présence de soude dans l'éthanol, à la température de reflux du mélange réactionnel.

Les esters de formule générale (XXXV) peuvent être préparés par application de la méthode décrite par R. BUCOURT et coll., Tetrahedron *34,* 2233 (1978).

Les acides de formule générale (XXXIV) dans laquelle R''° est un radical de formule générale (VI) protégé peuvent être préparés par action d'un produit de formule générale (VIa) dont la fonction acide est protégée sur un produit de formule générale:

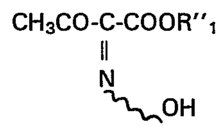

(XXXVI)

dans laquelle $R''_1$ est un radical protecteur d'acide tel que défini précédemment pour $R_1$, puis élimination du radical protecteur $R''_1$.

La réaction s'effectue généralement en présence d'une base minérale ou organique (par exemple la soude, la potasse, l'hydrure de sodium, le carbonate de potassium ou une base azotée telle que la triéthylamine) dans un solvant organique tel qu'un solvant chloré (par exemple dichlorométhane, dichloroéthane), un éther (par exemple tétrahydrofuranne, dioxanne), une cétone (par exemple acétone) ou un amide (par exemple diméthylformamide).

Il est nécessaire que les radicaux protecteurs d'acide $R''_1$ et le groupement protecteur du radical de formule générale (VIa) soient différents et éliminables sélectivement.

L'élimination du radical protecteur $R''_1$ s'effectue dans les conditions décrites précédemment.

Les produits de formule générale (XVIII) dans laquelle R° représente un atome d'hydrogène peuvent être obtenus à partir d'un produit de formule générale:

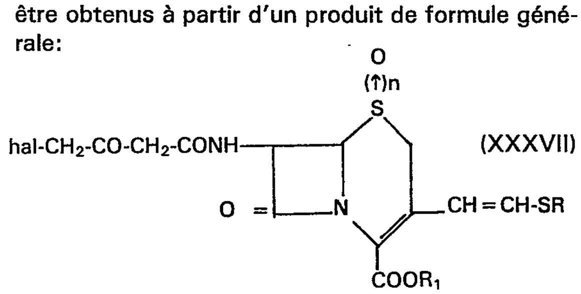

(XXXVII)

dans laquelle R, $R_1$, hal et n sont définis comme précédemment, par analogie avec la méthode décrite dans la demande de brevet français 2 399 418, puis éventuellement réduction du sulfoxyde et élimination des radicaux protecteurs.

Il est entendu que, lorsque le radical R du produit de formule générale (XXXVII) contient un radical amino, alcoylamino, ou formyle, celui-ci est protégé, et lorsque le radical R contient un substituant hydroxy, carboxy ou acylalcoyle, celui-ci est libre ou protégé.

Le cas échéant la réduction du sulfoxyde et l'élimination des radicaux protecteurs s'effectue dans les conditions décrites précédemment.

Les produits de formule générale (XXXVII) peuvent être obtenus à partir d'une amino-7 céphalosporine de formule générale (IX), par action d'un produit de formule générale:

$$\text{hal-}CH_2\text{-}COCH_2\text{-CO-hal} \qquad \text{(XXXVIII)}$$

dans laquelle hal est défini comme précédemment, (qui peut être formé in situ), en opérant dans les conditions décrites précédemment pour condenser un produit de formule générale (XXXII) avec un produit de formule générale (IX) ou par analogie avec la méthode décrite dans la demande de brevet français 2 399 418.

Les produits de formule générale (XXXVIII), qui peuvent être formés in situ, sont préparés comme décrit dans la demande française ci-dessus.

Les isomères des produits de formules générales (I), (IX), (XII), (XV), (XVI), (XVIII), (XX), (XXII), (XXV), (XXVI), (XXVIII) ou (XXXI) peuvent être séparés par chromatographie ou cristallisation.

Les nouveaux produits selon l'invention peuvent être transformés en sels d'addition avec les acides. Selon les procédés de la présente invention les produits peuvent être obtenus sous forme de trifluoroacétate, solvate avec l'acide formique ou l'eau, phosphate, méthanesulfonate ou paratoluènesulfonate. Les produits de formule générale (I), dans laquelle R est défini selon la présente invention, obtenus sous forme de ces sels, peuvent être libérés et transformés en sels d'autres acides selon les méthodes habituelles.

Les produits selon la présente invention peuvent aussi être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique.

Le sel formé précipite après concentration éventuelle de sa solution, il est séparé par filtration ou décantation. Il peut également être obtenu par lyophilisation de sa solution.

Comme exemples de sels pharmaceutiquement acceptables peuvent être cités les sels d'addition avec des acides minéraux (chlorhydrates, bromhydrates, sulfates, nitrates, phosphates) ou organiques (succinates, fumarates, maléates, p.toluènesulfonates), les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les nouveaux produits selon la présente invention peuvent être éventuellement purifiés par des méthodes physiques telles que la cristallisation ou la chromatographie.

Les nouveaux dérivés de la céphalosporine selon la présente invention et leurs sels pharmaceutiquement acceptables présentent des propriétés antibactériennes particulièrement intéressantes. Ils manifestent une activité remarquable in vitro et in vivo sur les germes Gram-positifs et Gram-négatifs.

In vitro, les produits de formule générale (I) se sont montrés actifs à une concentration comprise entre 0,5 et 10 $\mu g/cm^3$ sur des souches de staphylocoques sensibles à la pénicilline G (Staphylococcus aureus Smith) et à une concentration comprise entre 0,01 et 2 $\mu g/cm^3$ sur Escherichia coli souche NIHJ. De plus les produits de formule générale (I) définis en α/ se sont montrés actifs à une concentration comprise entre 2 et 125 $\mu g/cm^3$ sur Pseudomonas aeruginosa.

In vivo les produits de formule générale (I) se sont montrés actifs sur les infections expérimentales de la souris à Staphylococcus aureus Smith (sensible à la pénicilline G) à une dose comprise entre 0,5 et 15 mg/kg par jour par voie sous-cutanée et à Escherichia coli (souche NIHJ) à des doses comprises entre 0,01 et 10 mg/kg par jour par voie sous-cutanée.

Par ailleurs, la $DL_{50}$ des produits de formule générale (I) est comprise entre 1 g/kg et des doses supérieures à 2,5 g/kg par voie sous-cutanée chez la souris.

D'un intérêt particulier sont les produits de formule générale (I) dans laquelle

R' représente un atome d'hydrogène et, soit

R° représente le radical carboxyalcoyle de formule générale (VI) et

R représente un radical pyrimidinyle-2, un radical dioxo-5,6 tétrahydro-1,4,5,6 triazine-1,2,4 yle-3 substitué en position —4 par un radical formylalcoyle ou dihydroxy-2,3 propyle, un radical formylalcoyl-1 alcoyl-4 dioxo-5,6 tétrahydro-1,4,5,6 triazine-1,2,4 yle-3 ou un radical alcoyl-1 tétrazolyle-5, soit

R° représente un radical alcoyle ou un radical carboxyalcoyle de formule générale (VI) et

R représente un radical dioxo-5,6 hydroxyalcoylcarbamoylalcoyl-4 tétrahydro-1,4,5,6 triazine-1,2,4 yle-3, alcoyl-1 formylalcoyl-4 dioxo-5,6 tétrahydro-1,4,5,6 triazine-1,2,4 yle-3 ou formylalcoyl-1 alcoyl-4 dioxo-5,6 tétrahydro-1,4,5,6 triazine-1,2,4 yle-3.

Et parmi ces produits plus spécialement actifs sont les produits de formule générale (I) dans laquelle

R' représente un atome d'hydrogène et, soit

R° représente un radical de formule générale (VI) dans laquelle $R^{iv}$ et $R^{v}$ sont des atomes d'hydrogène, des radicaux méthyle ou forment ensemble un radical triméthylène, et
R représente un radical dioxo-5,6 tétrahydro-1,4,5,6 triazine-1,2,4 yle-3 substitué en position —4 par un radical formylméthyle ou dihydroxy-2,3 propyle, ou un radical formylalcoyl-1 alcoyl-4 dioxo-5,6 tétrahydro-1,4,5,6 triazine-1,2,4 yle-3, soit
R° représente un radical méthyle ou un radical de formule générale (VI) tel que défini ci-dessus, et
R représente un radical dioxo-5,6 hydroxyalcoylcarbamoylméthyl-4 tétrahydro-1,4,5,6 triazine-1,2,4 yle-3, alcoyl-1 formylalcoyl-4 dioxo-5,6 tétrahydro-1,4,5,6 triazine-1,2,4 yle-3 ou formylalcoyl-1 alcoyl-4 dioxo-5,6 tétrahydro-1,4,5,6 triazine-1,2,4 yle-3,
et notamment les produits de formule générale (I) dans laquelle
R' représente un atome d'hydrogène et, soit
R° représente un radical de formule générale (VI) tel que défini ci-dessus et
R représente un radical dioxo-5,6 formylméthyl-4 tétrahydro-1,4,5,6 triazine-1,2,4 yle-3, soit
R° représente un radical méthyle ou un radical de formule générale (VI) tel que défini ci-dessus, et
R représente un radical dioxo-5,6 hydroxyméthylcarbamoylméthyl-4 tétrahydro-1,4,5,6 triazine-1,2,4 yle-3, méthyl-1 formylméthyl-4 dioxo-5,6 tétrahydro-1,4,5,6 triazine-1,2,4 yle-3 ou formylméthyl-1 méthyl-4 dioxo-5,6 tétrahydro-1,4,5,6 triazine-1,2,4 yle-3.

Les exemples suivants, donnés à titre non limitatif, montrent comment l'invention peut être mise en pratique.

Dans ces exemples les produits sont désignés selon la nomenclature des Chemical Abstracts. Il est entendu que tous les produits selon la présente invention présentent la stéréochimie donnée par la formule générale partielle:

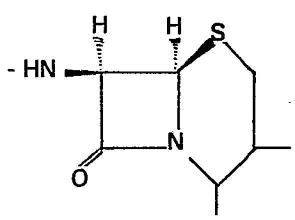

(XXXIX)

*Exemple 1*

On agite à 50°C sous azote pendant 6 heures un mélange de 16,56 g de benzhydryloxycarbonyl-2 [t.butoxycarbonylméthoxyimino-2 (tritylamino-2 thiazolyl-4)-2 acétamido]-7 oxo-8 oxyde-5 (tosyloxy-2 vinyl)-3 thia-5 aza-1 bicyclo[4.2.0] octène-2, isomère syn, forme E (produit 1a), 135 $cm^3$ de diméthylformamide, 3,67 g de (diméthoxy-2,2 éthyl)-4 dioxo-5,6 thioxo-3 perhydrotriazine-1,2,4 et 2,75 $cm^3$ de N,N-diisopropyléthylamine. On dilue par 600 $cm^3$ d'acétate d'éthyle, lave par 2 fois 150 $cm^3$ d'eau et 2 fois 150 $cm^3$ d'une solution saturée de chlorure de sodium, sèche sur sulfate de sodium, filtre et concentre à sec à 20°C sous 20 mm de mercure (2,7 kPa). Le produit est fixé sur 50 g de gel de silice Merck (0,06-0,2) et la poudre est déposée sur une colonne de 350 g de gel de silice (diamètre de la colonne: 4 cm, hauteur: 75 cm). On élue par 3,2 litres d'un mélange cyclohexaneacétate d'éthyle 50-50 (en volumes) et 12 litres d'un mélange 25-75 (en volumes) en recueillant des fractions de 500 $cm^3$. Les fractions 7 à 29 sont concentrées à sec à 20°C sous 20 mm de mercure (2,7 kPa), et on recueille 12,8 g de benzhydryloxycarbonyl-2 [t.butoxycarbonylméthoxyimino-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo[4.2.0] octène-2, isomère syn, forme E, sous la forme d'une meringue de couleur crème (produit 1b).

Spectre infra-rouge ($CHBr_3$), bandes caractéristiques ($cm^{-1}$) 1800, 1720, 1690, 1590, 1520, 1495, 1450, 1370, 1080, 1065, 1040, 945, 755, 700.

Spectre de RMN du proton (350 MHz, DMSO $d_6$, $\delta$ en ppm, J en Hz) 1,40 (s, 9H, $-C(CH_3)_3$); 3,24 (s, 6H, $(-OCH_3)_2$); 3,60 et 4,25 (2d, J = 18, 2H, $-SCH_2-$); 3,95 (d, J = 6, 2H, $>NCH_2-$); 4,52 (s, 2H, $=NOCH_2-$); 4,54 (t, J = 6, 1H, $-C\underline{H}(OCH_3)_2$); 5,08 (d, J = 4, 1H, H en 6); 5,91 (dd, J = 4 et 9, 1H, H en 7); 6,82 (s, 1H, H du thiazole); 6,97 (s, 1H, $-COOCH<$); 6,96 et 7,0 (2d, J = 16, 2H, $-CH=CH-$); 8,68 (d, J = 9, 1H, $-CONH-$); 8,74 (s, 1H, $-N\underline{H}\ C(C_6H_5)_3$); 12,35 (s, 1H, $=NNHCO-$ ou $=NN=C-OH$).
|

On traite à —5°C sous agitation pendant 1 heure 15 une solution de 12,7 g de produit (1b) dans 100 $cm^3$ de dichlorométhane et 4,34 $cm^3$ de N,N-diméthylacétamide par 1,91 $cm^3$ de trichlorure de phosphore. On dilue le mélange par 600 $cm^3$ d'acétate d'éthyle, lave par 2 fois 200 $cm^3$ d'une solution à 2% de bicarbonate de sodium et 2 fois 200 $cm^3$ d'une solution demi-saturée de chlorure de sodium, sèche sur sulfate de sodium, filtre et concentre à sec à 30°C sous 20 mm de mercure (2,7 kPa). Le produit mis en solution dans 50 $cm^3$ de dichlorométhane est fixé sur 25 g de gel de silice Merck (0,06-0,2) et la poudre obtenue est déposée sur une colonne de 175 g de gel de silice (diamètre de la colonne: 3 cm, hauteur: 60 cm). On élue par 1,2 litre d'un mélange cyclohexane-acétate d'éthyle 60-40 (en volumes) puis par 2,8 litres d'un mélange 40-60 (en volumes) en recueillant des fractions de 100 $cm^3$. Les fractions 13 à 28 sont réunies et évaporées à sec à 20°C sous 20 mm de mercure (2,7 kPa), on obtient 9,45 g de benzhydryloxycarbonyl-2 [t.butoxycarbonylméthoxyimino-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 thia-5 aza-1 bicyclo[4.2.0] octène-2, isomère syn, forme E, sous la forme d'une meringue jaune-orange (produit 1c).

Spectre infra-rouge ($CHBr_3$), bandes caractéristiques ($cm^{-1}$) 3280, 1800, 1725, 1690, 1590, 1530, 1495, 1440, 1370, 1160, 1080, 945, 755, 700.

Spectre de RMN du proton (350 MHz, DMSO $d_6$, $\delta$ en ppm, J en Hz) 1,45 (s, 9H, $-C(CH_3)_3$); 3,25 (s, 6H, $(-OCH_3)_2$); 3,63 et 3,81 (2d, J = 18, 2H, $-SCH_2-$); 3,95 (d, J = 6, 2H, $>NCH_2-$); 4,52 (s, 2H, $=NOCH_2-$); 4,57 (t, J = 6, 1H, $-C\underline{H}(OCH_3)_2$); 5,24 (d, J = 4, 1H, H en 6); 5,78 (dd, J = 4 et 9, 1H, H

en 7); 6,78 (s, 1H, H du thiazole); 6,95 (s, 1H, -COOCH<); 6,93 et 7,0 (2d, J = 16, 2H, -CH=CH-); 8,80 (s, 1H, -NH C($C_6H_5$)$_3$); 9,50 (d, J = 9, 1H, -CONH-); 12,62 (s, 1H, =NNHCO- ou =N-N=C-OH).

On agite à 20°C pendant 25 minutes une solution de 9,4 g de produit (1c) dans 95 cm$^3$ d'acide trifluoracétique et concentre à sec à 20°C sous 0,05 mm de mercure (0,007 kPa). Le résidu est trituré dans 200 cm$^3$ d'éther diéthylique, on filtre la suspension et obtient 6,7 g de poudre jaune. Le produit ainsi obtenu est mis en solution dans 190 cm$^3$ d'acide formique pur, on chauffe à 50°C pendant 40 minutes et concentre à sec à 30°C sous 0,05 mm de mercure (0,007 kPa). On reprend le résidu par 200 cm$^3$ d'acétone, concentre à sec à 20°C sous 30 mm de mercure (4 kPa) et répète l'opération une seconde fois. Le solide obtenu est traité par 300 cm$^3$ d'acétone à reflux pendant 15 minutes, on filtre à chaud et, après séchage, on obtient 4,45 g d'[(amino-2 thiazolyl-4)-2 carboxyméthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (produit 1) sous la forme d'une poudre jaune.

Spectre infra-rouge (KBr), bandes caractéristiques (cm$^{-1}$) 3600, 2200, 1775, 1815, 1680, 1635, 1585, 1195, 945, 800, 720.

Spectre de RMN du proton (350 MHz, $CF_3COOD$, δ en ppm, J en Hz) 3,88 (s, large, 2H, -$SCH_2$-); 5,12 (s, 2H, =$NOCH_2$-); 5,21 (s, 2H, -$CH_2$CHO); 5,39 (d, J = 4, 1H, H en 6); 6,10 (d, J = 4, 1H, H en 7); 7,24 et 7,75 (2d, J = 16, 2H, -CH=CH-); 7,52 (s, 1H, H du thiazole); 9,77 (s, 1H, -CHO).

Le produit (1a) peut être préparé de la manière suivante:

A un mélange refroidi à 5°C de 23,14 g d'amino-7 benzhydryloxycarbonyl-2 oxo-8 oxyde-5 (tosyloxy-2 vinyl)-3 thia-5 aza-1 bicyclo[4.2.0] octène-2, forme E (produit 1d) dans 400 cm$^3$ de dichlorométhane et de 26,10 g d'acide t.butoxycarbonylméthoxyimino-2 (tritylamino-2 thiazolyl-4)-2 acétique, isomère syn (préparé selon le procédé décrit dans le brevet belge 865 298) dans 100 cm$^3$ de diméthylformamide, on ajoute sous agitation 0,2 g de diméthylamino-4 pyridine et, goutte à goutte en 17 minutes, une solution de 9,90 g de N,N'-dicyclohexylcarbodiimide. On agite pendant 1 heure 1/2 à 5°C et 1 heure 1/2 à 20°C, ajoute 2 cm$^3$ d'acide acétique, agite pendant 15 minutes à 20°C et concentre à sec à 20°C sous 20 mm de mercure (2,7 kPa). On reprend le résidu dans 200 cm$^3$ d'acétate d'éthyle, filtre et lave la phase organique par 2 fois 250 cm$^3$ d'eau, 2 fois 250 cm$^3$ d'une solution aqueuse à 2% de bicarbonate de sodium et 2 fois 250 cm$^3$ d'une solution demi-saturée de chlorure de sodium, sèche sur sulfate de sodium, filtre et concentre à sec à 25°C sous 20 mm de mercure (2,7 kPa), on reprend le résidu dans 20 cm$^3$ de tétrahydrofuranne, laisse à 3°C pendant 12 heures, filtre et concentre à sec à 20°C sous 20 mm de mercure (2,7 kPa). On fixe le produit sur 200 g de gel de silice Merck (0,06-0,2) et dépose la poudre sur une colonne de 800 g de gel de silice (diamètre de la colonne: 6 cm, hauteur: 77 cm). On élue par 3 litres d'un mélange cyclohexane-acétate d'éthyle 80-20 (en volumes), 6 litres d'un mélange 70-30 et 9 litres d'un mélange 60-40 en recueillant des fractions de 600 cm$^3$. Les fractions 19 à 26 sont concentrées à sec à 20°C sous 20 mm de mercure, on recueille 27,96 g de produit (1a) sous forme d'une meringue jaune.

Spectre infra-rouge ($CHBr_3$), bandes caractéristiques (cm$^{-1}$) 1780, 1725, 1680, 1495, 1450, 1370, 1190, 1180, 815, 740.

Spectre de RMN du proton (350 MHz, $CDCl_3$, δ en ppm, J en Hz) 1,45 (s, 9H, -C($CH_3$)$_3$); 2,45 (s, 3H, -$CH_3$ tosyl); 3,14 et 3,73 (2d, J = 18, 2H, -S-$CH_2$-); 4,57 (d, J = 5, 1H, -H en 6); 4,72 (ab, J = 16, 2H, =$NOCH_2$COO-); 6,02 (dd, J = 5 et 9, 1H, -H en 7); 6,75 (s, 1H, H thiazole); 6,90 et 7,05 (2d, J = 12, 2H, -CH=CH-S-); 6,90 (s, 1H, -COO-CH($C_6H_5$)$_2$); 7,02 (s large, 1H, -NH-C($C_6H_5$)$_3$); 8,25 (d, J = 9, 1H, -CO-NH-).

Le produit (1d) peut être obtenu de la manière suivante:

On agite à 35°C pendant 2 heures une solution de 54,3 g de benzhydryloxycarbonyl-2 t.butoxycarbonylamino-7 oxo-8 oxyde-5 (tosyloxy-2 vinyl)-3 thia-5 aza-1 bicyclo[4.2.0] octène-2 (produit 1e), forme E et de 30,4 g d'acide p.toluènesulfonique hydraté dans 1,4 litre d'acétonitrile. On concentre à sec à 30°C sous 20 mm de mercure (2,7 kPa), reprend dans 1 litre d'acétate d'éthyle, lave par 2 fois 500 cm$^3$ d'une solution demi-saturée de bicarbonate de sodium et 2 fois 500 cm$^3$ d'une solution demi-saturée de chlorure de sodium, sèche sur sulfate de sodium et concentre à sec à 20°C sous 20 mm de mercure (2,7 kPa). Le résidu est trituré dans 200 cm$^3$ d'éther. On obtient 28,13 g de produit (1d) sous la forme d'une poudre brun clair.

Rf = 0,32 chromatoplaque de silicagel [dichlorométhane-méthanol 85-15 (vol.)].

Le produit (1e) peut être préparé de la manière suivante:

Dans une solution refroidie à —10°C de 180,56 g de benzhydryloxycarbonyl-2 t.butoxycarbonylamino-7 oxo-8 (tosyloxy-2 vinyl)-3 thia-5 aza-1 bicyclo[4.2.0] octène-2 (ou -3), (produit 1f, mélange des formes E et Z) dans 1,4 litre de dichlorométhane, on ajoute goutte à goutte en 2 heures une solution de 55,22 g d'acide m.chloroperbenzoïque à 85% dans 600 cm$^3$ de dichlorométhane. Le mélange est lavé par 1,5 litre d'une solution à 5% de bicarbonate de sodium et 2 fois 1,5 litre d'eau, séché sur sulfate de sodium et concentré à 20°C sous pression réduite (20 mm de mercure) jusqu'au volume de 300 cm$^3$. Cette solution est chromatographiée sur une colonne de 3 kg de gel de silice Merck (0,05-0,2) (diamètre de la colonne: 9,2 cm; hauteur: 145 cm). On élue par des mélanges cyclohexane-acétate d'éthyle, successivement: 15 litres [80-20 (en volumes)] et 32 litres [70-30 (en volumes)] en recueillant des fractions de 600 cm$^3$. Les fractions 27 et 28 sont recueillies et concentrées à sec, on obtient 5,56 g de la forme Z du produit (1e).

Spectre infra-rouge ($CHBr_3$), bandes caractéristi-

ques ($cm^{-1}$) 3420, 1800, 1720, 1505, 1380, 1370, 1195, 1180, 1050, 1010, 730.

Spectre de RMN du proton (350 MHz, $CDCl_3$, $\delta$ en ppm, J en Hz) 1,49 (s, 9H, $-C(CH_3)_3$); 2,44 (s, 3H, $-CH_3$); 3,36 et 4,04 (2d, J = 19, 2H, $-SCH_2-$); 4,44 (d, J = 4,5, 1H, H en 6); 5,73 (d, J = 9, 1H, -CONH-); 5,81 (dd, J = 4,5 et 9, 1H, H en 7); 6,42 (d, J = 7, 1H, $-C\underline{H}=CH\ OSO_2-$); 6,46 (d, J = 7, 1H, $=CH\ OSO_2-$); 6,89 (s, 1H, $-COOCH\langle$); 7,77 (d, J = 9, 2H, H en ortho du tosyle).

Dans les fractions 29 à 34, on obtient 26 g du mélange des formes Z et E.

Enfin dans les fractions 35 à 58, on obtient 43 g de la forme E du produit (1e):

Spectre infra-rouge ($CHBr_3$), bandes caractéristiques ($cm^{-1}$) 3420, 1800, 1720, 1505, 1380, 1370, 1195, 1180, 1075, 935, 745.

Spectre de RMN du proton (350 MHz, $CDCl_3$, $\delta$ en ppm, J en Hz) 1,48 (s, 9H, $(CH_3)_3C-$); 2,46 (s, 3H, $-CH_3$); 3,16 et 3,81 (2d, J = 18, 2H, $-SCH_2-$); 4,46 (d, J = 4,5, 1H, H en 6); 5,73 (d, J = 9, 1H, -CONH-); 5,8 (dd, J = 9 et 4,5, 1H, H en 7); 6,83 (d, J = 13, 1H, $-C\underline{H}=CH\ OSO_2-$); 6,83 (s, 1H, $-COOCH\langle$); 7,08 (d, J = 13, 1H, $=CH\ OSO_2-$); 7,73 (d, J = 9, 2H, H en ortho du tosyle).

Le produit (1f), mélange des formes E et Z, peut être obtenu de la manière suivante:

A une solution de 113,7 g de benzhydryloxycarbonyl-2 t.butoxycarbonylamino-7 (diméthylamino-2 vinyl)-3 oxo-8 thia-5 aza-1 bicyclo[4.2.0] octène-2 (forme E) (produit 1g) dans 1 litre de tétrahydrofuranne, on ajoute une solution de 50 $cm^3$ d'acide formique dans 500 $cm^3$ d'eau. On agite la solution homogène à 20°C pendant 20 minutes puis on la concentre au quart de son volume sous pression réduite (20 mm de mercure) à 20°C. On reprend le concentrat dans 2 litres d'acétate d'éthyle, lave par 2 fois 500 $cm^3$ d'une solution à 5% de bicarbonate de sodium, 2 fois 500 $cm^3$ d'eau et 2 fois 500 $cm^3$ d'une solution saturée de chlorure de sodium, sèche sur sulfate de sodium, filtre et évapore à sec à 20°C sous pression réduite (20 mm de mercure). On recueille 112,4 g de produit brut qui sont traités en solution dans 250 $cm^3$ de pyridine anhydre à 5°C par 57,2 g de chlorure de tosyle. Après 30 minutes à 5°C et 1 heure à 20°C, on verse la solution dans 1 litre d'un mélange eau-glace pilée. On sépare la phase aqueuse et lave l'insoluble par 300 $cm^3$ d'eau distillée. Le produit pâteux est mis en solution dans 200 $cm^3$ d'acétate d'éthyle, on lave par 2 fois 750 $cm^3$ d'acide chlorhydrique 1N, 2 fois 750 $cm^3$ d'une solution à 5% de bicarbonate de sodium et 4 fois 750 $cm^3$ d'eau, sèche sur sulfate de sodium et concentre à sec sous pression réduite (20 mm de mercure) à 20°C. On obtient 121 g de produit constitué principalement de produit (1f) (mélange des formes E et Z) sous la forme d'une meringue brune brute.

Le produit (1g) est préparé comme décrit ci-après à l'exemple 11 pour le produit (11i).

*Exemple 2*

On procède comme dans l'exemple 1 mais à partir de 11,04 g de benzhydryloxycarbonyl-2 [t.butoxycarbonylméthoxyimino-2 (tritylamino-2 thiazolyl-4)-2 acétamido]-7 oxo-8 oxyde-5 (tosyloxy-2 vinyl)-3 thia-5 aza-1 bicyclo[4.2.0] octène-2, isomère syn, forme E (produit 2a), 50 $cm^3$ de diméthylformamide, 2,72 g de (diméthyl-2,2 dioxolannyl-3) méthyl-4 dioxo-5,6 thioxo-3 perhydrotriazine-1,2,4 et 1,84 $cm^3$ de N,N-diisopropyléthylamine. On chromatographie le produit de la réaction sur une colonne de gel de silice Merck (0,04-0,06) (diamètre de la colonne: 6 cm, hauteur: 30 cm), en éluant par 4 litres d'un mélange cyclohexane-acétate d'éthyle 20-80 (en volumes), 2 litres d'acétate d'éthyle et 2 litres d'un mélange acétate d'éthyl-méthanol 90-10 (en volumes) sous une pression de 40 kPa et en recueillant des fractions de 125 $cm^3$. On évapore à sec les fractions 14 à 48 à 20°C sous 20 mm de mercure (2,7 kPa) et obtient 2,41 g de benzhydryloxycarbonyl-2 [t.butoxycarbonylméthoxyimino-2 (tritylamino-2 thiazolyl-4)-2 acétamido]-7 {[(diméthyl-2,2 dioxolannyl-3) méthyl-4 dioxo-5,6 tétrahydro-1,4,5,6 triazine-1,2,4 yl-3] thio-2 vinyl}-3 oxo-8 oxyde-5 thia-5 aza-1 bicyclo[4.2.0] octène-2, isomère syn, forme E (produit 2b) sous la forme d'une meringue brun-clair.

Spectre infra-rouge ($CHBr_3$), bandes caractéristiques ($cm^{-1}$) 3380, 3240, 1800, 1720, 1680, 1590, 1525, 1495, 1450, 1370, 1070, 1045, 940, 750, 700.

Spectre de RMN du proton (350 MHz, $CDCl_3$, $\delta$ en ppm, J en Hz) 1,40 (s, 9H, $-C(CH_3)_3$); 1,30 et 1,45 (2s, 6H, $(-CH_3)_2$); 3,32 et 4,10 (2d, J = 18, 2H, $-S-CH_2-$); 3,70 à 4,00 (mt, 2H, $-CH_2-O-$); 4 à 4,2 (mt, 2H, $>N-CH_2-$); 4,40 (mt, 1H, $-C\underline{H}\langle^{O-}_{CH_2-O-}$); 4,68 (mf, 3H, $=N-O-CH_2-$ + -H en 6); 5,87 (mt, 1H, -H en 7); 6,71 (s, 1H, -H thiazole); 6,82 (d, J = 16, 1H, $-C\underline{H}=C\underline{H}-S-$); 6,95 (s, 1H, $-COO-C\underline{H}(C_6H_5)_2$); 7,64 (mf, 1H, $-N\underline{H}-C(C_6H_5)_3$); 8,28 (d, J = 9, -CO-NH-); 11,90 (s large, 1H, =N-NH-CO- ou =N-N=C-OH).

On traite d'une façon similaire à l'exemple 1 2,38 g de produit (2b) en solution dans 15 $cm^3$ de dichlorométhane et 0,8 $cm^3$ de N,N-diméthylacétamide par 0,35 $cm^3$ de trichlorure de phosphore. On chromatographie le résidu obtenu après traitement sur une colonne de gel de silice Merck (0,04-0,06) (diamètre de la colonne: 4 cm, hauteur: 20 cm). On élue par 2 litres d'un mélange cyclohexane-acétate d'éthyle 35-65 (en volumes) sous une pression de 40 kPa en recueillant des fractions de 125 $cm^3$. On concentre à sec les fractions 4 à 10 à 25°C sous 20 mm de mercure (2,7 kPa) et recueille 1,17 g de benzhydryloxycarbonyl-2 [t.butoxycarbonylméthoxyimino-2 (tritylamino-2 thiazolyl-4)-2 acétamido]-7 {[(diméthyl-2,2 dioxolannyl-3) méthyl-4 dioxo-5,6 tétrahydro-1,4,5,6 triazine-1,2,4 yl-3] thio-2 vinyl}-3 oxo-8 thia-5 aza-1 bicyclo[4.2.0] octène-2 isomère syn, forme E (produit 2c) sous la forme d'une meringue jaune.

Spectre infra-rouge ($CHBr_3$), bandes caractéristiques ($cm^{-1}$) 3390 + 3260, 1790, 1725, 1680, 1585, 1530, 1495, 1450, 1370, 1070, 1045, 940, 760, 740, 700.

Spectre de RMN du proton (350 MHz, $CDCl_3$, $\delta$ en ppm, J en Hz) 1,42 (s, 9H, $-C(CH_3)_3$); 1,32 et 1,45

(2s, 6H, $(-CH_3)_2$); 3,58 et 3,62 (2d, J = 18, 2H, $-S-CH_2-$); 3,77 et 3,95 (2 mt, 2H, $-CH_2-O-$); 4,13 (mt, 2H, $>N-CH_2-$); 4,43 (mt, 1H, $-C\underline{H}<^{O-}_{CH_2-O-}$); 4,70 et 4,78 (2d, J = 16, 2H, $=N-OCH_2-$); 5,10 (d, J = 14, 1H, -H en 6); 5,86 (dd, J = 4 et 9, 1H, -H en 7); 6,78* (2d, J = 16, 1H, $-C\underline{H}=CH-S-$); 6,82 (s, 1H, H thiazole); 6,96 (s, 1H, $-COO-CH(C_6H_5)_2$); 8,78 (d, J = 9, 1H, -CO-NH-); 11,02 (s large, 1H, =N-NH-CO- ou =N-N=C-OH).

(* présence probable de deux conformères).

On laisse à 20°C pendant 20 minutes une solution de 1,15 g de produit (2c) dans 11,5 $cm^3$ d'acide trifluoroacétique, on concentre à sec à 20°C sous 0,05 mm de mercure (0,007 kPa), triture le résidu dans 30 $cm^3$ d'éther diéthylique et filtre. Le solide jaune obtenu est dissous dans un mélange de 25 $cm^3$ d'acide formique et de 5 $cm^3$ d'eau, on agite la solution à 50°C pendant 45 minutes et concentre à sec à 20°C sous 0,05 mm de mercure (0,007 kPa). On reprend le résidu par 3 fois 100 $cm^3$ d'acétone en concentrant à chaque fois à sec à 25°C sous 30 mm de mercure (4 kPa) puis chauffe le solide obtenu à reflux dans 40 $cm^3$ d'acétone et filtre à chaud. On recueille 0,61 g d'[(amino-2 thiazolyl-4)-2 carboxyméthoxyimino-2 acétamido]-7 carboxy-2 {[(dihydroxy-2,3 propyl)-4 dioxo-5,6 tétrahydro-1,4,5,6 triazine-1,2,4 yl-3] thio-2 vinyl}-3 oxo-8 thia-5 aza-1 bicyclo[4.2.0] octène-2, isomère syn, forme E (produit 2) sous la forme d'une poudre jaune. Dans ce produit le spectre de RMN montre la présence d'ester formique de l'une ou l'autre des fonctions alcool.

Spectre infra-rouge (KBr), bandes caractéristiques ($cm^{-1}$) 3600-2200, 1775, 1715, 1685, 1635, 1590, 1380, 1200, 945.

Spectre de RMN du proton (350 MHz, DMSO $d_6$, $\delta$ en ppm, J en Hz) pics caractéristiques: 4,62 (s, $=N-O-CH_2-$); 5,25 (d, J = 4, -H en 6); 5,85 (dd, J = 4 et 9, H en 7); 6,83 (s, H thiazole); 9,55 (d, J = 9, -CO-NH-); 12,62 (s, =N-NH-CO- ou =N-N=C-OH).

La (diméthyl-2,2 dioxolannyl-4 méthyl)-4 dioxo-5,6 thioxo-3 perhydrotriazine-1,2,4 peut être préparée de la manière suivante:

On prépare une solution de 1,12 g de sodium dans 50 $cm^3$ de méthanol anhydre, ajoute, sous azote et en agitant à 25°C, 10 g de (diméthyl-2,2 dioxolannyl-4 méthyl)-4 thiosemicarbazide puis, goutte à goutte en 10 minutes, 6,6 $cm^3$ d'oxalate de diéthyle et on chauffe à reflux pendant 2 heures. On laisse refroidir à 20°C, dilue par 1 litre d'éther diéthylique, filtre et recueille après séchage 3,7 g d'un solide blanc. Le produit est repris dans 200 $cm^3$ de dichlorométhane et agité en présence d'acide chlorhydrique 1N (10 $cm^3$). On décante, lave par 2 fois 50 $cm^3$ d'eau saturée de chlorure de sodium, sèche sur sulfate de sodium et concentre à sec à 20°C sous 20 mm de mercure (2,7 kPa). On reprend l'huile résiduelle dans 50 $cm^3$ de dichlorométhane, amorce la cristallisation par grattage et laisse à 4°C pendant 3 heures. Après filtration et séchage, on recueille 1,5 g de (diméthyl-2,2 dioxolannyl-4 méthyl)-4 dioxo-5,6 thioxo-3 perhydrotriazine-1,2,4 sous forme de cristaux blancs.

Spectre infra-rouge (KBr), bandes caractéristiques ($cm^{-1}$) 3600-3100, 1680, 1575, 1535, 1210, 1060.

Spectre de RMN du proton (80 MHz, DMSO $d_6$, $\delta$ en ppm, J en Hz) 1,30 et 1,42 (2s, 6H, $>C(CH_3)_2$); 3,95 (m, 2H, $-CH_2O-$); 4,50 (m, 3H, -CHO- et $-N-CH_2-$).

Le (diméthyl-2,2 dioxolannyl-4 méthyl)-4 thiosemicarbazide peut être préparé de la manière suivante:

On chauffe à reflux pendant 2 heures 30 minutes un mélange de 23,6 g de N-(diméthyl-2,2 dioxolannyl-4 méthyl) dithiocarbamate de méthyle préparé selon le brevet US 4 064 242, 500 $cm^3$ d'éthanol absolu et 5,6 g d'hydrate d'hydrazine. On concentre à sec à 20°C sous 20 mm de mercure (2,7 kPa) et reprend dans 100 $cm^3$ d'éther diéthylique. Après filtration et séchage on recueille 15,2 g de (diméthyl-2,2 dioxolannyl-4 méthyl)-4 thiosemicarbazide sous forme d'un solide de couleur crème fondant à 145°C.

Spectre infra-rouge (KBr), bandes caractéristiques ($cm^{-1}$) 3340, 3200, 1630, 1555, 1510, 1380, 1370, 1240, 1210, 1060.

Spectre de RMN du proton (80 MHz, $CDCl_3$, $\delta$ en ppm, J en Hz) 1,38 et 1,48 (2s, 6H, $>C(CH_3)_2$); 3,72 (dd, J = 5 et 6, 2H, $-CH_2N<$); 3,90 (s, 2H, $-NH_2$); 4,10 (dd, J = 6 et 7, 2H, $-CH_2O-$); 4,38 (m, 1H, >CHO-); 7,78 (t, J = 5, 1H, $-CH_2N\underline{H}-$); 7,98 (s, 1H, -NH-N).

*Exemple 3*

On procède comme décrit dans l'exemple 1 mais à partir de 7,18 g de benzhydryloxycarbonyl-2 [t.butoxycarbonylméthoxyimino-2 (tritylamino-2 thiazolyl-4)-2 acétamido]-7 oxo-8 oxyde-5 (tosyloxy-2 vinyl)-3 thia-5 aza-1 bicyclo[4.2.0] octène-2, isomère syn, forme E (produit 3a), 65 $cm^3$ de diméthylformamide, 0,91 g de méthyl-1 mercapto-5 tétrazole et 1,36 $cm^3$ de N,N-diisopropyléthylamine. Après ce traitement, le produit obtenu est chromatographié sur une colonne de 150 g de gel de silice Merck (0,06-0,2) (diamètre de la colonne: 3 cm, hauteur: 48 cm). On élue par 500 $cm^3$ d'un mélange cyclohexaneacétate d'éthyle 80-20 (en volumes); 1 litre d'un mélange 60-40 et 2 litres d'un mélange 40-60 en recueillant des fractions de 125 $cm^3$. On concentre à sec les fractions 17 à 26 à 25°C sous 25 mm de mercure (3,3 kPa) et on obtient 5,01 g de benzhydryloxycarbonyl-2 [t.butoxycarbonylméthoxyimino-2 (tritylamino-2 thiazolyl-4)-2 acétamido]-7 [(méthyl-1 tétrazolyl-5) thio-2 vinyl]-3 oxo-8 oxyde-5 thia-5 aza-1 bicyclo[4.2.0] octène-2, isomère syn, forme E (produit 3b) sous la forme d'une meringue de couleur crème.

Spectre infra-rouge ($CHBr_3$), bandes caractéristiques ($cm^{-1}$) 1800, 1730, 1685, 1520, 1495, 1450, 1215, 1155, 1095, 1045, 945, 750, 700.

Spectre de RMN du proton (350 MHz, DMSO $d_6$, $\delta$

en ppm, J en Hz) 1,42 (s, 9H, $-C(CH_3)_3$); 3,64 et 4,30 (2d, J = 18, 2H, $-SCH_2-$); 3,98 (s, 3H, >N $CH_3$); 4,53 (s, 2H, $=NOCH_2-$); 5,07 (d, J = 4, 1H, H en 6); 5,93 (dd, J = 4 et 9, 1H, H en 7); 6,83 (s, 1H, H du thiazole); 7,07 (d, J = 16, 1H, -CH=CHS-); 8,65 (d, J = 9, 1H, -CONH-); 8,71 (s, 1H, $-NHC(C_6H_5)_3$).

On opère comme dans l'exemple 1 à partir de 4,72 g de produit (3b) en solution dans 45 $cm^3$ de dichlorométhane et 1,8 $cm^3$ de diméthylacétamide et de 0,79 $cm^3$ de trichlorure de phosphore. Après ce traitement on chromatographie sur une colonne de 100 g de gel de silice Merck (0,06-0,2) (diamètre de la colonne: 3 cm, hauteur: 33,6 cm) en éluant par 250 $cm^3$ d'un mélange cyclohexane-acétate d'éthyle 80-20 (en volumes), 500 $cm^3$ d'un mélange 70-30 et 1,5 litre d'un mélange 60-40. Par évaporation des fractions 3 à 8 on obtient 3,45 g de benzhydryloxycarbonyl-2 [t.butoxycarbonylméthoxyimino-2 (tritylamino-2 thiazolyl-4)-2 acétamido]-7 [(méthyl-1 tétrazolyl-5) thio-2 vinyl]-3 oxo-8 thia-5 aza-1 bicyclo[4.2.0] octène-2, isomère syn, forme E (produit 3c) sous la forme d'une meringue jaune.

Spectre infra-rouge ($CHBr_3$), bandes caractéristiques ($cm^{-1}$) 1790, 1730, 1690, 1525, 1495, 1450, 1370, 1290, 1160, 945, 750, 700.

Spectre de RMN du proton (350 MHz, DMSO $d_6$, $\delta$ en ppm, J en Hz) 1,44 (s, 9H, $-C(CH_3)_3$); 3,64 et 3,87 (2d, J = 18, 2H, $-SCH_2-$); 3,98 (s, 3H, $>N-CH_3$); 4,50 (s, 2H, $=NOCH_2-$); 5,22 (d, J = 4, 1H, H en 6); 5,78 (dd, J = 4 et 9, 1H, H en 7); 6,77 (s, 1H, H du thiazole); 6,94 (s, 1H, -COOCH<); 7,0 et 7,08 (2d, J = 16, 2H, -CH=CH-); 8,78 (s, 1H, $-NHC(C_6H_5)_3$; 9,48 (d, J = 9, 1H, -CONH-).

A une solution de 3,2 g de produit (3c) dans 32 $cm^3$ d'acide formique pur, on ajoute 10 $cm^3$ d'eau et chauffe le mélange à 50°C pendant 30 minutes. On dilue ensuite par 22 $cm^3$ d'eau, filtre et concentre à sec à 30°C sous 0,05 mm de mercure (0,007 kPa). On reprend le résidu par 3 fois 150 $cm^3$ d'éthanol en évaporant à sec à chaque fois à 20°C sous 20 mm de mercure (2,7 kPa), triture dans 50 $cm^3$ d'éthanol et filtre. On dissout le solide jaune obtenu dans 11 $cm^3$ d'acide trifluoroacétique, laisse pendant 20 minutes à 20°C et concentre à sec à 30°C sous 0,05 mm de mercure (0,007 kPa). Le résidu est repris dans 100 $cm^3$ d'éther diéthylique, on filtre et obtient 1,26 g d'[(amino-2 thiazolyl-4)-2 carboxyméthoxyimino-2 acétamido]-7 carboxy-2 [(méthyl-1 tétrazolyl-5) thio-2 vinyl]-3 oxo-8 thia-5 aza-1 bicyclo[4.2.0] octène-2, isomère syn, forme E (produit 3) sous forme de trifluoroacétate.

Spectre infra-rouge (KBr), bandes caractéristiques ($cm^{-1}$) 3600, 2200, 1770, 1715, 1675, 1635, 1560, 1200, 945, 805, 720.

Spectre de RMN du proton (350 MHz, DMSO $d_6$, $\delta$ en ppm, J en Hz) 3,60 et 3,68 (2d, J = 18, 2H, $-SCH_2-$); 4,0 (s, 3H, $>N-CH_3$); 4,59 (s, 2H, $=NOCH_2-$); 5,20 (d, J = 4, 1H, H en 6); 5,82 (dd, J = 4 et 9, 1H, H en 7); 6,82 (s, 1H, H du thiazole); 6,98 et 7,11 (2d, J = 16, 2H, -CH=CH-); 9,52 (d, J = 9, 1H, -CONH-).

*Exemple 4*

On agite une solution de 1,2 g de benzhydryloxycarbonyl-2 [t.butoxycarbonylméthoxyimino-2 (tritylamino-2 thiazolyl-4)-2 acétamido]-7 oxo-8 [(pyrimidinyl-2) thio-2 vinyl]-3 thia-5 aza-1 bicyclo[4.2.0] octène-2, isomère syn, forme E (produit 4a) dans 15 $cm^3$ d'acide trifluoroacétique pendant 15 minutes à 23°C. On évapore la solution sous pression réduite (0,5 mm de mercure; 0,07 kPa) et ajoute 50 $cm^3$ d'éther. On sépare sur filtre le produit solidifié, lave le gâteau à l'éther et sèche à 20°C sous pression réduite (0,2 mm de mercure; 0,03 kPa). On dissout le solide obtenu dans 15 $cm^3$ d'acide formique, ajoute 5 $cm^3$ d'eau et chauffe pendant 15 minutes à 50°C. On concentre sous pression réduite (0,5 mm de mercure; 0,07 kPa), reprend le résidu par 3 fois 50 $cm^3$ d'acétone en évaporant chaque fois à sec à 20°C sous pression réduite (30 mm de mercure; 4 kPa). On triture le solide obtenu dans 20 $cm^3$ d'éther éthylique anhydre pendant 1 heure sous agitation, filtre, lave par 6 fois 20 $cm^3$ d'éther, sèche à 20°C sous pression réduite (0,2 mm de mercure; 0,03 kPa) pendant 15 heures. On obtient ainsi 0,55 g d'[(amino-2 thiazolyl-4)-2 carboxyméthoxyimino-2 acétamido]-7 carboxy-2 [(pyrimidinyl-2) thio-2 vinyl]-3 oxo-8 thia-5 aza-1 bicyclo[4.2.0] octène-2, isomère syn, forme E (produit 4).

Spectre infra-rouge (KBr), bandes caractéristiques ($cm^{-1}$) 3300 à 3200, 3150, 2100, 1775, 1730, 1680, 1635, 1565, 1550, 1380, 1040, 945.

Spectre de RMN du proton (350 MHz, DMSO $d_6$, $\delta$ en ppm, J en Hz) 3,75 et 3,94 (2d, J = 18, 2H, $-SCH_2-$); 4,63 (s, 2H, $=NOCH_2-$); 5,26 (d, J = 4, 1H, H en 6); 5,83 (dd, J = 4 et 9, 1H, H en 7); 6,86 (s, 1H, H du thiazole); 7,21 à 7,53 (2d, J = 16, 2H, -CH=CH-); 7,33 (t, J = 5, 1H, H en 4 de la pyrimidine); 8,72 (d, J = 5, 2H, H en 3 et 5 de la pyrimidine); 9,58 (d, J = 9, 1H, -CONH-).

En procédant comme dans l'exemple 1 à partir de 1,80 g de benzhydryloxycarbonyl-2 [t.butoxycarbonylméthoxyimino-2 (tritylamino-2 thiazolyl-4)-2 acétamido]-7 oxo-8 oxyde-5 [(pyrimidinyl-2) thio-2 vinyl]-3 thia-5 aza-1 bicyclo[4.2.0] octène-2, isomère syn, forme E (produit 4b) en solution dans 20 $cm^3$ de dichlorométhane sec et 0,85 $cm^3$ de diméthylacétamide, en présence de 0,40 $cm^3$ de trichlorure de phosphore, et après chromatographie sur une colonne de 30 g de silice Merck (0,06-0,2) (diamètre de la colonne: 2 cm, hauteur: 20 cm) en éluant avec un mélange cyclohexane-acétate d'éthyle (60-40) par fractions de 20 $cm^3$, et concentration des fractions 12 à 22 sous pression réduite (30 mm de mercure; 4 kPa) on obtient 1,20 g de produit (4a) sous forme d'une meringue jaune.

Spectre infra-rouge (KBr), bandes caractéristiques ($cm^{-1}$) 3300, 3060, 3030, 2980, 2920, 1780, 1725, 1690, 1560, 1550, 1525, 1450, 1380, 1300, 1245, 1150, 1090, 940, 745, 700.

Spectre de RMN du proton (350 MHz, DMSO $d_6$, $\delta$ en ppm, J en Hz) 1,47 (s, 9H, $-C(CH_3)_3$); 3,77 et 3,95 (2d, J = 18, 2H, $-SCH_2-$); 4,57 (s, 2H, $=NOCH_2-$); 5,28 (d, J = 4, 1H, H en 6); 5,82 (dd, J = 9, 1H, H en 7); 6,83 (s, 1H, H du thiazole); 6,98 (s, 1H, -COOCH<); 7,08 et 7,62 (2d, J = 16, 2H, -CH=CH-); 7,25 à 7,55 (protons aromatiques); 8,73 (d, J = 5, 2H, H en 3 et 5 de la pyrimidine); 8,82 (s, 1H, $-NH-C(C_6H_5)_3$); 9,58 (d, J = 9, 1H, -CONH-).

On ajoute sous azote pendant 2 heures à 20°C à une solution de 1,40 g de benzhydryloxycarbonyl-2 [t-butoxycarbonylméthoxyimino-2 (tritylamino-2 thiazolyl-4)-2 acétamido]-7 oxo-8 oxyde-5 (tosyloxy-2 vinyl)-3 thia-5 aza-1 bicyclo[4.2.0] octène-2, isomère syn, forme E (produit 4c) dans 15 $cm^3$ de diméthylformamide sec, 0,162 g de mercapto-2 pyrimidine puis 0,23 $cm^3$ de diisopropyléthylamine. On ajoute 100 $cm^3$ d'acétate d'éthyle, lave cette solution organique par 4 fois 100 $cm^3$ d'eau puis 2 fois 100 $cm^3$ de solution de chlorure de sodium à 180 g par litre. On sèche l'extrait organique sur sulfate de magnésium, concentre sous pression réduite (20 mm de mercure; 2,7 kPa) et sèche sous pression réduite (0,2 mm de mercure; 0,03 kPa) pendant 15 heures à 20°C. On obtient ainsi 1,40 g de produit (4b) sous forme d'une meringue jaune.

Spectre infra-rouge (KBr), bandes caractéristiques ($cm^{-1}$) 3300, 1800, 1730, 1690, 1600, 1590, 1565, 1550, 1525, 1495, 1450, 1380, 1065, 945.

*Exemple 5*

On procède comme décrit dans l'exemple 1 à partir de 6,79 g de benzhydryloxycarbonyl-2 {[(t.butoxycarbonyl-2 propyl-2) oxyimino]-2 (tritylamino-2 thiazolyl-4)-2 acétamido}-7 oxo-8 oxyde-5 (tosyloxy-2 vinyl)-3 thia-5 aza-1 bicyclo[4.2.0] octène-2, isomère syn, forme E (produit 5a), 60 $cm^3$ de diméthylformamide, 1,68 g de (diméthoxy-2,2 éthyl)-4 dioxo-5,6 thioxo-3 perhydrotriazine-1,2,4 et 1,25 $cm^3$ de N,N-diisopropyléthylamine. Le résidu obtenu après ce traitement est chromatographié sur une colonne de 125 g de gel de silice Merck (0,06-0,2) (diamètre de la colonne: 3 cm, hauteur: 43 cm). On élue par 1,5 litre d'un mélange cyclohexane-acétate d'éthyle 50-50 (en volumes) et 1 litre d'acétate d'éthyle en recueillant des fractions de 100 $cm^3$. Les fractions 16 à 21 sont concentrées à sec à 25°C sous 20 mm de mercure (2,7 kPa), on obtient 5,5 g de benzhydryloxycarbonyl-2 {[(t.butoxycarbonyl-2 propyl-2) oxyimino]-2 (tritylamino-2 thiazolyl-4)-2 acétamido}-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo[4.2.0] octène-2, isomère syn, forme E (produit 5b) sous la forme d'une meringue brune.

Spectre infra-rouge ($CHBr_3$), bandes caractéristiques ($cm^{-1}$) 1800, 1720, 1690, 1585, 1510, 1495, 1445, 1370, 1080, 1060, 1040, 940, 750, 700.

Spectre de RMN du proton (350 MHz, DMSO $d_6$, $\delta$ en ppm, J en Hz) 1,35 (s, 9H, $-C(CH_3)_3$); 1,44 et 1,45 (2s, 6H, $=N-O-C(CH_3)_2-$); 3,32 (s, 6H, $(-OCH_3)_2$); 3,65 et 4,36 (2d, J = 18, 2H, $-S-CH_2-$); 3,95 (d, J = 5, 2H, $\rangle N-CH_2-$); 4,56 (t, J = 5, 1H, $-CH\langle^{O-}_{O-}$); 5,09 (d, J = 5, 1H, -H en 6); 5,95 (dd, J = 5 et 9, 1H, -H en 7); 6,78 (s, 1H, -H thiazole); 7 (s, 1H, $-COO-C\underline{H}(C_6H_5)_2$); 7,02 et 7,1 (2d, J = 16, 2H, -CH=CH-S-); 8,23 (d, J = 9, 1H, -CO-NH-); 8,73 (s, 1H, $-N\underline{H}-C(C_6H_5)_3$); 12,65 (s, 1H, =N-NH-CO- ou =N-N=C-OH).

On procède comme décrit dans l'exemple 1 à partir de 5,37 g de produit (5b) en solution dans 45 $cm^3$ de dichlorométhane et 1,79 $cm^3$ de N,N-diméthylacétamide et de 0,79 $cm^3$ de trichlorure de phosphore. Le résidu obtenu après traitement est chromatographié sur une colonne de 80 g de gel de silice Merck (0,06-0,2) (diamètre de la colonne: 2 cm, hauteur: 20 cm), on élue par 250 $cm^3$ d'un mélange cyclohexane-acétate d'éthyle 40-60 (en volumes) et 1,5 litre d'un mélange 30-70 en recueillant des fractions de 100 $cm^3$. Les fractions 5 à 14 sont concentrées à sec à 25°C sous 20 mm de mercure, on obtient 4,02 g de benzhydryloxycarbonyl-2 {[(t.butoxycarbonyl-2 propyl-2) oxyimino]-2 (tritylamino-2 thiazolyl-4)-2 acétamido}-7 {[(diméthoxy-2,2 éthyl)-4 dioxo-5,6 tétrahydro-1,4,5,6 triazine-1,2,4 yl-3] thio-2 vinyl}-3 oxo-8 thia-5 aza-1 bicyclo[4.2.0] octène-2, isomère syn, forme E (produit 5c) sous la forme d'une meringue brun-clair.

Spectre infra-rouge (KBr), bandes caractéristiques ($cm^{-1}$) 1790, 1720, 1690, 1585, 1520, 1495, 1450, 1370, 1080, 940, 750, 700.

Spectre de RMN du proton (350 MHz, DMSO $d_6$, $\delta$ en ppm, J en Hz) 1,37 (s, 9H, $-C(CH_3)_3$); 1,42 (s, 6H, $=N-O-C(C\underline{H}_3)_2-$); 3,31 (s, 6H, $(-OCH_3)_2$); 3,64 et 3,89 (2d, J = 18, 2H, $-S-CH_2-$); 3,95 (d, J = 5, 2H, $\rangle N-CH_2-$); 4,56 (t, J = 5, 1H, $-CH\langle^{O-}_{O-}$); 5,26 (d, J = 4, 1H, -H en 6); 5,77 (dd, J = 4 et 9, 1H, -H en 7); 6,71 (s, 1H, -H thiazole); 6,90 et 7,03 (2d, J = 16, 2H, -CH=CH-S-); 6,97 (s, 1H, $-COO-C\underline{H}-(C_6H_5)_2$); 8,80 (s, 1H, $-N\underline{H}-C(C_6H_5)_3$); 9,39 (d, J = 9, 1H, -CO-NH-); 12,66 (s, 1H, =N-NH-CO- ou =N-N=C-OH).

On agite à 20°C pendant 20 minutes une solution de 3,89 g de produit (5c) dans 39 $cm^3$ d'acide trifluoracétique. On concentre à sec à 20°C sous 0,05 mm de mercure (0,007 kPa), reprend le résidu dans 100 $cm^3$ d'éther diéthylique, agite pendant 10 minutes et filtre. Le solide obtenu est traité à 50°C pendant 45 minutes par 80 $cm^3$ d'acide formique, on ajoute 16 $cm^3$ d'eau, maintient 30 minutes à 50°C et concentre à sec à 20°C sous 0,05 mm de mercure (0,007 kPa). On reprend le résidu par 3 fois 150 $cm^3$ d'acétone en évaporant à chaque fois à 20°C sous 30 mm de mercure (4 kPa) et chauffe le résidu à reflux sous agitation dans 100 $cm^3$ d'acétone. On filtre et recueille 2,15 g d'{(amino-2 thiazolyl-4)-2 [(carboxy-2 propyl-2) oxyimino]-2 acétamido}-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (produit 5) sous la forme d'une poudre jaune.

Spectre infra-rouge (KBr), bandes caractéristiques ($cm^{-1}$) 3400, 3300, 3200, 2200, 1780, 1720, 1685, 1585, 1540, 1000.

Spectre de RMN du proton (350 MHz, $CF_3COOD$, $\delta$ en ppm, J en Hz) 1,85 et 1,86 (2s, 6H, $-CH_3$); 3,90 (s large, 2H, $-SCH_2-$); 5,20 (s, 2H, $-C\underline{H}_2CHO$); 5,40 (d, J = 4, 1H, H en 6); 6,12 (d, J = 4, 1H, H en 7); 7,23 et 7,76 (2d, J = 16, 2H, -CH=CH-); 7,50 (s, 1H, H du thiazole); 9,73 (s, 1H, -CHO).

Le produit (5a) peut être obtenu selon l'une ou l'autre des méthodes suivantes:

A/ A une solution refroidie à 0°C de 3 g de benzhydryloxycarbonyl-2 {[(t.butoxycarbonyl-2 propyl-2) oxyimino]-2 (tritylamino-2 thiazolyl-4)-2 acétamido}-7 oxo-8 (tosyloxy-2 vinyl)-3 thia-5 aza-1 bicyclo[4.2.0] octène-2, isomère syn, (produit 5d) dans 50 $cm^3$ de dichlorométhane, on ajoute goutte à goutte en 25 minutes une solution de 0,464 g d'acide m.chloroperbenzoïque à 90% dans 10 $cm^3$ de dichlorométhane. On agite pendant 1 heure à 0°C, dilue par 500 $cm^3$ d'acétate d'éthyle, lave par 2 fois 100 $cm^3$ d'une solution à 2% de bicarbonate de sodium, 2 fois 100 $cm^3$ d'eau et 100 $cm^3$ d'eau saturée de chlorure de sodium, sèche sur sulfate de sodium, filtre et concentre à sec à 20°C sous 20 mm de mercure (2,7 kPa). Le résidu est chromatographié sur une colonne de 60 g de gel de silice Merck (0,06-0,2) (diamètre de la colonne: 2 cm, hauteur: 20 cm), on élue par 1 litre d'un mélange cyclohexane-acétate d'éthyle (70-30 en volumes) en recueillant des fractions de 60 $cm^3$. Les fractions 5 à 14 sont concentrées à sec à 20°C sous 20 mm de mercure (2,7 kPa), on recueille 1,9 g de produit (5a) sous la forme d'une meringue jaune.

Spectre infra-rouge ($CHBr_3$), bandes caractéristiques ($cm^{-1}$) 3380, 1800, 1720, 1680, 1590, 1580, 1510, 1490, 1445, 1375, 1190, 1175, 1070, 730.

Spectre de RMN du proton (350 MHz, DMSO $d_6$, $\delta$ en ppm, J en Hz) 1,37 (s, 9H, $-C(CH_3)_3$); 1,45 et 1,46 (2s, 6H, $-O\ C(CH_3)_2-$); 2,44 (s, 3H, $-CH_3$ tosyle); 3,60 et 4,41 (2d, J = 18, 2H, $-SCH_2-$); 5,06 (d, J = 4, 1H, H en 6); 5,96 (dd, J = 4 et 9, 1H, H en 7); 6,75 (d, J = 13, 1H, $-C\underline{H}=CHS-$); 6,73 (s, 1H, H du thiazole); 6,93 (s, 1H, -COOCH-); 7,48 et 7,84 (ab, 2H, J = 9); 8,16 (d, J = 9, 1H, -CONH-); 8,73 (s, 1H, $-N\underline{H}\ C(C_6H_5)_3$).

Le produit (5d) peut être obtenu de la manière suivante:

A une solution refroidie à 5°C de 4,45 g de benzhydryloxycarbonyl-2 {[(t.butoxycarbonyl-2 propyl-2) oxyimino]-2 (tritylamino-2 thiazolyl-4)-2 acétamido}-7 formylméthyl-3 oxo-8 thia-5 aza-1 bicyclo[4.2.0] octène-2, isomère syn (produit 5e) dans 50 $cm^3$ de pyridine, on ajoute 1,31 g de chlorure de p.toluènesulfonyle. On laisse remonter la température à 20°C en 30 minutes, agite pendant 1 heure à 20°C, verse dans 300 $cm^3$ d'eau glacée, décante l'eau et reprend le produit pâteux insoluble dans 300 $cm^3$ d'acétate d'éthyle. On lave par 100 $cm^3$ d'acide chlorhydrique 1N, 100 $cm^3$ d'une solution saturée de bicarbonate de sodium et 100 $cm^3$ d'une solution saturée de chlorure de sodium, sèche sur sulfate de sodium, filtre et concentre à sec à 20°C sous 20 mm de mercure (2,7 kPa). Le produit ainsi obtenu est utilisé tel quel pour la suite de la synthèse. 1 g de ce produit est purifié par chromatographie sur une colonne de 20 g de gel de silice (0,05-0,2) (diamètre de la colonne: 1,7 cm) en éluant par un mélange de cyclohexane et d'acétate d'éthyle 80-20 (en volumes) et en recueillant des fractions de 50 $cm^3$. Les fractions 4 à 12 contenant le produit pur sont évaporées à sec sous pression réduite (30 mm de mercure; 4 kPa) à 30°C. On obtient 0,43 g de produit (5d) mélange des formes E et Z (dans des proportions — déterminées par RMN — de 75% de forme E et 25% de forme Z) sous la forme d'un solide jaune pâle.

Spectre infra-rouge ($CHBr_3$), bandes caractéristiques ($cm^{-1}$) 3400, 3260, 1790, 1720, 1680, 1630, 1595, 1580, 1520, 1490, 1450, 1380, 1370, 1190, 1180, 1070, 835, 750.

Spectre de RMN du proton (350 MHz, $CDCl_3$, $\delta$ en ppm, J en Hz) mélange d'isomères E et Z dans les proportions 75/25

forme E: 1,45 (s, 9H, $-C(CH_3)_3$); 1,62 et 1,67 (2s, 6H, $=N-O-C(CH_3)_2-$); 2,48 (s, 3H, $-CH_3$ tosyl); 3,42 et 3,50 (2d, J = 18, 2H, $-S-CH_2-$); 5,08 (d, J = 4, 1H, -H en 6); 6,00 (dd, J = 4 et 9, 1H, -H en 7); 6,78 (s, 1H, H thiazole); 6,88 (mf, 1H, $-N\underline{H}-C(C_6H_5)_3$); 6,90 et 6,97 (2d, J = 12, 2H, -CH=CH-S-); 6,92 (s, 1H, $-COO-C\underline{H}(C_6H_5)_2$); 8,20 (d, J = 9, 1H, -CO-NH-)

forme Z: 6,20 et 6,47 (2d, J = 7, -CH=CH-S- Z); 2,45 (s, $-CH_3$ tosyl).

Le produit (5e) peut être obtenu de la manière suivante:

On agite pendant 1 heure à 20°C un mélange de 6,45 g de benzhydryloxycarbonyl-2 (diméthylamino-2 vinyl)-3 {[(t.butoxycarbonyl-2 propyl-2) oxyimino]-2 (tritylamino-2 thiazolyl-4)-2 acétamido}-7 oxo-8 thia-5 aza-1 bicyclo[4.2.0] octène-2, isomère syn, forme E (produit 5f) en solution dans 100 $cm^3$ d'acétate d'éthyle et 64 $cm^3$ d'acide chlorhydrique 1N. On décante, lave par 100 $cm^3$ d'eau, 100 $cm^3$ d'eau saturée de bicarbonate de sodium et 100 $cm^3$ d'eau saturée de chlorure de sodium, sèche sur sulfate de sodium, filtre et concentre à sec à 20°C sous 20 mm de mercure (2,7 kPa). On recueille 4,95 g de produit (5e) brut sous la forme d'une meringue brune.

Spectre infra-rouge ($CHBr_3$), bandes caractéristiques ($cm^{-1}$) 3400, 3270, 2720, 1770, 1725, 1685, 1525, 1495, 1450, 1370, 755, 700.

Spectre de RMN du proton (350 MHz, $CDCl_3$, $\delta$ en ppm, J en Hz) 1,45 (s, 9H, $-C(CH_3)_3$); 1,64 et 1,67 (2s, 6H, $(-CH_3)_2$); 3,25 et 3,54 (2d, J = 18, 2H, $-SCH_2-$); 3,50 et 3,73 (2d, J = 16, 2H, $-C\underline{H_2}CHO$); 5,10 (d, J = 4, 1H, H en 6); 6,06 (dd, J = 4 et 9, 1H, H en 7); 6,77 (s, 1H, H du thiazole); 6,90 (s, 1H, -COOCH<); 8,22 (d, J = 9, 1H, -CONH-); 9,58 (s, 1H, -CHO).

Le produit (5f) peut être obtenu de la manière suivante:

A une solution à 80°C sous azote de 6 g de benzhydryloxycarbonyl-2 {[(t.butoxycarbonyl-2 propyl-2) oxyimino]-2 (tritylamino-2 thiazolyl-4)-2 acétamido}-7 méthyl-3 oxo-8 thia-5 aza-1 bicyclo[4.2.0] octène-2, isomère syn (produit 5g) dans 64 $cm^3$ de diméthylformamide, on ajoute sous agitation 1,9 $cm^3$ de t.butoxy bis-diméthylaminométhane et poursuit la réaction pendant 15 minutes. On verse ensuite le mélange dans un mélange de 200 $cm^3$ d'acétate d'éthyle et 200 $cm^3$ d'eau, décante, lave par 3 fois 100 $cm^3$ d'eau et 100 $cm^3$ d'eau saturée de chlorure de sodium, sèche sur sulfate de sodium, filtre et concentre à sec à 20°C sous 20 mm de mercure (2,7 kPa). On obtient ainsi 6,5 g de produit (5f) brut sous la forme d'une meringue brune.

On purifie un échantillon (2,2 g) par chromatographie sur une colonne (diamètre 4 cm, hauteur 20

cm) de gel de silice Merck (0,04-0,06) en éluant par un mélange cyclohexane-acétate d'éthyle 65/35 (vol.) sous une pression de 40 kPa et en recueillant des fractions de 50 $cm^3$. Les fractions 14 à 24 sont concentrées à sec. On obtient 1,2 g de benzhydryloxycarbonyl-2 (diméthylamino-2 vinyl)-3 [[ [(t.butoxycarbonyl-2 propyl-2) oxyimino]-2 (tritylamino-2 thiazolyl-4)-2 acétamido]]-7 oxo-8 thia-5 aza-1 bicyclo[4.2.0] octène-2 isomère syn, forme E.

Spectre infra-rouge ($CHBr_3$), bandes caractéristiques ($cm^{-1}$) 3400, 3280, 1770, 1720, 1680, 1610, 1525, 1490, 1450, 1370, 940, 750, 735.

Spectre de RMN du proton (350 MHz, $CDCl_3$, $\delta$ en ppm, J en Hz) 1,45 (s, 9H, $-C(CH_3)_3$); 1,64 et 1,71 (2s, 6H, $=N-O-C(CH_3)_2-$); 2,93 (s, 6H, $-N(CH_3)_2$); 3,20 et 3,30 (2d, J = 14, 2H, $-S-CH_2-$); 5,18 (d, J = 4, 1H, -H en 6); 5,71 (dd, J = 4 et 9, 1H, -H en 7); 6,61 et 6,82 (2d, J = 14, 2H, -CH=CH-S-); 6,88 (s, 1H, H thiazole); 6,92 (s, 1H, $-COO-\underline{CH}(C_6H_5)_2$); 6,92 (s large, 1H, $-\underline{NH}-C(C_6H_5)_3$); 8,28 (d, J = 9, 1H, -CO-NH-).

Le benzhydryloxycarbonyl-2 [[ [(t.butoxycarbonyl-2 propyl-2) oxyimino]-2 (tritylamino-2 thiazolyl-4)-2 acétamido]]-7 méthyl-3 oxo-8 thia-5 aza-1 bicyclo[4.2.0] octène-2 isomère syn peut être préparé de la manière suivante:

A une solution de 25,58 g d'acide (t.butoxycarbonyl-2 propyl-2 oxyimino)-2 (tritylamino-2 thiazolyl-4)-2 acétique préparé selon le brevet belge 876 541 dans un mélange de 250 $cm^3$ de dichlorométhane et 13,9 $cm^3$ de diméthylacétamide, on ajoute à —10°C en 30 minutes et sous agitation 40 $cm^3$ d'une solution 1,3 M de phosgène dans du chlorobenzène. On agite encore pendant 3 heures à —10°C et ajoute goutte à goutte en 1 heure 15 une solution de 16,29 g d'amino-7 benzhydryloxycarbonyl-2 méthyl-3 oxo-8 thia-5 aza-1 bicyclo[4.2.0] octène-2 dans 250 $cm^3$ de dichlorométhane. Après 1 heure 15 à —10°C, on lave le mélange par 200 $cm^3$ d'une solution aqueuse à 2% de bicarbonate de sodium et 200 $cm^3$ d'eau, sèche sur sulfate de sodium et concentre à sec à 30°C sous 20 mm de mercure (2,7 kPa). Le résidu est chromatographié sur une colonne de 200 g de gel de silice Merck (0,05-0,2) (diamètre de la colonne: 4 cm). On élue par un mélange cyclohexane-acétate d'éthyle 70-30 (en volumes) en recueillant des fractions de 120 $cm^3$. Les fractions 6 à 10 sont concentrées à sec à 30°C sous 20 mm de mercure (2,7 kPa). On recueille 8,5 g de benzhydryloxycarbonyl-2 [(t.butoxycarbonyl-2 propyl-2 oxyimino)-2 (tritylamino-2 thiazolyl-4)-2 acétamido]-7 méthyl-3 oxo-8 thia-5 aza-1 bicyclo[4.2.0] octène-2, isomère syn, sous la forme d'une meringue jaune.

Spectre infra-rouge ($CHBr_3$), bandes caractéristiques ($cm^{-1}$) 3400, 3270, 1790, 1725, 1685, 1525, 1500, 1450, 1380, 1370, 760, 740.

B/ A une solution de 9,84 g d'amino-7 benzhydryloxycarbonyl-2 oxo-8 oxyde-5 (tosyloxy-2 vinyl)-3 thia-5 aza-1 bicyclo[4.2.0] octène-2, forme E (produit 5h) dans 170 $cm^3$ de dichlorométhane, on ajoute 11,66 g d'acide [(t.butoxycarbonyl-2 propyl-2) oxyimino]-2 (tritylamino-2 thiazolyl-4)-2 acétique, isomère syn (préparé selon le procédé décrit dans le brevet belge 876 541) et 0,1 g de diméthylamino-4 pyridine. On ajoute 50 $cm^3$ de diméthylformamide pour obtenir une solution limpide, refroidit entre 0°C et 5°C et ajoute en 15 minutes, sous agitation, une solution de 4,21 g de N,N'-dicyclohexylcarbodiimide dans 50 $cm^3$ de dichlorométhane. On agite pendant 2 heures à 5°C et 2 heures à 20°C et concentre le mélange à 20°C sous 20 mm de mercure (2,7 kPa). Le résidu est repris dans 150 $cm^3$ d'acétate d'éthyle, on filtre, lave par 3 fois 100 $cm^3$ d'eau, 2 fois 100 $cm^3$ d'eau demi-saturée de bicarbonate de sodium et 2 fois 100 $cm^3$ d'une solution demi-saturée de chlorure de sodium, sèche sur sulfate de sodium, filtre et concentre à sec à 20°C sous 20 mm de mercure (2,7 kPa). On chromatographie le résidu sur une colonne de 400 g de gel de silice Merck (0,06-0,2) (diamètre de la colonne: 4 cm, hauteur: 76 cm), on élue par 1 litre d'un mélange cyclohexane-acétate d'éthyle 80-20 (en volumes) et 4 litres d'un mélange 70-30 en recueillant des fractions de 250 $cm^3$. On concentre à sec les fractions 6 à 11 à 20°C sous 20 mm de mercure (2,7 kPa) et recueille 6,95 g de produit (5a) sous la forme d'une meringue jaune dont les caractéristiques sont identiques à celles du produit (5a) obtenu en A/.

La préparation du produit (5h) a été décrite précédemment dans l'exemple 1 comme produit (1d).

*Exemple 6*

On procède comme décrit dans l'exemple 1 mais à partir de 3,2 g de benzhydryloxycarbonyl-2 {[(t.butoxycarbonyl-2 propyl-2) oxyimino]-2 (tritylamino-2 thiazolyl-4)-2 acétamido}-7 oxo-8 oxyde-5 (tosyloxy-2 vinyl)-3 thia-5 aza-1 bicyclo[4.2.0] octène-2, isomère syn, forme E (produit 6a), 28 $cm^3$ de diméthylformamide, 0,37 g de mercapto-5 méthyl-1 tétrazole et 0,574 $cm^3$ de N,N-diisopropyléthylamine. Le résidu obtenu après traitement est chromatographié sur une colonne de 70 g de gel de silice Merck (0,06-0,2) (diamètre de la colonne: 2,3 cm, hauteur: 25 cm). On élue par 1 litre d'un mélange cyclohexane-acétate d'éthyle 50-50 (en volumes) en recueillant des fractions de 60 $cm^3$. Les fractions 6 à 10 sont concentrées à sec à 20°C sous 20 mm de mercure (2,7 kPa), on obtient 1,9 g de benzhydryloxycarbonyl-2 {[(t.butoxycarbonyl-2 propyl-2) oxyimino]-2 (tritylamino-2 thiazolyl-4)-2 acétamido}-7 [(méthyl-1 tétrazolyl-5) thio-2 vinyl]-3 oxo-8 oxyde-5 thia-5 aza-1 bicyclo[4.2.0] octène-2, isomère syn, forme E (produit 6b) sous la forme d'une meringue brune.

Spectre infra-rouge (KBr), bandes caractéristiques ($cm^{-1}$) 1800, 1730, 1680, 1515, 1495, 1450, 1370, 1045, 940, 750, 700.

Spectre de RMN du proton (350 MHz, $CDCl_3$, $\delta$ en ppm, J en Hz) 1,44 (s, 9H, $-C(CH_3)_3$); 1;58 et 1,60 (2s, 6H, $(-CH_3)_2$); 3,93 (s, 3H, $>N-CH_3$); 3,28 et 4,04 (2d, J = 18, 2H, $-SCH_2-$); 4,65 (d, J = 4, 1H, H en 6); 6,24 (dd, J = 4 et 9, 1H, H en 7); 6,76 (s, 1H, H du thiazole); 6,91 (s, 1H, -COO CH$<$); 7,0 et 7,59 (2d, J = 16, 2H, -CH=CH-); 7,9 (d, J = 9, 1H, -CONH-).

On procède comme décrit dans l'exemple 1 mais à partir de 1,9 g de produit (6b) en solution dans 17 $cm^3$ de dichlorométhane et 0,63 $cm^3$ de diméthylacétamide et 0,297 $cm^3$ de trichlorure de phos-

phore. On chromatographie le résidu (obtenu après traitement comme dans l'exemple 1) sur une colonne de 40 g de gel de silice Merck (0,06-0,2) (diamètre de la colonne: 1,75 cm, hauteur: 20 cm). On élue par 1 litre d'un mélange cyclohexane-acétate d'éthyle 50-50 (en volumes) en recueillant des fractions de 60 $cm^3$. On concentre à sec les fractions 4 à 6 et obtient 0,83 g de benzhydryloxycarbonyl-2 {[(t.butoxycarbonyl-2 propyl-2) oxyimino]-2 (tritylamino-2 thiazolyl-4)-2 acétamido}-7 [(méthyl-1 tétrazolyl-5) thio-2 vinyl]-3 oxo-8 thia-5 aza-1 bicyclo[4.2.0] octène-2, isomère syn, forme E (produit 6c) sous la forme d'une meringue de couleur crème.

Spectre infra-rouge (KBr), bandes caractéristiques ($cm^{-1}$) 1790, 1720, 1685, 1520, 1490, 1445, 1370, 750, 700.

Spectre de RMN du proton (350 MHz, $CDCl_3$, $\delta$ en ppm, J en Hz) 1,43 (s, 9H, $-C(CH_3)_3$); 1,62 et 1,66 (2s, 6H, $=N-O-C(CH_3)_2-$); 3,6 et 3,7 (2d, J = 18, 2H, $-S-CH_2-$); 3,96 (s, 3H, $>N-CH_3$); 5,12 (d, J = 4, 1H, -H en 6); 6,05 (dd, J = 4 et 9, 1H, -H en 7); 6,75 (s, 1H, -H thiazole); 6,9 (s large, 1H, $-N\underline{H}-C(C_6H_5)_3$); 6,95 (s, 1H, $-COO-C\underline{H}(C_6H_5)_2$); 7,04 (d, J = 16, 1H, $-C\underline{H}=CH-S-$); 8,23 (d, J = 9, 1H, -CO-NH-).

On agite à 20°C pendant 30 minutes, une solution de 0,82 g de produit (6c) dans 10 $cm^3$ d'acide trifluoracétique. On concentre à sec à 20°C sous 0,5 mm de mercure (0,007 kPa), reprend le résidu dans un mélange de 10 $cm^3$ d'acide formique et 5 $cm^3$ d'eau, agite à 60°C pendant 30 minutes et concentre à sec à 20°C sous 0,5 mm de mercure (0,007 kPa). Le résidu est repris par 3 fois 100 $cm^3$ d'éthanol en évaporant à sec à chaque fois à 20°C sous 30 mm de mercure (4 kPa). Le solide obtenu est dissous à 60°C dans 8 $cm^3$ d'éthanol, on ajoute 50 $cm^3$ d'éther diéthylique et filtre. On recueille 0,34 g d'{(amino-2 thiazolyl-4)-2 [(carboxy-2 propyl-2) oxyimino]-2 acétamido}-7 carboxy-2 [(méthyl-1 tétrazolyl-5) thio-2 vinyl]-3 oxo-8 thia-5 aza-1 bicyclo[4.2.0] octène-2, isomère syn, forme E (produit 6) sous la forme d'une poudre jaune.

Spectre infra-rouge (KBr), bandes caractéristiques, ($cm^{-1}$) 3600-2200, 1775, 1675, 1630, 1530, 1385, 990, 950, 700.

Spectre de RMN du proton (350 MHz, DMSO $d_6$, $\delta$ en ppm, J en Hz) 1,48 (s, 6H, $(-CH_3)_2$); 3,63 et 3,89 (2d, J = 18, 2H, $-SCH_2-$); 4,03 (s, 3H, $>N-CH_3$); 5,23 (d, J = 4, 1H, H en 6); 5,88 (dd, J = 4 et 9, 1H, H en 7); 6,73 (s, 1H, H du thiazole); 6,97 et 7,10 (2d, J = 16, 2H, -CH=CH-); 7,18 (s, 2H, $-NH_2$); 9,42 (d, J = 9, -CONH-).

*Exemple 7*

En procédant comme décrit dans l'exemple 1 mais à partir de 2,69 g de benzhydryloxycarbonyl-2 [(t.butoxycarbonyl-1 cyclobutyloxyimino)-2 (tritylamino-2 thiazolyl-4)-2 acétamido]-7 oxo-8 oxyde-5 (tosyloxy-2 vinyl)-3 thia-5 aza-1 bicyclo[4.2.0] octène-2, isomère syn, forme E (produit 7a), 30 $cm^3$ de diméthylformamide, 0,57 g de (diméthoxy-2,2 éthyl)-4 dioxo-5,6 thioxo-3 perhydrotriazine-1,2,4 et 0,43 $cm^3$ de N,N-diisopropyléthylamine, on obtient après traitement et chromatographie sur gel de silice Merck (0,06-0,2) [éluant: cyclohexane-acétate d'éthyle 40-60 (en volumes) 2,5 g de benzhydryloxycarbonyl-2 [(t.butoxycarbonyl-1 cyclobutyloxyimino)-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo[4.2.0] octène-2, isomère syn, forme E (produit 7b) sous la forme d'une meringue jaune.

Spectre infra-rouge ($CHBr_3$), bandes caractéristiques ($cm^{-1}$) 3360, 3240, 1800, 1720, 1680, 1585, 1520, 1490, 1450, 1080, 970, 940, 750, 735.

Spectre de RMN du proton (350 MHz, $CDCl_3$, $\delta$ en ppm, J en Hz) 1,4 (s, 9H, $-C(CH_3)_3$); 1,85 à 2,15 (mt, 2H, $-CH_2-C\underline{H_2}-CH_2-$); 2,3 à 2,8 (mt, 4H, $-C\underline{H_2}-CH_2-C\underline{H_2}-$); 3,42 à 3,43 (2s, 6H, $(-OCH_3)_2$); 3,48 et 4,19 (2d, J = 18, 2H, $-S-CH_2-$); 3,9 à 4,1 (mt, 2H, $>N-CH_2-$); 4,66 (t, J = 5, 1H, $-CH<^{O-}_{O-}$); 4,84 (d, J = 4, 1H, -H en 6); 5,85 (dd, J = 4 et 9, 1H, -H en 7); 6,63 (s, 1H, -H thiazole); 6,95 (s, 1H, $-COO-C\underline{H}(C_6H_5)_2$); 7,08 (d, J = 16, $-C\underline{H}=CH-S-$); 7,7 (mf, 1H, $-N\underline{H}-C(C_6H_5)_3$); 7,96 (d, J = 9, 1H, -CO-NH-); 12,35 (mf, 1H, =N-NH-CO ou =N-N=C-OH).

En procédant comme décrit dans l'exemple 1 mais à partir de 2,37 g de produit (7b), 40 $cm^3$ de dichlorométhane, 1,55 $cm^3$ de diméthylacétamide et 0,695 $cm^3$ de trichlorure de phosphore, on obtient après traitement et chromatographie sur gel de silice Merck (0,06-0,2) [éluant: cyclohexane-acétate d'éthyle 30-70 (en volumes)] 1,5 g de benzhydryloxycarbonyl-2 [(t.butoxycarbonyl-1 cyclobutyloxyimino)-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 thia-5 aza-1 bicyclo[4.2.0] octène-2, isomère syn, forme E (produit 7c) sous la forme d'une poudre de couleur crème.

Spectre infra-rouge ($CHBr_3$), bandes caractéristiques ($cm^{-1}$) 3390, 3280, 2840, 1790, 1720, 1695, 1590, 1520, 1495, 1450, 1370, 1080, 950, 750, 700.

Spectre de RMN du proton (350 MHz, $CDCl_3$, $\delta$ en ppm, J en Hz) 1,48 (s, 9H, $-C(CH_3)_3$); 2 à 2,10 (mt, 2H, $-CH_2-C\underline{H_2}-CH_2-$); 2,50 à 2,7 (mt, 4H, $-C\underline{H_2}-CH_2-C\underline{H_2}-$); 3,45 (s, 6H, $(-OCH_3)_2$); 3,61 (ab limite, 2H, $-S-CH_2-$); 4,03 (d, J = 6, 2H, $>N-CH_2-$); 4,69 (t, J = 6, 1H, $-CH<^{O-}_{O-}$); 5,13 (d, J = 4, 1H, -H en 6); 5,98 (dd, J = 4 et 9, 1H, -H en 7); 6,79 (s, 1H, H thiazole); 6,83 (d, J = 15, 1H, $-C\underline{H}=CH-S-$); 6,98 (s, 1H, $-COO-C\underline{H}(C_6H_5)_2$); 8,60 (d, J = 9, 1H, -CO-NH-); 10,70 (mf large, 1H, =N-NH-CO- ou =N-N=C-OH).

On agite à 20°C pendant 30 minutes une solution de 1,5 g de produit (7c) dans 15 $cm^3$ d'acide trifluoracétique, concentre à sec à 20°C sous 0,5 mm de mercure (0,007 kPa), triture dans 50 $cm^3$ d'éther diéthylique et filtre. Le solide de couleur crème obtenu est chauffé pendant 15 minutes à 50°C dans un mélange de 15 $cm^3$ d'acide formique et de 7 $cm^3$ d'eau, on concentre à sec à 30°C sous 0,5 mm de mercure, (0,007 kPa) et reprend le résidu dans 3 fois

20 $cm^3$ d'acétone en concentrant à chaque fois à sec à 20 °C sous 20 mm de mercure (2,7 kPa). Le résidu est chauffé à reflux dans 20 $cm^3$ d'acétone, on filtre et recueille 0,75 g d'[(amino-2 thiazolyl-4)-2 (carboxy-1 cyclobutyloxyimino)-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (produit 7) sous la forme d'une poudre jaune.

Spectre infra-rouge (KBr), bandes caractéristiques ($cm^{-1}$) 3400, 3300, 3220, 3100, 2200, 1780, 1715, 1685, 1585, 1535, 1055, 945.

Spectre de RMN du proton (350 MHz, $CF_3COOD$, $\delta$ en ppm, J en Hz) 2,20 à 2,32 (mt, 2H, $-CH_2-C\underline{H}_2-CH_2-$); 2,65 à 2,95 (mt, 4H, $-C\underline{H}_2-CH_2-C\underline{H}_2-$); 3,90 (s large, 2H, $-S-CH_2-$); 5,20 (s, 2H, $>N-CH_2-$); 5,40 (d, J = 4, 1H, -H en 6); 6,14 (d, J = 4, 1H, -H en 7); 7,24 et 7,75 (2d, J = 16, 2H, -CH=CH-S-);

7,49 (s, 1H, H thiazole); 9,78 (s, 1H, 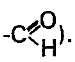).

Le produit (7a) peut être préparé de la manière suivante:

A une solution de 4,34 g d'amino-7 benzhydryloxycarbonyl-2 oxo-8 oxyde-5 (tosyloxy-2 vinyl)-3 thia-5 aza-1 bicyclo[4.2.0] octène-2, forme E (produit 7d) dans 50 $cm^3$ de dichlorométhane, on ajoute 4,42 g d'acide (t.butoxycarbonyl-1 cyclobutyloxyimino)-2 (tritylamino-2 thiazolyl-4)-2 acétique, isomère syn préparé selon le brevet belge 876 541 et 0,25 g de diméthylamino-4 pyridine. On refroidit à 5 °C et ajoute goutte à goutte en 10 minutes une solution de 1,69 g de N,N'-dicyclohexylcarbodiimide dans 30 $cm^3$ de dichlorométhane. On laisse remonter la température, agite pendant 4 heures à 20 °C, concentre à sec à 20 °C sous 20 mm de mercure (2,7 kPa) et reprend dans 200 $cm^3$ d'acétate d'éthyle. On lave par 2 fois 100 $cm^3$ d'eau, 100 $cm^3$ d'acide chlorhydrique 0,1 N, 100 $cm^3$ d'eau saturée de bicarbonate de sodium et 100 $cm^3$ d'eau, sèche sur sulfate de sodium, filtre et concentre à sec à 25 °C sous 20 mm de mercure (2,7 kPa). Le résidu est chromatographié sur une colonne de 180 g de gel de silice Merck (0,06-0,2) (diamètre de la colonne: 3 cm, hauteur: 60 cm). On élue par 1 litre d'un mélange cyclohexane-acétate d'éthyle 70-30 (en volumes) en recueillant des fractions de 60 $cm^3$. Les fractions 6 à 10 sont concentrées à sec à 20 °C sous 20 mm de mercure (2,7 kPa), on obtient 2,7 g de produit (7a) sous la forme d'une poudre de couleur crème.

Spectre infra-rouge ($CHBr_3$), bandes caractéristiques ($cm^{-1}$) 3390, 1800, 1720, 1680, 1595, 1495, 1450, 1520, 1370, 1190, 1180, 1070, 940, 830, 750, 700.

Spectre de RMN du proton (350 MHz, $CDCl_3$, $\delta$ en ppm, J en Hz) 1,45 (s, 9H, $-C(CH_3)_3$); 1,92 à 2,12 (mt, 2H, $-CH_2-C\underline{H}_2-CH_2-$); 2,40 à 2,65 (mt, 4H, $-C\underline{H}_2-CH_2-C\underline{H}_2-$); 2,48 (s, 3H, $-CH_3$ tosyl); 3,15 et 3,71 (2d, J = 18, 2H, $-S-CH_2-$); 4,6 (d, J = 4, 1H, -H en 6); 6,17 (dd, J = 4 et 9, 1H, -H en 7); 6,75 (s, 1H, -H thiazole); 6,91 (s, 1H, $-COO-C\underline{H}(C_6H_5)_2$); 6,92 et 7,09 (2d, J = 12, 2H, -CH=CH-S-); 6,99 (s, 1H, $-N\underline{H}-C(C_6H_5)_3$); 8,02 (d, J = 9, 1H, -CO-NH-).

*Exemple 8*

Une solution, dans 120 $cm^3$ de N,N diméthylformamide, de 5,85 g de benzhydryloxycarbonyl-2 [méthoxyimino-2 (tritylamino-2 thiazolyl-4)-2 acétamido]-7 oxo-8 (tosyloxy-2 vinyl)-3 thia-5 aza-1 bicyclo[4.2.0] octène-2, isomère syn, forme E (produit 8a, préparé comme décrit dans le brevet belge 883 415), et de 1,59 g de sel de sodium de la dioxo-5,6 [N-(hydroxy-2 éthyl) carbamoylméthyl]-4 thioxo-3 perhydrotriazine-1,2,4 est chauffée à 60 °C pendant 1 heure 1/2 puis versée dans 800 $cm^3$ d'eau distillée. On extrait par 3 fois 200 $cm^3$ d'acétate d'éthyle et lave les extraits par 2 fois 200 $cm^3$ de solution demi-saturée de chlorure de sodium. Après séchage sur sulfate de magnésium et évaporation du solvant sous pression réduite (30 mm de mercure; 4 kPa) à 30 °C on chromatographie le résidu sur une colonne de silice (0,04-0,06) (diamètre de la colonne: 4 cm, hauteur: 30 cm) en éluant sous 60 kPa par un mélange de dichlorométhane et de méthanol (95-5 en volumes) et en recueillant des fractions de 100 $cm^3$. Les fractions 15 à 21 contenant le produit pur sont rassemblées et concentrées à sec sous pression réduite (30 mm de mercure; 4 kPa) à 30 °C. On obtient 2 g de benzhydryloxycarbonyl-2 {[dioxo-5,6 [N-(hydroxy-2 éthyl) carbamoylméthyl]-4 tétrahydro-1,4,5,6 triazine-1,2,4 yl-3} thio-2 vinyl}-3 [méthoxyimino-2 (tritylamino-2 thiazolyl-4)-2 acétamido]-7 oxo-8 thia-5 aza-1 bicyclo[4.2.0] octène-2, isomère syn, forme E (produit 8b) sous la forme d'une meringue jaune.

Spectre infra-rouge ($CHBr_3$), bandes caractéristiques ($cm^{-1}$) 3360, 3200, 1780, 1720, 1675, 1590, 1520, 1490, 1445, 1045, 940, 750, 740.

Spectre de RMN du proton (350 MHz, DMSO $d_6$, $\delta$ en ppm, J en Hz) 3,18 (mt, 2H, $-CONH-C\underline{H}_2-$); 3,42 (mt, 2H, $-CH_2-O-$); 3,62 et 3,87 (2d, J = 18, 2H, $-S-CH_2-$); 3,83 (s, 3H, $=N-OCH_3$); 4,48 (s large, 2H, $>N-CH_2-$); 4,71 (t, J = 5, 1H, -OH); 5,25 (d, J = 4, 1H, -H en 6); 5,76 (dd, J = 4 et 9, 1H, -H en 7); 6,74 (s, 1H, H thiazole); 6,88 et 6,97 (2d, J = 16, 2H, -CH=CH-S-); 6,96 (s, 1H, $-COO-C\underline{H}(C_6H_5)_2$); 8,30 (t, J = 5,5, 1H, $-CO-N\underline{H}-CH_2-$); 8,80 (s, 1H, $-N\underline{H}-C(C_6H_5)_3$); 9,59 (d, J = 9, 1H, -CO-NH-); 12,69 (s, 1H, =N-NH-CO- ou =N-N=C-OH).

Une solution de 2 g de produit (8b) dans 25 $cm^3$ d'acide formique et 10 $cm^3$ d'eau distillée est chauffée à 50 °C pendant 50 minutes puis refroidie à 25 °C. Après filtration de l'insoluble le filtrat est concentré sous pression réduite (2 mm de mercure; 0,27 kPa) à 30 °C. Le résidu est trituré avec 30 $cm^3$ d'éthanol anhydre que l'on évapore ensuite sous pression réduite (30 mm de mercure; 4 kPa) à 30 °C. Cette opération est répétée 3 fois puis le résidu est repris dans 30 $cm^3$ d'éthanol. Le solide est essoré, lavé 3 fois par 20 $cm^3$ d'éthanol et 3 fois par 20 $cm^3$ d'éther éthylique. On obtient 1,22 g d'[(amino-2 thiazolyl-4)-2 méthoxyimino-2 acétamido]-7 carboxy-2 {[dioxo-5,6 [N-(hydroxy-2 éthyl) carbamoylméthyl]-4 tétrahydro-1,4,5,6 triazine-1,2,4 yl-3} thio-2 vinyl}-3 oxo-8 thia-5 aza-1 bicyclo[4.2.0] octène-2, isomère syn, forme E (produit 8) sous la forme d'un solide jaune.

Spectre infra-rouge (KBr), bandes caractéristiques

($cm^{-1}$) 3600-2700, 1770, 1715, 1675, 1590, 1550, 1040, 945.

Spectre de RMN du proton (350 MHz, DMSO $d_6$, $\delta$ en ppm, J en Hz) 3,20 à 3,60 (mt, >N-$CH_2$-$CH_2$-O-); 3,62 et 3,84 (2d, J = 18, 2H, -S-$CH_2$-); 3,85 (s, 3H, =N-$OCH_3$); 4,12 (t, J = 7, 1H, -OH); 4,50 (s, 2H, >N-$CH_2$-); 5,20 (d, J = 4, 1H, -H en 6); 5,79 (dd, J = 4 et 9, 1H, -H en 7); 6,74 (s, 1H, H thiazole); 6,85 et 7,07 (2d, J = 16, 2H, -CH=CH-S-); 7,18 (s, 2H, -$NH_2$); 8,45 (t, J = 6, -CO-N<u>H</u>-$CH_2$-); 9,60 (d, J = 9, 1H, -CO-NH-); 12,70 (s, 1H, =N-NH-CO- ou =N-N=C-OH).
|

La dioxo-5,6 [N-(hydroxy-2 éthyl) carbamoylméthyl]-4 thioxo-3 perhydrotriazine-1,2,4 peut être préparée de la manière suivante:

On ajoute à une solution de 0,23 g de sodium dans 15 $cm^3$ de méthanol sec à 25°C, 1,92 g de N-(hydroxy-2 éthyl) carbamoylméthyl-4 thiosemicarbazide et 1,33 $cm^3$ d'oxalate d'éthyle. Le mélange réactionnel est chauffé à la température de reflux pendant 2 heures 1/2. Après refroidissement vers 25°C on isole le solide sur filtre à vide, on le lave par 5 $cm^3$ de méthanol et 20 $cm^3$ d'éther éthylique et on le sèche. On obtient 1,65 g de sel de sodium de dioxo-5,6 N-(hydroxy-2 éthyl) carbamoylméthyl-4 thioxo-3 perhydrotriazine-1,2,4 sous la forme d'un solide rosé.

Spectre infra-rouge (KBr), bandes caractéristiques ($cm^{-1}$) 3400, 3300, 1695, 1660, 1645, 1590, 1070.

La N-(hydroxy-2 éthyl) carbamoylméthyl-4 thiosemicarbazide peut être préparée de la manière suivante:

5 g d'éthoxycarbonylméthyl-4 thiosemicarbazide sont traités dans 60 $cm^3$ d'éthanol par 1,7 g d'hydroxy-2 éthylamine pendant 1 heure à la température de reflux du mélange réactionnel, qui est ensuite concentré à sec sous pression réduite (30 mm de mercure; 4 kPa) à 40°C. On obtient 5,4 g de N-(hydroxy-2 éthyl) carbamoylméthyl-4 thiosemicarbazide sous la forme d'une huile qui cristallise lentement.

Spectre de RMN du proton (60 MHz, DMSO $d_6$, $\delta$ en ppm, J en Hz) 3,25 (t, J = 6, 2H, -C<u>$H_2$</u>OH); 3,40 (q, J = 6, 2H, -NHC<u>$H_2$</u>$CH_2$OH); 4,13 (s large, 2H, -NHC<u>$H_2$</u>CO-); 4,66 (m, 3H, -$NH_2$ et -OH); 7,9 (m, 2H, -N<u>H</u> C N<u>H</u>-).
||
S

*Exemple 9*

On traite 6 g de benzhydryloxycarbonyl-2 [méthoxyimino-2 (tritylamino-2 thiazolyl-4)-2 acétamido]-7 oxo-8 oxyde-5 (tosyloxy-2 vinyl)-3 thia-5 aza-1 bicyclo[4.2.0] octène-2, isomère syn, forme E (produit 9a) par 1,65 g de (diéthoxy-2,2 éthyl)-1 dioxo-5,6 méthyl-4 thioxo-3 perhydrotriazine-1,2,4 dans 60 $cm^3$ de N,N-diméthylformamide à 60°C en présence de 1,05 $cm^3$ de N,N-diisopropyléthylamine conformément au mode opératoire décrit à l'exemple 1. On obtient 2,57 g de benzhydryloxycarbonyl-2 {[(diéthoxy-2,2 éthyl)-1 dioxo-5,6 méthyl-4 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme E (produit 9b) sous la forme d'une meringue orangée.

Spectre infra-rouge ($CHBr_3$), bandes caractéristiques ($cm^{-1}$) 3380, 2820, 1800, 1715, 1680, 1585, 1515, 1495, 1445, 1050, 940, 750.

Spectre de RMN du proton (350 MHz, DMSO $d_6$, $\delta$ en ppm, J en Hz) 1,07 et 1,08 (2t, J = 7, 6H, 2-$CH_3$); 3,28 (s, >N-$CH_3$); 3,40 à 3,70 (2mt, 4H, (-O-C<u>$H_2$</u>-$CH_3$)$_2$); 3,62 et 4,36 (2d, J = 18, 2H, -S-$CH_2$-); 3,85 (s, 3H, =N-$OCH_3$); 3,95 (AB limite, 2H, >N-$CH_2$-); 4,85 (t, J = 6, 1H, -CH<$^{O-}_{O-}$); 5,08 (d, J = 4, 1H, -H en 6); 5,87 (dd, J = 4 et 9, 1H, -H en 7); 6,80 (s, 1H, -H thiazole); 6,99 (s, 1H, -COO-C<u>H</u>($C_6H_5$)$_2$); 7,05 et 7,18 (2d, J = 16, 2H, -CH=CH-S-); 8,79 (s, 1H, -N<u>H</u>-C($C_6H_5$)$_3$); 9,12 (d, J = 9, 1H, -CO-NH-).

Rf = 0,3 chromatoplaque de gel de silice Merck [éluant: mélange de cyclohexane et d'acétate d'éthyle 20-80 en volumes].

On traite 2,3 g de produit (9b) par 0,44 $cm^3$ de trichlorure de phosphore conformément au mode opératoire de l'exemple 1. On obtient 2 g de benzhydryloxycarbonyl-2 {[(diéthoxy-2,2 éthyl)-1 dioxo-5,6 méthyl-4 tétrahydro-1,4,5,6 triazine-1,2,4 yl-3] thio-2 vinyl}-3 [méthoxyimino-2 (tritylamino-2 thiazolyl-4)-2 acétamido]-2 oxo-8 thia-5 aza-1 bicyclo-[4.2.0] octène-2, isomère syn, forme E (produit 9c) sous la forme d'une meringue jaune.

Spectre infra-rouge ($CHBr_3$), bandes caractéristiques ($cm^{-1}$) 3390, 2820, 1785, 1715, 1680, 1585, 1515, 1490, 1455, 1050, 940, 750, 740.

Spectre de RMN du proton (350 MHz, DMSO $d_6$, $\delta$ en ppm, J en Hz) 1,07 (t, J = 7, 6H, 2-$CH_3$); 3,30 (s, >N-$CH_3$); 3,40 à 3,68 (2 mt, 4H, (-O-C<u>$H_2$</u>-$CH_3$)$_2$); 3,68 et 3,92 (2d, J = 18, 2H, -S-$CH_2$-); 3,83 (s, 3H, =N-$OCH_3$); 3,95 (d, J = 6, 2H, >N-$CH_2$-); 4,85 (t, J = 6, 1H, -CH<$^{O-}_{O-}$); 5,27 (d, J = 4, 1H, -H en 6); 5,79 (dd, J = 4 et 9, 1H, -H en 7); 6,75 (s, 1H, -H thiazole); 6,96 (s, 1H, -COO-C<u>H</u>($C_6H_5$)$_2$); 6,98 et 7,05 (2d, J = 16, 2H, -CH=CH-S-); 8,82 (s, 1H, -N<u>H</u>-C($C_6H_5$)$_3$); 9,63 (d, J = 9, 1H, -CO-NH-).

Une solution de 1,9 g de produit (9c) dans 20 $cm^3$ d'acide formique est agitée pendant 30 minutes à 50°C puis diluée par 2 $cm^3$ d'eau et agitée pendant 10 minutes à 50°C. Après refroidissement à 20°C le mélange réactionnel est filtré et le filtrat concentré sous pression réduite (0,1 mm de mercure; 0,013 kPa) à 35°C. Le résidu est trituré avec 20 $cm^3$ d'éthanol que l'on évapore sous pression réduite (30 mm de mercure; 4 kPa) à 40°C. Cette opération est répétée 3 fois puis le résidu est repris par 30 $cm^3$ d'éthanol. Le solide est isolé sur filtre à vide et lavé par 3 fois 10 $cm^3$ d'éthanol et par 3 fois 10 $cm^3$ d'éther éthylique et séché. On obtient 0,96 g d'[(amino-2 thiazolyl-4)-2 méthoxyimino-2 acétamido]-7 carboxy-2 [(dioxo-5,6 formylméthyl-1 méthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (produit 9) sous la forme d'un solide de couleur crème.

Spectre infra-rouge (KBr), bandes caractéristiques ($cm^{-1}$) 3700-2200, 1770, 1710, 1675, 1580, 1550, 1040.

Spectre de RMN du proton (350 MHz, $CF_3COOD$, $\delta$ en ppm, J en Hz) 3,66 (s, 3H, >N-$CH_3$); 3,85 (s large, 2H, -S-$CH_2$-); 4,31 (s, 3H, =N-O-$CH_3$); 5,13 (s large, 2H, >N-$CH_2$-); 5,38 (d, J = 4, 1H, -H en 6); 6,02 (d, J = 4, 1H, -H en 7); 7,22 et 7,73 (2d, J = 16, 2H, -CH=CH-S-); 7,48 (s, 1H, -H thiazole); 9,74 (s, 1H, -C(=O)H).

La (diéthoxy-2,2 éthyl)-1 dioxo-5,6 méthyl-4 thioxo-3 perhydrotriazine-1,2,4 peut être préparée de la manière suivante:

8,8 g de (diéthoxy-2,2 éthyl)-1 éthoxalyl-1 méthyl-4 thiosemicarbazide sont traités par 3,08 g de t.butylate de potassium dans 60 $cm^3$ de t.butanol sec pendant 2 heures à 25°C. Le mélange réactionnel est dilué par 50 $cm^3$ d'éther. Le précipité de sel de potassium du produit attendu est isolé sur filtre, lavé par 10 $cm^3$ d'éther puis repris par 30 $cm^3$ d'eau. La solution obtenue est acidifiée à pH 3 par de l'acide chlorhydrique 4N. Le précipité est isolé sur filtre, lavé par 10 $cm^3$ d'eau et 10 $cm^3$ d'éther éthylique, puis séché sous pression réduite (2 mm de mercure; 0,27 kPa) à 20°C. On obtient 3,65 g de (diéthoxy-2,2 éthyl)-1 dioxo-5,6 méthyl-4 thioxo-3 perhydrotriazine-1,2,4 sous la forme de cristaux blancs F = 136-138°C.

La (diéthoxy-2,2 éthyl)-1 éthoxalyl-1 méthyl-4 thiosemicarbazide peut être obtenue de la manière suivante:

19,4 g de (diéthoxy-2,2 éthyl)-1 méthyl-4 thiosemicarbazide dans 200 $cm^3$ de dichlorométhane sec sont traités par 7,1 $cm^3$ de pyridine et 9,85 $cm^3$ de chlorure d'éthoxalyle pendant 1 heure à 2°C puis 16 heures à 25°C; après lavage par 100 $cm^3$ d'eau, séchage sur sulfate de magnésium et concentration à sec sous pression réduite (30 mm de mercure; 4 kPa) à 30°C, le résidu est cristallisé dans 40 $cm^3$ d'éther éthylique. On obtient 8,5 g de (diéthoxy-2,2 éthyl)-1 éthoxalyl-1 méthyl-4 thiosemicarbazide sous la forme de cristaux blancs (F = 122°C).

La (diéthoxy-2,2 éthyl)-1 méthyl-4 thiosemicarbazide peut être préparée de la manière suivante:

39,3 g de (diéthoxy-2,2 acétyl)-1 méthyl-4 thiosemicarbazide sont réduits par 18 g d'hydrure de lithium et d'aluminium dans 500 $cm^3$ de tétrahydrofuranne sec pendant 1 heure 30 à 25°C puis 1 heure au reflux. On obtient 26,4 g de (diéthoxy-2,2 éthyl)-1 méthyl-4 thiosemicarbazide sous la forme d'une huile incolore.

Spectre infra-rouge ($CCl_4$), bandes caractéristiques ($cm^{-1}$) 3360, 3200, 1550, 1240, 1130, 1060.

Spectre de masse m/e = 221, 175, 146.

La (diéthoxy-2,2 acétyl)-1 méthyl-4 thiosemicarbazide peut être préparée de la manière suivante:

31,5 g d'hydrazide de l'acide diéthoxyacétique sont traités dans 200 $cm^3$ d'éther éthylique par 14,6 g d'isothiocyanate de méthyle pendant 20 heures entre 20 et 30°C. La (diéthoxy-2,2 acétyl)-1 méthyl-4 thiosemicarbazide cristallise dans le milieu. (Poids: 42 g; F = 116-118°C).

*Exemple 10*

En opérant de façon analogue à l'exemple 9 on obtient l'[(amino-2 thiazolyl-4)-2 méthoxyimino-2 acétamido]-7 carboxy-2 {[dioxo-5,6 formylméthyl-4 méthyl-1 tétrahydro-1,4,5,6 triazine-1,2,4 yl-3] thio-2 vinyl}-3 oxo-8 thia-5 aza-1 bicyclo[4.2.0] octène-2, isomère syn, forme E sous la forme d'une poudre jaune (produit 10).

Spectre infra-rouge (KBr), bandes caractéristiques ($cm^{-1}$) 3600 à 2300, 1770, 1710, 1670, 1575, 1040, 945.

Spectre de RMN du proton (350 MHz, $CF_3COOD$, $\delta$ en ppm, J en Hz) 3,82 (s large, 2H, -$SCH_2$-); 3,88 (s, 3H, >N-$CH_3$); 4,32 (s, 3H, -$OCH_3$); 5,18 (s, 2H, >$NCH_2$); 5,40 (d, J = 4, 1H, H en 6); 6,04 (d, J = 4, 1H, H en 7); 7,24 et 7,73 (2d, J = 16, 2H, -CH=CH-S-); 7,48 (s, 1H, H hétérocycle); 9,73 (s, 1H, -CHO).

*Exemple 11*

On agite pendant 10 minutes à 20°C un mélange de 0,14 g de méthanesulfonate d'amino-7 benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo[4.2.0] octène-2, forme E (produit 11a), 30 $cm^3$ de dichlorométhane et 10 $cm^3$ d'une solution demi-saturée de bicarbonate de sodium, on décante, lave la phase organique par 15 $cm^3$ d'une solution saturée de chlorure de sodium, sèche sur sulfate de sodium et filtre. La solution filtrée est additionnée de 0,156 g d'acide t.butoxycarbonylméthoxyimino-2 (tritylamino-2 thiazolyl-4)-2 acétique, isomère syn (préparé selon le brevet belge 865 298) et de 5 mg de diméthylamino-4 pyridine. On refroidit à 5°C, ajoute goutte à goutte en 3 minutes une solution de 50 mg de N,N'-dicyclohexylcarbodiimide dans 1 $cm^3$ de dichlorométhane et laisse remonter la température à 20°C en 3 heures. On lave le mélange par 20 $cm^3$ d'une solution à 2% de bicarbonate de sodium et 20 $cm^3$ d'une solution demi-saturée de chlorure de sodium, sèche sur sulfate de sodium, filtre et concentre à sec à 20°C sous 20 mm de mercure (2,7 kPa). On chromatographie le résidu sur une colonne de gel de silice Merck (0,04-0,06) (diamètre de la colonne: 1,8 cm, hauteur: 9 cm); on élue par 200 $cm^3$ d'un mélange cyclohexane-acétate d'éthyle 20-80 (en volumes) sous une pression de 40 kPa en recueillant des fractions de 10 $cm^3$. On concentre à sec les fractions 6 à 14 à 20°C sous 20 mm de mercure (2,7 kPa) et recueille 50 mg de benzhydryloxycarbonyl-2 [t.butoxycarbonylméthoxyimino-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo[4.2.0] octène-2, isomère syn, forme E (produit 11b) dont les caractéristiques infra-rouge et RMN sont identiques à celles du produit (1b) décrit à l'exemple 1.

En opérant comme décrit à l'exemple 1 on obtient le benzhydryloxycarbonyl-2 [t.butoxycarbonylméthoxyimino-2 (tritylamino-2 thiazolyl-4)-2 acétamido]-7 {[(diméthoxy-2,2 éthyl)-4 dioxo-5,6 tétrahydro-1,4,5,6 triazine-1,2,4 yl-3] thio-2 vinyl}-3 oxo-8 thia-5 aza-1 bicyclo[4.2.0] octène-2, isomère syn,

forme E (produit 11c), puis le carboxy-2 [(amino-2 thiazolyl-4)-2 carboxyméthoxyimino-2 acétamido]-7 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (produit 11) dont les caractéristiques sont identiques à celles du produit (1) décrit précédemment à l'exemple 1.

Le produit (11a) peut être préparé de la manière suivante:

On ajoute 5 g de benzhydryloxycarbonyl-2 t.butoxycarbonylamino-7 {[(diméthoxy-2,2 éthyl)-4 dioxo-5,6 tétrahydro-1,4,5,6 triazine-1,2,4 yl-3] thio-2 vinyl}-3 oxo-8 oxyde-5 thia-5 aza-1 bicyclo-[4.2.0] octène-2, isomère E (produit 11d) à 80 $cm^3$ d'un mélange acétonitrile-méthanol (85-15 en volumes) puis ajoute 6 g d'acide méthanesulfonique. Le mélange est devenu limpide. On laisse sous agitation pendant 5 heures à 20°C et on filtre le précipité blanc formé. Le précipité est lavé par 2 fois 10 $cm^3$ d'acétonitrile, par 2 fois 25 $cm^3$ d'acétate d'éthyle puis par 2 fois 25 $cm^3$ d'éther éthylique. Après séchage sous vide (0,5 mm de mercure; 0,07 kPa) on obtient 4,4 g de méthanesulfonate du produit (11a) sous forme de cristaux blancs.

Spectre infra-rouge (KBr), bandes caractéristiques ($cm^{-1}$) 3300, 2200, 1800, 1720, 1590, 1495, 1455, 1220, 1125, 1085, 1080, 1045, 945, 760, 710, 620, 600, 560.

Spectre de RMN du proton (80 MHz, DMSO $d_6$, $\delta$ en ppm, J en Hz) 2,48 (s, 3H, $\underline{CH_3}SO_3H$); 3,38 (s, 6H, $(-OCH_3)_2$); 3,74 et 4,45 (2d, J = 18, 2H, $-SCH_2-$); 3,98 (d, J = 5, 2H, $>NCH_2-$); 4,58 (t, J = 5, 1H, $-\underline{CH}(OCH_3)_2$); 5,06 (d, J = 4, 1H, H en 6); 5,38 (d, J = 4, 1H, H en 7); 7,0 (s, 1H, $-COO\underline{CH}<$).

Le produit (11d) peut être obtenu selon l'une ou l'autre des méthodes suivantes:

A/ Une solution de 0,54 g de benzhydryloxycarbonyl-2 t.butoxycarbonylamino-7 (chloro-2 vinyl)-3 oxo-8 oxyde-5 thia-5 aza-1 bicyclo[4.2.0] octène-2, forme E (produit 11e), 0,25 g de (diméthoxy-2,2 éthyl)-4 dioxo-5,6 thioxo-3 perhydrotriazine-1,2,4 et 0,19 $cm^3$ de N,N-diisopropyléthylamine dans 8 $cm^3$ de N,N-diméthylformamide sec est portée à 60°C pendant 4 heures puis agitée pendant 16 heures à 25°C et diluée par 100 $cm^3$ de dichlorométhane. La solution est lavée successivement par 2 fois 50 $cm^3$ de solution demi-saturée de chlorure de sodium, par 2 fois 50 $cm^3$ de solution demi-saturée de bicarbonate de sodium et 2 fois 50 $cm^3$ d'eau distillée. Après séchage de la couche organique sur sulfate de sodium et concentration à sec sous pression réduite (30 mm de mercure; 4 kPa) à 35°C, le résidu est chromatographié sur une colonne de gel de silice (0,04-0,06) (diamètre de la colonne: 2 cm, hauteur: 30 cm) en éluant sous 50 kPa par de l'acétate d'éthyle et recueillant des fractions de 30 $cm^3$. Les fractions 12 à 16 sont rassemblées et concentrées à sec sous pression réduite (30 mm de mercure; 4 kPa) à 35°C. On obtient 0,4 g de produit (11d) sous la forme d'un solide orangé amorphe. Ce produit est cristallisé dans 20 $cm^3$ de mélange d'oxyde d'isopropyle et d'acétonitrile (75-25 en volumes). On obtient 0,15 g du produit cristallisé dont les caractéristiques sont les suivantes:

Spectre infra-rouge (KBr), bandes caractéristiques ($cm^{-1}$) 3410, 1790, 1715, 1580, 1490, 1450, 1155, 1115, 1075, 1040, 935, 750, 740, 695.

Spectre de RMN du proton (80 MHz, DMSO $d_6$, $\delta$ en ppm, J en Hz) 1,45 (s, 9H, $(CH_3)_3C-$); 3,62 et 4,42 (2d, J = 18,5, 2H, $-S(O)CH_2-$); 3,33 (s, 6H, $-CH(O\underline{CH_3})_2$); 3,97 (d, J = 5, 2H, $-\underline{CH_2}CH(OCH_3)_2$); 4,55 (t, J = 5, 1H, $-\underline{CH}(OCH_3)_2$); 5,04 (d, J = 4, 1H, H en 6); 5,80 (dd, J = 4 et 9, 1H, H en 7); 6,40 (d, J = 9, $-CON\underline{H}-C_7$); 6,98 (s, 1H, $-\underline{CH}(C_6H_5)_2$); 7,08 (AB limite, 2H, -CH=CH-S-); 7,2 à 7,50 (m, 10H, aromatiques); 12,68 (s, 1H, -N=COH).

Le produit (11e) peut être préparé de la manière suivante:

On agite à 25°C pendant 16 heures une solution de 3,3 g de benzhydryloxycarbonyl-2 t.butoxycarbonylamino-7 (dichloro-2,2 éthyl)-3 oxo-8 oxyde-5 thia-5 aza-1 bicyclo[4.2.0] octène-2 (produit 11f) et de 0,88 $cm^3$ de triéthylamine dans 50 $cm^3$ de tétrahydrofuranne sec puis dilue le mélange réactionnel par 200 $cm^3$ d'acétate d'éthyle. La solution organique est lavée par 5 fois 60 $cm^3$ d'eau distillée, séchée sur sulfate de sodium et concentrée à sec sous pression réduite (30 mm de mercure; 4 kPa) à 35°C. On obtient 3,1 g de produit (11e) sous la forme d'une meringue orangée.

Spectre infra-rouge ($CHBr_3$), bandes caractéristiques ($cm^{-1}$) 3400, 1800, 1715, 1590, 1570, 1500, 1450, 1390, 1365, 1040, 960, 755.

Spectre de RMN du proton (350 MHz, $CDCl_3$, $\delta$ en ppm, J en Hz) 1,48 (s, 9H, $(CH_3)_3C-$); 3,20 et 3,94 (2d, J = 18, 2H, $-S(O)CH_2-$); 4,53 (d, J = 4, 1H, H en 6); 5,78 (d, J = 9, 1H, -CONH-); 5,86 (dd, J = 4 et 9, 1H, H en 7); 6,45 (d, J = 14, 1H, $-\underline{CH}=CHCl$); 7,03 (s, 1H, $-CO_2CH(C_6H_5)_2$); 7,49 (d, J = 14, 1H, $-CH=\underline{CH}Cl$); 7,20 à 7,60 (m, 10H, aromatiques).

B/ Une solution de 1,09 g de produit (11f), 0,49 g de (diméthoxy-2,2 éthyl)-4 dioxo-5,6 thioxo-3 perhydrotriazine-1,2,4 et 0,77 $cm^3$ de N,N-diisopropyléthylamine dans 20 $cm^3$ de N,N-diméthylformamide est agitée pendant 16 heures à 25°C et 4 heures à 60°C puis refroidie et diluée par 200 $cm^3$ de dichlorométhane. La solution obtenue est lavée successivement par 2 fois 100 $cm^3$ de solution saturée de chlorure de sodium, 2 fois 100 $cm^3$ de solution saturée de bicarbonate de sodium et 2 fois 100 $cm^3$ d'eau distillée puis séchée sur sulfate de sodium et concentrée à sec sous pression réduite; le résidu est chromatographié sur une colonne de gel de silice (0,04-0,06) (diamètre de la colonne: 3 cm, hauteur: 30 cm) en éluant sous une pression de 50 kPa par de l'acétate d'éthyle et recueillant des fractions de 50 $cm^3$ environ. Les fractions 7 à 15 contenant le produit pur sont rassemblées et évaporées à sec. On obtient 0,7 g de produit (11d) dont les caractéristiques sont identiques à celle du produit décrit en A/.

On prépare une solution de composé d'addition du chlore et du triphénylphosphite en ajoutant, en 15 minutes, à 4 $cm^3$ d'une solution à 10% (poids/volume) de chlore dans du dichlorométhane refroidie à —5°C, une solution de 1,55 g de triphénylphosphite

dans 5 $cm^3$ de dichlorométhane. Cette solution est ajoutée en 90 minutes à —10°C à une solution de 2,4 g de benzhydryloxycarbonyl-2 t.butoxycarbonylamino-7 oxo-8 (oxo-2 éthyl)-3 thia-5 aza-1 bicyclo[4.2.0] octène-2 (produit 11g) et de 0,4 $cm^3$ de pyridine dans 15 $cm^3$ de dichlorométhane. Le mélange réactionnel est lavé par 20 $cm^3$ d'eau distillée, 20 $cm^3$ de solution saturée de bicarbonate de sodium et par 2 fois 20 $cm^3$ d'eau distillée puis séchée sur sulfate de magnésium et concentrée à sec sous pression réduite (30 mm de mercure; 4 kPa) à 30°C. On obtient 4,6 g d'un mélange équimoléculaire de benzhydryloxycarbonyl-2 t.butoxycarbonylamino-7 (dichloro-2,2 éthyl)-3 oxo-8 thia-5 aza-1 bicyclo-[4.2.0] octène-2 (produit 11h) et de phosphate de triphényle. Ce mélange peut être utilisé sans purification supplémentaire pour la suite de la synthèse en le redissolvant dans 15 $cm^3$ de dichlorométhane sec et en ajoutant en 20 minutes à la solution refroidie à —10°C une solution de 1,22 g d'acide métachloroperbenzoïque à 85% dans 30 $cm^3$ de dichlorométhane. L'insoluble est éliminé par filtration et le filtrat est lavé par 2 fois 25 $cm^3$ de solution saturée de bicarbonate de sodium et 2 fois 25 $cm^3$ de solution saturée de chlorure de sodium puis séché sur sulfate de magnésium. Le résidu obtenu après évaporation du solvant sous pression réduite (30 mm de mercure; 4 kPa) à 35°C est chromatographié sur une colonne de silice (0,04-0,06) (diamètre de la colonne: 2 cm, hauteur: 30 cm) en éluant sous 50 kPa avec un mélange de cyclohexane et d'acétate d'éthyle (60-40 en volumes) et en recueillant des fractions de 30 $cm^3$. Les fractions 6 à 12 sont rassemblées et concentrées à sec sous pression réduite (30 mm de mercure; 4 kPa) à 40°C. On obtient 1,3 g d'une meringue jaune constituée principalement de produit (11f).

Spectre de masse: pic moléculaire m/e = 578.

Spectre de RMN du proton (350 MHz, $CDCl_3$, δ en ppm, J en Hz) 1,48 (s, 9H, $(CH_3)_3C$-); 3,10 (dd, J = 5 et 14, 1H, -C$\underline{H}_2$-$CHCl_2$); 3,66 (dd, J = 7 et 14, 1H, 2ème H du -C$\underline{H}_2$-$CHCl_2$); 3,41 et 3,96 (2d, J = 18, 2H, -S(O)C$\underline{H}_2$-); 4,53 (d, J = 4, 1H, H en 6); 5,78 (d, J = 9, 1H, -CONH-); 5,83 (dd, J = 4 et 9, 1H, H en 7); 5,87 (dd, J = 5 et 7, 1H, -$CH_2$C$\underline{H}Cl_2$); 6,98 (s, 1H, -C$\underline{H}Ar_2$); 7,2 à 7,5 (m, 10H, aromatiques).

On obtient le produit (11h) purifié en chromatographiant 4,6 g de mélange équimoléculaire de ce produit avec le phosphate de triphényle, tel qu'obtenu ci-dessus, sur une colonne de 30 g de gel de silice (0,2-0,06) en éluant par du dichlorométhane et en recueillant des fractions de 10 $cm^3$. Les fractions 2 à 11 sont réunies et concentrées à sec sous pression réduite (30 mm de mercure; 4 kPa) à 30°C. Le résidu obtenu est soumis à une nouvelle chromatographie sur une colonne de silice (0,04-0,06) (diamètre de la colonne: 2 cm, hauteur: 30 cm) en éluant sous 50 kPa par un mélange de cyclohexane et d'acétate d'éthyle (80-20 en volumes) et recueillant des fractions de 10 $cm^3$. Les fractions 7 et 8 contenant le produit pur sont concentrées à sec sous pression réduite (30 mm de mercure; 4 kPa) à 35°C et le produit séché sous pression réduite (0,2 mm de mercure; 0,027 kPa) à 25°C.

Spectre de masse: pic moléculaire m/e = 562.

Spectre de RMN du proton (350 MHz, $CDCl_3$, δ en ppm, J en Hz) 1,46 (s, 9H, $(CH_3)_3C$-); 3,20 et 3,28 (2dd, J = 9 et 14, 2H, -C$\underline{H}_2$-$CHCl_2$); 3,65 (AB limite, J = 18, 2H, -$SCH_2$-); 5,0 (d, J = 4, 1H, H en 6); 5,25 (d, J = 9, 1H, -CONH-); 5,67 (dd, J = 4 et 9, 1H, H en 7); 5,98 (dd, J = 9 et 4, 1H, -$CH_2$-C$\underline{H}Cl_2$); 6,95 (s, 1H, -$CO_2CH(C_6H_5)_2$); 7,20 à 7,50 (m, 10H, aromatiques).

Le produit (11g) peut être obtenu de la façon suivante:

Une solution de 1,07 g de benzhydryloxycarbonyl-2 t.butoxycarbonylamino-7 (diméthylamino-2 vinyl)-3 oxo-8 thia-5 aza-1 bicyclo[4.2.0] octène-2, forme E (produit 11i) dans 10 $cm^3$ d'acétate d'éthyle est agitée pendant 1 heure à 25°C avec 5 $cm^3$ de solution aqueuse 1N d'acide chlorhydrique. La phase organique est décantée, lavée 4 fois par 50 $cm^3$ de solution aqueuse saturée de chlorure de sodium puis séchée sur sulfate de magnésium et filtrée. L'évaporation à sec du solvant sous pression réduite donne 1 g d'un produit dont le spectre IR montre qu'il s'agit principalement de produit (11g).

Rf = 0,57 [chromatoplaque de gel de silice; éluant: cyclohexane-acétate d'éthyle 60-40 (en volumes)].

Spectre infra-rouge (solution $CHBr_3$), bandes caractéristiques ($cm^{-1}$) 2840, 1785, 1720.

Spectre de RMN du proton (350 MHz, $CDCl_3$, δ en ppm, J en Hz) 1,47 (s, 9H, $(CH_3)_3C$-O-); 3,24 et 3,55 (AB, J = 18, 2H, -$SCH_2$-); 3,50 et 3,66 (AB, J = 16, 2H, -C$\underline{H}_2$CHO); 4,98 (d, J = 4,5, 1H, H en 6); 5,25 (d, J = 9, 1H, -CONH-); 5,65 (dd, J = 4,5 et 9, 1H, H en 7); 6,87 (s, 1H, -$CO_2$C$\underline{H}$<); 7,2 à 7,5 (massif, 10H, aromatiques); 9,54 (s, 1H, -CHO).

Le produit (11i) peut être obtenu en opérant de la manière suivante:

Une solution de 1,0 g de benzhydryloxycarbonyl-2 t.butoxycarbonylamino-7 méthyl-3 oxo-8 thia-5 aza-1 bicyclo[4.2.0] octène-3 (produit 11j) dans 100 $cm^3$ de N,N-diméthylformamide anhydre est chauffée à 80°C en atmosphère d'azote. On ajoute alors rapidement 0,86 $cm^3$ de bisdiméthylamino t.butoxyméthane. Le mélange réactionnel est maintenu à 80°C pendant 5 minutes puis versé dans 50 $cm^3$ d'acétate d'éthyle. Après addition de 25 $cm^3$ d'eau distillée, la phase organique est décantée, lavée par 4 fois 25 $cm^3$ d'eau distillée, séchée sur sulfate de magnésium et filtrée. On concentre à sec sous pression réduite (20 mm de mercure; 2,7 kPa) à 30°C et obtient 1,10 g d'un produit constitué principalement de produit (11i) sous la forme d'une meringue orangée.

Rf = 0,29; chromatoplaque de silicagel [cyclohexane-acétate d'éthyle 50-50 (en volumes)].

Spectre infra-rouge ($CHBr_3$), bandes caractéristiques ($cm^{-1}$) 3430, 3350, 2820, 1765, 1715, 1690, 1615, 1540, 1505, 1495, 1465, 1370, 1240, 940, 745, 600.

Spectre UV visible — Ethanol.

λ max = 390 nm ε = 29000 (c = $2.10^{-5}$M).

Spectre de masse: pic moléculaire = 535 fragments caractéristiques m/e = 378 et 379 (coupure du β-lactame).

Spectre de RMN du proton (350 MHz, $CDCl_3$, δ en ppm, J en Hz) 1,48 (s, $(CH_3)_3C$-OCO-, 9H); 2,89 (s,

6H, $(CH_3)_2N$-); 3,17 (AB, J = 14, 2H, -S-$CH_2$- céphème); 5,02 (d, J = 4, 1H, H en 6); 5,27 (dd, J = 4 et 9, 1H, H en 7); 5,60 (d, J = 9, 1H, -OCONH-); 6,71 (d, J = 14, 1H, -CH=CH-N<); 6,49 (d, J = 14, 1H, -CH=CH-N<); 6,95 (s, 1H, -C<u>H</u>$(C_6H_5)_2$); 7,2 à 7,5 (massif, aromatiques, 10H).

Le produit (11j) peut être préparé par estérification de 3,2 g de t.butoxycarbonylamino-7 carboxy-2 méthyl-3 oxo-8 thia-5 aza-1 bicyclo[4.2.0] octène-3, (produit 11k), par 2,1 g de diphényldiazométhane entre 25 et 30 °C pendant 16 heures. Après recristallisation dans un mélange cyclohexane-acétate d'éthyle 90-10 (en volumes) on obtient 2,3 g de produit (11j) sous la forme de cristaux blancs (F = 161 °C).

Le produit (11k) peut être préparé par conversion de 8,28 g du t.butoxycarbonylamino-7 méthoxycarbonyl-2 méthyl-3 oxo-8 thia-5 aza-1 bicyclo-[4.2.0] octène-2 (produit 11 1) en utilisant la méthode de R.B. MORIN et coll., J. Amer. Chem. Soc., *91*(6), 1401 (1969). On obtient 5,4 g de produit (11k).

F = 200°C (déc.) (après recristallisation dans l'acétate d'éthyle).

Rf = 0,59 [chromatoplaque de gel de silice; éluant: mélange acétate d'éthyle-acétone-eau-acide formique 60-20-1-1 (en volumes)].

Le produit (11 1) peut être préparé en estérifiant 16,7 g de t.butoxycarbonylamino-7 carboxy-2 méthyl-3 oxo-8 thia-5 aza-1 bicyclo[4.2.0] octène-2 (produit 11m) par une solution éthérée de diazométhane selon R.B. MORIN et coll., J. Amer. Chem. Soc., *91*(6), 1401 (1969). On obtient 13,6 g de produit (11 1) sous la forme de cristaux blancs (F = 148°C).

Rf = 0,45 [chromatoplaque de gel de silice; éluant: cyclohexane-acétate d'éthyle 60-40 (en volumes)].

Le produit (11 m) peut être préparé de la manière suivante:

371 g d'amino-7 carboxy-2 méthyl-3 oxo-8 thia-5 aza-1 bicyclo[4.2.0] octène-2 sont dissous dans une solution de 307 g de bicarbonate de sodium dans un mélange de 2 litres d'eau distillée et 2 litres de dioxanne. On ajoute en 10 minutes une solution de 421 g de pyrocarbonate de di t.butyle dans 2 litres de dioxanne. Le mélange réactionnel est agité pendant 48 heures à 25°C. La suspension obtenue est concentrée sous pression réduite (20 mm de mercure; 2,7 kPa) à 50 °C jusqu'à volume résiduel d'environ 2 litres, puis diluée par 1 litre d'acétate d'éthyle et 2 litres d'eau distillée. La phase aqueuse est décantée, lavée par 500 $cm^3$ d'acétate d'éthyle et acidifiée à pH = 2 par de l'acide chlorhydrique 6N en présence de 1500 $cm^3$ d'acétate d'éthyle. La phase aqueuse est extraite 2 fois par 1 litre d'acétate d'éthyle. Les phases organiques rassemblées sont lavées par 2 fois 250 $cm^3$ de solution saturée de chlorure de sodium et séchées sur sulfate de sodium. Après filtration, le solvant est évaporé sous pression réduite (20 mm de mercure; 2,7 kPa) à 50 °C. On obtient 486 g de t.butoxycarbonylamino-7 carboxy-2 méthyl-3 oxo-8 thia-5 aza-1 bicyclo[4.2.0] octène-2 sous la forme de cristaux jaunes.

(F = 190°C, décomposition).

*Exemple 12*

Une solution de 0,9 g de benzhydryloxycarbonyl-2 (chloro-2 vinyl)-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme E (produit 12a), 0,23 g de (diéthoxy-2,2 éthyl)-1 dioxo-5,6 méthyl-4 thioxo-3 perhydrotriazine-1,2,4 et 0,19 $cm^3$ de N,N-diisopropyléthylamine dans 10 $cm^3$ de N,N-diméthylformamide est agitée pendant 16 heures à 25 °C puis 4 heures à 60 °C, puis refroidie et diluée par 100 $cm^3$ de dichlorométhane. La solution obtenue est lavée successivement par 2 fois 50 $cm^3$ de solution saturée de chlorure de sodium, 2 fois 50 $cm^3$ de solution saturée de bicarbonate de sodium et 2 fois 50 $cm^3$ d'eau distillée puis séchée sur sulfate de sodium et concentrée à sec sous pression réduite (30 mm de mercure; 4 kPa) à 30°C. Le résidu est chromatographié sur une colonne de gel de silice (0,04-0,06) (diamètre de la colonne: 3 cm, hauteur: 30 cm) en éluant sous une pression de 50 kPa par 2,5 litres d'un mélange de cyclohexane et d'acétate d'éthyle (25-75 en volumes) en recueillant des fractions de 50 $cm^3$. Les fractions 19 à 42 contenant le produit pur sont réunies et concentrées à sec. On obtient 0,8 g de benzhydryloxycarbonyl-2 {[(diéthoxy-2,2 éthyl)-1 dioxo-5,6 méthyl-4 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxyde-5 thia-5 aza-1 bicyclo-[4.2.0] octène-2, isomère syn, forme E (produit 12b) dont les caractéristiques sont identiques à celles du produit (9b) obtenu dans l'exemple 9.

En opérant comme décrit à l'exemple 9 et après réduction et élimination des radicaux protecteurs on obtient l'[(amino-2 thiazolyl-4)-2 méthoxyimino-2 acétamido]-7 carboxy-2 [(dioxo-5,6 formylméthyl-1 méthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (produit 12) dont les caractéristiques sont décrites à l'exemple 9, produit (9).

Le produit (12a) peut être obtenu de la manière suivante:

A une solution de 3,4 g de benzhydryloxycarbonyl-2 t.butoxycarbonylamino-7 (chloro-2 vinyl)-3 oxo-8 oxyde-5 thia-5 aza-1 bicyclo[4.2.0] octène-2, forme E (produit 12c) dans 15 $cm^3$ d'acétonitrile à 40°C on ajoute en 15 minutes une solution de 2,2 g d'acide paratoluènesulfonique (hydrate) dans 15 $cm^3$ d'acétonitrile. Après 30 minutes à 40°C le mélange réactionnel est concentré à sec sous pression réduite (30 mm de mercure; 4 kPa) à 30 °C. Le résidu est repris dans 100 $cm^3$ d'acétate d'éthyle et la solution obtenue est lavée par 2 fois 50 $cm^3$ de solution saturée de bicarbonate de sodium et 2 fois 50 $cm^3$ d'eau distillée puis séchée sur sulfate de sodium et concentrée à sec sous pression réduite (30 mm de mercure; 4 kPa) à 35 °C. Le résidu est séché sous pression réduite (0,2 mm de mercure; 0,03 kPa) à 25 °C pendant 1 heure. On obtient 2,5 g d'amino-7 benzhydryloxycarbonyl-2 (chloro-2 vinyl)-3 oxo-8 oxyde-5 thia-5 aza-1 bicyclo[4.2.0] octène-2, forme E (produit 12d) brut sous la forme d'une meringue orangée. Ce produit est redissous dans 45 $cm^3$ de dichlorométhane sec et on ajoute une solution de 2,5

g d'acide méthoxyimino-2 (tritylamino-2 thiazolyl-4)-2 acétique (isomère syn) et de 0,03 g de diméthylamino-4 pyridine dans 30 $cm^3$ de dichlorométhane sec puis (après refroidissement vers 4°C) 1,3 g de N,N'-dicyclohexylcarbodiimide et 10 $cm^3$ de dichlorométhane sec. Le mélange réactionnel est agité pendant 40 minutes vers 6°C puis 16 heures à 25°C. On filtre le précipité de N,N'-dicyclohexylurée, que l'on rince par 2 fois 5 $cm^3$ de dichlorométhane sec. Le filtrat et les lavages rassemblés sont concentrés à sec sous pression réduite (30 mm de mercure; 4 kPa) à 30°C et le résidu obtenu est repris dans 200 $cm^3$ d'acétate d'éthyle. La solution obtenue est lavée successivement par 50 $cm^3$ d'acide chlorhydrique 0,2N, 2 fois 50 $cm^3$ de solution demi-saturée de bicarbonate de sodium et 50 $cm^3$ de solution saturée de chlorure de sodium puis séchée sur sulfate de sodium. Le résidu obtenu après évaporation à sec sous pression réduite (30 mm de mercure; 4 kPa) à 30°C est chromatographié sur une colonne de silice (0,04-0,06) (diamètre de la colonne: 4 cm, hauteur: 30 cm) en éluant sous une pression de 50 kPa par 800 $cm^3$ de dichlorométhane puis par 1200 $cm^3$ de mélange de dichlorométhane et d'acétate d'éthyle (95-5 en volumes) en recueillant des fractions de 60 $cm^3$. Les fractions 12 à 25 contenant le produit pur sont rassemblées et concentrées à sec sous pression réduite. On obtient 1,7 g de produit (12a) sous la forme d'une meringue de couleur crème.

Spectre infra-rouge ($CHBr_3$), bandes caractéristiques ($cm^{-1}$) 3380, 2820, 1800, 1725, 1680, 1595, 1585, 1570, 1515, 1495, 1450, 1210, 1040, 930, 750.

Spectre de RMN du proton (350 MHz, $CDCl_3$, $\delta$ en ppm, J en Hz) 3,22 et 3,92 (2d, J = 18, 2H, $-S-CH_2-$); 4,12 (s, 3H, $=N-O-CH_3$); 4,63 (d, J = 5, 1H, -H en 6); 6,21 (dd, J = 5 et 9, 1H, -H en 7); 6,44 et 7,50 (2d, J = 14, 2H, -CH=CHCl); 6,74 (s, 1H, -H thiazole); 7,01 (s, 1H, $-COO-\underline{CH}(C_6H_5)_2$); 7,11 (mf, 1H, $-\underline{NH}-C(C_6H_5)_3$); 7,2 à 7,6 (mt, aromatiques); 7,54 (d, J = 9, -CO-NH-).

Le produit (12c) peut être obtenu comme décrit précédemment à l'exemple 11 (produit 11e).

*Exemple 13*

On ajoute à une solution de 0,09 g de benzhydryloxycarbonyl-2 (dichloro-2,2 éthyl)-3 [méthoxyimino-2 (tritylamino-2 thiazolyl-4)-2 acétamido]-7 oxo-8 oxyde-5 thia-5 aza-1 bicyclo[4.2.0] octène-2, isomère syn (produit 13a) et de 0,03 g de (diéthoxy-2,2 éthyl)-1 dioxo-5,6 méthyl-4 thioxo-3 perhydrotriazine-1,2,4 dans 2 $cm^3$ de N,N diméthylformamide sec 0,04 $cm^3$ de N,N-diisopropyléthylamine et agite le mélange réactionnel pendant 16 heures à 25°C puis 4 heures à 60°C. L'examen chromatographique met en évidence la formation du benzhydryloxycarbonyl-2 {[(diéthoxy-2,2 éthyl)-1 dioxo-5,6 méthyl-4 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme E (produit 13b).

Rf = 0,3 [chromatoplaque de gel de silice Merck; éluant: mélange de cyclohexane et d'acétate d'éthyle 20-80 (en volumes)].

En opérant comme à l'exemple 9 et après réduction et élimination des radicaux protecteurs, on obtient l'[(amino-2 thiazolyl-4)-2 méthoxyimino-2 acétamido]-7 carboxy-2 [(dioxo-5,6 formylméthyl-1 méthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (produit 13), identique au produit (9).

Le produit (13a) peut être obtenu de la manière suivante:

En opérant selon le mode opératoire décrit dans l'exemple 11B on traite une solution de 2,3 g de benzhydryloxycarbonyl-2 [méthoxyimino-2 (tritylamino-2 thiazolyl-4)-2 acétamido]-7 oxo-8 (oxo-2 éthyl)-3 thia-5 aza-1 bicyclo[4.2.0] octène-2, isomère syn (produit 13c) dans 15 $cm^3$ de dichlorométhane refroidie à —40°C par 5,5 $cm^3$ d'une solution 0,5 M dans le dichlorométhane du composé d'addition du chlore et du triphénylphosphite. Le mélange réactionnel est agité pendant 3 heures à une température comprise entre —40°C et —20°C puis dilué par 200 $cm^3$ d'acétate d'éthyle. La solution organique décantée est lavée par 50 $cm^3$ de solution demi-saturée en bicarbonate de sodium et en chlorure de sodium puis 3 fois 50 $cm^3$ de solution saturée de chlorure de sodium. Les phases aqueuses sont réextraites par 50 $cm^3$ d'acétate d'éthyle et les solutions organiques rassemblées sont concentrées à sec sous pression réduite (30 mm de mercure; 4 kPa) à 30°C. Le résidu est agité pendant 16 heures avec 150 $cm^3$ d'oxyde d'isopropyle. Le précipité est essoré et lavé par 50 $cm^3$ d'oxyde d'isopropyle puis redissous dans 50 $cm^3$ de dichlorométhane et fixé sur 8 g de gel de silice (0,2-0,06) que l'on dépose sur une colonne de gel de silice (0,06-0,04) (diamètre de la colonne: 1,8 cm; hauteur: 35 cm). On élue sous une pression de 80 kPa par un mélange de cyclohexane et d'acétate d'éthyle (75-25 en volumes) en recueillant des fractions de 50 $cm^3$. Les fractions 5 à 7 contenant le produit pur sont réunies et concentrées à sec sous pression réduite (30 mm de mercure; 4 kPa) à 30°C. On obtient 0,25 g de benzhydryloxycarbonyl-2 (dichloro-2,2 éthyl)-3 [méthoxyimino-2 (tritylamino-2 thiazolyl-4)-2 acétamido]-7 oxo-8 thia-5 aza-1 bicyclo[4.2.0] octène-2, isomère syn (produit 13d) sous la forme d'un solide blanchâtre.

Spectre de RMN du proton (350 MHz, $CDCl_3$, $\delta$ en ppm, J en Hz) 3,17 + 3,33 (2dd, J = 14 et 9 + J = 14 et 5, 2H, $-CH_2$ -exo); 3,62 (AB limite, J = 18, 2H, $-CH_2-S-$); 4,08 (s, 3H, $=N-O-CH_3$); 5,06 (d, J = 5, 1H, -H en 6); 5,94 à 6 (mt, 2H, -H en 7 et $-CHCl_2$); 6,76 (s, 1H, -H thiazole); 6,8 (d, J = 9, 1H, -CO-NH-); 6,92 (s, 1H, $-COO-\underline{CH}(C_6H_5)_2$); 7,04 (mf, 1H, $-\underline{NH}-C(C_6H_5)_3$); 7,15 à 7,5 (mt, aromatiques).

A une solution refroidie à —10°C de 0,2 g de produit (13d) dans 5 $cm^3$ de dichlorométhane, on ajoute d'un coup une solution de 0,05 g d'acide métachloroperbenzoïque (à 85%) dans 1 $cm^3$ de dichlorométhane. Après 30 minutes à —10°C le mélange réactionnel est dilué par 20 $cm^3$ d'acétate d'éthyle et lavé par 2 fois 10 $cm^3$ de solution saturée de bicarbonate de sodium et 2 fois 10 $cm^3$ de solu-

tion saturée de chlorure de sodium. Après séchage sur sulfate de sodium et concentration à sec sous pression réduite (30 mm de mercure; 4 kPa) à 30°C on obtient 0,15 g de produit (13a) brut sous la forme d'une meringue orangée.

Spectre de RMN du proton (350 MHz, $CDCl_3$, $\delta$ en ppm, J en Hz) 3,14 + 3,56 (2dd, J = 15 et 5 + J = 15 et 7, 2H, $-CH_2$-exo); 3,39 et 3,96 (2d, J = 18, 2H, $-CH_2-S\overset{\nearrow O}{}$); 4,09 (s, 3H, $=N-O-CH_3$); 4,58 (d, J = 5, 1H, -H en 6); 5,87 (dd, J = 5 et 7, 1H, $-CHCl_2$); 6,19 (dd, J = 5 et 9, 1H, -H en 7); 6,72 (s, 1H, -H thiazole); 6,94 (s, 1H, $-COO-\underline{CH}(C_6H_5)_2$); 7,08 (mf, 1H, $-\underline{NH}-C(C_6H_5)_3$); 7,10 à 7,60 (mt, aromatiques + -CO-NH).

*Exemple 14*

En traitant (comme dans l'exemple 1) 1,9 g de benzhydryloxycarbonyl-2 [t.butoxycarbonylméthoxyimino-2 (tritylamino-2 thiazolyl-4)-2 acétamido]-7 {[(diéthoxy-2,2 éthyl)-1 dioxo-5,6 méthyl-4 tétrahydro-1,4,5,6 triazine-1,2,4 yl-3] thio-2 vinyl}-3 oxo-8 thia-5 aza-1 bicyclo[4.2.0] octène-2, isomère syn, forme E successivement par 19 $cm^3$ d'acide trifluoroacétique et par un mélange de 19 $cm^3$ d'acide formique et 9,5 $cm^3$ d'eau, on obtient 0,75 g d'[(amino-2 thiazolyl-4)-2 carboxyméthoxyimino-2 acétamido]-7 carboxy-2 [(dioxo-5,6 méthyl-4 formylméthyl-1 tétrahydro-1,4,5,6 triazine-1,2,4 yl-3) thio-2 vinyl]-3 oxo-8 thia-5 aza-1 bicyclo[4.2.0] octène-2, isomère syn, forme E sous la forme d'une poudre jaune.

Spectre infra-rouge (KBr), bandes caractéristiques ($cm^{-1}$) 3600, 2200, 1775, 1705, 1675, 1580, 1405, 945.

Le benzhydryloxycarbonyl-2 [t.butoxycarbonylméthoxyimino-2 (tritylamino-2 thiazolyl-4)-2 acétamido]-7 {[(diéthoxy-2,2 éthyl)-1 dioxo-5,6 méthyl-4 tétrahydro-1,4,5,6 triazine-1,2,4 yl-3] thio-2 vinyl}-3 oxo-8 thia-5 aza-1 bicyclo[4.2.0] octène-2, isomère syn, forme E peut être obtenu de la manière suivante:

On réduit (comme dans l'exemple 1) 2,9 g de benzhydryloxycarbonyl-2 [t.butoxycarbonylméthoxyimino-2 (tritylamino-2 thiazolyl-4)-2 acétamido]-7 {[(diéthoxy-2,2 éthyl)-1 dioxo-5,6 méthyl-4 tétrahydro-1,4,5,6 triazine-1,2,4 yl-3] thio-2 vinyl}-3 oxo-8 oxyde-5 thia-5 aza-1 bicyclo[4.2.0] octène-2, isomère syn, forme E dans un mélange de 80 $cm^3$ de dichlorométhane et 0,889 $cm^3$ de diméthylacétamide par 0,428 $cm^3$ de trichlorure de phosphore. Après chromatographie sur une colonne de 60 g de gel de silice Merck (0,06-0,2) (diamètre de la colonne: 2 cm) en éluant par 600 $cm^3$ d'un mélange cyclohexane-acétate d'éthyle (35-65 en volumes) et en recueillant des fractions de 60 $cm^3$, on recueille dans les fractions 3 à 9 1,9 g de benzhydryloxycarbonyl-2 [t.butoxycarbonylméthoxyimino-2 (tritylamino-2 thiazolyl-4)-2 acétamido]-7 {[(diéthoxy-2,2 éthyl)-1 dioxo-5,6 méthyl-4 tétrahydro-1,4,5,6 triazine-1,2,4 yl-3] thio-2 vinyl}-3 oxo-8 thia-5 aza-1 bicyclo[4.2.0] octène-2, isomère syn, forme E sous la forme d'une meringue jaune.

Spectre infra-rouge ($CHBr_3$), bandes caractéristiques ($cm^{-1}$) 3380, 3260, 1770, 1720, 1680, 1585, 1525, 1490, 1450, 1095, 1065, 940, 750, 735.

Spectre de RMN du proton (350 MHz, $CDCl_3$, $\delta$ en ppm, J en Hz) 1,16 (t, J = 7, 6H, $-CH_3$); 1,42 (s, 9H, $-C(CH_3)_3$); 3,43 (s, 3H, $>N-CH_3$); 3,5 à 3,8 (mt, 6H, $(-O\underline{CH_2}CH_3)_2$ et $-SCH_2-$); 4,07 (d, J = 5, 2H, $>N-CH_2-$); 4,74 et 4,86 (2d, J = 16, 2H, $-OCH_2COO-$); 4,91 (t, J = 5, 1H, $-CH\langle{}^{O-}_{O-}$); 5,11 (d, J = 4,5 1H, H en 6); 5,92 (dd, J = 4,5 et 9, 1H, H en 7); 6,81 (d, J = 16, 1H, $-\underline{CH}=CHS-$); 6,83 (s, 1H, H du thiazole); 6,95 (s, 1H, $-COOCH-$); 7 (s large, 1H, $-NHC(C_6H_5)_3$); 8,75 (d, J = 9, 1H, -CONH-).

Le benzhydryloxycarbonyl-2 [t.butoxycarbonylméthoxyimino-2 (tritylamino-2 thiazolyl-4)-2 acétamido]-7 {[(diéthoxy-2,2 éthyl)-1 dioxo-5,6 méthyl-4 tétrahydro-1,4,5,6 triazine-1,2,4 yl-3] thio-2 vinyl}-3 oxo-8 oxyde-5 thia-5 aza-1 bicyclo[4.2.0] octène-2, isomère syn, forme E peut être obtenu de la manière suivante:

On traite (comme dans l'exemple 1) 3,3 g de benzhydryloxycarbonyl-2 [t.butoxycarbonylméthoxyimino-2 (tritylamino-2 thiazolyl-4)-2 acétamido]-7 oxo-8 oxyde-5 (tosyloxy-2 vinyl)-3 thia-5 aza-1 bicyclo[4.2.0] octène-2, isomère syn, forme E en solution dans 60 $cm^3$ de diméthylformamide par 0,864 g de (diéthoxy-2,2 éthyl)-1 dioxo-5,6 mercapto-3 méthyl-4 tétrahydro-1,4,5,6 triazine-1,2,4 en présence de 0,522 $cm^3$ de diisopropyléthylamine. Après chromatographie sur une colonne de 200 g de gel de silice Merck (0,06-0,2) (diamètre de la colonne: 2,2 cm) en éluant par 600 $cm^3$ d'un mélange cyclohexane-acétate d'éthyle (20-80 en volumes), on recueille dans les fractions 4 à 9 (chacune de 60 $cm^3$) 2,9 g de benzhydryloxycarbonyl-2 [t.butoxycarbonylméthoxyimino-2 (tritylamino-2 thiazolyl-4)-2 acétamido]-7 {[(diéthoxy-2,2 éthyl)-1 dioxo-5,6 méthyl-4 tétrahydro-1,4,5,6 triazine-1,2,4 yl-3] thio-2 vinyl}-3 oxo-8 oxyde-5 thia-5 aza-1 bicyclo-[4.2.0] octène-2, isomère syn, forme E sous la forme d'une meringue jaune.

Spectre infra-rouge ($CHBr_3$), bandes caractéristiques ($cm^{-1}$) 3380, 3240, 1805, 1720, 1680, 1580, 1520, 1490, 1445, 1390, 1370, 1060, 940, 750, 735.

Spectre de RMN du proton (350 MHz, $CDCl_3$, $\delta$ en ppm, J en Hz) [1,17 (t, J = 7) et 1,18 (t, J = 7): 6H, $-CH_3$]; 1,44 (s, 9H, $-C(CH_3)_3$); 3,22 et 4,02 (2d, J = 18, 2H, $-SCH_2-$); 3,43 (s, 1H, $>N-CH_3$); 3,53 (mt, 2H, $O\underline{CH_2}CH_3$); 3,72 (mt, 2H, $-O\underline{CH_2}CH_3$); 4,07 (mt, 2H, $>NCH_2-$); 4,64 (d, J = 4,5, 1H, H en 6); 3,72 et 3,79 (2d, J = 16, 2H, $=NOCH_2-$); 4,91 (t, J = 5, 1H, $-CH\langle{}^{O-}_{O-}$); 6,06 (dd, J = 4,5 et 9, 1H, H en 7); 6,78 (s, 1H, H du thiazole); 6,82 (d, J = 16, 1H, $-\underline{CH}=CHS-$); 6,97 (s, 1H, $-COOCH\langle$); 7,03 (s large, 1H, $-NHC(C_6H_5)_3$); 8,37 (d, J = 9, 1H, -CONH-).

La présente invention concerne également les médicaments qui contiennent comme produit actif au moins un produit de formule générale (I) à l'état pur (sous forme libre ou sous forme de sel) ou sous forme d'une composition en association avec un ou plusieurs adjuvants pharmaceutiquement acceptables. Ces médicaments peuvent être utilisés par voie orale, parentérale (notamment intra-musculaire ou intra-veineuse) ou rectale.

Comme compositions solides pour administration orale peuvent être utilisés des comprimés, des pilules, des poudres (généralement présentées dans des capsules, par exemple en gélatine) ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également préparées sous forme de compositions solides stériles qui seront dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou des glycérides semi-synthétiques.

En thérapeutique humaine, les médicaments selon la présente invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 1 et 10 g par jour de produit actif par voie intramusculaire pour un adulte.

L'exemple suivant, donné à titre non limitatif, illustre une composition selon la présente invention.

*Exemple*

On prépare 100 $cm^3$ d'une solution aqueuse isotonique contenant 2,63 g de bicarbonate de sodium et, comme produit actif, 10 g d'[(amino-2 thiazolyl-4)-2 carboxyméthoxyimino-2 acétamido]-7 carboxy-2 [(dioxo-5,6 formylméthyl-4 tétrahydro-1,4,5,6 triazine-1,2,4 yl-3) thio-2 vinyl]-3 oxo-8 thia-5 aza-1 bicyclo[4.2.0] octène-2, isomère syn, forme E (aldéhyde libre). Après filtration sur filtre bactériologique, on répartit aseptiquement cette solution en ampoules (à raison de 10 $cm^3$ par ampoule), on lyophilise et on scelle les ampoules.

Chaque ampoule contient l'équivalent de 1 g du produit actif, sous forme de son sel disodique, hydrate d'aldéhyde.

**Revendications pour les Etats contractants:**
BE, CH, DE, FR, GB, IT, LI, LU, NL, SE

1. Une thiovinyl-3 céphalosporine caractérisée en ce qu'elle répond à la formule générale

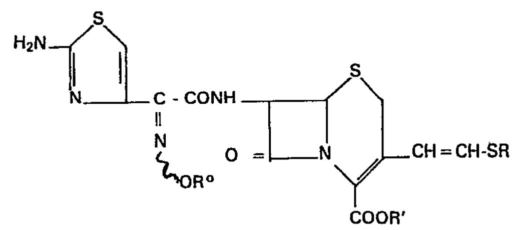

dans laquelle

$\alpha$/ le symbole R est choisi parmi les significations suivantes:

1) alcoyle, L-amino-2 carboxy-2 éthyle, phényle,

2) pyridyle-2, pyridyle-3 ou pyridyle-4 et leurs N-oxydes,

3) pyrimidinyle-2, pyridazinyle-3 substitué en position —6 (par un radical alcoyle, méthoxy, amino ou acylamino) et éventuellement N-oxydé ou tétrazolo [4,5-b] pyridazinyle-6,

4) dioxo-5,6 tétrahydro-1,4,5,6 triazine-1,2,4 yle-3 substitué en position —4; triazol-1,3,4 yle-5 ou alcoyloxycarbonyl-2 triazol-1,3,4 yle-5 substitués en position —1

a) par un radical alcoyle, non substitué ou substitué par un radical alcoyloxy, alcoylthio, phényle, formyle, carbamoyle, alcoylcarbamoyle, dialcoylcarbamoyle, acyle, alcoyloxycarbonyle ou thiazolidinyle-2,

b) par un radical allyle; dihydroxy-2,3 propyle; dihydroxy-1,3 propyle-2; formyl-2 hydroxy-2 éthyle; formyloxy-3 hydroxy-2 propyle; bis-formyloxy-2,3 propyle ou bisformyloxy-1,3 propyle-2;

c) par un radical alcoyle contenant 2 à 4 atomes de carbone, substitué par un radical hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales:

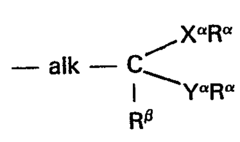

ou 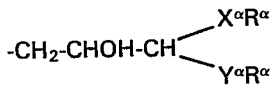

ou 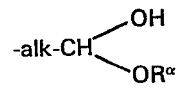

dans lesquelles alk est un radical alcoylène contenant 1 à 4 atomes de carbone, $X^\alpha$ et $Y^\alpha$ sont identiques et représentent des atomes d'oxygène ou de soufre, et $R^\alpha$ représente un radical alcoyle, ou bien $X^\alpha$ et $Y^\alpha$ sont identiques ou différents et représentent des atomes d'oxygène ou de soufre et les radicaux $R^\alpha$ forment ensemble un radical alcoylène contenant 2 ou 3 atomes de carbone, et $R^\beta$ représente un atome d'hydrogène ou un radical alcoyle contenant 1 à 3 atomes de carbone.

e) par un radical alcoyle contenant 2 à 5 atomes de carbone substitué par un radical alcoyloxyimino ou hydroxyimino,

5) dialcoyl-1,4 dioxo-5,6 tétrahydro-1,4,5,6 triazine-1,2,4 yle-3; alcoyl-1 dioxo-5,6 tétrahydro-1,4,5,6 triazine-1,2,4 yle-3; alcoyl-2 dioxo-5,6 tétrahydro-1,2,5,6 triazine-1,2,4 yle-3;

6) triazol-1,3,4 yle-5; triazol-1,2,3 yle-5 ou alcoyl-1 triazol-1,2,4 yle-5 non substitué ou substitué en position —3 par alcoyloxycarbonyle,

7) a) thiadiazol-1,3,4 yle-5 non substitué ou substitué par un radical alcoyle, trifluorométhyle, alcoyloxy, alcoylthio, hydroxyalcoylthio dont la partie alcoyle contient 2 à 4 atomes de carbone, alcoylsulfonyle, hydroxy, hydroxyalcoyle, carboxy, carboxyalcoyle, amino, alcoylamino, dialcoylamino, aminoalcoyle, alcoylaminoalcoyle, dialcoylaminoalcoyle, acylamino ou acylaminoalcoyle,

b) thiadiazol-1,2,4 yle-5 substitué par un radical alcoyle ou alcoyloxy,

8) a) oxadiazol-1,3,4 yle-5 non substitué ou substitué par un radical alcoyle, trifluorométhyle, phényle, aminoalcoyle, alcoylaminoalcoyle, dialcoylaminoalcoyle ou acylaminoalcoyle,

b) oxazolyle-2 ou alcoyl-4 oxazolyle-2,

9) tétrazolyle-5 non substitué ou substitué en position —1 par

a) un radical alcoyle non substitué ou substitué par alcoyloxy, sulfo, carboxy, formyle ou sulfamoyle,

b) un radical alcoyle contenant 2 à 4 atomes de carbone substitué par hydroxy, amino, alcoylamino, dialcoylamino, acylamino, carboxyalcoylamino, sulfamoylamino, sulfoamino, uréido, alcoyluréido ou dialcoyluréido,

c) un radical alcoyle contenant 2 à 5 atomes de carbone substitué par hydroxyimino ou alcoyloxyimino,

d) un radical phényle, dihydroxy-2,3 propyle, dihydroxy-1,3 propyle-2; formyl-2 hydroxy-2 éthyle; formyloxy-3 hydroxy-2 propyle; bisformyloxy-2,3 propyle ou bisformyloxy-1,3 propyle-2, ou

e) un radical de formules générales

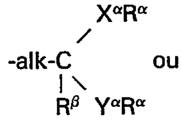 ou 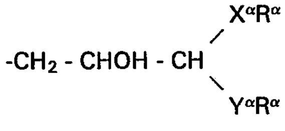

dans lesquelles $R^\beta$ est und atome d'hydrogène et $X^\alpha$, $Y^\alpha$ et $R^\alpha$ sont définis comme en (4d) ci-dessus et le symbole $R^\circ$ représente un radical carboxyalcoyle de formule générale:

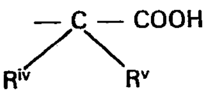

dans lesquelles $R^\beta$ est un atome d'hydrogène et $X^\alpha$, rents, représentent des atomes d'hydrogène ou des radicaux alcoyle ou forment ensemble un radical alcoylène contenant 2 ou 3 atomes de carbone ou bien

β/ le symbole R est choisi parmi

1) dioxo-5,6 tétrahydro-1,4,5,6 triazine-1,2,4 yle-3 substitué en position —1, dioxo-5,6 tétrahydro-1,2,5,6 triazine-1,2,4 yle-3 substitué en position —2, triazol-1,2,4 yle-5 ou alcoyloxycarbonyl-3 triazol-1,2,4 yle-5 substitués en position —1 par

a) un radical alcoyle lui-même substitué par un radical alcoyloxy, alcoylthio, phényle, formyle, carbamoyle, alcoylcarbamoyle, dialcoylcarbamoyle, hydroxyalcoylcarbamoyle (dont la partie alcoyle contient 2 à 4 atomes de carbone), acyle, alcoyloxycarbonyle ou thiazolidinyle-2,

b) un radical allyle; dihydroxy-2,3 propyle; dihydroxy-1,3 propyle-2; formyl-2 hydroxy-2 éthyle; formyloxy-3 hydroxy-2 propyle; bis-formyloxy-2,3 propyle ou bisformyloxy-1,3 propyle-2,

c) un radical alcoyle contenant 2 à 4 atomes de carbone, substitué par un radical hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) un radical répondant à l'une des formules générales citées ci-dessus en α-4-d) ou

e) un radical alcoyle contenant 2 à 5 atomes de carbone substitué par un radical alcoyloxyimino ou hydroxyimino, ou bien

2) dioxo-5,6 hydroxyalcoylcarbamoylalcoyl-4 tétrahydro-1,4,5,6 triazine-1,2,4 yle-3 dont la portion hydroxyalcoyle contient 2 à 4 atomes de carbone, ou bien

3) alcoyl-1 dioxo-5,6 tétrahydro-1,4,5,6 triazine-1,2,4 yle-3 substitué en position —4, alcoyl-1 dioxo-5,6 tétrahydro-1,2,5,6 triazine-1,2,4 yle-3 substitué en position —2, alcoyl-2 dioxo-5,6 tétrahydro-1,2,5,6 triazine-1,2,4 yle-3 substitué en position —1 ou alcoyl-4 dioxo-5,6 tétrahydro-1,4,5,6 triazine-1,2,4 yle-3 substitué en position —1 par un radical formylalcoyle ou par un radical de formule générale

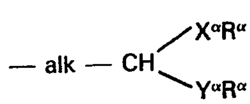

dans laquelle alk, $X^\alpha$, $Y^\alpha$ et $R^\alpha$ sont définis comme ci-dessus et le symbole $R^\circ$ représente un atome d'hydrogène, un radical alcoyle, vinyle, cyanométhyle ou un radical de formule générale

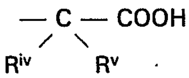

telle que définie ci-avant,
et le symbole R' représente un atome d'hydrogène ou un radical facilement éliminable par voie enzymatique de formule générale:

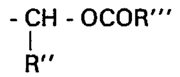

dans laquelle R'' représente un atome d'hydrogène ou un radical alcoyle et R''' représente un radical alcoyle ou le radical cyclohexyle, étant entendu que les radicaux et portions alcoyles ou acyles cités ci-dessus sont (sauf mention spéciale) droits ou ramifiés et contiennent 1 à 4 atomes de carbone, sous ses formes syn ou anti et E ou Z et leurs mélanges, ainsi que ses sels d'addition avec les acides, ses sels métalliques et ses sels d'addition avec les bases azotées.

2. Procédé de préparation d'un produit selon la revendication 1, pour lequel le radical R ne contient pas de substituant de formule générale -alk-CH(OH)OR$^{\alpha}$, caractérisé en ce que l'on fait agir un acide de formule générale:

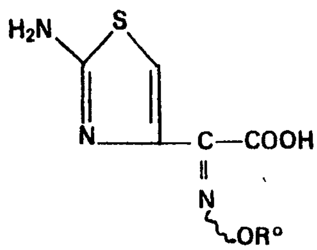

dans laquelle R° est défini selon la revendication 1, et dont la fonction amine est préalablement protégée (ainsi que l'oxime lorsque R° représente l'hydrogène ou la fonction acide lorsque R° contient un groupement carboxy) ou un dérivé réactif de cet acide, sur une amino-7 céphalosporine de formule générale:

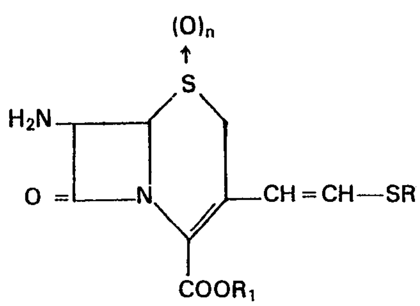

dans laquelle R est défini selon la revendication 1, $R_1$ représente un atome d'hydrogène, un radical de formule générale:

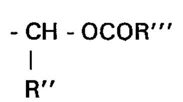

tel que défini dans la revendication 1, ou un radical protecteur facilement éliminable, et n représente 0 ou 1 puis, lorsque n = 1, on réduit l'oxyde obtenu et élimine les radicaux protecteurs puis transforme éventuellement le produit obtenu en un sel d'addition avec un acide, en un sel métallique, ou en un sel d'addition avec une base azotée.

3. Procédé selon la revendication 2, caractérisé en ce que l'on utilise un acide de formule générale:

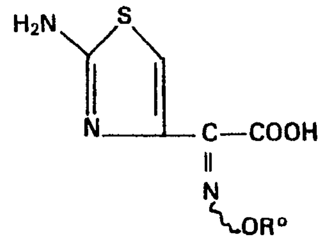

dans laquelle R° est défini selon la revendication 1, et dont la fonction amine est préalablement protégée par un groupement t.butoxycarbonyle, trichloro-2,2,2 éthoxycarbonyle, trichloracétyle, chloracétyle, trityle, dibenzyle, benzyle, benzyloxycarbonyle, p.nitrobenzyloxycarbonyle, p.méthoxybenzyloxycarbonyle, formyle ou trifluoracétyle.

4. Procédé selon la revendication 2, caractérisé en ce que l'on utilise un acide de formule générale:

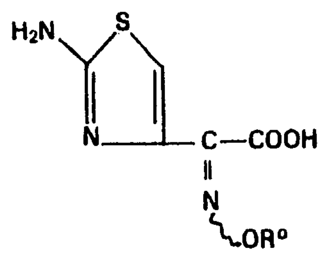

dans laquelle R° est un groupement trityle, tétrahydropyrannyle ou méthoxy-2 propyle- protecteur de la fonction oxime.

5. Procédé selon la revendication 2, caractérisé en ce que l'on utilise un acide de formule générale:

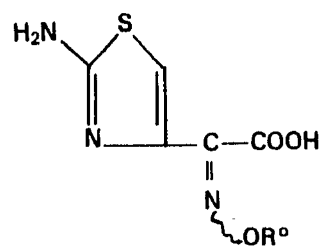

dans laquelle R° contient un radical carboxy, protégé par un groupement méthoxyméthyle, t.butyle, benzhydryle, benzyle, nitrobenzyle ou p.méthoxybenzyle.

6. Procédé selon la revendication 2, caractérisé en ce que l'on utilise une amino-7 céphalosporine dans laquelle le radical facilement éliminable représente par $R_1$ est choisi parmi les radicaux méthoxyméthyle, t.butyle, benzhydryle, nitrobenzyle et p.méthoxybenzyle.

7. Procédé selon la revendication 2, caractérisé en ce que l'on utilise un dérivé réactif de l'acide de formule générale:

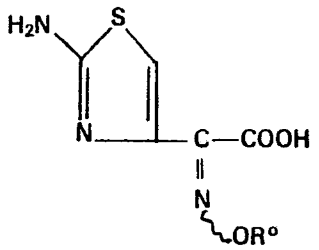

dans laquelle $R^o$ est défini selon la revendication 1, choisi parmi l'anhydride, un anhydride mixte, un ester réactif et un halogénure de cet acide.

8. Procédé de préparation d'un produit selon la revendication 1, pour lequel le radical R ne contient pas de substituant de formule générale -alk-CH(OH)OR$^\alpha$, caractérisé en ce que l'on fait agir un thiol (libre ou à l'état de sel alcalin ou alcalino-terreux) de formule générale:

R - SH

(dans laquelle le substituant de R, qui est défini comme ci-dessus, est protégé à l'état d'acétal [défini par les formules générales

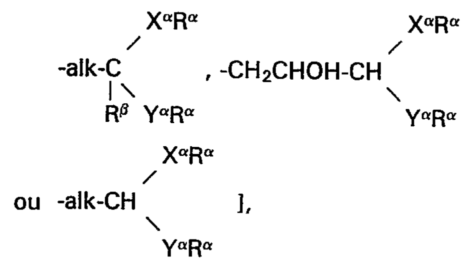

lorsque l'on veut obtenir un produit selon la revendication 1 pour lequel R contient un radical formyle ou acylalcoyle), sur un dérivé de céphalosporine (ou, le cas échéant, sur un mélange des isomères) de formule générale:

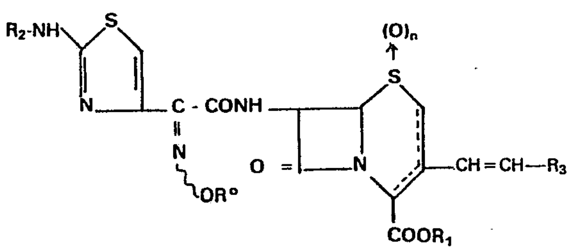

dans laquelle, $R^o$ étant défini selon la revendication 1, $R_1$ et n étant définis selon la revendication 2,
lorsque n = 0 ce dérivé se présente sous forme bicyclooctène-2 ou -3,
lorsque n = 1 ce dérivé se présente sous forme bicyclooctène-2,
le substituant sur l'atome de carbone en position —3 du bicyclooctène présente la stéréoisomérie E ou Z, $R_2$ représente un atome d'hydrogène ou un radical protecteur du radical amino, et $R_3$ représente un atome d'halogène choisi parmi le chlore, le brome ou l'iode ou un radical de formules générales:

$-O-SO_2R'_3$
ou $-O-CO\ R''_3$

dans lesquelles $R'_3$ est un radical alcoyle droit ou ramifié contenant 1 à 4 atomes de carbone, trifluorométhyle, trichlorométhyle, ou phényle non substitué ou substitué par un atome d'halogène ou par un radical alcoyle ou nitro, et
$R''_3$ est défini comme $R'_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle,
puis lorsque n = 1 on réduit l'oxyde obtenu, élimine s'il y a lieu les radicaux protecteurs et transforme éventuellement le produit obtenu en sel d'addition avec un acide, un sel métallique ou un sel d'addition avec une base azotée.

9. Procédé selon la revendication 8, caractérisé en ce que la réaction est effectuée en présence d'une pyridine ou d'une base organique tertiaire.

10. Procédé de préparation d'un produit selon la revendication 1, pour lequel le radical R ne contient pas de substituant de formule générale -alk-CH(OH)-OR$^\alpha$, caractérisé en ce que l'on fait agir un thioloester de formule générale:

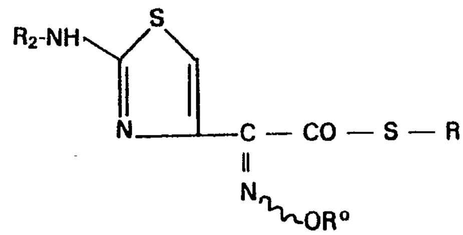

dans laquelle $R^o$ et $R_2$ sont définis comme dans la revendication 8, et R est défini comme ci-dessus, [étant entendu que lorsque $R^o$ est un atome d'hydrogène, l'oxime peut être protégée, lorsque $R^o$ contient un radical carboxy, ce dernier est protégé, lorsque R contient un substituant amino, alcoylamino, formyle ou acylalcoyle, celui-ci est protégé, et lorsque R contient un substituant hydroxy ou carboxy, celui-ci est libre ou protégé], sur une amino-7 céphalosporine de formule générale:

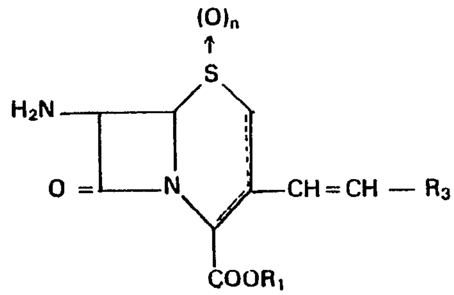

dans laquelle $R_1$, $R_3$ et n sont définis comme dans la revendication 8, et qui présente la même stéréoisomérie que le produit défini dans la revendication 8 puis, lorsque n = 1, on réduit l'oxyde obtenu, élimine s'il y a lieu les radicaux protecteurs et transforme éventuellement le produit obtenu en un sel d'addition avec un acide, en sel métallique, ou en sel d'addition avec une base azotée.

11. Procédé selon la revendication 10, caractérisé en ce que l'on utilise un thioloester dans lequel le cas échéant le radical R est protégé par un radical protecteur d'amino tel que défini dans la revendication 3, ou sous forme d'acétal par un radical de formule générale:

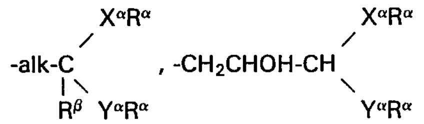

ou

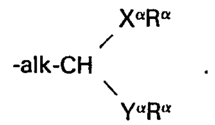

12. Procédé de préparation d'un produit selon la revendication 1, pour lequel le radical R ne contient pas de substituant de formule générale -alk-CH-(OH)OR$^{\alpha}$ et R° est autre que vinyle, caractérisé en ce que lon fait agir une thiourée de formule générale:

$R_2NH$ CS $NH_2$

dans laquelle $R_2$ est un atome d'hydrogène ou un radical protecteur d'amine, sur un produit de formule générale:

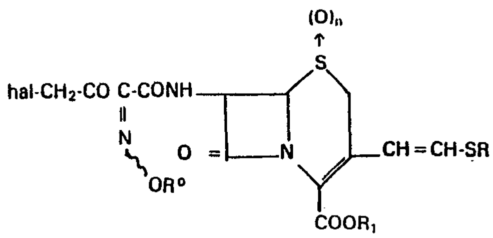

dans laquelle R°, $R_1$ et n sont définis comme dans la revendication 2 (à l'exception, pour R°, de représenter un radical vinyle),
R est défini comme ci-dessus et hal représente un atome de chlore ou de brome, lorsque n = 1, réduit l'oxyde obtenu, élimine s'il y a lieu les radicaux protecteurs et transforme éventuellement le produit obtenu en un sel d'addition avec un acide, en sel métallique, ou en sel d'addition avec une base azotée.

13. Procédé selon la revendication 12, caractérisé en ce que l'on fait agir une thiourée dans la formule de laquelle le radical $R_2$ est choisi parmi les radicaux t.butoxycarbonyle, trichloro-2,2,2 éthoxycarbonyle, trityle, dibenzyle, benzyle, benzyloxycarbonyle, p.nitrobenzyloxycarbonyle, p.méthoxybenzyloxycarbonyle, formyle ou trifluoracétyle.

14. Procédé de préparation d'un produit selon la revendication 1 pour le quel le radical R représente un radical dioxo-5,6 tétrahydro-1,4,5,6 triazine-1,2,4 yle-3 substitué en position —1 ou —4, dioxo-5,6 tétrahydro-1,2,5,6 triazine-1,2,4 yle-3 substitué en position -2, triazol-1,3,4 yle-5 ou alcoyloxycarbonyl-2 triazol-1,3,4 yle-5 ou triazol-1,2,4 yle-5 ou alcoyloxycarbonyl-3 triazol-1,2,4 yle-5 substitués en position —1, par un radical alcoyle contenant 2 à 4 atomes de carbone lui-même substitué par un groupement carbamoyloxy ou acyloxy (dont la partie acyle est éventuellement substituée par un radical amino, alcoylamino ou dialcoylamino), caractérisé en ce qu'on carbamoyle ou estérifie un alcool de formule générale:

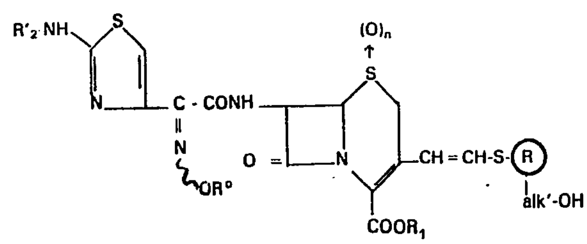

(dans laquelle R° , $R_1$ et n sont définis comme dans la revendication 2, $R'_2$ est un radical protecteur d'amine, et -Ⓡ-alk'-OH représente un radical dioxo-5,6 hydroxyalcoyl-1 (ou -4) tétrahydro-1,4,5,6 triazine-1,2,4 yle-3, dioxo-5,6 hydroxyalcoyl-2 tétrahydro-1,2,5,6 triazine-1,2,4 yle-3 ou bien hydroxyalcoyl-1 triazol-1,3,4 yle-5, alcoyloxycarbonyl-2 hydroxyalcoyl-1 triazol-1,3,4 yle-5, hydroxyalcoyl-1 triazol-1,2,4 yle-5 ou alcoyloxycarbonyl-3 hydroxyalcoyl-1 triazol-1,2,4 yle-5) par toute méthode connue pour obtenir un carbamate ou un ester à partir d'un alcool sans toucher au reste de la molécule, on réduit le cas échéant l'oxyde obtenu, élimine les groupements protecteurs, puis transforme éventuellement le produit obtenu en un sel d'addition avec un acide, en un sel métallique ou en un sel d'addition avec une base azotée.

15. Procédé selon la revendication 14, caractérisé en ce qu'on utilise une céphalosporine dans la formule de laquelle $R'_2$ est choisi parmi les groupements protecteurs d'amino cités dans la revendication 3.

16. Procédé de préparation d'un produit selon la revendication 1 pour lequel R représente un radical dioxo-5,6 tétrahydro-1,4,5,6 triazine-1,2,4 yle-3 substitué en position —1 (ou —4), dioxo-5,6 tétrahydro-1,2,5,6 triazine-1,2,4 yle-3 substitué en position —2, triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position —1, par un radical alcoyle contenant 2 à 4 atomes de carbone, lui-même substitué par un groupement sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido ou dialcoyluréido, ou représente un radical thiadiazol-1,3,4 yle-5 substitué par un radical acylamino ou acylaminoalcoyle, ou représente un radical oxadiazol-1,3,4 yle-5 substitué par un radical acylaminoalcoyle, ou représente un radical tétrazolyle-5 substitué en position —1 par un radical alcoyle contenant 2 à 4 atomes de carbone substitué par un groupement acylamino, sulfamoylamino, sulfoamino, uréido, alcoyluréido ou dialcoyluréido, caractérisé en ce qu'on traite une amine de formule générale:

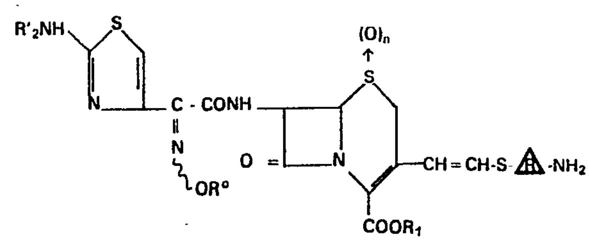

dans laquelle R°, $R_1$, et n sont définis comme dans la revendiation 2, $R'_2$ est un radical protecteur d'amine, et -R-$NH_2$ représente un radical dioxo-5,6 tétrahydro-1,4,5,6 triazine-1,2,4 yle-3 substitué en position —1 (ou —4), dioxo-5,6 tétrahydro-1,2,5,6 triazine-1,2,4 yle-3 substitué en position —2, triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position —1, par un radical aminoalcoyle (contenant 2 à 4 atomes de carbone), ou un radical thiadiazol-1,3,4 yle-5 substitué par un radical amino ou aminoalcoyle, ou un radical oxadiazol-1,3,4 yle-5 substitué par un radical aminoalcoyle, ou un radical tétrazolyle-5 substitué en position —1 par un radical aminoalcoyle (contenant 2 à 4 atomes de carbone), par toute méthode connue pour former une fonction amide, sulfamide, carbamate ou urée, sans toucher au reste de la molécule, puis lorsque n = 1 réduit l'oxyde obtenu, élimine les groupements protecteurs, puis transforme éventuellement le produit obtenu en un sel d'addition avec un acide, en un sel métallique, ou en un sel d'addition avec une base azotée.

17. Procédé selon la revendication 16, caractérisé en ce qu'on utilise une céphalosporine dans la formule de laquelle $R'_2$ est défini comme dans la revendication 15.

18. Procédé selon la revendication 16, caractérisé en ce que, lorsque l'on veut préparer un produit défini dans la revendication 16 pour lequel R contient un substituant hydroxy ou amino, ces fonctions sont protégées dans le réactif utilisé.

19. Procédé de préparation d'un produit selon la revendication 1 pour lequel R représente un radical dioxo-5,6 tétrahydro-1,4,5,6 triazine-1,2,4 yle-3 substitué en position —1 (ou —4), dioxo-5,6 tétrahydro-1,2,5,6 triazine-1,2,4 yle-3 substitué en position —2, triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position —1, par un radical thiazolidinyl-2 alcoyle, par un radical -alk-CH(OH)$OR^{\alpha}$ ou par un radical hydroxyiminoalcoyle ou alcoyloxyiminoalcoyle dont la partie iminoalcoyle contient 2 à 5 atomes de carbone, ou bien représente un radical tétrazolyle-5 substitué en position —1 par un radical hydroxyiminoalcoyle ou alcoyloxyiminoalcoyle dont la partie iminoalcoyle contient 2 à 5 atomes de carbone, caractérisé en ce qu'on effectue une réaction d'addition de cystéamine, d'un alcool, d'hydroxylamine ou d'une alcoyloxyamine, sur un aldéhyde de formule générale:

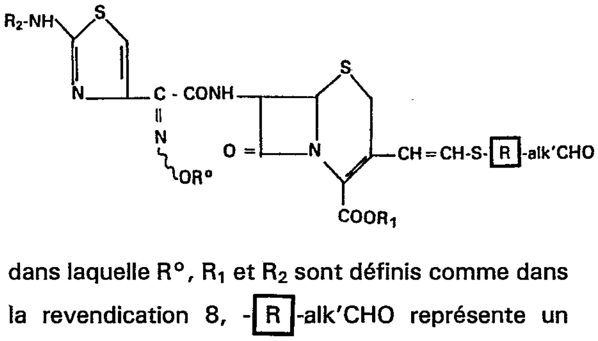

dans laquelle R°, $R_1$ et $R_2$ sont définis comme dans la revendication 8, -R-alk'CHO représente un radical dioxo-5,6 formylalcoyl-1 (ou -4) tétrahydro-1,4,5,6 triazine-1,2,4 yle-3, dioxo-5,6 formylalcoyl-2 tétrahydro-1,2,5,6 triazine-1,2,4 yle-3 ou bien formylalcoyl-1 triazine-1,3,4 yle-5, alcoyloxycarbonyl-2 formylalcoyl-1 triazol-1,3,4 yle-5, formylalcoyl-1 triazol-1,2,4 yle-5, alcoyloxycarbonyl-3 formylalcoyl-1 triazol-1,2,4 yle-5 ou formylalcoyl-1 tétrazolyle-5, par toute méthode connue pour former de tels dérivés d'addition de fonctions carbonylées, puis élimine les radicaux protecteurs et transforme éventuellement le produit obtenu en un sel d'addition avec un acide, en un sel métallique ou en un sel d'addition avec une base azotée.

20. Procédé de préparation selon la revendication 19, caractérisé en ce qu'on utilise une céphalosporine dans la formule de laquelle $R_2$ est un groupement protecteur choisi parmi les radicaux protecteurs d'amino cités dans la revendication 3.

21. Procédé de préparation d'un produit selon la revendication 1, pour lequel R' représente un radical de formule générale:

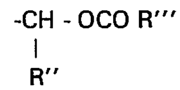

tel que défini dans la revendication 1, caractérisé en ce que l'on estérifie un produit selon la revendication 1 pour lequel R' est un atome d'hydrogène, par toute méthode connue en soi pour préparer un ester à partir d'un acide sans toucher au reste de la molécule, puis transforme éventuellement le produit obtenu en sel d'addition avec un acide.

22. Procédé de préparation d'un produit selon la revendication 1 pour lequel R et R° sont définis comme dans la revendication 1 en $\alpha$/, [étant entendu que R ne contient pas de substituant de formule générale -alk-CH(OH)$OR^{\alpha}$], caractérisé en ce que l'on fait agir un produit de formule générale

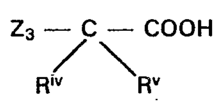

dans laquelle $R^{iv}$ et $R^{v}$ sont définis comme dans la revendication 1 et $Z_3$ représente un atome d'halogène ou un radical sulfate ou sulfonate, et dont la fonction acide est protégée, sur une céphalosporine de formule générale

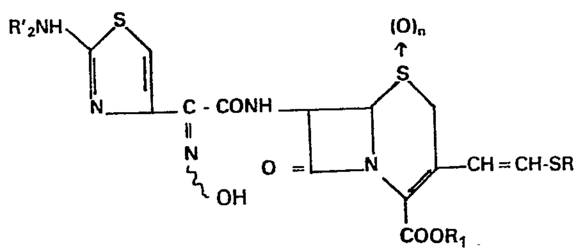

dans laquelle R est défini comme ci-dessus, (étant entendu que, lorsque R contient un substituant amino ou alcoylamino celui-ci est protégé, et lorsqu'il contient un radical formyle celui-ci est libre ou protégé), $R_1$ et n sont définis comme dans la revendication 2 et $R'_2$ est défini comme dans la revendication 14, puis on réduit le cas échéant l'oxyde obtenu, élimine les radicaux protecteurs et transforme éventuellement le produit obtenu en un sel d'addition avec un acide, en sel métallique ou en sel d'addition avec une base azotée.

23. Procédé selon la revendication 22, caractérisé en ce que l'on utilise une céphalosporine dans laquelle $R'_2$ est un radical protecteur d'amino choisi parmi les radicaux cités dans la revendication 3.

24. Procédé de préparation d'un produit selon la revendication 1 pour lequel le radical R ne contient pas de substituant de formule générale -alk-CH-(OH)OR$^\alpha$, caractérisé en ce que l'on fait agir un thiol (libre ou à l'état de sel alcalin ou alcalino-terreux) tel que défini dans la revendication 8, sur un dérivé de céphalosporine (ou un mélange des isomères) de formule générale

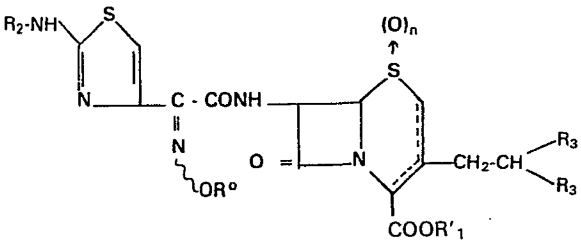

dans laquelle R° représente un radical alcoyle, vinyle ou cyanométhyle, un radical protecteur d'oxime ou un radical de formule générale

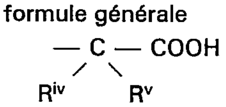

(dans laquelle les radicaux $R^{iv}$ et $R^{v}$, qui sont identiques ou différents, représentent des atomes d'hydrogène ou des radicaux alcoyle ou forment ensemble un radical alcoylène contenant 2 ou 3 atomes de carbone) protégé, $R'_1$ est défini comme $R_1$ dans la revendication 2 à l'exception de représenter un atome d'hydrogène, $R_2$ est défini comme dans la revendication 8, n représente 0 ou 1 (lorsque n = 0 ce dérivé se présente sous forme bicyclooctène-1 ou -3, lorsque n = 1 ce dérivé se présente sous forme bicyclooctène-2) et $R_3$ représente un atome de chlore ou de brome, puis lorsque n = 1 on réduit le produit obtenu, élimine les radicaux protecteurs, et transforme éventuellement le produit obtenu en un sel d'addition avec un acide, en un sel métallique ou en sel d'addition avec une base azotée.

25. Médicament caractérisé en ce qu'il contient au moins un produit selon la revendication 1 à l'état pur ou sous forme de composition pharmaceutique en association avec un ou plusieurs diluants ou adjuvants compatibles et pharmaceutiquement acceptables.

**Revendication pour l'Etat contractant:** AT

Procédé de préparation d'une thiovinyl-3 céphalosporine de formule générale

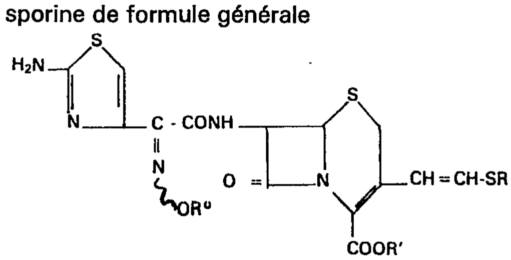

dans laquelle

$\alpha$/ le symbole R est choisi parmi les significations suivantes:

1) alcoyle, L-amino-2 carboxy-2 éthyle, phényle,

2) pyridyle-2, pyridyle-3 ou pyridyle-4 et leurs N-oxydes,

3) pyrimidinyle-2, pyridazinyle-3 substitué en position —6 (par un radical alcoyle, méthoxy, amino ou acylamino) et éventuellement N-oxydé ou tétrazolo [4,5-b] pyridazinyle-6,

4) dioxo-5,6 tétrahydro-1,4,5,6 triazine-1,2,4 yle-3 substitué en position —4; triazol-1,3,4 yle-5 ou alcoyloxycarbonyl-2 triazol-1,3,4 yle-5 substitués en position —1

a) par un radical alcoyle, non substitué ou substitué par un radical alcoyloxy, alcoylthio, phényle, formyle, carbamoyle, alcoylcarbamoyle, dialcoylcarbamoyle, acyle, alcoyloxycarbonyle ou thiazolidinyle-2,

b) par un radical allyle; dihydroxy-2,3 propyle; dihydroxy-1,3 propyle-2; formyl-2 hydroxy-2 éthyle; formyloxy-3 hydroxy-2 propyle; bisformyloxy-2,3 propyle ou bisformyloxy-1,3 propyle-2;

c) par un radical alcoyle contenant 2 à 4 atomes de carbone, substitué par un radical hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) par un radical répondant à l'une des formules générales:

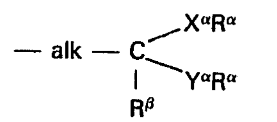

ou 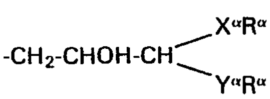

ou 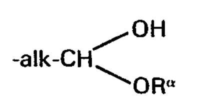

dans lesquelles alk est un radical alcoylène contenant 1 à 4 atomes de carbone, $X^\alpha$ et $Y^\alpha$ sont identiques et représentent des atomes d'oxygène ou de soufre, et $R^\alpha$ représente un radical alcoyle, ou bien $X^\alpha$ et $Y^\alpha$ sont identiques ou différents et représentent des atomes d'oxygène ou de soufre et les radicaux $R^\alpha$ forment ensemble un radical alcoylène contenant 2 ou 3 atomes de carbone, et $R^\beta$ représente un atome d'hydrogène ou un radical alcoyle contenant 1 à 3 atomes de carbone.

e) par un radical alcoyle contenant 2 à 5 atomes de carbone substitué par un radical alcoyloxyimino ou hydroxyimino,

5) dialcoyl-1,4 dioxo-5,6 tétrahydro-1,4,5,6 triazine-1,2,4 yle-3; alcoyl-1 dioxo-5,6 tétrahydro-1,4,5,6 triazine-1,2,4 yle-3; alcoyl-2 dioxo-5,6 tétrahydro-1,2,5,6 triazine-1,2,4 yle-3;

6) triazol-1,3,4 yle-5; triazol-1,2,3 yle-5 ou alcoyl-1 triazol-1,2,4 yle-5 non substitué ou substitué en position —3 par alcoyloxycarbonyle,

7) a) thiadiazol-1,3,4 yle-5 non substitué ou substitué par un radical alcoyle, trifluorométhyle, alcoyloxy, alcoylthio, hydroxyalcoylthio dont la partie alcoyle contient 2 à 4 atomes de carbone, alcoylsulfonyle, hydroxy, hydroxyalcoyle, carboxy, carboxyalcoyle, amino, alcoylamino, dialcoylamino, aminoalcoyle, alcoylaminoalcoyle, dialcoylaminoalcoyle, acylamino ou acylaminoalcoyle,

b) thiadiazol-1,2,4 yle-5 substitué par un radical alcoyle ou alcoyloxy,

8) a) oxadiazol-1,3,4 yle-5 non substitué ou substitué par un radical alcoyle, trifluorométhyle, phényle, aminoalcoyle, alcoylaminoalcoyle, dialcoylaminoalcoyle ou acylaminoalcoyle,

b) oxazolyle-2 ou alcoyl-4 oxazolyle-2,

9) tétrazolyle-5 non substitué ou substitué en position —1 par

a) un radical alcoyle non substitué ou substitué par alcoyloxy, sulfo, carboxy, formyle ou sulfamoyle,

b) un radical alcoyle contenant 2 à 4 atomes de carbone substitué par hydroxy, amino, alcoylamino, dialcoylamino, acylamino, carboxyalcoylamino, sulfamoylamino, sulfoamino, uréido, alcoyluréido ou dialcoyluréido,

c) un radical alcoyle contenant 2 à 5 atomes de carbone substitué par hydroxyimino ou alcoyloxyimino,

d) un radical phényle, dihydroxy-2,3 propyle, dihydroxy-1,3 propyle-2; formyl-2 hydroxy-2 éthyle; formyloxy-3 hydroxy-2 propyle; bisformyloxy-2,3 propyle ou bisformyloxy-1,3 propyle-2, ou

e) un radical de formules générales

```
         XαRα                        XαRα
        /                           /
-alk-CH         ou  -CH2-CHOH-CH
  |   \                             \
  Rβ   YαRα                          YαRα
```

dans lesquelles $R^\beta$ est un atome d'hydrogène et $X^\alpha$, $Y^\alpha$ et $R^\alpha$ sont définis comme en (4d) ci-dessus et le symbole R° représente un radical carboxyalcoyle de formule générale

```
 — C — COOH
  / \
Riv  Rv
```

dans laquelle $R^{iv}$ et $R^{v}$, qui sont identiques ou différents, représentent des atomes d'hydrogène ou des radicaux alcoyle ou forment ensemble un radical alcoylène contenant 2 ou 3 atomes de carbone ou bien

β/ le symbole R est choisi parmi

1) dioxo-5,6 tétrahydro-1,4,5,6 triazine-1,2,4 yle-3 substitué en position —1, dioxo-5,6 tétrahydro-1,2,5,6 triazine-1,2,4 yle-3 substitué en position —2, triazol-1,2,4 yle-5 ou alcoyloxycarbonyl-3 triazol-1,2,4 yle-5 substitués en position —1 par

a) un radical alcoyle lui-même substitué par un radical alcoyloxy, alcoylthio, phényle, formyle, carbamoyle, alcoylcarbamoyle, dialcoylcarbamoyle, hydroxyalcoylcarbamoyle (dont la partie alcoyle contient 2 à 4 atomes de carbone), acyle, alcoyloxycarbonyle ou thiazolidinyle-2,

b) un radical allyle; dihydroxy-2,3 propyle; dihydroxy-1,3 propyle-2; formyl-2 hydroxy-2 éthyle; formyloxy-3 hydroxy-2 propyle; bis-formyloxy-2,3 propyle ou bisformyloxy-1,3 propyle-2,

c) un radical alcoyle contenant 2 à 4 atomes de carbone, substitué par un radical hydroxy, carbamoyloxy, acyloxy (dont la partie acyle peut être substituée par un radical amino, alcoylamino ou dialcoylamino), alcoylsulfinyle, alcoylsulfonyle, amino, alcoylamino, dialcoylamino, sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido, dialcoyluréido,

d) un radical répondant à l'une des formules générales citées ci-dessus en α-4-d) ou

e) un radical alcoyle contenant 2 à 5 atomes de carbone substitué par un radical alcoyloxyimino ou hydroxyimino, ou bien

2) dioxo-5,6 hydroxyalcoylcarbamoylalcoyl-4 tétrahydro-1,4,5,6 triazine-1,2,4 yle-3 dont la portion hydroxyalcoyle contient 2 à 4 atomes de carbone, ou bien

3) alcoyl-1 dioxo-5,6 tétrahydro-1,4,5,6 triazine-1,2,4 yle-3 substitué en position —4, alcoyl-1 dioxo-5,6 tétrahydro-1,2,5,6 triazine-1,2,4 yle-3 substitué en position —2, alcoyl-2 dioxo-5,6 tétrahydro-1,2,5,6 triazine-1,2,4 yle-3 substitué en position —1 ou alcoyl-4 dioxo-5,6 tétrahydro-1,4,5,6 triazine-1,2,4 yle-3 substitué en position —1 par un radical formylalcoyle ou par un radical de formule générale

```
            XαRα
           /
— alk — CH
           \
            YαRα
```

dans laquelle alk, $X^\alpha$, $Y^\alpha$ et $R^\alpha$ sont définis comme ci-dessus et le symbole R° représente un atome d'hydrogène, un radical alcoyle, vinyle, cyanométhyle ou un radical de formule générale

```
 — C — COOH
  / \
Riv  Rv
```

telle que définie ci-avant,

et le symbole R' représente un atome d'hydrogène ou un radical facilement éliminable par voie enzymatique de formule générale:

```
- CH OCOR'''
  |
  R''
```

dans laquelle R'' représente un atome d'hydrogène ou un radical alcoyle et R''' représente un radical alcoyle ou le radical cyclohexyle, étant entendu que les radicaux et portions alcoyles ou acyles cités ci-dessus sont (sauf mention spéciale) droits ou ramifiée et contiennent 1 à 4 atomes de carbone, sous ses formes syn ou anti et E ou Z et leurs mélanges, ainsi que ses sels d'addition avec les acides, ses sels métalliques et ses sels d'addition avec les bases azotées, caractérisé en ce que pour la préparation d'un produit dans la formule duquel le radical R ne contient pas de substituant de formule générale -alk-$CH(OH)OR^{\alpha}$

A - on fait agir un acide de formule générale:

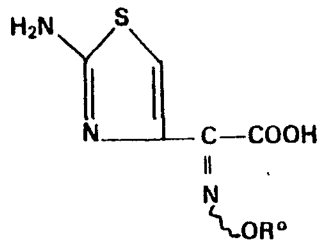

dans laquelle R° est défini comme ci-dessus, et dont la fonction amine est préalablement protégée (ainsi que l'oxime lorsque R° représente l'hydrogène ou la fonction acide lorsque R° contient un groupement carboxy) ou un dérivé réactif de cet acide, sur une amino-7 céphalosporine de formule générale:

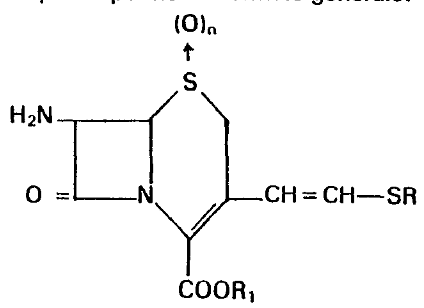

dans laquelle R est défini comme ci-dessus, $R_1$ représente un atome d'hydrogène, un radical de formule générale:

- CH - OCOR'''
|
R''

tel que défini ci-dessus ou un radical protecteur facilement éliminable, et n représente 0 ou 1, ou

B - on fait agir un thiol (libre ou à l'état de sel alcalin ou alcalino-terreux) de formule générale:

R - SH

(dans laquelle le substituant de R, qui est défini comme ci-dessus, est protégé à l'état d'acétal [défini par les formules générales

$-alk-C(R^{\beta})(X^{\alpha}R^{\alpha})(Y^{\alpha}R^{\alpha})$ , $-CH_2CHOH-CH(X^{\alpha}R^{\alpha})(Y^{\alpha}R^{\alpha})$ ou

$-alk-CH(X^{\alpha}R^{\alpha})(Y^{\alpha}R^{\alpha})$ ],

lorsque l'on veut obtenir un produit pour lequel R contient un radical formyle ou acylalcoyle), sur un dérivé de céphalosporine (ou, le cas échéant, sur un mélange des isomères) de formule générale:

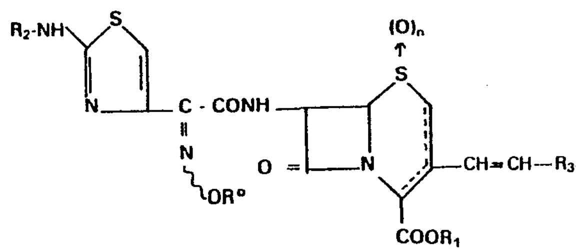

dans laquelle, R°, $R_1$ et n étant définis comme ci-dessus en A

lorsque n = 0 ce dérivé se présente sous forme bicyclooctène-2 ou -3,

lorsque n = 1 ce dérivé se présente sous forme bicyclooctène-2,

le substituant sur l'atome de carbone en position —3 du bicyclooctène présente la stéréoisomérie E ou Z, $R_2$ représente un atome d'hydrogène ou un radical protecteur du radical amino, et $R_3$ représente un atome d'halogène choisi parmi le chlore, le brome ou l'iode ou un radical de formules générales:

$-O-SO_2R'_3$
ou $-O-CO\ R''_3$

dans lesquelles $R'_3$ est un radical alcoyle droit ou ramifié contenant 1 à 4 atomes de carbone, trifluorométhyle, trichlorométhyle, ou phényle non substitué ou substitué par un atome d'halogène ou par un radical alcoyle ou nitro, et

$R''_3$ est défini comme $R'_3$ ou représente un radical acylméthyle, acyl-2 éthyle, acyl-2 propyle, alcoyloxycarbonylméthyle, alcoyloxycarbonyl-2 éthyle ou alcoyloxycarbonyl-2 propyle, ou

C - on fait agir un thioloester de formule générale:

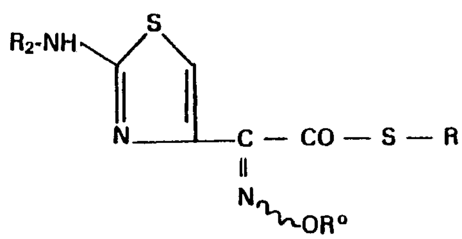

dans laquelle R, R° et $R_2$ sont définis comme ci-dessus en B, [étant entendu que lorsque R° est un atome d'hydrogène, l'oxime peut être protégée, lorsque R° contient un radical carboxy, ce dernier est protégé, lorsque R contient un substituant amino, alcoylamino, formyle ou acylalcoyle, celui-ci est protégé, et lorsque R contient un substituant hydroxy ou carboxy, celui-ci est libre ou protégé], sur une amino-7 céphalosporine de formule générale:

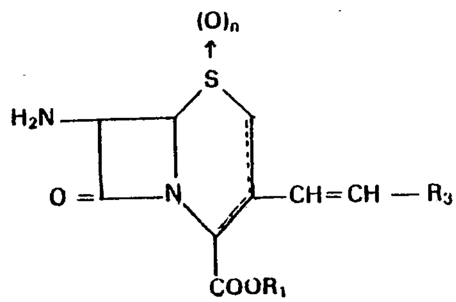

dans laquelle $R_1$, $R_3$ et n sont définis comme ci-dessus en B, et qui présente la même stéréoisomérie que le produit défini ci-dessus en B ou,

D - on fait agir un thiol (libre ou à l'état de sel alcalin ou alcalino-terreux) tel que défini en B sur un dérivé de céphalosporine (ou un mélange des isomères) de formule générale:

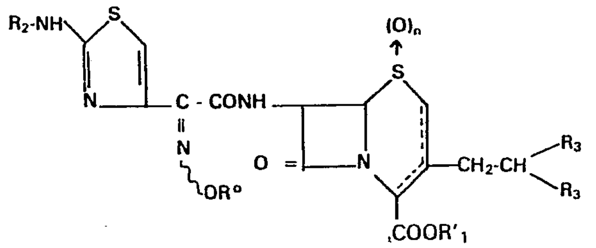

dans laquelle R° représente un radical alcoyle, vinyle ou cyanométhyle, un radical protecteur d'oxime ou un radical de formule générale

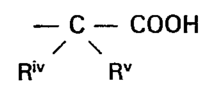

(dans laquelle les radicaux $R^{iv}$ et $R^{v}$, qui sont identiques ou différents, représentent des atomes d'hydrogène ou des radicaux alcoyle ou forment ensemble un radical alcoylène contenant 2 ou 3 atomes de carbone) protégé, $R'_1$ est défini comme $R_1$ en A à l'exception de représenter un atome d'hydrogène, $R_2$ est défini comme en B,

n représente 0 ou 1 (lorsque n = 0 ce dérivé se présente sous forme bicyclooctène-2 ou -3, lorsque n = 1 ce dérivé se présente sous forme bicyclooctène-2) et $R_3$ représente un atome de chlore ou de brome, puis lorsque n = 1 on réduit le produit obtenu, élimine les radicaux protecteurs, et transforme éventuellement le produit obtenu en un sel d'addition avec un acide, en un sel métallique ou en sel d'addition avec une base azotée ou

E - lorsque R° est défini comme ci-dessus excepté vinyle, on fait agir une thiourée de formule générale:

$R_2NH\ CS\ NH_2$

dans laquelle $R_2$ est un atome d'hydrogène ou un radical protecteur d'amine, sur un produit de formule générale:

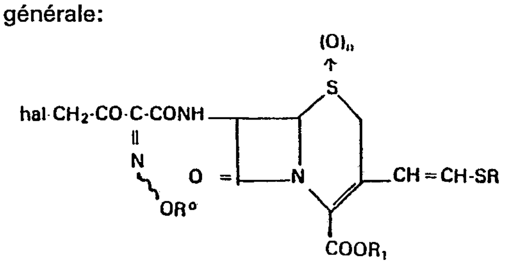

dans laquelle R°, $R_1$ et n sont définis comme ci-dessus en A (à l'exception, pour R°, de représenter un radical vinyle),
R est défini comme ci-dessus et hal représente un atome de chlore ou de brome,
puis, lorsque n = 1, on réduit l'oxyde obtenu, on élimine s'il y a lieu les radicaux protecteurs et transforme éventuellement le produit obtenu en un sel d'addition avec un acide, en sel métallique, ou en sel d'addition avec une base azotée,ou

F - pour la préparation d'un produit dans la formule duquel R et R° sont définis comme en α/, [étant entendu que R ne contient pas de substituant de formule générale -alk-CH/OH)OR$^{\alpha}$], on prépare selon l'invention une céphalosporine de formule générale

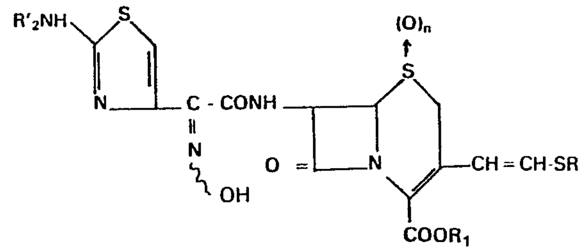

dans laquelle R est défini comme ci-dessus, (étant entendu que, lorsque R contient un substituant amino ou alcoylamino celui-ci est protégé, et lorsqu'il contient un radical formyle celui-ci est libre ou protégé), $R_1$ et n sont définis comme en A et $R'_2$ est un radical protecteur d'amine, sur laquelle ensuite on fait agir un produit de formule générale

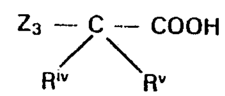

dans laquelle $R^{iv}$ et $R^{v}$ sont définis comme en α/ et $Z_3$ représente un atome d'halogène ou un radical sulfate ou sulfonate, et dont la fonction acide est protégée, puis on réduit le cas échéant l'oxyde obtenu, élimine les radicaux protecteurs et transforme éventuellement le produit obtenu en un sel d'addition avec un acide, en un sel métallique ou en sel d'addition avec une base azotée, ou

G - pour la préparation d'un produit dans la formule duquel le radical R représente un radical dioxo-5,6 tétrahydro-1,4,5,6 triazine-1,2,4 yle-3 substitué en position —1 ou —4, dioxo-5,6 tétra-hydro-1,2,5,6 triazine-1,2,4 yle-3 substitué en position —2, triazol-1,3,4 yle-5 ou alcoyloxycarbonyl-2 triazol-1,3,4 yle-5 ou triazol-1,2,4 yle-5 ou alcoyloxycarbonyl-3 triazol-1,2,4 yle-5 substitués en position —1, par un radical alcoyle contenant 2 à 4 atomes de carbone lui-même substitué par un groupement carbamoyloxy ou acyloxy (dont la partie acyle est éventuellement substituée par un radical amino, alcoylamino ou dialcoylamino), on prépare, selon l'invention, un alcool de formule générale:

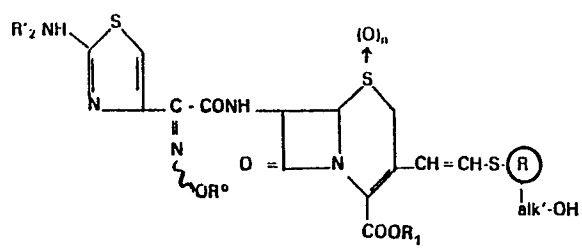

(dans laquelle R° , $R_1$ et n sont définis comme en A, $R'_2$ est un radical protecteur d'amine, et

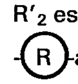-alk'-OH représente un radical dioxo-5,6 hydroxyalcoyl-1 (ou -4) tétrahydro-1,4,5,6 triazine-1,2,4 yle-3, dioxo-5,6 hydroxyalcoyl-2 tétrahydro-1,2,5,6 triazine-1,2,4 yle-3 ou bien hydroxyalcoyl-1 triazol-1,3,4 yle-5, alcoyloxycarbonyl-2 hydroxyalcoyl-1 triazol-1,3,4 yle-5, hydroxyalcoyl-1 triazol-1,2,4 yle-5 ou alcoyloxycarbonyl-3 hydroxyalcoyl-1 triazol-1,2,4 yle-5) puis on transforme cet alcool en l'ester où carbamate correspondant, on réduit le cas échéant l'oxyde obtenu, élimine les groupements protecteurs, puis transforme éventuellement le produit obtenu en un sel d'addition avec un acide, en un sel métallique ou en un sel d'addition avec une base azotée ou

H - pour la préparation d'un produit dans la formule duquel R représente un radical dioxo-5,6 tétrahydro-1,4,5,6 triazine-1,2,4 yle-3 substitué en position —1 (ou —4), dioxo-5,6 tétrahydro-1,2,5,6 triazine-1,2,4 yle-3 substitué en position —2, triazol-1,3,4 yle-5, alcoyloxycarbonyl-2 triazol-1,3,4 yle-5 triazol-1,2,4 yle-5 ou alcoyloxycarbonyl-3 triazol-1,2,4 yle-5 substitués en position —1, par un radical alcoyle contenant 2 à 4 atomes de carbone, lui-même substitué par un groupement sulfoamino, alcoylsulfonylamino, sulfamoylamino, acylamino (dont la partie acyle est éventuellement substituée par hydroxy, amino, alcoylamino ou dialcoylamino), alcoyloxycarbonylamino, uréido, alcoyluréido ou dialcoyluréido, ou représente un radical thiadiazol-1,3,4 yle-5 substitué par un radical acylamino ou acylaminoalcoyle, ou représente un radical oxadiazol-1,3,4 yle-5 substitué par un radical acylaminoalcoyle, ou représente un radical tétrazolyle-5 substitué en position —1 par un radical alcoyle contenant 2 à 4 atomes de carbone substitué par un groupement acylamino, sulfamoylamino, sulfoamino, uréido, alcoyluréido ou dialcoyluréido, on prépare selon l'invention une amine de formule générale:

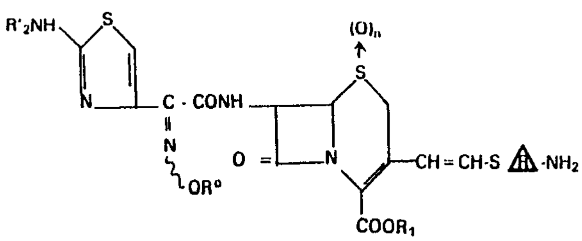

dans laquelle R°, $R_1$ et n sont définis comme en A, $R'_2$ est un radical protecteur d'amine, et -$\triangle$R-$NH_2$ représente un radical dioxo-5,6 tétrahydro-1,4,5,6 triazine-1,2,4 yle-3 substitué en position —1 (ou —4), dioxo-5,6 tétrahydro-1,2,5,6 triazine-1,2,4 yle-3 substitué en position —2, triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position —1, par un radical aminoalcoyle (contenant 2 à 4 atomes de carbone), ou un radical thiadiazol-1,3,4 yle-5 substitué par un radical amino ou aminoalcoyle, ou un radical oxadiazol-1,3,4 yle-5 substitué par un radical aminoalcoyle, ou un radical tétrazolyle-5 substitué en position —1 par un radical aminoalcoyle (contenant 2 à 4 atomes de carbone), puis on transforme l'amine en l'amide, sulfamide, carbamate ou urée correspondant, puis, lorsque n = 1 réduit l'oxyde obtenu, élimine les groupements protecteurs, puis transforme éventuellement le produit obtenu en un sel d'addition avec un acide, en un sel métallique, ou en un sel d'addition avec une base azotée ou

I - pour la préparation d'un produit dans la formule duquel R représente un radical dioxo-5,6 tétrahydro-1,4,5,6 triazine-1,2,4 yle-3 substitué en position —1 (ou —4), dioxo-5,6 tétrahydro-1,2,5,6 triazine-1,2,4 yle-3 substitué en position —2, triazol-1,3,4 yle-5, alcoyloxycarbonyl-2 triazol-1,3,4 yle-5, triazol-1,2,4 yle-5 ou alcoyloxycarbonyl-3 triazol-1,2,4 yle-5 substitués en position —1, par un radical thiazolidinyl-2 alcoyle, par un radical -alk-CH(OH)$OR^{\alpha}$ ou par un radical hydroxyiminoalcoyle ou alcoyloxyiminoalcoyle dont la partie iminoalcoyle contient 2 à 5 atomes de carbone, ou bien représente un radical tétrazolyle-5 substitué en position —1 par un radical hydroxyiminoalcoyle ou alcoyloxyiminoalcoyle dont la partie iminoalcoyle contient 2 à 5 atomes de carbone, on prépare selon l'invention un aldéhyde de formule générale:

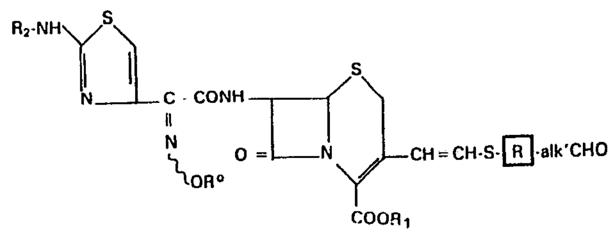

dans laquelle R°, $R_1$ et $R_2$ sont définis comme en B, -$\boxed{R}$-alk'CHO représente un radical dioxo-5,6 formylalcoyl-1 (ou -4) tétrahydro-1,4,5,6 triazine-1,2,4 yle-3, dioxo-5,6 formylalcoyl-2 tétrahydro-1,2,5,6 triazine-1,2,4 yle-3 ou bien formylalcoyl-1 triazol-1,3,4 yle-5, alcoyloxycarbonyl-2 formylalcoyl-1 triazol-1,3,4 yle-5, formylalcoyl-1 triazol-1,2,4 yle-5, alcoyloxycarbonyl-3 formylalcoyl-1 triazol-1,2,4 yle-5 ou formylalcoyl-1 tétrazolyle-5, puis on effectue sur l'aldéhyde une réaction d'addition de cystéamine, d'un alcool, d'hydroxyamine ou d'une alcoyloxyamine, puis élimine les radicaux protecteurs et transforme éventuellement le produit obtenu en un sel d'addition avec un acide, en un sel métallique ou en un sel d'addition avec une base azotée, ou

J - pour la préparation d'un produit dans la formule duquel R' représente un radical de formule générale:

- CH - OCO R'''
|
R''

on prépare selon l'invention l'acide correspondant pour lequel R' est un atome d'hydrogène, puis estérifie ledit acide et transforme éventuellement le produit en sel d'addition avec un acide.

**Patentansprüche für die Vertragsstaaten:**
BE, CH, DE, FR, GB, IT, LI, LU, NL, SE

1. Ein 3-Thiovinylcephalosporin, dadurch gekennzeichnet, dass es der allgemeinen Formel

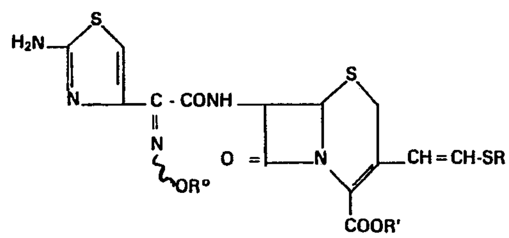

entspricht, worin bedeuten

α/ das Symbol R ist ausgewählt unter den folgenden Bedeutungen:

1) Alkyl, L-2-Amino-2-carboxyethyl, Phenyl,

2) Pyridyl-2, Pyridyl-3 oder Pyridyl-4 und deren N-Oxide,

3) Pyrimidinyl-2, Pyridazinyl-3, substituiert in 6-Stellung (durch einen Alkyl-, Methoxy-, Amino- oder Acylaminorest) und gegebenenfalls N-oxidiert, oder Tetrazolo[4,5-b]pyridazinyl-6,

4) 5,6-Dioxo-1,4,5,6-tetrahydro-1,2,4-triazin-3-yl, substituiert in 4-Stellung; 1,3,4-Triazol-5-yl oder 2-Alkyloxycarbonyl-1,3,4-triazol-5-yl, substituiert in 1-Stellung

a) durch einen Alkylrest, nichtsubstituiert oder substituiert durch einen Alkyloxy-, Alkylthio-, Phenyl-, Formyl-, Carbamoyl-, Alkylcarbamoyl-, Dialkylcarbamoyl-, Acyl-, Alkyloxycarbonyl- oder Thiazolidinyl-2-Rest,

b) durch einen Allylrest; 2,3-Dihydroxypropyl; 1,3-Dihydroxypropyl-2; 2-Formyl-2-hydroxyethyl; 3-Formyloxy-2-hydroxypropyl; 2,3-Bisformyloxypropyl oder 1,3-Bis-formyloxypropyl-2;

c) durch einen Alkylrest, enthaltend 2 bis 4 Kohlenstoffatome, substituiert durch einen Hydroxy-, Carbamoyloxy-, Acyloxy-(dessen Acylteil substituiert sein kann durch einen Amino-, Alkylamino- oder Dialkylaminorest), Alkylsulfinyl-, Alkylsulfonyl-, Amino-, Alkylamino-, Dialkylamino-, Sulfoamino-, Alkylsulfonylamino-, Sulfamoylamino-, Acylamino-(dessen Acylteil gegebenenfalls substituiert ist durch Hydroxy, Amino, Alkylamino oder Dialkylamino), Alkyloxycarbonylamino-, Ureido-, Alkylureido-, Dialkylureido-Rest,

d) durch einen Rest, entsprechend einer der allgemeinen Formeln:

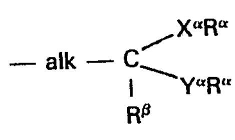

oder 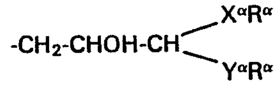

oder 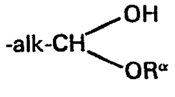

worin alk einen Alkylenrest mit 1 bis 4 Kohlenstoffatomen bedeutet, $X^\alpha$ und $Y^\alpha$ identisch sind und Sauerstoff- oder Schwefelatome bedeuten und $R^\alpha$ einen Alkylrest bedeutet oder $X^\alpha$ und $Y^\alpha$ identisch oder voneinander verschieden sind und Sauerstoff- oder Schwefelatome bedeuten und die Reste $R^\alpha$ zusammen einen Alkylenrest mit 2 oder 3 Kohlenstoffatomen bilden und $R^\beta$ ein Wasserstoffatom oder einen Alkylrest mit 1 bis 3 Kohlenstoffatomen bedeutet,

e) durch einen Alkylrest mit 2 bis 5 Kohlenstoffatomen, substituiert durch einen Alkyloxyimino- oder Hydroxyiminorest;

5) 1,4-Dialkyl-5,6-dioxo-1,4,5,6-tetrahydro-1,2,4-triazin-3-yl; 1-Alkyl-5,6-dioxo-1,4,5,6-tetrahydro-1,2,4-triazin-3-yl; 2-Alkyl-5,6-dioxo-1,2,5,6-tetrahydro-1,2,4-triazin-3-yl;

6) 1,3,4-Triazol-5-yl; 1,2,3-Triazol-5-yl oder 1-Alkyl-1,2,4-triazol-5-yl, nichtsubstituiert oder substituiert in 3-Stellung durch Alkyloxycarbonyl,

7) a) 1,3,4-Thiadiazol-5-yl, nichtsubstituiert oder substituiert durch einen Alkyl-, Trifluormethyl-, Alkyloxy-, Alkylthio-, Hydroxyalkylthio-, dessen Alkylteil 2 bis 4 Kohlenstoffatome enthält, Alkylsulfonyl-, Hydroxy-, Hydroxyalkyl-, Carboxy-, Carboxyalkyl-, Amino-, Alkylamino-, Dialkylamino-, Aminoalkyl-, Alkylaminoalkyl-, Dialkylaminoalkyl-, Acylamino- oder Acylaminoalkyl-Rest,

b) 1,2,4-Thiadiazol-5-yl, substituiert durch einen Alkyl- oder Alkyloxyrest,

8) a) 1,3,4-Oxadiazol-5-yl, nichtsubstituiert oder substituiert durch einen Alkyl-, Trifluormethyl-, Phenyl-, Aminoalkyl-, Alkylaminoalkyl-, Dialkylaminoalkyl- oder Acylaminoalkyl-Rest,

b) Oxazolyl-2 oder 4-Alkyloxazolyl-2,

9) Tetrazolyl-5, nichtsubstituiert oder substituiert in 1-Stellung durch

a) einen nichtsubstituierten oder durch Alkyloxy, Sulfo, Carboxy, Formyl oder Sulfamoyl substituierten Alkylrest,

b) einen Alkylrest mit 2 bis 4 Kohlenstoffatomen, substituiert durch Hydroxy, Amino, Alkylamino, Dialkylamino, Acylamino, Carboxyalkylamino, Sulfamoylamino, Sulfoamino, Ureido, Alkylureido oder Dialkylureido,

c) einen Alkylrest mit 2 bis 5 Kohlenstoffatomen, substituiert durch Hydroxyimino oder Alkyloxyimino,

d) einen Phenyl-, 2,3,-Dihydroxy-propyl-, 1,3-Dihydroxy-propyl-2-Rest; 2-Formyl-2-hydroxy-ethyl; 3-Formyloxy-2-hydroxy-propyl; 2,3-Bisformyloxy-propyl oder 1,3-Bisformyloxy-propyl-2, oder

e) einen Rest der allgemeinen Formeln

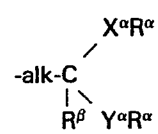 oder 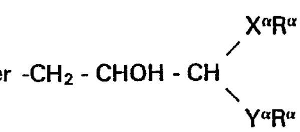

worin $R^\beta$ ein Wasserstoffatom ist und $X^\alpha$, $Y^\alpha$ und $R^\alpha$ wie oben unter (4d) definiert sind,

und das Symbol $R^o$ bedeutet einen Carboxyalkylrest der allgemeinen Formel

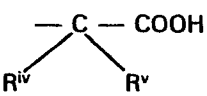

worin $R^{iv}$ und $R^v$, welche identisch oder verschieden sind, Wasserstoffatome oder Alkylrest bedeuten oder zusammen einen Alkylenrest mit 2 oder 3 Kohlenstoffatomen bilden, oder auch

β/ das Symbol R ist ausgewählt unter

1) 5,6-Dioxo-1,4,5,6-tetrahydro-1,2,4-triazin-3-yl, substituiert in 1-Stellung, 5,6-Dioxo-1,2,5,6-tetrahydro-1,2,4-triazin-3-yl, substituiert in 2-Stellung, oder 1,2,4-Triazol-5-yl oder 3-Alkyloxycarbonyl-1,2,4-triazol-5-yl, substituiert in 1-Stellung durch

a) einen Alkylrest der seinerseits substituiert ist durch einen Alkyloxy-, Alkylthio-, Phenyl-, Formyl-, Carbamoyl-, Alkylcarbamoyl-, Dialkylcarbamoyl-, Hydroxyalkylcarbamoyl- (dessen Alkylteil 2 bis 4 Kohlenstoffatome enthält), Acyl-, Alkyloxycarbonyl- oder Thiazolidinyl-2-Rest,

b) einen Allylrest; 2,3-Dihydroxypropyl; 1,3-Dihydroxy-propyl-2; 2-Formyl-2-hydroxy-ethyl; 3-Formyloxy-2-hydroxy-propyl; 2,3-Bisformyloxy-propyl oder 1,3-Bisformyloxy-propyl-2,

c) einen Alkylrest mit 2 bis 4 Kohlenstoffatomen, substituiert durch einen Hydroxy-, Carbamoyloxy-, Acyloxy-(dessen Acylteil substituiert sein kann durch einen Amino-, Alkylamino- oder Dialkylaminorest), Alkylsulfinyl-, Alkylsulfonyl-, Amino-, Alkylamino-, Dialkylamino-, Sulfoamino-, Alkylsulfonylamino-, Sulfamoylamino-, Acylamino-(dessen Acylteil gegebenenfalls substituiert ist durch Hydroxy, Amino, Alkylamino oder Dialkylamino), Alkyloxycarbonylamino-, Ureido-, Alkylureido-, Dialkylureido-Rest,

d) einen Rest, entsprechend einer der oben unter α-4-d) erwähnten allgemeinen Formeln oder

e) einen Alkylrest mit 2 bis 5 Kohlenstoffatomen, substituiert durch einen Alkyloxyimino- oder Hydroxyimino-Rest, oder auch

2) 5,6-Dioxo-4-hydroxyalkylcarbamoylalkyl-1,4,5,6-tetrahydro-1,2,4-triazin-3-yl, dessen Hydroxyalkylteil 2 bis 4 Kohlenstoffatome enthält, oder auch

3) 1-Alkyl-5,6-dioxo-1,4,5,6-tetrahydro-1,2,4-triazin-3-yl, substituiert in 4-Stellung, 1-Alkyl-5,6-dioxo-1,2,5,6-tetrahydro-1,2,4-triazin-3-yl, substituiert in 2-Stellung, 2-Alkyl-5,6-dioxo-1,2,5,6-tetrahydro-1,2,4-triazin-3-yl, substituiert in 1-Stellung, oder 4-Alkyl-5,6-dioxo-1,4,5,6-tetrahydro-1,2,4-triazin-3-yl, substituiert in 1-Stellung,
durch einen Formylalkylrest oder durch einen Rest der allgemeinen Formel

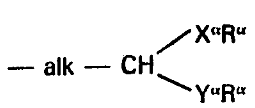

worin alk, $X^\alpha$, $Y^\alpha$ und $R^\alpha$ wie oben definiert sind, und das Symbol $R°$ bedeutet ein Wasserstoffatom, einen Alkylrest, Vinylrest, Cyanomethylrest oder einen Rest der allgemeinen Formel

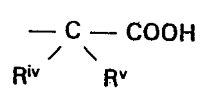

wie oben definiert,
und das Symbol R' bedeutet ein Wasserstoffatom oder einen auf enzymatischem Wege leicht entfernbaren Rest der allgemeinen Formel

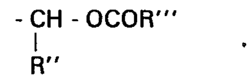

worin R'' ein Wasserstoffatom oder einen Alkylrest bedeutet und R''' einen Alkylrest oder den Cyclohexylrest bedeutet, wobei die oben erwähnten Alkyl- oder Acylreste und -teile (wenn nichts anderes angegeben ist) gerade oder verzweigt sind und 1 bis 4 Kohlenstoffatome enthalten,
in seinen syn- oder anti-Formen und E- oder Z-Formen und ihre Gemische,
sowie seine Additionssalze mit Säuren, seine Metallsalze und seine Additionssalze mit Stickstoffbasen.

2. Verfahren zur Herstellung eines Produktes gemäss Anspruch 1, für das der Rest R keinen Substituenten der allgemeinen Formel -alk-CH(OH)OR$^\alpha$ enthält, dadurch gekennzeichnet, dass man eine Säure der allgemeinen Formel

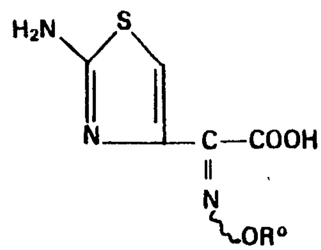

worin R° wie in Anspruch 1 definiert ist und dessen Aminfunktion vorher geschützt wurde (sowie das Oxim, falls R° Wasserstoff oder die Säurefunktion bedeutet, falls R° eine Carboxygruppe enthält), oder ein reaktives Derivat dieser Säure auf ein 7-Aminocephalosporin der allgemeinen Formel

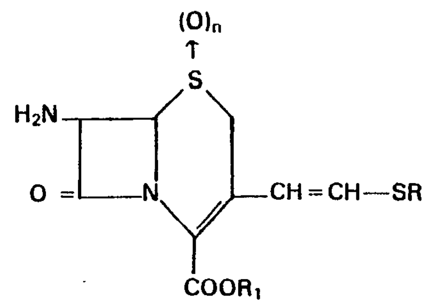

einwirken lässt, worin R wie in Anspruch 1 definiert ist, $R_1$ ein Wasserstoffatom, einen Rest der allgemeinen Formel

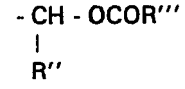

wie in Anspruch 1 definiert, oder einen leicht entfernbaren Schutzrest, bedeutet, und n für 0 oder 1 steht, dass man dann, wenn n = 1, das erhaltene Oxid reduziert und die Schutzreste entfernt, und dann gegebenenfalls das erhaltene Produkt in ein Additionssalz mit einer Säure, in ein Metallsalz oder in ein Additionssalz mit einer Stickstoffbase überführt.

3. Verfahren gemäss Anspruch 2, dadurch gekennzeichnet, dass man eine Säure der allgemeinen Formel

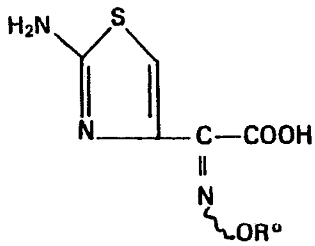

verwendet, worin R° wie in Anspruch 1 definiert ist, und deren Aminfunktion vorher geschützt ist durch eine tert.-Butoxycarbonyl-, 2,2,2,-Trichlor-ethoxycarbonyl-, Trichloracetyl-, Chloracetyl-, Trityl-, Dibenzyl-, Benzyl-, Benzyloxycarbonyl-, p-Nitrobenzyloxycarbonyl-, p-Methoxybenzyloxycarbonyl-, Formyl- oder Trifluoracetylgruppe.

4. Verfahren gemäss Anspruch 2, dadurch gekennzeichnet, dass man eine Säure der allgemeinen Formel

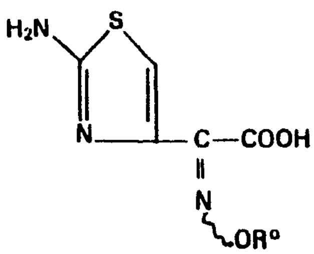

verwendet, worin R° eine Trityl-, Tetrahydropyranyl- oder 2-Methoxy-propyl-2-Schutzgruppe der Oximfunktion ist.

5. Verfahren gemäss Anspruch 2, dadurch gekennzeichnet, dass man eine Säure der allgemeinen Formel

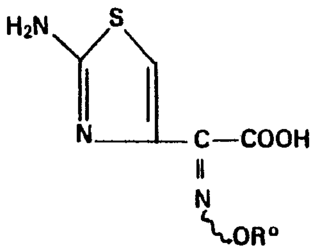

verwendet, worin R° einen Carboxyrest enthält, der durch eine Methoxymethyl-, tert.-Butyl-, Benzhydryl-, Benzyl-, Nitrobenzyl- oder p-Methoxybenzylgruppe geschützt ist.

6. Verfahren gemäss Anspruch 2, dadurch gekennzeichnet, dass man ein 7-Aminocephalosporin verwendet, worin der leicht entfernbare, durch $R_1$ wiedergegebene Rest ausgewählt ist unter den Methoxymethyl-, tert.-Butyl-, Benzhydryl-, Nitrobenzyl- und p-Methoxybenzylresten.

7. Verfahren gemäss Anspruch 2, dadurch gekennzeichnet, dass man ein reaktives Derivat der Säure der allgemeinen Formel

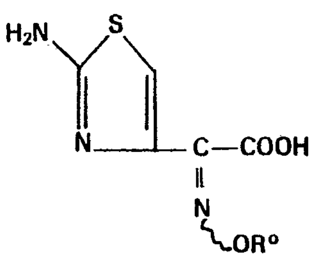

verwendet, worin R° wie in Anspruch 1 definiert ist, ausgewählt unter dem Anhydrid, einem gemischten Anhydrid, einem reaktiven Ester und einem Halogenid dieser Säure.

8. Verfahren zur Herstellung eines Produktes gemäss Anspruch 1, für das der Rest R keinen Substituenten der allgemeinen Formel -alk-CH(OH)OR$^\alpha$ enthält, dadurch gekennzeichnet, dass man ein Thiol (frei oder im Zustand des Alkali- oder Erdalkalisalzes) der allgemeinen Formel

$$R — SH$$

(worin der Substituent von R, der oben definiert ist, im Zustand des Acetals geschützt ist [definiert durch die allgemeinen Formeln

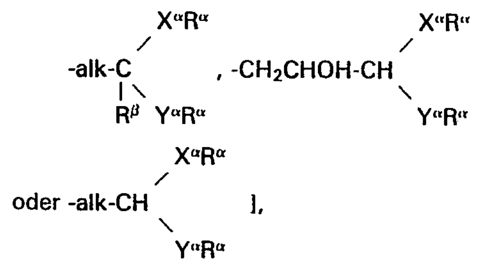

, oder ],

wenn man ein Produkt gemäss Anspruch 1 erhalten will, für das R einen Formyl- oder einen Acylalkylrest enthält) auf ein Cephalosporinderivat (oder gegebenenfalls auf ein Gemisch der Isomeren) der allgemeinen Formel

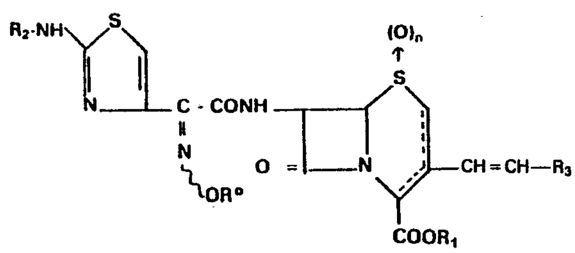

einwirken lässt, worin R° wie in Anspruch 1 definiert ist, $R_1$ und n wie in Anspruch 2 definiert sind, wenn n = 0, dieses Derivat unter der Bicycloocten-2- oder -3-Form vorliegt,
wenn n = 1, dieses Derivat in Bycycloocten-2-Form vorliegt,
der Substituent am Kohlenstoffatom in 3-Stellung des Bicyclooctens die E- oder Z-Stereoisomerie aufweist, $R_2$ ein Wasserstoffatom oder einen Schutzrest des Aminorestes bedeutet und $R_3$ ein Halogenatom, ausgewählt unter Chlor, Brom oder Jod, oder einen Rest der allgemeinen Formeln

$$-O-SO_2R'_3$$

oder $$-O-CO\ R''_3$$

bedeutet, worin $R'_3$ ein gerader oder verzweigter Alkylrest mit 1 bis 4 Kohlenstoffatomen ist, Trifluormethyl, Trichlormethyl oder Phenyl, nichtsubstituiert oder substituiert durch ein Halogenatom oder durch einen Alkyl- oder Nitrorest, und
$R''_3$ wie $R'_3$ definiert ist oder einen Acylmethyl-, 2-Acylethyl-, 2-Acyl-propyl-, Alkyloxycarbonylmethyl-, 2-Alkyloxycarbonylethyl- oder 2-Alkyloxycarbonylpropyl-Rest bedeutet, dann, falls n = 1, man das erhaltene Oxid reduziert, die Schutzreste gegebenenfalls entfernt und dass man gegebenenfalls das erhaltene Produkt in ein Additionssalz mit einer Säure, ein Metallsalz oder ein Additionssalz mit einer Stickstoffbase überführt.

9. Verfahren gemäss Anspruch 8, dadurch gekennzeichnet, dass die Reaktion in Gegenwart eines Pyridins oder einer tertiären organischen Base durchgeführt wird.

10. Verfahren zur Herstellung eines Produktes gemäss Anspruch 1, für das R keinen Substituenten der allgemeinen Formel -alk-CH(OH)OR$^\alpha$ enthält, dadurch gekennzeichnet, dass man einen Thiolester der allgemeinen Formel

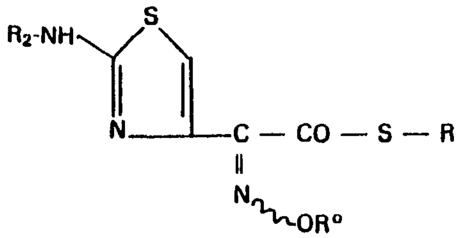

worin R° und $R_2$ wie in Anspruch 8 definiert sind und R wie vorstehend definiert ist [wobei, falls R° ein Wasserstoffatom ist, das Oxim geschützt sein kann, falls R° einen Carboxyrest enthält, der letztere geschützt ist, falls R einen Amino-, Alkylamino-, Formyl- oder Acylalkyl-Substituenten enthält, dieser geschützt ist, und falls R einen Hydroxy- oder Carboxy-Substituenten enthält, dieser frei oder geschützt ist] auf ein 7-Aminocephalosporin der allgemeinen Formel

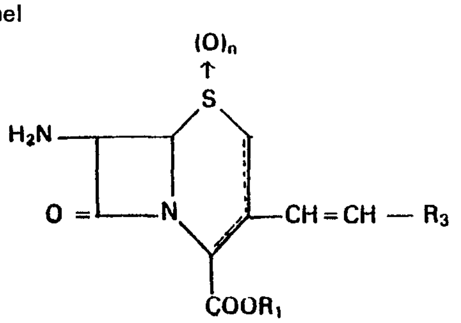

einwirken lässt, worin $R_1$, $R_3$ und n wie in Anspruch 8 definiert sind, und das dieselbe Stereoisomerie besitzt wie das in Anspruch 8 definierte Produkt, dann, falls n = 1, das erhaltene Oxid reduziert, gegebenenfalls die Schutzreste entfernt und gegebenenfalls das erhaltene Produkt in ein Additionssalz mit einer Säure, in ein Metallsalz oder in ein Additionssalz mit einer Stickstoffbase überführt.

11. Verfahren gemäss Anspruch 10, dadurch gekennzeichnet, dass man einen Thiolester verwendet, worin gegebenenfalls der Rest R geschützt ist durch einen Aminoschutzrest, wie in Anspruch 3 definiert, oder in Acetalform durch einen Rest der allgemeinen Formel

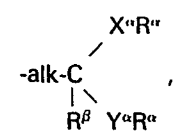 , 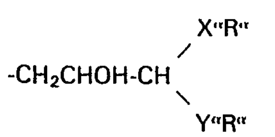 oder

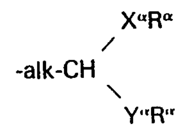

12. Verfahren zur Herstellung eines Produktes gemäss Anspruch 1, für das der Rest R keinen Substituenten der allgemeinen Formel -alk-CH(OH)OR$^\alpha$ enthält und R° anders als Vinyl ist, dadurch gekennzeichnet, dass man einen Thioharnstoff der allgemeinen Formel

$$R_2NH\ CS\ NH_2$$

worin $R_2$ ein Wasserstoffatom oder ein Amin-Schutzrest ist, auf ein Produkt der allgemeinen Formel

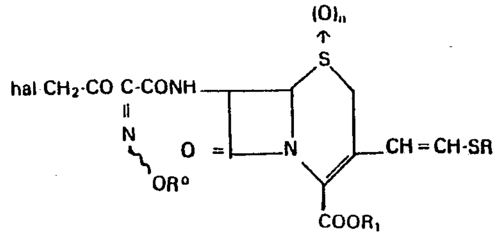

einwirken lässt, worin R°, $R_1$ und n wie in Anspruch 2 definiert sind (mit Ausnahme der Bedeutung eines Vinylrestes für R°), R wie vorstehend definiert ist und hal ein Chlor- oder Brom-atom bedeutet, dass man dann, wenn n = 1, das erhaltene Oxid reduziert, gegebenenfalls die Schutzreste entfernt und gegebenenfalls das erhaltene Produkt in ein Additionssalz mit einer Säure, in ein Metallsalz oder ein Additionssalz mit einer Stickstoffbase überführt.

13. Verfahren gemäss Anspruch 12, dadurch gekennzeichnet, dass man einen Thioharnstoff zur Reaktion bringt, in dessen Formel der Rest $R_2$ ausgewählt ist unter den tert.-Butoxycarbonyl-, 2,2,2-Trichlor-ethoxycarbonyl-, Trityl-, Dibenzyl-, Benzyl-, Benzyloxycarbonyl-, p-Nitrobenzyloxycarbonyl-, p-Methoxybenzyloxycarbonyl-, Formyl- oder Trifluoracetyl-Resten.

14. Verfahren zur Herstellung eines Produktes gemäss Anspruch 1, für das der Rest R einen 5,6-Dioxo-1,4,5,6-tetrahydro-1,2,4-triazin-3-yl-Rest, substituiert in 1- oder 4-Stellung, 5,6-Dioxo-1,2,5,6-tetrahydro-1,2,4-triazin-3-yl-Rest, substituiert in 2-Stellung, 1,3,4-Triazol-5-yl- oder 2-Alkyloxycarbonyl-1,3,4-triazol-5-yl- oder 1,2,4-Triazol-5-yl- oder 3-Alkyloxycarbonyl-1,2,4-triazol-5-yl-Rest, substituiert in 1-Stellung durch einen Alkylrest mit 2 bis 4 Kohlenstoffatomen, der seinerseits durch eine Carbamoyloxy, oder Acyloxygruppe substituiert ist (dessen Acylteil gegebenenfalls durch einen Amino-, Alkylamino- oder Dialkylaminorest substituiert ist), dadurch gekennzeichnet, dass man einen Alkohol der allgemeinen Formel

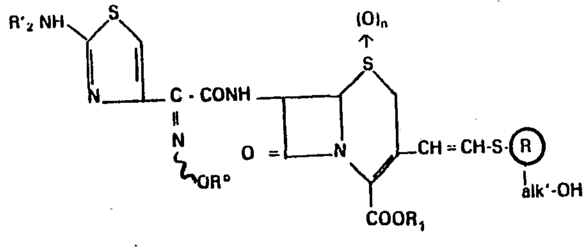

(worin $R^o$, $R_1$ und n wie in Anspruch 2 definiert sind, $R'_2$ ein Amin-Schutzrest ist und -(R)-alk'-OH einen 5,6-Dioxo-1(oder 4)-hydroxyalkyl-1,4,5,6-tetrahydro-1,2,4-triazin-3-yl-Rest, 5,6-Dioxo-2-hydroxyalkyl-1,2,5,6-tetrahydro-1,2,4-triazin-3-yl-Rest oder auch 1-Hydroxyalkyl-1,3,4-triazol-5-yl-Rest, 2-Alkyloxycarbonyl-1-hydroxyalkyl-1,3,4-triazol-5-yl-Rest, 1-Hydroxyalkyl-1,2,4-triazol-5-yl-Rest oder 3-Alkyloxycarbonyl-1-hydroxyalkyl-1,2,4-triazol-5-yl-Rest bedeutet) carbamoyliert oder verestert nach jeder Methode, die zur Erzielung eines Carbamats oder eines Esters aus einem Alkohol bekannt ist, ohne den Rest des Moleküls zu berühren, dass man gegebenenfalls das erhaltene Oxid reduziert, die Schutzgruppen entfernt und dann gegebenenfalls das erhaltene Produkt in ein Additionssalz mit einer Säure, in ein Metallsalz oder in ein Additionssalz mit einer Stickstoffbase überführt.

15. Verfahren gemäss Anspruch 14, dadurch gekennzeichnet, dass man ein Cephalosporin verwendet, in dessen Formel $R'_2$ ausgewählt ist unter den in Anspruch 3 genannten Amino-Schutzgruppen.

16. Verfahren zur Herstellung eines Produktes gemäss Anspruch 1, wofür R einen Rest 5,6-Dioxo-1,4,5-tetrahydro-1,2,4-triazin-3-yl, substituiert in 1(oder 4)-Stellung, 5,6-Dioxo-1,2,5,6-tetrahydro-1,2,4-triazin-3-yl, substituiert in 2-Stellung, 1,3,4-Triazol-5-yl, 2-Alkoxycarbonyl-1,3,4-triazol-5-yl, 1,2,4-Triazol-5-yl oder 3-Alkoxycarbonyl-1,2,4-triazol-5-yl, substituiert in 1-Stellung, durch einen Alkylrest mit 2 bis 4 Kohlenstoffatomen, der seinerseits substituiert ist durch eine Sulfoamino-, Alkylsulfoamino-, Sulfamoylamino-, Acylamino-(dessen Acylteil gegebenenfalls substituiert ist durch Hydroxy, Amino, Alkylamino oder Dialkylamino), Alkyloxycarbonylamino-, Ureido-, Alkylureido- oder Dialkylureido-Gruppe, oder einen 1,3,4-Thiadiazol-5-yl-Rest, substituiert durch einen Acylamino- oder Acylaminoalkylrest, bedeutet oder einen 1,3,4-Oxadiazol-5-yl-Rest, substituiert durch einen Acylaminoalkylrest, bedeutet oder einen Tetrazolyl-5-Rest, substituiert in 1-Stellung durch einen Alkylrest mit 2 bis 4 Kohlenstoffatomen, substituiert durch eine Acylamino-, Sulfamoylamino-, Sulfoamino-, Ureido-, Alkylureido- oder Dialkylureidogruppe, bedeutet, dadurch gekennzeichnet, dass man ein Amin der allgemeinen Formel

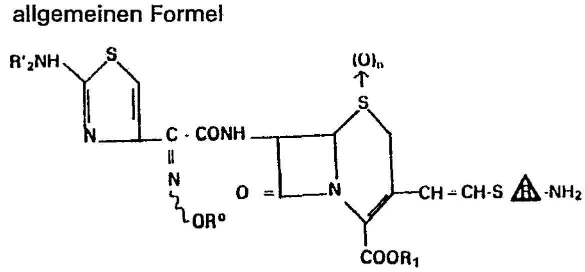

worin $R^o$, $R_1$ und n wie in Anspruch 2 definiert sind, $R'_2$ ein Amin-Schutzrest ist und -(R)-$NH_2$ einen 5,6-Dioxo-1,4,5,6-tetrahydro-1,2,4-triazin-3-yl-Rest, substituiert in 1(oder 4)-Stellung, 5,6-Dioxo-1,2,-5,6-tetrahydro-1,2,4-triazin-3-yl-Rest, substituiert in 2-Stellung, 1,3,4-Triazol-5-yl-, 2-Alkyloxycarbonyl-1,3,4-triazol-5-yl-, 1,2,4-Triazol-5-yl- oder 3-Alkyloxycarbonyl-1,2,4-triazol-5-yl-Rest, substituiert in 1-Stellung, durch einen Aminoalkylrest (enthaltend 2 bis 4 Kohlenstoffatome) oder einen 1,3,4-Thiadiazol-5-yl-Rest, substituiert durch einen Amino- oder Aminoalkylrest, oder einen 1,3,4-Oxadiazol-5-yl-Rest, substituiert durch einen Aminoalkylrest, oder einen Tetrazolyl-5-Rest, substituiert in 1-Stellung durch einen Aminoalkylrest (enthaltend 2 bis 4 Kohlenstoffatome), bedeutet, nach jeder Methode behandelt, die zur Bildung einer Amid-, Sulfamid-, Carbamat- oder Harnstoff-Funktion bekannt ist, ohne dass der Rest des Moleküls berührt wird, dass man dann, wenn n = 1, das erhaltene Oxid reduziert, die Schutzgruppen entfernt und dann gegebenenfalls das erhaltene Produkt in ein Additionssalz mit einer Säure, in ein Metallsalz oder in ein Additionssalz mit einer Stickstoffbase überführt.

17. Verfahren gemäss Anspruch 16, dadurch gekennzeichnet, dass man ein Cephalosporin verwendet, in dessen Formel $R'_2$ wie in Anspruch 15 definiert ist.

18. Verfahren gemäss Anspruch 16, dadurch gekennzeichnet, dass, falls man ein in Anspruch 16 definiertes Produkt herstellen will, wofür R einen Hydroxy- oder Amino-Substituenten enthält, diese Funktionen in dem verwendeten Reaktionsteilnehmer geschützt sind.

19. Verfahren zur Herstellung eines Produktes gemäss Anspruch 1, wofür R einen 5,6-Dioxo-1,4,-5,6-tetrahydro-1,2,4-triazin-3-yl-Rest, substituiert in 1(oder 4)-Stellung, 5,6-Dioxo-1,2,5,6-tetrahydro-1,2,4-triazin-3-yl-Rest, substituiert in 2-Stellung, 1,3,4-Triazol-5-yl-, 2-Alkyloxycarbonyl-1,3,4-triazol-5-yl-, 1,2,4-Triazol-5-yl- oder 3-Alkyloxycarbonyl-1,2,4-triazol-5-yl-Rest, substituiert in 1-Stellung, durch einen 2-Thiazolidinylalkylrest, durch einen Rest -alk-CH(OH)$OR^\alpha$ oder durch einen Hydroxyiminoalkyl- oder Alkyloxyiminoalkyl-Rest, dessen Iminoalkylteil 2 bis 5 Kohlenstoffatome enthält, oder auch einen Tetrazolyl-5-Rest, substituiert in 1-Stellung durch einen Hydroxyiminoalkyl- oder Alkyloxyiminoalkylrest, dessen Iminoalkylteil 2 bis 5 Kohlenstoffatome enthält, bedeutet, dadurch gekennzeichnet, dass man eine Additionsreaktion von Cysteamin, einem Alkohol, Hydroxylamin oder einem Alkyloxyamin auf einem Aldehyd der allgemeinen Formel

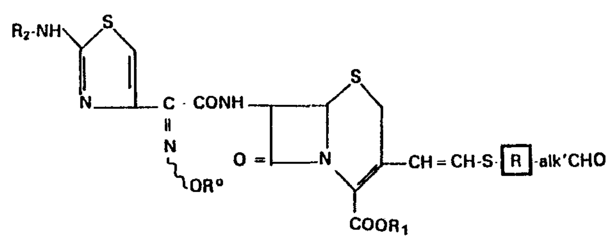

bewirkt, worin $R^o$, $R_1$ und $R_2$ wie in Anspruch 8 definiert sind, -[R]-alk'CHO einen 5,6-Dioxo-1(oder 4)-formylalkyl-1,4,5,6-tetrahydro-1,2,4-triazin-3-yl-Rest, 5,6-Dioxo-2-formylalkyl-1,2,5,6-tetrahydro-1,2,4-triazin-3-yl-Rest oder auch 1-Formylalkyl-1,3,4-triazol-5-yl, 2-Alkyloxycarbonyl-1-formylalkyl-1,3,4-triazol-5-yl, 1-Formylalkyl-1,2,4-triazol-5-yl, 3-Alkyloxycarbonyl-1-formylalkyl-1,2,4-triazol-5-yl oder 1-Formylalkyl-tetrazolyl-5 bedeutet, nach jeder Methode, die zur Bildung derartiger Additionsderivate der Carbonylfunktionen bekannt ist, dass man dann die Schutzgruppen entfernt und gegebenenfalls das erhaltene Produkt in ein Additionssalz mit einer Säure, in ein Metallsalz oder ein Salz mit einer Stickstoffbase überführt.

20. Verfahren zur Herstellung gemäss Anspruch 19, dadurch gekennzeichnet, dass man ein Cephalosporin verwendet, in dessen Formel $R_2$ eine Schutzgruppe ist, ausgewählt unter den Amino-Schutzgruppen, die in Anspruch 3 angegeben sind.

21. Verfahren zur Herstellung eines Produktes gemäss Anspruch 1, für das R' einen Rest der allgemeinen Formel

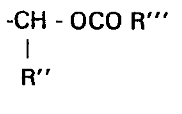

wie in Anspruch 1 definiert, bedeutet, dadurch gekennzeichnet, dass man ein Produkt gemäss Anspruch 1, für das R' ein Wasserstoffatom ist, nach jeder Methode verestert, die an sich zur Herstellung eines Esters aus einer Säure bekannt ist, ohne den Rest des Moleküls zu berühren, und dass man dann gegebenenfalls das erhaltene Produkt in ein Additionssalz mit einer Säure überführt.

22. Verfahren zur Herstellung eines Produktes gemäss Anspruch 1, für das R und R° wie in Anspruch 1 in α/ definiert sind [wobei R keinen Substituenten der allgemeinen Formel -alk-CH(OH)OR$^{\alpha}$ enthält], dadurch gekennzeichnet, dass man ein Produkt der allgemeinen Formel

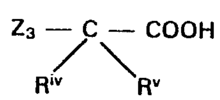

worin R$^{iv}$ und R$^{v}$ wie in Anspruch 1 definiert sind und $Z_3$ ein Halogenatom oder einen Sulfat- oder Sulfonatrest bedeutet und dessen Säurefunktion geschützt ist, auf ein Cephalosporin der allgemeinen Formel

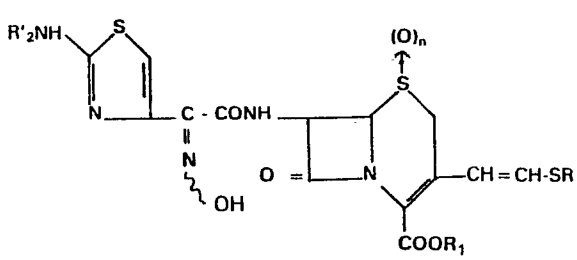

einwirken lässt, worin R wie vorstehend definiert ist (wobei, falls R einen Amino- oder Alkylamino-Substituenten enthält, dieser geschützt ist, und falls es einen Formylrest enthält, dieser frei oder geschützt ist), $R_1$ und n wie in Anspruch 2 definiert sind und $R'_2$ wie in Anspruch 14 definiert ist, dass man dann gegebenenfalls das erhaltene Oxid reduziert, die Schutzgruppen entfernt und gegebenenfalls das erhaltene Produkt in ein Additionssalz mit einer Säure, in ein Metallsalz oder in ein Additionssalz mit einer Stickstoffbase überführt.

23. Verfahren gemäss Anspruch 22, dadurch gekennzeichnet, dass man ein Cephalosporin verwendet, worin $R'_2$ ein Amino-Schutzrest ist, ausgewählt unter den in Anspruch 3 genannten Resten.

24. Verfahren zur Herstellung eines Produktes gemäss Anspruch 1, wofür R keinen Substituenten der allgemeinen Formel -alk CH(OH)OR$^{\alpha}$ enthält, dadurch gekennzeichnet, dass man ein Thiol (frei oder im Zustand des Alkali- oder Erdalkalisalzes), wie in Anspruch 8 definiert, auf ein Cephalosporinderivat (oder ein Gemisch der Isomeren) der allgemeinen Formel

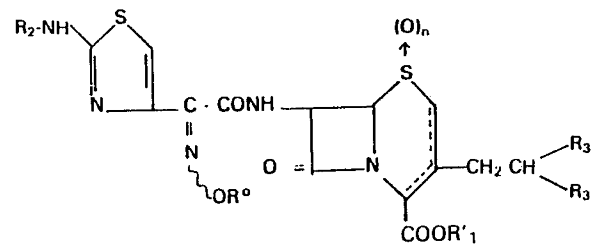

einwirken lässt, worin R° einen Alkyl-, Vinyl- oder Cyanomethylrest, einen Oxim-Schutzrest oder einen Rest der allgemeinen Formel

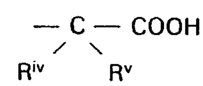

bedeutet (worin die Reste R$^{iv}$ und R$^{v}$, welche identisch oder verschieden sind, Wasserstoffatome oder Alkylreste bedeuten oder zusammen einen Alkylenrest mit 2 oder 3 Kohlenstoffatomen bilden), der geschützt ist, $R'_1$ wie $R_1$ in Anspruch 2 definiert ist, mit Ausnahme der Bedeutung eines Wasserstoffatoms, $R_2$ wie in Anspruch 8 definiert ist, n 0 oder 1 bedeutet (wenn n = 0, liegt dieses Derivat in Bicycloocten-2- oder -3-Form vor, wenn n = 1, liegt dieses Derivat in Bicycloocten-2-Form vor) und $R_3$ ein Chlor- oder Bromatom bedeutet, dass man dann, wenn n = 1, das erhaltene Produkt reduziert, die Schutzgruppen entfernt und das erhaltene Produkt gegebenenfalls in ein Additionssalz mit einer Säure, in ein Metallsalz oder ein Additionssalz mit einer Stickstoffbase überführt.

25. Arzneimittel, dadurch gekennzeichnet, dass es wenigstens ein Produkt gemäss Anspruch 1 in reinem Zustand oder in Form einer pharmazeutischen Zusammensetzung zusammen mit einem oder mehreren verträglichen und pharmazeutisch annehmbaren Verdünnungs- oder Hilfsmitteln enthält.

**Patentanspruch für den Vertragsstaat:** AT

Verfahren zur Herstellung eines 3-Thiovinyl-Cephalosporins der allgemeinen Formel

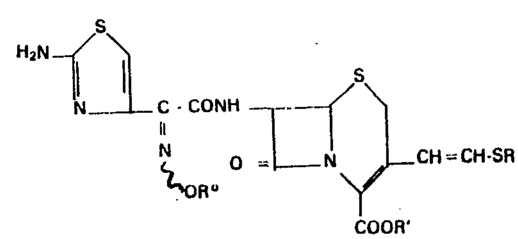

in welcher

α/ das Symbol R ausgewählt ist aus folgenden Bedeutungen:

1) Alkyl, 2-L-Amino-2-carboxy-äthyl, Phenyl,

2) 2-Pyridyl, 3-Pyridyl oder 4-Pyridyl und ihren N-Oxiden,

3) 2-Pyrimidinyl, 3-Pyridazinyl, substituiert in 6-Stellung (durch einen Alkyl-, Methoxy-, Amino- oder Acylaminorest) und gegebenenfalls N-oxidiert, oder Tetrazolo[4,5-b]-6-pyridazinyl,

4) 5,6-Dioxo-1,4,5,6-tetrahydro-1,2,4-triazin-3-yl, substituiert in 4-Stellung; 1,3,4-Triazol-5-yl oder 2-Alkyloxycarbonyl-1,3,4-triazol-5-yl, substituiert in 1-Stellung

a) durch einen Alkylrest, gegebenenfalls substituiert durch einen Alkyloxy-, Alkylthio-, Phenyl-, Formyl-, Carbamoyl-, Alkylcarbamoyl-, Dialkylcarbamoyl-, Acylrest; Alkyloxycarbonyl oder 2-Thiazolidinyl,

b) durch einen Allyl-, 2,3-Dihydroxypropyl-, 1,3-Dihydroxy-2-propyl-, 2-Formyl-2-hydroxyäthyl-, 3-Formyloxy-2-hydroxypropyl-, 2,3-bis-Formyloxypropyl oder 1,3-bis-Formyloxy-2-propylrest,

c) durch einen Alkylrest mit 2 bis 4 Kohlenstoffatomen, substituiert durch einen Hydroxy-, Carbamoyloxy-, Acyloxy- (dessen Acylteil durch einen Amino-, Alkylamino- oder Dialkylaminorest substituiert sein kann), Alkylsulfinyl-, Alkylsulfonyl-, Amino-, Alkylamino-, Dialkylamino-, Sulfoamino-, Alkylsulfonylamino-, Sulfamoylamino-, Acylamino (dessen Acylteil gegebenenfalls substituiert ist durch Hydroxy, Amino, Alkylamino oder Dialkylamino), Alkyloxycarbonylamino-, Ureido-, Alkylureido-, Dialkylureidorest,

d) durch einen Rest, entsprechend einer der allgemeinen Formeln:

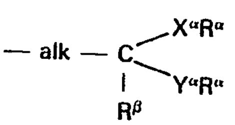

oder 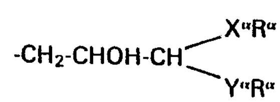

oder 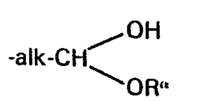

in welchen alk ein Alkylrest mit 1 bis 4 Kohlenstoffatomen ist, $X^{\alpha}$ und $Y^{\alpha}$ gleich sind und Sauerstoff- oder Schwefelatome darstellen und $R^{\alpha}$ einen Alkylrest darstellt, oder $X^{\alpha}$ und $Y^{\alpha}$ gleich oder voneinander verschieden sind und Sauerstoff- oder Schwefelatome darstellen und die Reste $R^{\alpha}$ gemeinsam einen Alkylenrest mit 2 oder 3 Kohlenstoffatomen bilden und $R^{\beta}$ ein Wasserstoffatom oder einen Alkylrest mit 1 bis 3 Kohlenstoffatomen darstellt,

e) durch einen Alkylrest mit 2 bis 5 Kohlenstoffatomen, substituiert durch einen Alkyloxyimino- oder Hydroxyiminorest,

5) 1,4-Dialkyl-5,6-dioxo-1,4,5,6-tetrahydro-1,2,4-triazin-3-yl; 1-Alkyl-5,6-dioxo-1,4,5,6-tetrahydro-1,2,4-triazin-3-yl; 2-Alkyl-5,6-dioxo-1,2,5,6-tetrahydro-1,2,4-triazin-3-yl;

6) 1,3,4-Triazol-5-yl; 1,2,3-Triazol-5-yl oder 1-Alkyl-1,2,4-triazol-5-yl, gegebenenfalls substituiert in 3-Stellung durch Alkyloxycarbonyl,

7) a) 1,3,4-Thiadiazol-5-yl, gegebenenfalls substituiert durch einen Alkyl-, Trifluormethyl-, Alkyloxy-, Alkylthio-, Hydroxyalkylthio-, dessen Alkylteil 2 bis 4 Kohlenstoffatome enthält, Alkylsulfonyl-, Hydroxy-, Hydroxyalkyl-, Carboxy-, Carboxyalkyl-, Amino-, Alkylamino-, Dialkylamino-, Aminoalkyl-, Alkylaminoalkyl-, Dialkylaminoalkyl-, Acylamino- oder Acylaminoalkylrest,

b) 1,2,4-Thiadiazol-5-yl, substituiert durch einen Alkyl- oder Alkyloxyrest,

8) a) 1,3,4-Oxadiazol-5-yl, gegebenenfalls substituiert durch einen Alkyl-, Trifluormethyl-, Phenyl-, Aminoalkyl-, Alkylaminoalkyl-, Dialkylaminoalkyl- oder Acylaminoalkylrest,

b) 2-Oxazolyl- oder 4-Alkyl-2-oxazolylrest,

9) 5-Tetrazolyl, gegebenenfalls substituiert in 1-Stellung durch

a) einen Alkylrest, gegebenenfalls substituiert durch Alkyloxy, Sulfo, Carboxy, Formyl oder Sulfamoyl

b) einen Alkylrest mit 2 bis 4 Kohlenstoffatomen, substituiert durch Hydroxy, Amino, Alkylamino, Dialkylamino, Acylamino, Carboxyalkylamino, Sulfamoylamino, Sulfoamino, Ureido, Alkylureido oder Dialkylureido,

c) einen Alkylrest mit 2 bis 5 Kohlenstoffatomen, substituiert durch Hydroxyimino oder Alkyloxyimino,

d) einen Phenyl-, 2,3,-Dihydroxypropyl-, 1,3-Dihydroxy-2-propyl-, 2-Formyl-2-hydroxyäthyl-, 3-Formyloxy-2-hydroxypropyl-, 2,3-bis-Formyloxypropyl oder 1,3-bis-Formyloxy-2-propylrest oder

e) einen Rest der allgemeinen Formeln

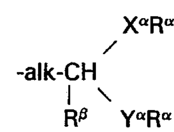 oder 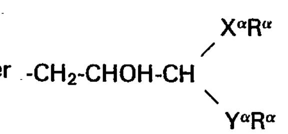

worin $R^{\beta}$ ein Wasserstoffatom ist und $X^{\alpha}$, $Y^{\alpha}$ und $R^{\alpha}$ die oben unter (4d) angegebene Bedeutung haben, und das Symbol $R^{\circ}$ einen Carboxyalkylrest der allgemeinen Formel

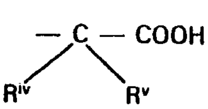

worin die Reste $R^{iv}$ und $R^{v}$, die gleich oder voneinander verschieden sind, Wasserstoffatome oder Alkylreste darstellen oder zusammen einen Alkylenrest mit 2 oder 3 Kohlenstoffatomen bilden, darstellt, oder

β/ R ausgewählt ist aus

1) 5,6-Dioxo-1,4,5,6-tetrahydro-1,2,4-triazin-3-yl, substituiert in 1-Stellung, 5,6-Dioxo-1,2,5,6-tetrahydro-1,2,4-triazin-3-yl, substituiert in 2-Stellung, 1,2,4-Triazol-5-yl oder 3-Alkyloxycarbonyl-1,2,4-triazol-5-yl, substituiert in 1-Stellung

a) durch einen Alkylrest, substituiert durch einen Alkyloxy-, Alkylthio-, Phenyl-, Formyl-, Carbamoyl-, Alkylcarbamoyl-, Dialkylcarbamoyl-, Hydroxyalkylcarbamoyl- (dessen Alkylteil 2 bis 4 Kohlenstoffatome enthält), Acyl-, Alkyloxycarbonyl- oder 2--Thiazolidinylrest,

b) durch einen Allyl-, 2,3-Dihydroxypropyl-, 1,3--Dihydroxy-2-propyl-, 2-Formyl-2-hydroxyäthyl-, 3--Formyloxy-2-hydroxypropyl-, 2,3-bis-Formyloxypropyl- oder 1,3-bis-Formyloxy-2-propylrest,

c) durch einen Alkylrest mit 2 bis 4 Kohlenstoffatomen, substituiert durch einen Hydroxy-, Carbamoyloxy-, Acyloxy- (dessen Acylteil durch einen Amino-, Alkylamino- oder Dialkylaminorest substituiert sein kann), Alkylsulfinyl-, Alkylsulfonyl-, Amino-, Alkylamino-, Dialkylamino-, Sulfoamino-, Alkylsulfonylamino-, Sulfamoylamino-, Acylamino- (dessen Acylteil gegebenenfalls durch Hydroxy, Amino, Alkylamino oder Dialkylamino substituiert ist), Alkyloxycarbonylamino-, Ureido-, Alkylureido-, Dialkylureidorest,

d) durch einen Rest entsprechend einer der oben unter $\alpha$-4-d) erwähnten allgemeinen Formeln oder

e) durch einen Alkylrest mit 2 bis 5 Kohlenstoffatomen, substituiert durch einen Alkyloxyimino- oder Hydroxyiminorest, oder

2) 5,6-Dioxo-4-hydroxyalkylcarbamoylalkyl-1,-4,5,6-tetrahydro-1,2,4-triazin-3-yl, dessen Hydroxyalkylteil 2 bis 4 Kohlenstoffatome enthält, oder

3) 1-Alkyl-5,6-dioxo-1,4,5,6-tetrahydro-1,2,4--triazin-3-yl, substituiert in 4-Stellung, 1-Alkyl-5,6--dioxo-1,2,5,6-tetrahydro-1,2,4-triazin-3-yl, substituiert in 2-Stellung, 2-Alkyl-5,6-dioxo-1,2,5,6-tetrahydro-1,2,4-triazin-3-yl, substituiert in 1-Stellung, oder 4-Alkyl-5,6-dioxo-1,4,5,6-tetrahydro--1,2,4-triazin-3-yl, substituiert in 1-Stellung durch einen Formylalkylrest oder durch einen Rest der allgemeinen Formel

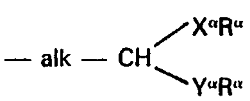

in welcher alk, $X^\alpha$, $Y^\alpha$ und $R^\alpha$ die obige Bedeutung haben und das Symbol $R^\circ$ ein Wasserstoffatom, einen Alkyl-, Vinyl- oder Cyanomethylrest oder einen Rest der allgemeinen Formel

$$-\underset{R^{iv}\ \ R^{v}}{C}-COOH$$

wie zuvor definiert, darstellt und das Symbol R' ein Wasserstoffatom oder einen leicht auf enzymatischem Wege entfernbaren Rest der allgemeinen Formel

$$-\underset{R''}{CH}\ OCOR'''$$

darstellt, worin R'' ein Wasserstoffatom oder einen Alkylrest bedeutet und R''' einen Alkylrest oder den Cyclohexylrest darstellt, wobei es selbstverständlich ist, dass die oben erwähnten Alkyl- oder Acylreste und -teile (ohne besondere Erwähnung) geradkettig oder verzweigt sind und 1 bis 4 Kohlenstoffatome enthalten,

in ihren syn- oder anti- und E- oder Z-Formen und deren Mischungen, sowie seiner Additionssalze mit Säuren, seiner Metallsalze und seiner Additionssalze mit Stickstoffbasen, dadurch gekennzeichnet, dass man zur Herstellung einer Verbindung, in deren Formel R keinen Substituenten der allgemeinen Formel -alk-CH(OH)OR$^\alpha$ enthält,

A - eine Säure allgemeinen Formel

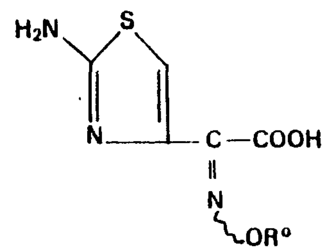

worin $R^\circ$ die obige Bedeutung hat und deren Aminfunktion zuvor geschützt wurde (ebenso wie das Oxim, wenn $R^\circ$ Wasserstoff darstellt, oder die Säurefunktion, wenn $R^\circ$ eine Carboxygruppe enthält) oder ein reaktionsfähiges Derivat dieser Säure auf ein 7-Aminocephalosporin der allgemeinen Formel

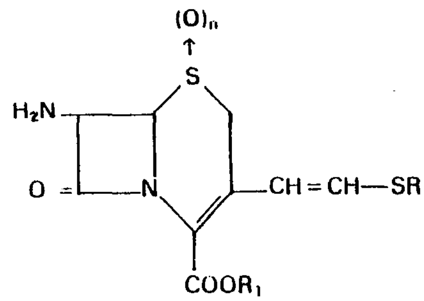

worin R die obige Bedeutung hat, $R_1$ ein Wasserstoffatom, einen Rest der allgemeinen Formel

$$-\underset{R''}{CH}-OCOR'''$$

wie oben definiert oder eine leicht entfernbare Schutzgruppe darstellt und n für 0 oder 1 steht, einwirken lässt, oder

B - einen Thiol (frei oder im Zustand des Alkali- oder Erdalkalisalzes) der allgemeinen Formel

R - SH

(worin der Substituent R, der die obige Bedeutung hat, im Acetalzustand geschützt ist [ausgedrückt durch die allgemeinen Formeln

$$-alk-\overset{X^\alpha R^\alpha}{\underset{R^\beta\ Y^\alpha R^\alpha}{C}}\ ,\ -CH_2CHOH-CH\begin{matrix}X^\alpha R^\alpha\\Y^\alpha R^\alpha\end{matrix}\quad \text{oder}$$

$$-alk-CH\begin{matrix}X^\alpha R^\alpha\\Y^\alpha R^\alpha\end{matrix}\ ],$$

wenn man eine Verbindung erhalten will, worin R einen Formyl- oder Acylalkylrest enthält) auf ein Cephalosporinderivat (oder gegebenenfalls auf eine Mischung von Isomeren) der allgemeinen Formel

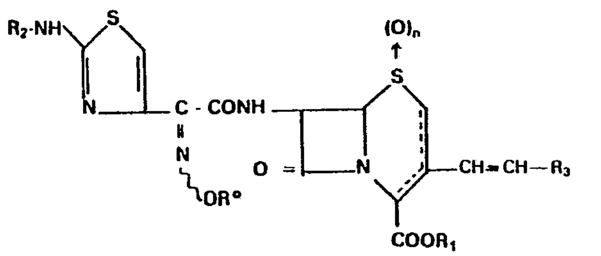

worin R°, $R_1$ und n die oben unter A engegebene Bedeutung haben,
wenn n = 0, dieses Derivat in 2- oder 3-Bicyclo-octen-Form vorliegt,
wenn n = 1, dieses Derivat in 2-Bycycloocten-Form vorliegt,
der Substituent am Kohlenstoffatom in 3-Stellung des Bicyclooctens die E- oder Z-Stereoisomerie aufweist, $R_2$ ein Wasserstoffatom oder eine Aminoschutzgruppe darstellt und $R_3$ ein Halogenatom, ausgewählt aus Chlor, Brom oder Jod, oder einen Rest der allgemeinen Formeln

$$-O-SO_2R'_3$$

oder $$-O-CO\ R''_3$$

darstellt, worin $R'_3$ ein geradkettiger oder verzweigter Alkylrest mit 1 bis 4 Kohlenstoffatomen, ein Trifluormethyl, Trichlormethylrest oder Phenylrest, gegebenenfalls substituiert durch ein Wasserstoffatom oder durch einen Alkyl- oder Nitrorest, ist, und $R''_3$ die Bedeutung von $R'_3$ hat oder einen Acylmethyl-, 2-Acyläthyl-, 2-Acylpropyl-, Alkyloxycarbonylmethyl-, 2-Alkyloxycarbonyläthyl- oder 2--Alkyloxycarbonyl-propylrest darstellt, einwirken lässt oder
C - einen Thiolester der allgemeinen Formel

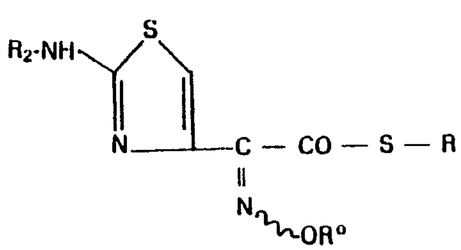

worin R, R° und $R_2$ die oben unter B angegebene Bedeutung haben [selbstverständlich kann, wenn R° ein Wasserstoffatom ist, das Oxim geschützt sein, ist, wenn R° einen Carboxyrest enthält, dieser geschützt, ist, wenn R einen Amino-, Alkylamino-, Formyl- oder Acylalkylrest enthält, dieser geschützt und ist, wenn R einen Hydroxy- oder Carboxysubstituenten enthält, dieser frei oder geschützt], auf ein 7-Amino-cephalosporin der allgemeinen Formel

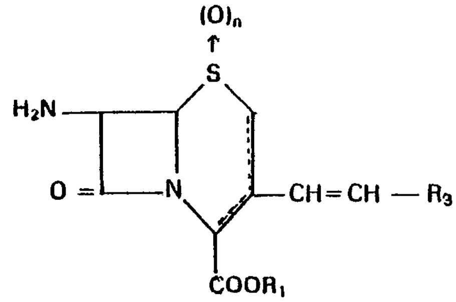

worin $R_1$, $R_3$ und n die oben unter B angegebene Bedeutung haben und welches die gleiche Stereoisomerie wie die oben unter B angegebene Verbindung hat, einwirken lässt oder
D - einen Thiol (frei oder im Zustand des Alkali- oder Erdalkalisalzes), wie unter B angegeben, auf ein Cephalosporinderivat (oder eine Isomerenmischung) der allgemeinen Formel

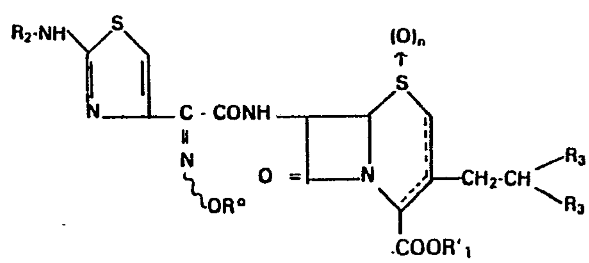

worin R° einen Alkyl-, Vinyl- oder Cyanomethylrest, eine Oximschutzgruppe oder einen geschützten Rest der allgemeinen Formel

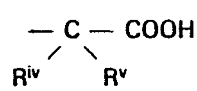

(worin die Reste $R^{iv}$ und $R^{v}$, die gleich oder voneinander verschieden sind, Wasserstoffatome oder Alkylreste darstellen oder gemeinsam einen Alkylenrest mit 2 oder 3 Kohlenstoffatomen bilden) darstellt,
$R'_1$ die Bedeutung von $R_1$ unter A, mit Ausnahme eines Wasserstoffatoms, hat, $R_2$ die unter B angegebene Bedeutung hat,
n für 0 oder 1 steht (wenn n = 0, weist dieses Derivat 2- oder 3-Bicyclooctenform auf, wenn n = 1, weist dieses Derivat 2-Bicyclooctenform auf) und $R_3$ ein Chlor- oder Bromatom darstellt, einwirken lässt, dass man dann die erhaltene Verbindung, wenn n = 1, reduziert, die Schutzgruppen entfernt und die erhaltene Verbindung gegebenenfalls in ein Säureadditionssalz, ein Metallsalz oder ein Salz mit einer Stickstoffbase überführt, oder
E - wenn R° die obige Bedeutung mit Ausnahme von Vinyl hat, einen Thioharnstoff der allgemeinen Formel

$$R_2NH\ CS\ NH_2$$

worin $R_2$ ein Wasserstoffatom oder eine Aminschutzgruppe ist, auf eine Verbindung der allgemeinen Formel

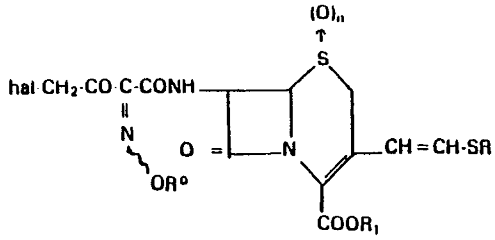

worin R°, $R_1$ und n die oben unter A angegebene Bedeutung haben (mit Ausnahme eines Vinylrests für R°), R die obige Bedeutung hat und hal ein Chlor- oder Bromatom darstellt, einwirken lässt und dass man dann, wenn n = 1, das erhaltene Oxid reduziert, dass man vorhandene Schutzgruppen entfernt und gegebenenfalls die erhaltene Verbindung in ein Säureadditionssalz, ein Metallsalz oder ein Additionssalz mit einer Stickstoffbase überführt, oder

F - zur Herstellung einer Verbindung der Formel, worin R und R° die unter α/ angegebene Bedeutung haben [selbstverständlich enthält R keinen Substituenten der allgemeinen Formel -alk-CH(OH)OR$^{\alpha}$], erfindungsgemäss ein Cephalosporin der allgemeinen Formel

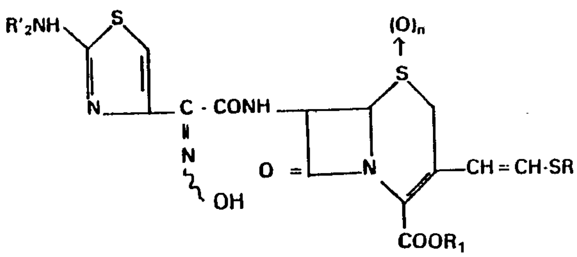

herstellt, worin R die obige Bedeutung hat (selbstverständlich ist ein in R enthaltener Amino- oder Alkylaminosubstituent geschützt und ein enthaltener Formylrest frei oder geschützt), $R_1$ und n die unter A angegebene Bedeutung haben und $R'_2$ eine Aminschutzgruppe ist, auf welches man dann eine Verbindung der allgemeinen Formel

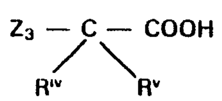

in welcher $R^{iv}$ und $R^{v}$ die unter α/ angegebene Bedeutung haben und $Z_3$ ein Halogenatom oder einen Sulfat- oder Sulfonatrest darstellt und deren Säurefunktion geschützt ist, einwirken lässt und dass man dann zutreffendenfalls das erhaltene Oxid reduziert, die Schutzgruppen entfernt und die erhaltene Verbindung gegebenenfalls in ein Säureadditionssalz, in ein Metallsalz oder ein Additionssalz mit einer Stickstoffbase überführt, oder

G - zur Herstellung einer Verbindung, in deren Formel der Rest R einen 5,6-Dioxo-1,4,5,6-tetrahydro-1,2,4-triazin-3-yl-rest, substituiert in 1- oder 4-Stellung, einen 5,6-Dioxo-1,2,5,6-tetrahydro-1,2,4-triazin-3-yl-rest, substituiert in 2-Stellung, einen 1,3,4-Triazol-5-yl-, 2-Alkyloxycarbonyl-1,3,4-triazol-5-yl- oder 1,2,4-Triazol-5-yl- oder 3-Alkyloxycarbonyl-1,2,4-triazol-5-yl-rest, substituiert in 1-Stellung durch einen Alkylrest mit 2 bis 4 Kohlenstoffatomen, selbst substituiert durch eine Carbamoyloxy- oder Acyloxygruppe (deren Acylteil gegebenenfalls durch einen Amino-, Alkylamino- oder Dialkylaminorest substituiert ist) erfindungsgemäss einen Alkohol der allgemeinen Formel

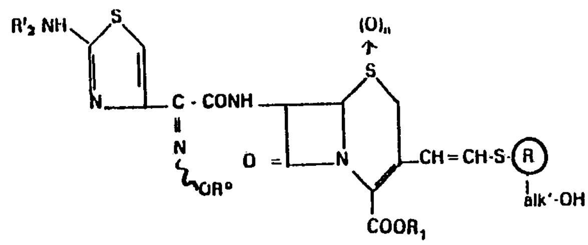

(in welcher R°, $R_1$ und n die unter A angegebene Bedeutung haben, $R'_2$ eine Aminschutzrest ist und -Ⓡ-alk'-OH einen 5,6-Dioxo-1 (oder -4)-hydroxyalkyl-1,4,5,6-tetrahydro-1,2,4-triazin-3-yl-, 5,6-Dioxo-2-hydroxyalkyl-1,2,5,6-tetrahydro-1,2,4-triazin-3-yl- oder 1-Hydroxyalkyl-1,3,4-triazol-5-yl-, 2-Alkylcarbonyl-1-hydroxyalkyl-1,3,4-triazol-5-yl-, 1-Hydroxyalkyl-1,2,4-triazol-5-yl- oder 3-Alkyloxycarbonyl-1-hydroxyalkyl-1,2,4-triazol-5-yl-rest darstellt) herstellt, dass man diesen Alkohol dann in den Ester oder das entsprechende Carbamat überführt, gegebenenfalls das erhaltene Oxid reduziert, die Schutzgruppen entfernt und dann gegebenenfalls die erhaltene Verbindung in ein Säureadditionssalz, ein Metallsalz oder ein Additionssalz mit einer Stickstoffbase überführt oder

H - zur Herstellung einer Verbindung in deren Formel R einen 5,6-Dioxo-1,4,5,6-tetrahydro-1,2,4-triazin-3-yl-rest, substituiert in 1 (oder 4)-Stellung, 5,6-Dioxo-1,2,5,6-tetrahydro-1,2,4-triazin-3-yl-rest, substituiert in 2-Stellung, 1,3,4-Triazol-5-yl-, 2-Alkyloxycarbonyl-1,3,4-triazol-5-yl-, 1,2,4-Triazol-5-yl oder 3-Alkyloxycarbonyl-1,2,4-triazol-5-ylrest, substituiert in 1-Stellung durch einen Alkylrest mit 2 bis 4 Kohlenstoffatomen, selbst substituiert durch eine Sulfoamino-, Alkylsulfonylamino-, Sulfamoylamino- Acylamino- (deren Acylteil gegebenenfalls durch Hydroxy, Amino, Alkylamino oder Dialkylamino substituiert ist), Alkyloxycarbonylamino-, Ureido-, Alkylureido- oder Dialkylureidogruppe, darstellt oder einen 1,3,4-Thiadiazol-5-yl-rest, substituiert durch einen Acylamino- oder Acylaminoalkylrest darstellt oder einen 1,3,4-Oxadiazol-5-yl-rest, substituiert durch einen Acylaminoalkylrest darstellt, oder einen 5-Tetrazolylrest, substituiert in 1-Stellung durch einen Alkylrest mit 2 bis 4 Kohlenstoffatomen, substituiert durch eine Acylamino-, Sulfamoylamino-, Sulfoamino-, Ureido-, Alkylureido- oder Dialkylureidogruppe, darstellt, erfindungsgemäss ein Amin der allgemeinen Formel

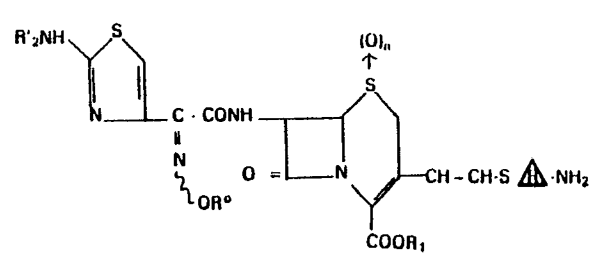

worin R°, $R_1$ und n die unter A angegebene Bedeutung haben, $R'_2$ eine Aminschutzgruppe ist und $-\triangle\!\!\!\!R-NH_2$ einen 5,6-Dioxo-1,4,5,6-tetrahydro-1,2,4-triazin-3-yl-rest, substituiert in 1- (oder 4)-Stellung, 5,6-Dioxo-1,2,5,6-tetrahydro-1,2,4-triazin-3-yl-rest, substituiert in 2-Stellung, 1,3,4-Triazol-5-yl-, 2-Alkyloxycarbonyl-1,3,4-triazol-5-yl-, 1,2,4-Triazol-5-yl- oder 3-Alkyloxycarbonyl-1,2,4-triazol-5-yl-rest, substituiert in 1-Stellung durch einen Aminoalkylrest (der 2 bis 4 Kohlenstoffatome enthält) oder einen 1,3,4-Thiadiazol-5-yl-rest, substituiert durch einen Amino- oder Aminoalkylrest, oder einen 1,3,4-Oxadiazol-5-yl-rest, substituiert durch einen Aminoalkylrest, oder einen 5-Tetrazolylrest, substituiert in 1-Stellung durch einen Aminoalkylrest (der 2 bis 4 Kohlenstoffatome enthält), herstellt, dann das Amin in das entsprechende Amid, Sulfamid, Carbamat oder Carbamid umwandelt, dann, wenn n = 1, das erhaltene Oxid reduziert, die Schutzgruppen entfernt, dann gegebenenfalls die erhaltene Verbindung in ein Säureadditionssalz, ein Metallsalz oder ein Additionssalz mit einer Stickstoffbase überführt oder

I - zur Herstellung einer Verbindung, in deren Formel R einen 5,6-Dioxo-1,4,5,6-tetrahydro-1,2,4-triazin-3-yl-rest, substituiert in 1- (oder 4)-Stellung, 5,6-Dioxo-1,2,5,6-tetrahydro-1,2,4-triazin-3-yl-rest, substituiert in 2-Stellung, oder 1,3,4-Triazol-5-yl-, 2-Alkyloxycarbonyl-1,3,4-triazol-5-yl-, 1,2,4-Triazol-5-yl- oder 3-Alkyloxycarbonyl-1,2,4-triazol-5-yl-rest, substituiert in 1-Stellung durch einen 2-Thiazolidinyl-alkylrest, durch einen Rest -alk-CH(OH)-$OR^{\alpha}$ oder durch einen Hydroxyiminoalkyl- oder Alkyloxyiminoalkylrest, deren Iminoalkylteil 2 bis 5 Kohlenstoffatome enthält, darstellt oder einen 5-Tetrazolylrest, substituiert in 1-Stellung durch einen Hydroxyiminoalkyl- oder Alkyloxyiminoalkylrest, deren Iminoalkylteil 2 bis 5 Kohlenstoffatome enthält, darstellt, erfindungsgemäss einen Aldehyd der allgemeinen Formel

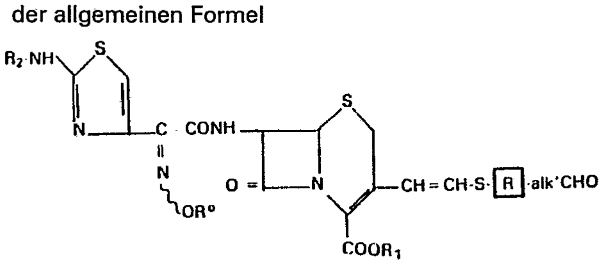

worin R°, $R_1$ und $R_2$ die unter B angegebene Bedeutung haben, -$\boxed{R}$-alk'CHO einen 5,6-Dioxo-1-(oder 4)-formylalkyl-1,4,5,6-tetrahydro-1,2,4-triazin-3-yl-, 5,6-Dioxo-2-formylalkyl-1,2,5,6-tetrahydro-1,2,4-triazin-3-yl- oder 1-Formylalkyl-1,3,4-triazol-5-yl-, 2-Alkyloxycarbonyl-1-formylalkyl-1,3,4-triazol-5-yl-, 1-Formylalkyl-1,2,4-triazol-5-yl-, 3-Alkyloxycarbonyl-1-formylalkyl-1,2,4-triazol-5-yl- oder 1-Formylalkyl-5-tetrazolyl-rest darstellt, herstellt, dann an dem Aldehyd eine Additionsreaktion mit Cysteamin, einem Alkohol, Hydroxyamin oder einem Alkyloxyamin ausführt, dann die Schutzgruppen entfernt und gegebenenfalls die erhaltene Verbindung in ein Säureadditionssalz, ein Metallsalz oder ein Additionssalz mit einer Stickstoffbase überführt oder

J - zur Herstellung einer Verbindung, in deren Formel R' einen Rest der allgemeinen Formel

$$-\underset{\displaystyle R''}{\underset{|}{CH}}-OCO\,R'''$$

darstellt, erfindungsgemäss die entsprechende Säure herstellt, worin R' ein Wasserstoffatom ist, diese Säure dann verestert und die erhaltene Verbindung gegebenenfalls in ein Säureadditionssalz überführt.

**Claims for the Contracting States:**
BE, CH, DE, FR, GB, IT, LI, LU, NL, SE

1. A 3-thiovinylcephalosporin of the general formula:

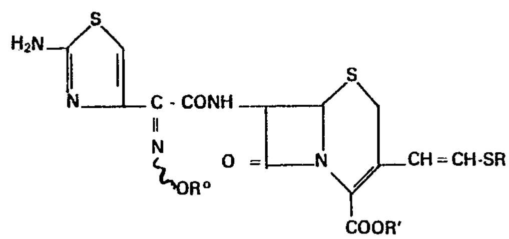

in which:

α. the symbol R is chosen amongst the following meanings:

1) alkyl, L-2-amino-2-carboxyethyl or phenyl,

2) pyrid-2-yl, pyrid-3-yl or pyrid-4-yl and their N-oxides,

3) pyrimidin-2-yl, pyridazin-3-yl substituted in the 6-position (by an alkyl, methoxy, amino or acylamino radical) and optionally N-oxidised, or tetrazolo[4,5-b]-pyridazin-6-yl,

4) 5,6-dioxo-1,4,5,6-tetrahydro-1,2,4-triazin-3-yl substituted in the 4-position, or 1,3,4-triazol-5-yl or 2-alkoxycarbonyl-1,3,4-triazol-5-yl substituted in the 1-position, by

a) an alkyl radical which is unsubstituted or substituted by an alkoxy, alkylthio, phenyl, formyl, carbamoyl, alkylcarbamoyl, dialkylcarbamoyl, acyl, alkoxycarbonyl, or thiazolidin-2-yl radical,

b) an allyl, 2,3-dihydroxypropyl, 1,3-dihydroxy-prop-2-yl, 2-formyl-2-hydroxyethyl, 3-formyloxy-2-hydroxypropyl, 2,3-bis-formyloxypropyl or 1,3-bis-formyloxyprop-2-yl radical,

c) an alkyl radical containing 2 to 4 carbon atoms, which is substituted by a hydroxyl or carbamoyloxy radical, an acyloxy radical (the acyl part of which can be substituted by an amino, alkylamino or dialkylamino radical), an alkylsulphinyl, alkylsulphonyl, amino, alkylamino, dialkylamino, sulphoamino, alkylsulphonylamino or sulphamoylamino radical, an acylamino radical (the acyl part of which is optionally substituted by hydroxyl, amino, alkylamino or dialkylamino) or an alkoxycarbonylamino, ureido, alkylureido or dialkylureido radical,

d) a radical corresponding to one of the general formulae:

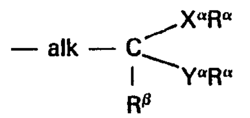

or 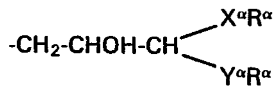

or 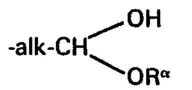

in which alk is an alkylene radical containing 1 to 4 carbon atoms, $X^\alpha$ and $Y^\alpha$ are identical and represent oxygen or sulphur atoms and $R^\alpha$ represents an alkyl radical, or alternatively $X^\alpha$ and $Y^\alpha$ are identical or diffrent and represent oxygen or sulphur atoms and the radicals $R^\alpha$ together form an alkylene radical containing 2 or 3 carbon atoms, and $R^\beta$ represent a hydrogen atom or an alkyl radical containing 1 to 3 carbon atoms, or

e) an alkyl radical containing 2 to 5 carbon atoms, which is substituted by an alkoxyimino or hydroxyimino radical,

5) 1,4-dialkyl-5,6-dioxo-1,4,5,6-tetrahydro-1,2,4-triazin-3-yl, 1-alkyl-5,6-dioxo-1,4,5,6-tetrahydro-1,2,4-triazin-3-yl or 2-alkyl-5,6-dioxo-1,2,5,-6-tetrahydro-1,2,4-triazin-3-yl,

6) 1,3,4-triazol-5-yl, 1,2,3-triazol-5-yl or 1-alkyl-1,2,4-triazol-5-yl which is unsubstituted or substituted in the 3-position by alkoxycarbonyl,

7) a) 1,3,4-thiadiazol-5-yl which is unsubstituted or substituted by an alkyl, trifluoromethyl, alkoxy or alkylthio radical, a hydroxyalkylthio radical, the alkyl part of which contains 2 to 4 carbon atoms, or an alkylsulphonyl, hydroxyl, hydroxyalkyl, carboxyl, carboxyalkyl, amino, alkylamino, dialkylamino, aminoalkyl, alkylaminoalkyl, dialkylaminoalkyl, acylamino or acylaminoalkyl radical, or

b) 1,2,4-thiadiazol-5-yl substituted by an alkyl or alkoxy radical,

8) a) 1,3,4-oxadiazol-5-yl which is unsubstituted or substituted by an alkyl, trifluoromethyl, phenyl, aminoalkyl, alkylaminoalkyl, dialkylaminoalkyl or acylaminoalkyl radical, or

b) oxazol-2-yl or 4-alkyloxazol-2-yl, and

9) tetrazol-5-yl which is unsubstituted or substituted in the 1-position by

a) an alkyl radical which is unsubstituted or substituted by alkoxy, sulpho, carboxyl, formyl or sulphamoyl,

b) an alkyl radical containing 2 to 4 carbon atoms, which is substituted by hydroxyl, amino, alkylamino, dialkylamino, acylamino, carboxyalkylamino, sulphamoylamino, sulphoamino, ureido, alkylureido or dialkylureido,

c) an alkyl radical containing 2 to 5 carbon atoms, which is substituted by hydroxyimino or alkoxyimino,

d) a phenyl, 2,3-dihydroxypropyl, 1,3-dihydroxyprop-2-yl, 2-formyl-2-hydroxyethyl, 3-formyloxy-2-hydroxypropyl, 2,3-bis-formyloxypropyl or 1,3-bis-formyloxyprop-2-yl radical, or

e) a radical of the general formulae:

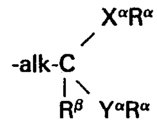 or 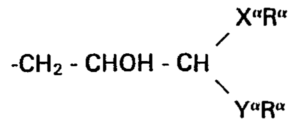

in which $R^\beta$ is a hydrogen atom and $X^\alpha$, $Y^\alpha$ and $R^\alpha$ are defined as under (4d) above, and the symbol $R^\circ$ represents a carboxyalkyl radical of the general formula:

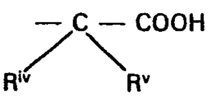

in which the radicals $R^{iv}$ and $R^{v}$, which are identical or different, represent hydrogen atoms or alkyl radicals, or together form an alkylene radical containing 2 or 3 carbon atoms, or alternatively

β. the symbol R is chosen from amongst:

1. 5,6-dioxo-1,4,5,6-tetrahydro-1,2,4-triazin-3-yl substituted in the 1-position, 5,6-dioxo-1,2,5,6-tetrahydro-1,2,4-triazin-3-yl substituted in the 2-position, or 1,2,4-triazol-5-yl or 3-alkoxycarbonyl-1,2,4-triazol-5-yl substituted in the 1-position by

a) an alkyl radical which is itself substituted by an alkoxy, alkylthio, phenyl, formyl, carbamoyl, alkylcarbamoyl or dialkylcarbamoyl radical, a hydroxyalkylcarbamoyl radical (the alkyl part of which contains 2 to 4 carbon atoms) or an acyl, alkoxycarbonyl or thiazolidin-2-yl radical,

b) an allyl, 2,3-dihydroxypropyl, 1,3-dihydroxyprop-2-yl, 2-formyl-2-hydroxyethyl, 2-formyloxy-2-hydroxypropyl, 2,3-bis-formyloxypropyl or 1,3-bis-formyloxyprop-2-yl radical,

c) an alkyl radical containing 2 to 4 carbon atoms, which is substituted by a hydroxyl or carbamoyloxy radical, an acyloxy radical (the acyl part of which can be substituted by an amino, alkylamino or dialkylamino radical), an alkylsulphinyl, alkylsulphonyl, amino, alkylamino, dialkylamino, sulphoamino, alkylsulphonylamino or sulphamoylamino radical, an acylamino radical (the acyl part of which is optionally substituted by hydroxyl, amino, alkylamino or dialkylamino or an alkoxycarbonylamino, ureido, alkylureido or dialkylureido radical,

d) a radical corresponding to one of the general formulae mentioned above under α.4d), or

e) an alkyl radical containing 2 to 5 carbon atoms, which is substituted by an alkoxyimino or hydroxyimino radical, or alternatively,

2. 5,6-dioxo-4-hydroxyalkylcarbamoylalkyl-1,-4,5,6-tetrahydro-1,2,4-triazin-3-yl of which the hydroxyalkyl portion contains 2 to 4 carbon atoms, or alternatively

3. 1-alkyl-5,6-dioxo-1,4,5,6-tetrahydro-1,2,4-triazin-3-yl substituted in the 4-position, 1-alkyl-5,6-dioxo-1,2,5,6-tetrahydro-1,2,4-triazin-3-yl substituted in the 2-position, 2-alkyl-5,6-dioxo-1,2,5,6-tetrahydro-1,2,4-triazin-3-yl substituted in the 1-position, or 4-alkyl-5,6-dioxo-1,4,5,6-tetrahydro-1,2,4-triazin-3-yl substituted in the 1-position by a formylalkyl radical or by a radical of the general formula:

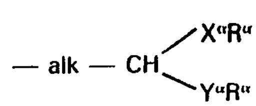

in which alk, $X^{\alpha}$, $Y^{\alpha}$ and $R^{\alpha}$ are defined as above, and the symbol $R^{o}$ represents a hydrogen atom, an alkyl, vinyl or cyanomethyl radical or a radical of the general formula:

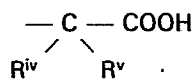

as defined above,
and the symbol R′ represents a hydrogen atom or a radical which can easily be removed by an enzymatic method, of the general formula

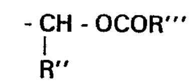

in which R″ represents a hydrogen atom or an alkyl radical and R‴ represents an alkyl radical or the cyclohexyl radical, it being understood that the alkyl or acyl radicals and portions mentioned above are linear or branched (unless otherwise mentioned) and contain 1 to 4 carbon atoms, in its syn or anti forms and E or Z forms, and mixtures thereof and also its addition salts with acids, its metal salts and its addition salts with nitrogen-containing bases.

2. A process for the preparation of a product according to claim 1 in which the radical R does not contain a substituent of the general formula -alk-CH(OH)$OR^{\alpha}$, which comprises reacting an acid of the general formula:

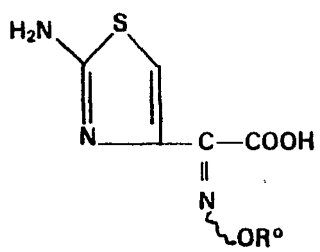

in which $R^{o}$ is defined according to claim 1 and in which the amine group has been protected beforehand (and also the oxime if $R^{o}$ represents hydrogen, or the acid group if $R^{o}$ contains a carboxyl group), or a reactive derivative of this acid, with a 7-aminocephalosporin of the general formula:

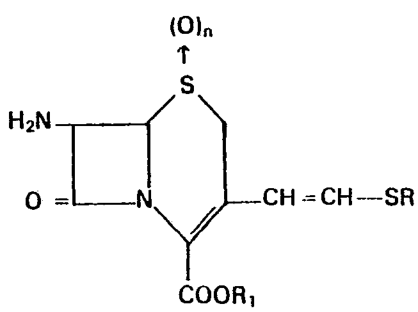

in which R is defined according to claim 1, $R_1$ represents a hydrogenm atom, a radical of the general formula:

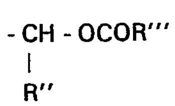

as defined in claim 1, or a protective radical which can easily be removed, and n represents 0 or 1, and then, if n = 1, reducing the oxide obtained and removing the protective radicals, and then, if appropriate, converting the product obtained to an addition salt salt with an acid, to a metal salt or to an addition salt with a nitrogen-containing base.

3. A process according to claim 2, which comprises using an acid of the general formula:

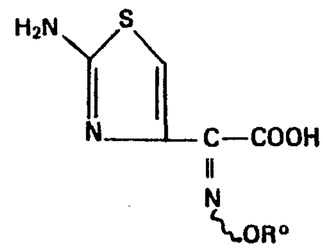

in which $R^{o}$ is defined according to claim 1 and in which the amine group has been protected beforehand by a t-butoxycarbonyl, 2,2,2-trichloroethoxycarbonyl, trichloroacetyl, chloroacetyl, trityl, dibenzyl, benzyl, benzyloxycarbonyl, p-nitrobenzyloxycarbonyl, p-methoxybenzyloxycarbonyl, formyl or trifluoroacetyl group.

4. A process according to claim 2, which comprises using an acid of the general formula:

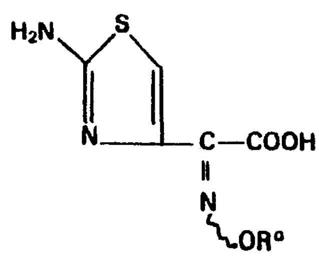

in which $R^{o}$ is a trityl, tetrahydropyranyl or 2-methoxy-prop-2-yl protective group of the oxime group.

5. A process according to claim 2, which comprises using an acid of the general formula:

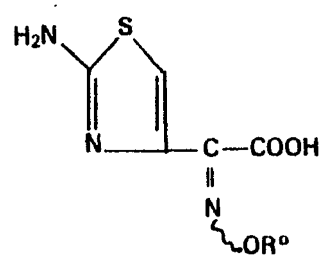

in which $R^{o}$ contains a carboxyl radical protected by a methoxymethyl, t-butyl, benzhydryl benzyl, nitrobenzyl or p-methoxybenzyl group.

6. A process according to claim 2, with comprises using a 7-aminocephalosporin in which the radical which can easily be removed, represented by $R_1$, is chosen from amongst methoxymethyl, t-butyl, benzhydryl, nitrobenzyl and p-methoxybenzyl radicals.

7. A process according to claim 2, which comprises using a reactive derivative of the acid of the general formula:

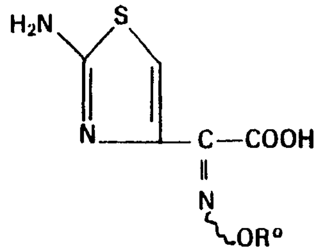

in which R° is defined according to claim 1, which reactive derivative is chosen from amongst the anhydride, a mixed anhydride, a reactive ester and a halide of this acid.

8. A process for the preparation of a product according to claim 1 in which the radical R does not contain a substituent of the general formula -alk-CH-(OH)OR$^{\alpha}$, which comprises reacting a thiol (free or in the form of an alkali metal salt or alkaline earth metal salt) of the general formula:

R - SH

(in which the substituent of R, which is defined as above, is protected in the form of an acetal (defined by the general formulae:

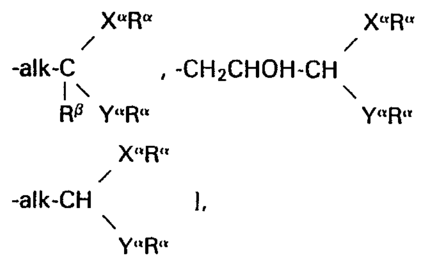

or

if it is desired to obtain a product according to claim 1 in which R contains a formyl or acylalkyl radical, with a cephalosporin derivative (or, if necessary, with a mixture of the isomers) of the general formula:

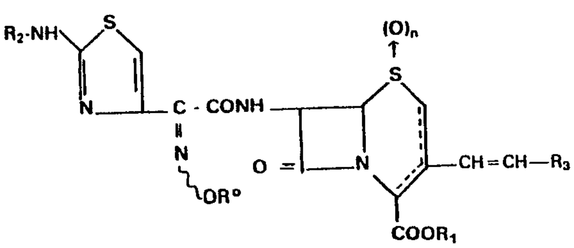

in which, R° being defined according to claim 1 and $R_1$ and n being defined according to claim 2, if n = 0, this derivative is in the form of a bicyclooct-2-ene or bicyclooct-3-ene, and if n = 1, this derivative is in the form of a bicyclooct-2-ene, the substituent on the carbon atom in the 3-position of the bicyclooctene exhibits E/Z stereoisomerism, $R_2$ represents a hydrogen atom or a protective radical of the amino radical, and $R_3$ represents a halogen atom chosen from amongst chlorine, bromine or iodine, or a radical of the general formulae:

$-O-SO_2R'_3$

or $-O-CO\ R''_3$

in which $R'_3$ is a linear or branched alkyl radical containing 1 to 4 carbon atoms, a trifluoromethyl or trichloromethyl radical or a phenyl radical which is unsubstituted or substituted by a halogen atom or by an alkyl or nitro radical, and $R''_3$ is defined in the same way as $R'_3$ or represents an acylmethyl, 2-acylethyl, 2-acylpropyl, alkoxycarbonylmethyl, 2-alkoxycarbonylethyl or 2-alkoxycarbonylpropyl radical, and then, if n = 1, reducing the oxide obtained and, if necessary, removing the protective radicals, and, if appropriate, converting the product obtained to an addition salt with an acid, a metal salt or an addition salt with a nitrogen-containing base.

9. A process according to claim 8, wherein the reaction is carried out in the presence of a pyridine or a tertiary organic base.

10. A process for the preparation of a product according to claim 1 in which the radical R does not contain a substituent of the general formula -alk-CH-(OH)OR$^{\alpha}$, which comprises reacting a thioloester of the general formula:

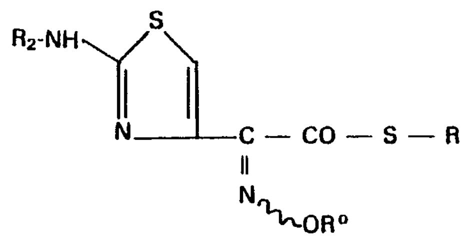

in which R° and $R_2$ are defined as in claim 8 and R is defined as above [it being understood that, if R° is a hydrogen atom, the oxime can be protected, if R° contains a carboxyl radical, the latter is protected, if R contains an amino, alkylamino, formyl or acylalkyl substituent, the latter is protected, and if R contains a hydroxyl or carboxyl substituent, the latter is free or protected], with a 7-aminocephalosporin of the general formula:

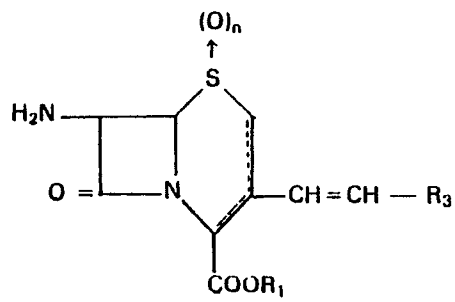

in which $R_1$, $R_3$ and n are defined as in claim 8 and which exhibits the same stereoisomerism as the product defined in claim 8, and then, if n = 1, reducing the oxide obtained and, if necessary, removing the protective radicals, and, if appropriate, converting the product obtained to an addition salt with an acid, to a metal salt or to an addition salt with a nitrogen-containing base.

11. A process according to claim 10, which comprises using a thioloester in which, if necessary, the radical R is protected by an amino-protecting radical as defined in claim 3, or in the form of an acetal by a radical of the general formula:

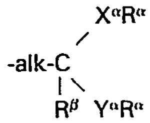

, 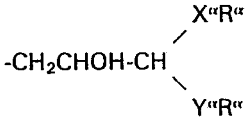 or

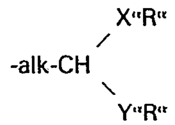 .

12. A process for the preparation of a product according to claim 1 in which the radical R does not contain a substituent of the general formula -alk-CH-(OH)OR$^\alpha$ and R° is not vinyl, which comprises reacting a thiourea of the general formula:

$$R_2NHCSNH_2$$

in which $R_2$ is a hydrogen atom or a protective radical, with a product of the general formula:

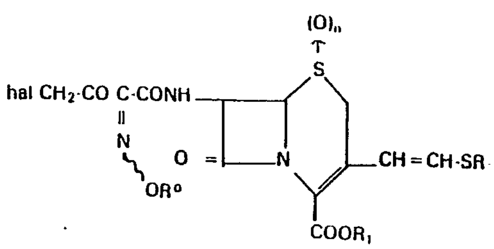

in which R°, $R_1$ and n are defined as in claim 2 (except that R° cannot represent a vinyl radical), R is defined as above and hal represents a chlorine or bromine atom, and then, if n = 1, reducing the oxide obtained and, if necessary, removing the protective radicals, and, if appropriate, converting the product obtained to an addition salt with an acid, to a metal salt or to an addition salt with a nitrogen-containing base.

13. A process according to claim 12, which comprises reacting a thiourea, in the formula of which the radical $R_2$ is chosen from amongst t-butoxycarbonyl-2,2,2-trichloroethoxycarbonyl, trityl, dibenzyl, benzyl, benzyloxycarbonyl, p-nitrobenzyloxycarbonyl, p-methoxybenzyloxycarbonyl, formyl or trifluoroacetyl radicals.

14. A process for the preparation of a product according to claim 1 in which the radical R represents a 5,6-dioxo-1,4,5,6-tetrahydro-1,2,4-triazin-3-yl radical substituted in the 1-position or 4-position, a 5,6-dioxo-1,2,5,6-tetrahydro-1,2,4-triazin-3-yl radical substituted in the 2-position, or a 1,3,4-triazol-5-yl or 2-alkoxycarbonyl-1,3,4-triazol-5-yl or 1,2,4-triazol-5-yl or 3-alkoxycarbonyl-1,2,4-triazol-5-yl radical substituted in the 1-position, by an alkyl radical containing 2 to 4 carbon atoms, which is itself substituted by a carbamoyloxy group or an acyloxy group (the acyl part of which is optionally substituted by an amino, alkylamino or dialkylamino radical), which comprises carbamoylating or esterifying an alcohol of the general formula:

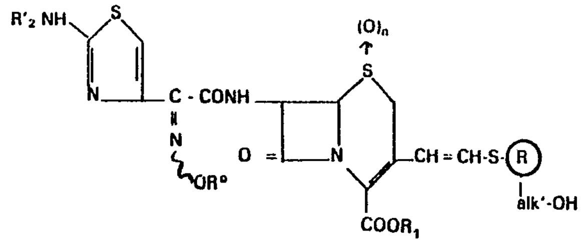

(in which R°, $R_1$ and n are defined as in claim 2, $R'_2$ is an amine-protecting radical and (R)-alk'-OH represents a 5,6-dioxo-1-(or 4)-hydroxyalkyl-1,4,5,6-tetrahydro-1,2,4-triazin-3-yl, 5,6-dioxo-2-hydroxyalkyl-1,2,5,6-tetrahydro-1,2,4-triazin-3-yl, 1-hydroxyalkyl-1,3,4-triazol-5-yl, 2-alkoxycarbonyl-1-hydroxyalkyl-1,3,4-triazol-5-yl, 1-hydroxyalkyl-1,2,4-triazol-5-yl or 3-alkoxycarbonyl-1-hydroxyalkyl-1,2,4-triazol-5-yl radical) by any method known for obtaining a carbamate or an ester from an alcohol without affecting the rest of the molecule, reducing the oxide obtained, if necessary, removing the protective groups and then, if appropriate, converting the product obtained to an addition salt with an acid, to a metal salt or to an addition salt with a nitrogen-containing base.

15. A process according to claim 14, which comprises using a cephalosporin, in the formula of which $R'_2$ is chosen from amongst the amino-protecting groups mentioned in claim 3.

16. A process for the preparation of a product according to claim 1 in which R represents a 5,6-dioxo-1,4,5,6-tetrahydro-1,2,4-triazin-3-yl radical substituted in the 1-position (or 4-position), a 5,6-dioxo-1,2,5,6-tetrahydro-1,2,4-triazin-3-yl radical substituted in the 2-postion, or a 1,3,4-triazol-5-yl, 2-alkoxycarbonyl-1,3,4-triazol-5-yl, 1,2,4-triazol-5-yl or 3-alkoxycarbonyl-1,2,4-triazol-5-yl radical substituted in the 1-position, by an alkyl radical containing 2 to 4 carbon atoms, which is itself substituted by a sulphoamino alkylsulphonylamino or sulphamoylamino group, an acylamino group (the acyl part of which is optionally substituted by hydroxyl, amino, alkylamino or dialkylamino), or an alkoxycarbonylamino, ureido, alkylureido or dialkylureido group, or represents a 1,3,4-thiadiazol-5-yl radical substituted by an acylamino or acylaminoalkyl radical, or represents a 1,3,4-oxadiazol-5-yl radical substituted by an acylaminoalkyl radical, or represents a tetrazol-5-yl radical substituted in the 1-position by an alkyl radical containing 2 to 4 carbon atoms, which is substituted by an acylamino, sulphamoylamino, sulphoamino, ureido, alkylureido or dialkylureido group, which comprises treating an amine of the general formula:

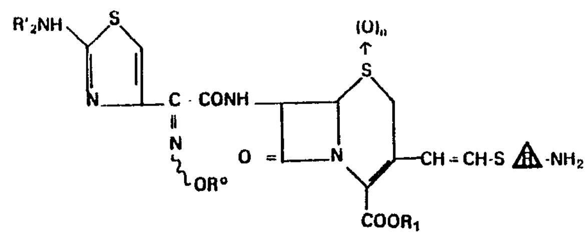

in which R°, $R_1$ and n are defined as in claim 2, $R'_2$ is an amine-protecting radical and -R-$NH_2$ represents a 5,6-dioxo-1,4,5,6-tetrahydro-1,2,4-triazin-3-yl radical substituted in the 1-position (or 4-position), a 5,6-dioxo-1,2,5,6-tetrahydro-1,2,4-triazin-3-yl radical substituted in the 2-position, or a 1,3,4-triazol-5-yl, 2-alkoxycarbonyl-1,3,4-triazol-5-yl, 1,2,4-triazol-5-yl or 3-alkoxycarbonyl-1,2,4-triazol-5-yl radical substituted in the 1-position, by an aminoalkyl radical (containing 2 to 4 carbon atoms) or a 1,3,4-thiadiazol-5-yl radical substituted by an amino or aminoalkyl radical, or a 1,3,4-oxadiazol-5-yl radical substituted by an aminoalkyl radical, or a tetrazol-5-yl radical substituted in the 1-position by an aminoalkyl radical (containing 2 to 4 carbon atoms), by any method known for forming an amide, sulphamide, carbamate or urea group without affecting the rest of the molecule, and then, if n = 1, reducing the oxide obtained and removing the protective groups, and then, if appropriate, converting the product obtained to an addition salt with an acid, to a metal salt or to an addition salt with a nitrogen-containing base.

17. A process according to claim 16, which comprises using a cephalosporin, in the formula of which $R'_2$ is defined as in claim 15.

18. A process according to claim 16, wherein, if it is desired to prepare a product defined in claim 16 in which R contains a hydroxyl or amino substituent, these groups are protected in the reactant used.

19. A process for the preparation of a product according to claim 1 in which R represents a 5,6-dioxo-1,4,5,6-tetrahydro-1,2,4-triazin-3-yl radical substituted in the 1-position (or 4-position), a 5,6-dioxo-1,2,5,6-tetrahydro-1,2,4-triazin-3-yl radical substituted in the 2-postion, or a 1,3,4-triazol-5-yl, 2-alkoxycarbonyl-1,3,4-triazol-5-yl, 1,2,4-triazol-5-yl or 3-alkoxycarbonyl-1,2,4-triazol-5-yl radical substituted in the 1-position, by a thiazolidin-2-yl alkyl radical, by a radical -alk-CH(OH)$OR^{\alpha}$ or by a hydroxyiminoalkyl or alkoxyiminoalkyl radical, the iminoalkyl part of which contains 2 to 5 carbon atoms, or alternatively represents a tetrazol-5-yl radical substituted in the 1-position by a hydroxyiminoalkyl or alkoxyiminoalkyl radical, the iminoalkyl part of which contains 2 to 5 carbon atoms, which comprises carrying out an addition reaction of cysteamine, an alcohol, hydroxylamine or an alkoxyamine onto an aldehyde of the general formula:

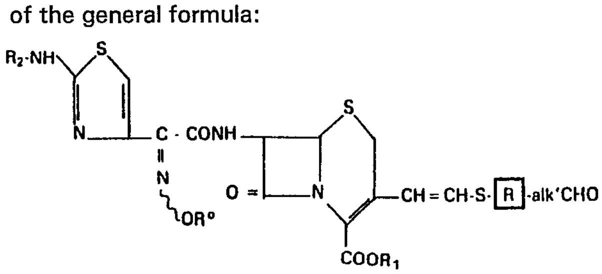

in which R°, $R_1$ and $R_2$ are defined as in claim 8 and -R-alk'CHO represents a 5,6-dioxo-1-(or 4-)formylalkyl-1,4,5,6-tetrahydro-1,2,4-triazin-3-yl, 5,6-dioxo-2-formylalkyl-1,2,5,6-tetrahydro-1,2,4-triazin-3-yl, or alternatively 1-formylalkyl-1,3,4-triazol-5-yl, 2-alkoxycarbonyl-1-formylalkyl-1,3,4-triazol-5-yl, 1-formylalkyl-1,2,4-triazol-5-yl, 3-alkoxycarbonyl-1-formylalkyl-1,2,4-triazol-5-yl or 1-formylalkyltetrazol-5-yl radical, by any method known for forming such addition derivatives of carbonylated groups, and then removing the protective radical and, if appropriate, converting the product obtained to an addition salt with an acid, to a metal salt or to an addition salt with a nitrogen-containing base.

20. A process of preparation according to claim 19, which comprises using a cephalosporin, in the formula of which $R_2$ is a protective group chosen from amongst the amino-protecting radicals mentioned in claim 3.

21. A process for the preparation of a product according to claim 1 in which R' represents a radical of the general formula:

-CH - OCO R'''
|
R''

as defined in claim 1, which comprises esterifying a product according to claim 1 in which R' is a hydrogen atom, by any method which is in itself known for preparing an ester from an acid without affecting the rest of the molecule, and then, if appropriate, converting the product obtained to an addition salt with an acid.

22. A process for the preparation of a product according to claim 1 in which R and R° are defined as in claim 1 under α. [it being understood that R does not contain a substituent of the general formula -alk-CH(OH)$OR^{\alpha}$], which comprises reacting a product of the general formula:

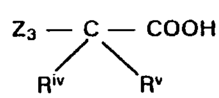

in which $R^{iv}$ and $R^{v}$ are defined as in claim 1 and $Z_3$ represents a halogen atom or a sulphate or sulphonate radical, and in which the acid group is protected, with a cephalosporin of the general formula:

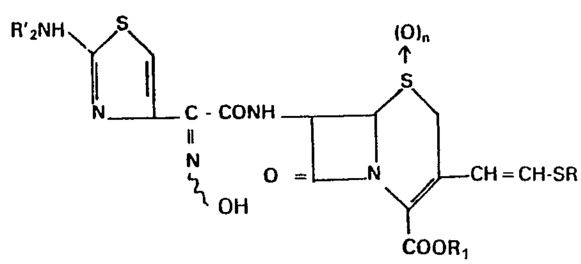

in which R is defined as above (it being understood that, if R contains an amino or alkylamino substituent, the latter is protected, and if it contains a formyl radical, the latter is free or protected), $R_1$ and n are defined as in claim 2 and $R'_2$ is defined as in claim 14, and then, if necessary, reducing the oxide obtained and removing the protective radicals, and, if appropriate, converting the product obtained to an addition salt with an acid, to a metal salt or to an addition salt with a nitrogen-containing base.

23. A process according to claim 22, which comprises using a cephalosporin in which $R'_2$ is an

amino-protecting radical chosen from amongst the radicals mentioned in claim 3.

24. A process for the preparation of a product according to claim 1 in which the radical R does not contain a substituent of the general formula -alk--CH(OH)OR$^{\alpha}$, which comprises reacting a thiol (free or in the form of an alkali metal salt or alkaline earth metal salt) as defined in claim 8, with a cephalosporin derivative (or a mixture of the isomers) of the general formula:

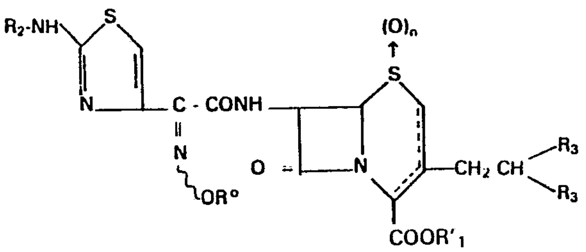

in which R$^{\circ}$ represents an alkyl, vinyl or cyanomethyl radical, an oxime-protecting radical or a protected radical of the general formula:

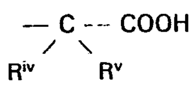

(in which the radicals R$^{iv}$ and R$^{v}$, which are identical or different, represent hydrogen atoms or alkyl radicals, or together form an alkylene radical containing 2 or 3 carbon atoms), R'$_1$ is defined in the same way as R$_1$ in claim 2, except that it cannot represent a hydrogen atom, R$_2$ is defined as in claim 8, n represents 0 or 1 (if n = 0, this derivative is in the form of a bicyclooct-2-ene or bicyclooct-3-ene, and if n = 1, this derivative is in the form of a bicyclooct-2-ene) and R$_3$ represents a chlorine or bromine atom, and then, if n = 1, reducing the product obtained, removing the protective radicals and, if appropriate, converting the product obtained to an addition salt with an acid, to a metal salt or to an addition salt with a nitrogen-containing base.

25. A medicament which contains at least one product according to claim 1, in the pure form or in the form of a pharmaceutical composition in association with one or more compatible and pharmaceutically acceptable diluents or adjuvants.

**Claim for the Contracting State:** AT

Process for the preparation of a 3-thiovinylcephalosporin of the general formula:

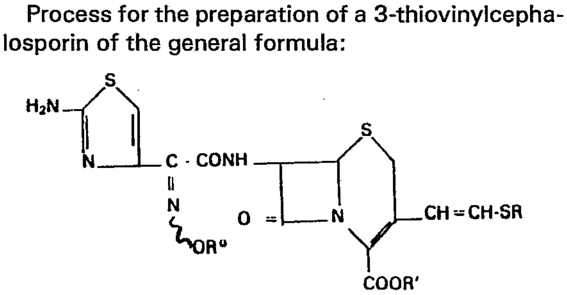

in which:

α. the symbol R is chosen amongst the following meanings:

1) alkyl, L-2-amino-2-carboxyethyl or phenyl,

2) pyrid-2-yl, pyrid-3-yl or pyrid-4-yl and their N-oxides,

3) pyrimidin-2-yl, pyridazin-3-yl substituted in the 6-position (by an alkyl, methoxy, amino or acylamino radical) and optionally N-oxidised, or tetrazolo[4,5-b]-pyridazin-6-yl,

4) 5,6-dioxo-1,4,5,6-tetrahydro-1,2,4-triazin-3--yl substituted in the 4-position, or 1,3,4-triazol-5-yl or 2-alkoxycarbonyl-1,3,4-triazol-5-yl substituted in the 1-position, by

a) an alkyl radical which is unsubstituted or substituted by an alkoxy, alkylthio, phenyl, formyl, carbamoyl, alkylcarbamoyl, dialkylcarbamoyl, acyl, alkoxycarbonyl, or thiazolidin-2-yl radical,

b) an allyl, 2,3-dihydroxypropyl, 1,3-dihydroxy--prop-2-yl, 2-formyl-2-hydroxyethyl, 3-formyloxy--2-hydroxypropyl, 2,3-bis-formyloxypropyl or 1,3--bis-formyloxyprop-2-yl radical,

c) an alkyl radical containing 2 to 4 carbon atoms, which is substituted by a hydroxyl or carbamoyloxy radical, an acyloxy radical (the acyl part of which can be substituted by an amino, alkylamino or dialkylamino radical), an alkylsulphinyl, alkylsulphonyl, amino, alkylamino, dialkylamino, sulphoamino, alkylsulphonylamino or sulphamoylamino radical, an acylamino radical (the acyl part of which is optionally substituted by hydroxyl, amino, alkylamino or dialkylamino) or an alkoxycarbonylamino, ureido, alkylureido or dialkylureido radical,

d) a radical corresponding to one of the general formulae:

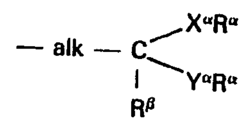

or 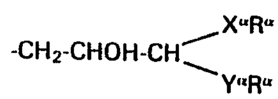

or 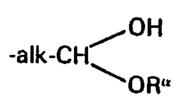

in which alk is an alkylene radical containing 1 to 4 carbon atoms, X$^{\alpha}$ and Y$^{\alpha}$ are identical and represent oxygen or sulphur atoms and R$^{\alpha}$ represents an alkyl radical, or alternatively X$^{\alpha}$ and Y$^{\alpha}$ are identical or different and represent oxygen or sulphur atoms and the radicals R$^{\alpha}$ together form an alkylene radical containing 2 or 3 carbon atoms, and R$^{\beta}$ represent a hydrogen atom or an alkyl radical containing 1 to 3 carbon atoms, or

e) an alkyl radical containing 2 to 5 carbon atoms, which is substituted by an alkoxyimino or hydroxyimino radical,

5) 1,4-dialkyl-5,6-dioxo-1,4,5,6-tetrahydro--1,2,4-triazin-3-yl, 1-alkyl-5,6-dioxo-1,4,5,6-tetrahydro-1,2,4-triazin-3-yl or 2-alkyl-5,6-dioxo-1,2,5,-6-tetrahydro-1,2,4-triazin-3-yl,

6) 1,3,4-triazol-5-yl, 1,2,3-triazol-5-yl or 1-alkyl--1,2,4-triazol-5-yl which is unsubstituted or substituted in the 3-position by alkoxycarbonyl,

7) a) 1,3,4-thiadiazol-5-yl which is unsubstituted or substituted by an alkyl, trifluoromethyl,

alkoxy or alkylthio radical, a hydroxyalkylthio radical, the alkyl part of which contains 2 to 4 carbon atoms, or an alkylsulphonyl, hydroxyl, hydroxyalkyl, carboxyl, carboxyalkyl, amino, alkylamino, dialkylamino, aminoalkyl, alkylaminoalkyl, dialkylaminoalkyl, acylamino or acylaminoalkyl radical, or

b) 1,2,4-thiadiazol-5-yl substituted by an alkyl or alkoxy radical,

8) a) 1,3,4-oxadiazol-5-yl which is unsubstituted or substituted by an alkyl, trifluoromethyl, phenyl, aminoalkyl, alkylaminoalkyl, dialkylaminoalkyl or acylaminoalkyl radical, or

b) oxazol-2-yl or 4-alkyloxazol-2-yl, and

9) tetrazol-5-yl which is unsubstituted or substituted in the 1-position by

a) an alkyl radical which is unsubstituted or substituted by alkoxy, sulpho, carboxyl, formyl or sulphamoyl,

b) an alkyl radical containing 2 to 4 carbon atoms, which is substituted by hydroxyl, amino, alkylamino, dialkylamino, acylamino, carboxyalkylamino, sulphamoylamino, sulphoamino, ureido, alkylureido or dialkylureido,

c) an alkyl radical containing 2 to 5 carbon atoms, which is substituted by hydroxyimino or alkoxyimino,

d) a phenyl, 2,3-dihydroxypropyl, 1,3-dihydroxyprop-2-yl, 2-formyl-2-hydroxyethyl, 3-formyloxy-2-hydroxypropyl, 2,3-bis-formyloxypropyl or 1,3-bis-formyloxyprop-2-yl radical, or

e) a radical of the general formulae:

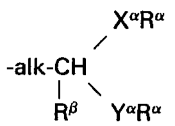

or

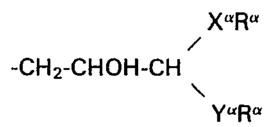

in which $R^{\beta}$ is a hydrogen atom and $X^{\alpha}$, $Y^{\alpha}$ and $R^{\alpha}$ are defined as under (4d) above, and the symbol $R^{o}$ represents a carboxyalkyl radical of the general formula:

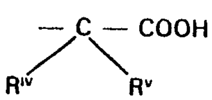

in which the radicals $R^{iv}$ and $R^{v}$, which are identical or different, represent hydrogen atoms or alkyl radicals, or together form an alkylene radical containing 2 or 3 carbon atoms, or alternatively

β. the symbol R is chosen from amongst:

1. 5,6-dioxo-1,4,5,6-tetrahydro-1,2,4-triazin-3-yl substituted in the 1-position, 5,6-dioxo-1,2,5,6-tetrahydro-1,2,4-triazin-3-yl substituted in the 2-position, or 1,2,4-triazol-5-yl or 3-alkoxycarbonyl-1,2,4-triazol-5-yl substituted in the 1-position, by

a) an alkyl radical which is itself substituted by an alkoxy, alkylthio, phenyl, formyl, carbamoyl, alkylcarbamoyl or dialkylcarbamoyl radical, a hydroxyalkylcarbamoyl radical (the alkyl part of which contains 2 to 4 carbon atoms) or an acyl, alkoxycarbonyl or thiazolidin-2-yl radical,

b) an allyl, 2,3-dihydroxypropyl, 1,3-dihydroxyprop-2-yl, 2-formyl-2-hydroxyethyl, 2-formyloxy-2-hydroxypropyl, 2,3-bis-formyloxypropyl or 1,3-bis-formyloxyprop-2-yl radical,

c) an alkyl radical containing 2 to 4 carbon atoms, which is substituted by a hydroxyl or carbamoyloxy radical, an acyloxy radical (the acyl part of which can be substituted by an amino, alkylamino or dialkylamino radical), an alkylsulphinyl, alkylsulphonyl, amino, alkylamino, dialkylamino, sulphoamino, alkylsulphonylamino or sulphamoylamino radical, an acylamino radical (the acyl part of which is optionally substituted by hydroxyl, amino, alkylamino or dialkylamino or an alkoxycarbonylamino, ureido, alkylureido or dialkylureido radical,

d) a radical corresponding to one of the general formulae mentioned above under α.4d), or

e) an alkyl radical containing 2 to 5 carbon atoms, which is substituted by an alkoxyimino or hydroxyimino radical, or alternatively,

2. 5,6-dioxo-4-hydroxyalkylcarbamoylalkyl-1,4,5,6-tetrahydro-1,2,4-triazin-3-yl of which the hydroxyalkyl portion contains 2 to 4 carbon atoms, or alternatively

3. 1-alkyl-5,6-dioxo-1,4,5,6-tetrahydro-1,2,4-triazin-3-yl substituted in the 4-position, 1-alkyl-5,6-dioxo-1,2,5,6-tetrahydro-1,2,4-triazin-3-yl substituted in the 2-position, 2-alkyl-5,6-dioxo-1,2,5,6-tetrahydro-1,2,4-triazin-3-yl substituted in the 1-position, or 4-alkyl-5,6-dioxo-1,4,5,6-tetrahydro-1,2,4-triazin-3-yl substituted in the 1-position by a formylalkyl radical or by a radical of the general formula:

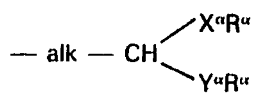

in which alk, $X^{\alpha}$, $Y^{\alpha}$ and $R^{\alpha}$ are defined as above, and the symbol $R^{o}$ represents a hydrogen atom, an alkyl, vinyl or cyanomethyl radical or a radical of the general formula:

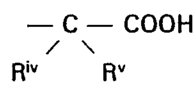

as defined above,
and the symbol R' represents a hydrogen atom or a radical which can easily be removed by an enzymatic method, of the general formula

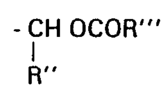

in which R'' represents a hydrogen atom or an alkyl radical and R''' represents an alkyl radical or the cyclohexyl radical, it being understood that the alkyl or acyl radicals and portions mentioned above are linear or branched (unless otherwise mentioned) and contain 1 to 4 carbon atoms, in its syn or anti forms and E or Z forms, and mixtures thereof, and also its addition salts with acids, its metal salts and its addition salts with nitrogen-containing bases, characterised in that for the preparation of a product in the formula of which the radical R does not contain a substituent of the general formula -alk-CH(OH)$OR^{\alpha}$,

A - an acid of the general formula:

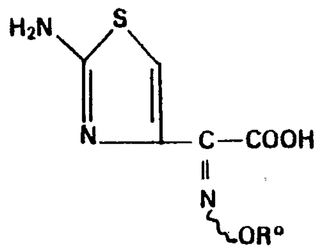

in which R° is defined as above and in which the amine group has been protected beforehand (and also the oxime if R° represents hydrogen, or the acid group if R° contains a carboxyl group), or a reactive derivative of this acid, is reacted with a 7-aminocephalosporin of the general formula:

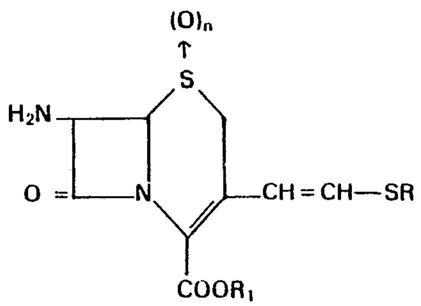

in which R is defined as above, $R_1$ represents a hydrogen atom, a radical of the general formula:

$$\begin{array}{l} -CH-OCOR''' \\ \;\;| \\ \;\;R'' \end{array}$$

as defined above, or an acid-protective radical which can easily be removed, and n represents 0 or 1, or

B - a thiol (free or in the form of an alkali metal salt or alkaline earth metal salt) of the general formula:

R - SH

(in which the substituent of R, which is defined as above, is protected in the form of an acetal [defined by the general formulae:

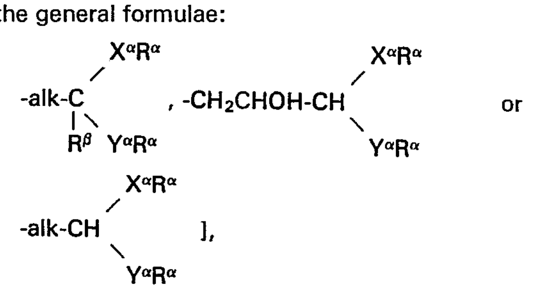

if it is desired to obtain a product in which R contains a formyl or acylalkyl radical, is reacted with a cephalosporin derivative (or, if necessary, with a mixture of the isomers) of the general formula:

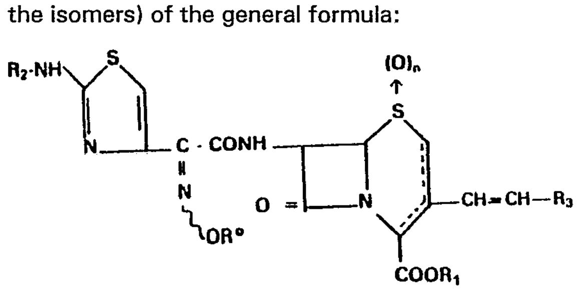

in which, R° being defined R° and $R_1$ and n being defined as above in A, if n = 0, this derivative is in the form of a bicyclooct-2-ene or bicyclooct-3-ene, and if n = 1, this derivative is in the form of a bicyclooct-2-ene, the substituent on the carbon atom in the 3-position of the bicyclooctene exhibits E/Z stereoisomerism, $R_2$ represents a hydrogen atom or a protective radical of the amino radical, and $R_3$ represents a halogen atom chosen from amongst chlorine, bromine or iodine, or a radical of the general formulae:

$-O-SO_2R'_3$
or $-O-CO\,R''_3$

in which $R'_3$ is a linear or branched alkyl radical containing 1 to 4 carbon atoms, a trifluoromethyl or trichloromethyl radical or a phenyl radical which is unsubstituted or substituted by a halogen atom or by an alkyl or nitro radical, and $R''_3$ is defined in the same way as $R'_3$ or represents an acylmethyl, 2-acylethyl, 2-acylpropyl, alkoxycarbonylmethyl, 2-alkoxycarbonylethyl or 2-alkoxycarbonylpropyl radical, or

C - a thioloester of the general formula:

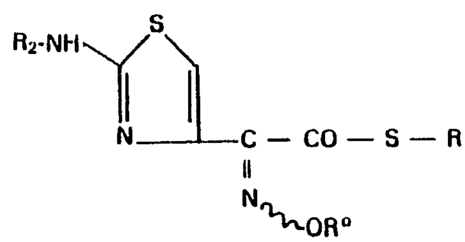

in which R, R° and $R_2$ are defined as above in B [it being understood that, if R° is a hydrogen atom, the oxime can be protected, if R° contains a carboxyl radical, the latter is protected, if R contains an amino, alkylamino, formyl or acylalkyl substituent, the latter is protected, and if R contains a hydroxyl or carboxyl substituent, the latter is free or protected], is reacted with a 7-aminocephalosporin of the general formula:

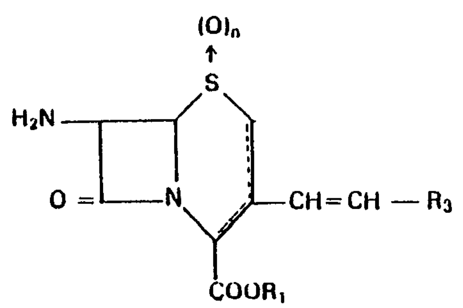

in which $R_1$, $R_3$ and n are defined as above in B and which exhibits the same stereoisomerism as the product defined above in B, or

D - a thiol (free or in the form of an alkali metal salt or alkaline earth metal salt) as defined in B, is reacted with a cephalosporin derivative (or a mixture of the isomers) of the general formula:

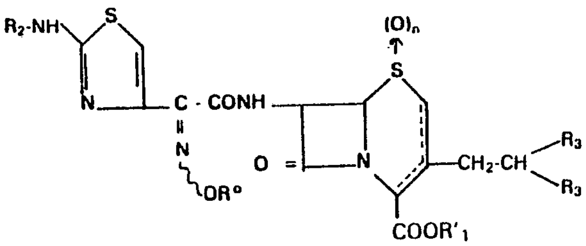

in which $R^o$ represents an alkyl, vinyl or cyanomethyl radical, an oxime-protecting radical or a protected radical of the general formula:

— C — COOH
/ \
$R^{iv}$ $R^{v}$

(in which the radicals $R^{iv}$ and $R^{v}$, which are identical or different, represent hydrogen atoms or alkyl radicals, or together form an alkylene radical containing 2 or 3 carbon atoms), $R'_1$ is defined in the same way as $R_1$ in A, except that it cannot represent a hydrogen atom, $R_2$ is defined as in B, n represents 0 or 1 (if n = 0, this derivatives is in the form of a bicyclooct-2-ene or bicyclooct-3-ene, and if n = 1, this derivative is in the form of a bicyclooct-2-ene) and $R_3$ represents a chlorine or bromine atom, and then, if n = 1, the product obtained is reduced, the protective radicals are removed and, if appropriate, the product obtained is converted to an addition salt with an acid, to a metal salt or to an addition salt with a nitrogen-containing base or

E - if $R^o$ is defined as above but is not vinyl, a thiourea of the general formula:

$R_2NHCSNH_2$

in which $R_2$ is a hydrogen atom or an amine-protective radical, is reacted with a product of the general formula:

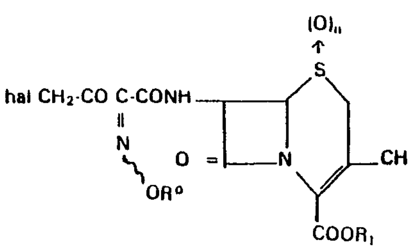

in which $R^o$, $R_1$ and n are defined as above in A (except that $R^o$ cannot represent a vinyl radical), R is defined as above and hal represents a chlorine or bromine atom, and then, if n = 1, reducing the oxide obtained is reduced and, if necessary, the protective radicals are removed, and, if appropriate, the product obtained is converted to an addition salt with an acid, to a metal salt or to an addition salt with a nitrogen-containing base or

F - for the preparation of a product in the formula of which R and $R^o$ are defined as in $\alpha$. [it being understood that R does not contain a substituent of the general formula -alk-CH(OH)$OR^{\alpha}$], a cephalosporin of the general formula:

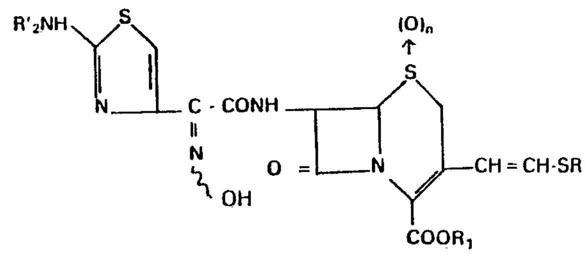

in which R is defined as above (it being understood that, if R contains an amino or alkylamino substituent, this is protected, and if it contains a formyl radical, this is free or protected), $R_1$ and n are defined as in A and $R'_2$ is an amine-protecting radical, is prepared according to the invention and is then reacted with a product of the general formula

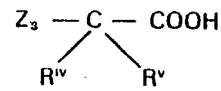

in which $R^{iv}$ and $R^{v}$ are defined as in $\alpha$. and $Z_3$ represents a halogen atom or a sulphate or sulphonate radical, whose acid group is protected, and if necessary, the oxide obtained is reduced, the protective radicals are removed and, if appropriate, the product obtained is converted to an addition salt with an acid, to a metal salt or to an addition salt with a nitrogen-containing base, or

G - for the preparation of a product in the formula of which the radical R represents a 5,6-dioxo-1,4,-5,6-tetrahydro-1,2,4-triazin-3-yl radical substituted in the 1-position or 4-position, a 5,6-dioxo-1,2,5,6-tetrahydro-1,2,4-triazin-3-yl radical substituted in the 2-position, or a 1,3,4-triazol-5-yl or 2-alkoxycarbonyl-1,3,4-triazol-5-yl or 1,2,4-triazol-5-yl or 3-alkoxycarbonyl-1,2,4-triazol-5-yl radical substituted in the 1-position, by an alkyl radical containing 2 to 4 carbon atoms, which is itself substituted by a carbamoyloxy group or an acyloxy group (the acyl part of which is optionally substituted by an amino, alkylamino or dialkylamino radical), an alcohol of the general formula:

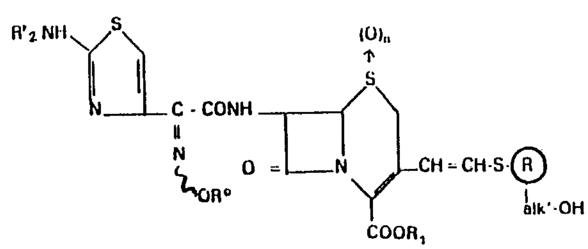

(in which $R^o$, $R_1$ and n are defined as in A, $R'_2$ is an amine-protecting radical and -(R)-alk'-OH represents a 5,6-dioxo-1-(or 4)-hydroxyalkyl-1,4,5,6-tetrahydro-1,2,4-triazin-3-yl, 5,6-dioxo-2-hydroxyalkyl-1,2,5,6-tetrahydro-1,2,4-triazin-3-yl, 1-hydroxyalkyl-1,3,4-triazol-5-yl, 2-alkoxycarbonyl-1-hydroxyalkyl-1,3,4-triazol-5-yl, 1-hydroxyalkyl-1,2,4-triazol-5-yl or 3-alkoxycarbonyl-1-hydroxyalkyl-1,2,4-triazol-5-yl radical) is prepared, and this alcohol is converted into the corresponding ester or carbamate, and, if necessary, the oxide obtained is reduced, the protective groups are removed and then, if appropriate, the product obtained is converted to an addition salt with an acid, to a metal salt or to an addition salt with a nitrogen-containing base or

H - for the preparation of a product in the formula of which R represents a 5,6-dioxo-1,4,5,6-tetrahydro-1,2,4-triazin-3-yl radical substituted in the 1-position (or 4-position), a 5,6-dioxo-1,2,5,6-tetrahydro-1,2,4-triazin-3-yl radical substituted in the 2-postion, or a 1,3,4-triazol-5-yl, 2-alkoxycarbonyl-1,3,4-triazol-5-yl, 1,2,4-triazol-5-yl or 3-alkoxycarbonyl-1,2,4-triazol-5-yl radical substituted in the 1-position, by an alkyl radical containing 2 to 4 carbon atoms, which is itself substituted by a sulphoamino alkylsulphonylamino or sulphamoylamino group, an acylamino group (the acyl part of which is optionally substituted by hydroxyl, amino, alkylamino or dialkylamino), or an alkoxycarbonylamino, ureido, alkylureido or dialkylureido group, or represents a 1,3,4-thiadiazol-5-yl radical substituted by an acylamino or acylaminoalkyl radical, or represents a 1,3,4-oxadiazol-5-yl radical substituted by an acylaminoalkyl radical, or represents a tetrazol-5-yl radical substituted in the 1-position by an alkyl radical containing 2 to 4 carbon atoms, which is substituted by an acylamino, sulphamoylamino, sulphoamino, ureido, alkylureido or dialkylureido group, an amine of the general formula:

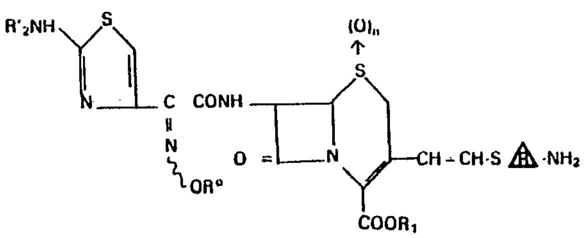

in which R°, $R_1$ and n are defined as in A, R'2 is an amine-protecting radical and -[A]-$NH_2$ represents a 5,6-dioxo-1,4,5,6-tetrahydro-1,2,4-triazin-3-yl radical substituted in the 1-position (or 4-position), a 5,6-dioxo-1,2,5,6-tetrahydro-1,2,4-triazin-3-yl radical substituted in the 2-position, or a 1,3,4-triazol-5-yl, 2-alkoxycarbonyl-1,3,4-triazol-5-yl, 1,2,4-triazol-5-yl or 3-alkoxycarbonyl-1,2,4-triazol-5-yl radical substituted in the 1-position, by an aminoalkyl radical (containing 2 to 4 carbon atoms) or a 1,3,4-thiadiazol-5-yl radical substituted by an amino or aminoalkyl radical, or a 1,3,4-oxadiazol-5-yl radical substituted by an aminoalkyl radical, or a tetrazol-5-yl radical substituted in the 1-position by an aminoalkyl radical (containing 2 to 4 carbon atoms) is prepared according to the invention, and the amine is converted into the corresponding amide, sulphamide, carbamate or urea, and then, if n = 1, the oxide obtained is reduced, the protective groups are removed, and then, if appropriate, the product obtained is converted to an addition salt with an acid, to a metal salt or to an addition salt with a nitrogen-containing base or

I - for the preparation of a product in the formula of which R represents a 5,6-dioxo-1,4,5,6-tetrahydro-1,2,4-triazin-3-yl radical substituted in the 1-position (or 4-position), a 5,6-dioxo-1,2,5,6-tetrahydro-1,2,4-triazin-3-yl radical substituted in the 2-position, or a 1,3,4-triazol-5-yl, 2-alkoxycarbonyl-1,3,4-triazol-5-yl, 1,2,4-triazol-5-yl or 3-alkoxycarbonyl-1,2,4-triazol-5-yl radical substituted in the 1-position, by a thiazolidin-2-yl alkyl radical, by a radical -alk-CH(OH)$OR^{\alpha}$ or by a hydroxyiminoalkyl or alkoxyiminoalkyl radical, the iminoalkyl part of which contains 2 to 5 carbon atoms, or alternatively represents a tetrazol-5-yl radical substituted in the 1-position by a hydroxyiminoalkyl or alkoxyiminoalkyl radical, the iminoalkyl part of which contains 2 to 5 carbon atoms, an aldehyde of the general formula:

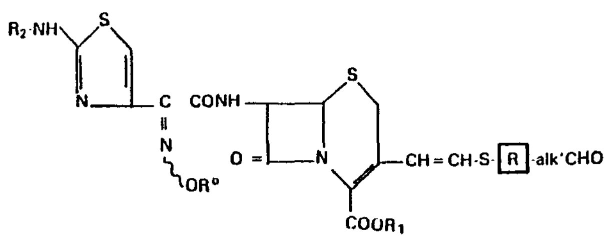

in which R°, $R_1$ and $R_2$ are defined as in B and -[R]-alk'CHO represents a 5,6-dioxo-1-(or 4-)formylalkyl-1,4,5,6-tetrahydro-1,2,4-triazin-3-yl, 5,6-dioxo-2-formylalkyl-1,2,5,6-tetrahydro-1,2,4-triazin-3-yl, 1-formylalkyl-1,3,4-triazol-5-yl, 2-alkoxycarbonyl-1-formylalkyl-1,3,4-triazol-5-yl, 1-formylalkyl-1,2,4-triazol-5-yl, 3-alkoxycarbonyl-1-formylalkyl-1,2,4-triazol-5-yl or 1-formylalkyltetrazol-5-yl radical is prepared according to the invention, and an addition reaction with cysteamine, an alcohol, hydroxylamine or an alkoxyamine is carried out on the aldehyde, and then the protective radicals are removed and, if appropriate, the product obtained is converted to an addition salt with an acid, to a metal salt or to an addition salt with a nitrogen-containing base or

J - for the preparation of a product in the formula of which R' represents a radical of the general formula:

$$-\underset{\substack{| \\ R''}}{CH}-OCO\ R'''$$

the corresponding acid, in which R' is a hydrogen atom, is prepared according to the invention, and the said acid is esterified and, if appropriate, the product obtained is converted into an addition salt with an acid.